# EUROPEAN PATENT SPECIFICATION

(11) **EP 1 117 807 B1**
(45) Date of publication and mention of the grant of the patent: **21.07.2010**
(21) Application number: 99947153.5
(22) Date of filing: 07.10.1999
(51) Int. Cl.: C12N 15/70, C12N 15/31, C12N 1/21, C07K 14/285, A61K 31/70, A61K 38/16

(54) **PROTECTIVE RECOMBINANT HAEMOPHILUS INFLUENZAE HIGH MOLECULAR WEIGHT PROTEINS**
SCHÜTZENDE HOCHMOLEKULARGEWICHTSPROTEINE AUS HAEMOPHILUS INFLUENZAE
PROTEINES DE HAUT POIDS MOLECULAIRE RECOMBINANTES PROTECTRICES, EXTRAITES D'HAEMOPHILUS INFLUENZAE

(30) Priority: 07.10.1998 US 167568; 08.12.1998 US 206942
(43) Date of publication of application: 25.07.2001
(73) Proprietor: Sanofi Pasteur Limited, Toronto, ON M2R 3T4 (CA)
(72) Inventor: LOOSMORE, Sheena, M., Aurora, Ontario L4G 4R4 (CA); YANG, Yan-Ping, Willowdale, Ontario M2R 3N7 (CA); KLEIN, Michel, H., Toronto,Ontario, M4N 1B9 (CA)
(74) Representative: Bizley, Richard Edward
(86) International application number: PCT/CA1999/000938
(87) International publication number: WO 2000/020609

(56) References cited:
- EP-A- 0 502 637
- WO-A-93/19090
- WO-A-94/21290
- WO-A-94/21290
- WO-A-97/36914
- WO-A-97/36914
- BARENKAMP S.J.: "Immunization with High-Molecular-Weight adhesion proteins of nontypeable Haemophilus influenzae modifies experimental otitis media in chinchillas" INF. IMMUN., vol. 64, no. 4, April 1996 (1996-04), pages 1245-1251, XP002133637
- BARENKAMP S J ET AL: "GENES ENCODING HIGH-MOLECULAR-WEIGHT ADHESION PROTEINS OF NONTYPEABLE HAEMOPHILUS INFLUENZAE ARE PART OF GENE CLUSTERS" INFECTION AND IMMUNITY,US,AMERICAN SOCIETY FOR MICROBIOLOGY. WASHINGTON, vol. 62, no. 8, 1 August 1994 (1994-08-01), pages 3320-3328, XP000578342 ISSN: 0019-9567
- YANG Y-P ET AL: "Nasopharyngeal colonization with nontypeable Haemophilus influenzae in chinchillas" INFECTION AND IMMUNITY 1998 UNITED STATES, vol. 66, no. 5, May 1998 (1998-05), pages 1973-1980, XP002421309 ISSN: 0019-9567
- ST. GEME III J.W. ET AL.: "Prevalence and distribution of the hmw and hia genes and the HMW and Hia adhesins among genetically diverse strains of nontypeable Haemophilus influenzae" INFECTION AND IMMUNITY, vol. 66, no. 1, January 1998 (1998-01), pages 364-368, XP002137980
- BARENKAMP S.J.: "Immunization with High-Molecular-Weight adhesion proteins of nontypeable Haemophilus influenzae modifies experimental otitis media in chinchillas" INF. IMMUN., vol. 64, no. 4, April 1996 (1996-04), pages 1245-1251, XP002133637 cited in the application
- BARENKAMP S J ET AL: "GENES ENCODING HIGH-MOLECULAR-WEIGHT ADHESION PROTEINS OF NONTYPEABLE HAEMOPHILUS INFLUENZAE ARE PART OF GENE CLUSTERS" INFECTION AND IMMUNITY,US,AMERICAN SOCIETY FOR MICROBIOLOGY. WASHINGTON, vol. 62, no. 8, 1 August 1994 (1994-08-01), pages 3320-3328, XP000578342 ISSN: 0019-9567 cited in the application
- ST. GEME III, J.W. ET AL.: "Secretion of the Haemophilus influenzae HMW1 and HMW2 adhesins involves a periplasmic intermediate and requires the HMWB and HMWC proteins." MOL. MICROBIOL., vol. 27, no. 3, February 1998 (1998-02), pages 617-630, XP000892544 cited in the application
- DAWID S.R. ET AL.: "High Molecular Weight adhesins of nontypeable Haemophilus influenzae: characterization of a novel mechanism of regulation" ABSTRACTS OF THE 1998 GENERAL MEETING OF THE AMERICAN SOCIETY FOR MICROBIOLOGY, vol. 98, 17 May 1998 (1998-05-17), page 74 XP002133638 cited in the application
- ST. GEME III J.W. ET AL.: "Prevalence and distribution of the hmw and hia genes and the HMW and Hia adhesins among genetically diverse strains of nontypeable Haemophilus influenzae" INFECTION AND IMMUNITY, vol. 66, no. 1, January 1998 (1998-01), pages 364-368, XP002137980

## Description

### FIELD OF INVENTION

The present invention relates to the field of molecular genetics and, in particular, to the production of recombinant *Haemophilus influenzae* high molecular weight proteins and nucleic acid molecules and vectors employed therein.

### BACKGROUND TO THE INVENTION

Encapsulated *Haemophilus influenzae* type b strains are a major cause of bacterial meningitis and other invasive infections in young children. However, the non-encapsulated or nontypeable *H. influenzae* (NTHi) are responsible for a wide range of human diseases, including otitis media, epiglottitis, pneumonia and tracheobronchitis. Vaccines based upon *H. influenzae* type b capsular polysaccharide conjugated to diphtheria toxoid (ref. 1. Throughout this application, various references are referred to in parenthesis to more fully describe the state of the art to which this invention pertains. Full bibliographic information for each citation is found at the end of the specification, immediately preceding the claims.), tetanus toxoid (ref . 2 and US patent 4,496,538), or *Neisseria meningitidis* outer membrane protein (ref. 3) have been effective in reducing *H. influenzae* type b-induced meningitis, but not NTHi-induced disease (ref. 4).

Otitis media is the most common illness of early childhood, with 60 to 70% of all children, of less than 2 years of age, experiencing between one and three ear infections (ref. 5). Chronic otitis media is responsible for hearing, speech and cognitive impairments in children. *H. influenzae* infections account for about 30% of the cases of acute otitis media and about 60% of chronic otitis media. In the United States alone, treatment of otitis media costs between 1 and 2 billion dollars per year for antibiotics and surgical procedures, such as tonsillectomies, adenoidectomies and insertion of tympanostomy tubes. It is estimated that an additional $30 billion is spent per annum on adjunct therapies, such as speech therapy and special education classes. Furthermore, many of the causative organisms of otitis media are becoming resistant to antibiotic treatment. An effective prophylactic vaccine against otitis media is thus desirable.

During natural infection by NTHi, surface-exposed outer membrane proteins that stimulate an antibody response are potentially important targets for bactericidal and/or protective antibodies and, therefore, potential vaccine candidates. Barenkamp and Bodor (ref. 6) demonstrated that convalescent sera from children suffering from otitis media due to NTHi contained antibodies to high molecular weight (HMW) proteins. About 70 to 75% of NTHi strains express the HMW proteins and most of these strains contain two gene clusters termed *hmw1ABC* and *hmw2ABC.* The *hmwA* genes encode the structural HMWA proteins and the *hmwB* and *hmwC* genes are accessory genes responsible for the processing and secretion of the HMWA proteins (refs. 7, 8, 9; US Patent No. 5,603,938; WO 97/36914). The HMWA proteins have been demonstrated to be adhesins mediating attachment to human epithelial cells (ref. 10) and only properly processed HMWA proteins appear to be effective adhesins (ref. 8). Immunization with a mixture of native HMW1A and HMW2A proteins resulted in protection in the chinchilla intrabulla challenge model of otitis media (ref. 11; WO 97/36914). The prototype *hmw1A* gene from NTHi strain 12 encodes a 160 kDa HMW1A protein that is processed by cleavage of a 35 kDa amino terminal fragment, generating the mature 125 kDa HMW1A protein. Similarly, the NTHi strain 12 *hmw2A* gene encodes a 155 kDa HMW2A protein that is processed by cleavage of a nearly identical 35 kDa amino terminal fragment to produce the mature 120 kDa HMW2A protein.

Plasmid pHMW1-15 (ref. 8) has a pT7-7 backbone (ref. 12) and contains the complete NTHi strain 12 *hmw1ABC* operon with 5'- and 3'-flanking regions. There are about 400 bp of 5'-flanking sequences located between the T7 promoter and the start of the *hmw1A* structural gene. Plasmid pHMW2-21 (ref. 10) has a pT7-7 backbone and contains the complete *hmw2ABC* operon with 5'- and 3'-flanking sequences. There are about 800 bp of 5'-flanking sequences located between the T7 promoter and the start of the *hmw2A* structural gene. The rHMW1A and rHMW2A proteins are produced in relatively low yield from plasmids pHMW1-15 and pHMW2-21.

The *H. influenzae hmw1 ABC* or *hmw2 ABC* genes can be genetically engineered to produce the mature recombinant HMW1A or HMW2A proteins by deleting the sequence encoding the 35 kDa leader sequence, that is normally removed by processing in *H. influenzae.* Since the leader sequence has been deleted, there should be no necessity for the *hmw1lBC* or *hmw2BC* genes which serve to process and secrete the mature HMW1A and HMW2A structural proteins in *H. influenzae* (ref. 9). The yield of rHMW1A or rHMW2A protein can be significantly increased by deletion of the leader sequence and processing genes, however, the purified recombinant proteins are not protective. As set forth herein, the *hmw1BC* and *hmw2BC* genes or their protein products apparently contribute to the protective ability of rHMW1A and rHMW2A proteins. Such a requirement for otherwise redundant accessory genes, is unexpected.

The *E. coli* cer gene is thought to stabilize plasmids by preventing multimerization (ref. 13). For expression vectors with large inserts, the cer gene may be used to stabilize the plasmids.

WO 97/36914 discloses high molecular weight surface proteins of non-typable *Haemophilus* influenzae which exhibit immunogenic properties and genes encoding the same. Genes coding for two immunodominent high molecular weight proteins, HMW1, HMW2, WMW3 and HMW4 were cloned, expressed and sequenced.

### SUMMARY OF THE INVENTION

The present invention is directed towards the provision of recombinant non-typeable *H. influenzae* high molecular weight proteins that are protective by providing certain nucleic acid molecules and vectors containing the same.

It has now been found that, in order to obtain recombinant high molecular weight (HMW) proteins of non-typeable *Haemophilus* which are protective, it is necessary to provide a vector containing only the segment of the A portion of the operon which encodes the mature HMW protein, i. e. lacking the segment of the A gene which encodes the leader sequence, and the B and C portions of the operon. It has also been found that the level of expression of the mature protein may be enhanced by including in the vector at least one additional segment which encodes the mature protein, the cer gene from *E. coli* or both.

Accordingly, in one aspect of the present invention, there is provided a nucleic acid molecule comprising a promoter functional in *E. coli* and operatively coupled to a modified operon of a non-typeable strain of *Haemophilus* comprising *A*, *B* and *C* genes, wherein the A gene of the operon contains only a nucleic acid sequence which encodes a mature high molecular weight protein of the non-typeable strain of *Haemophilus.* Hence from which nucleic acid molecule the portion of the A gene encoding the leader sequence is absent.

Any suitable promoter may be used to effect expression of the mature HMW protein in *E. coli.* However, it is preferred to use the T7 promoter.

The encoded mature high molecular weight protein may be HMW1 or HMW2 protein of the non-typeable *Haemophilus* strain. The non-typeable *Haemophilus* strain may be selected from the group consisting of strains 12, Joyc, K21, LCDC2, PMH1 and 15 of non-typeable *Haemophilus influenzae*.

There is described the nucleotide sequences for the *hmw1A* and/or *hmw2A* genes of certain non-typeable strains of *Haemophilus influenzae* which have not been previously isolated, purified and expressed, along with the deduced amino acid sequences of the corresponding HMW1 and HMW2 proteins of the non-typeable *Haemophilus* strains.

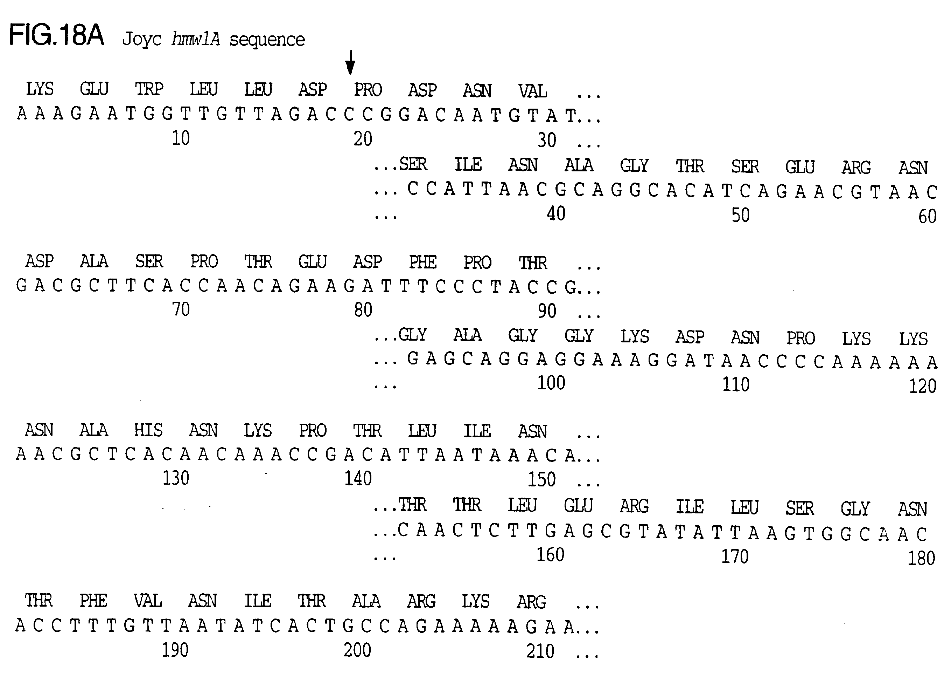

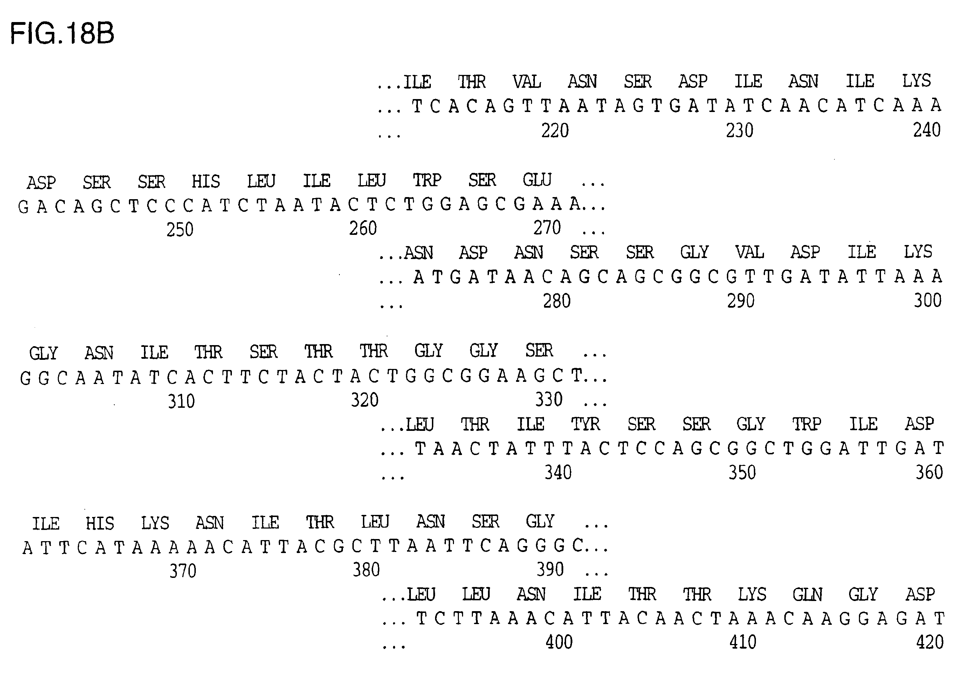

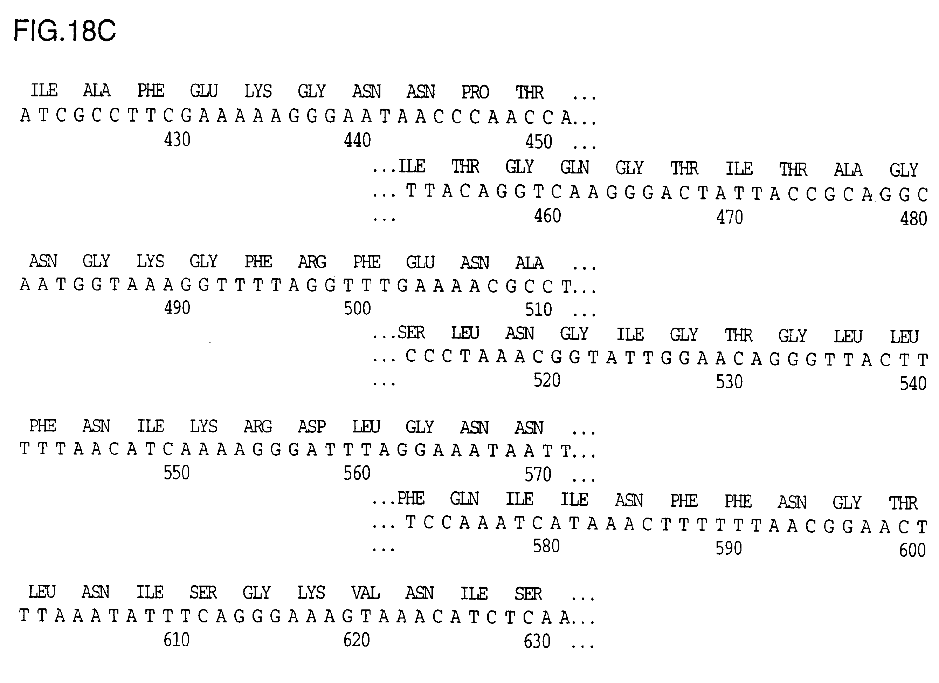

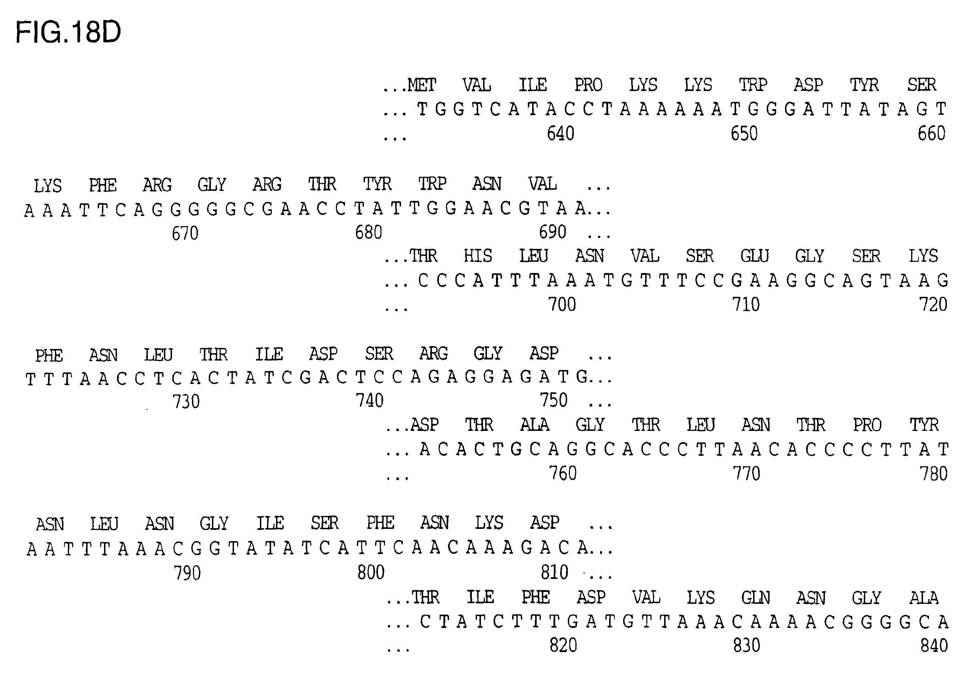

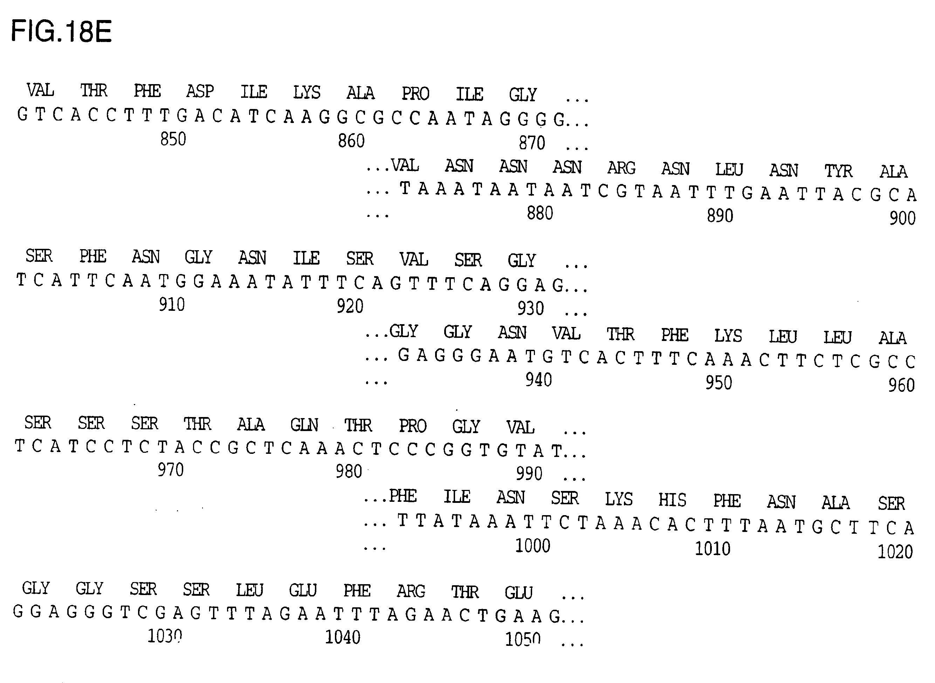

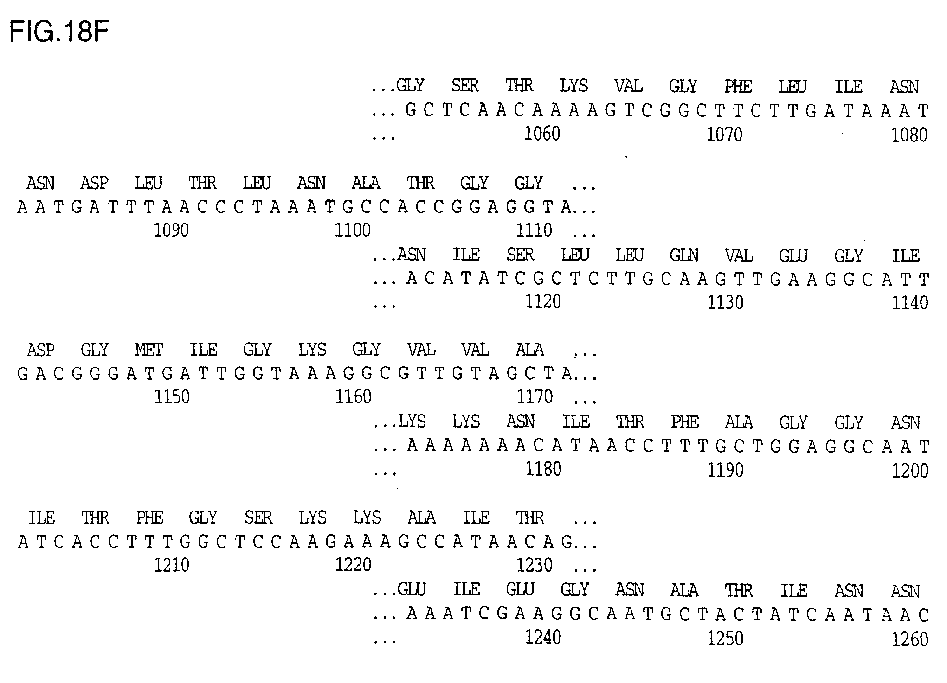

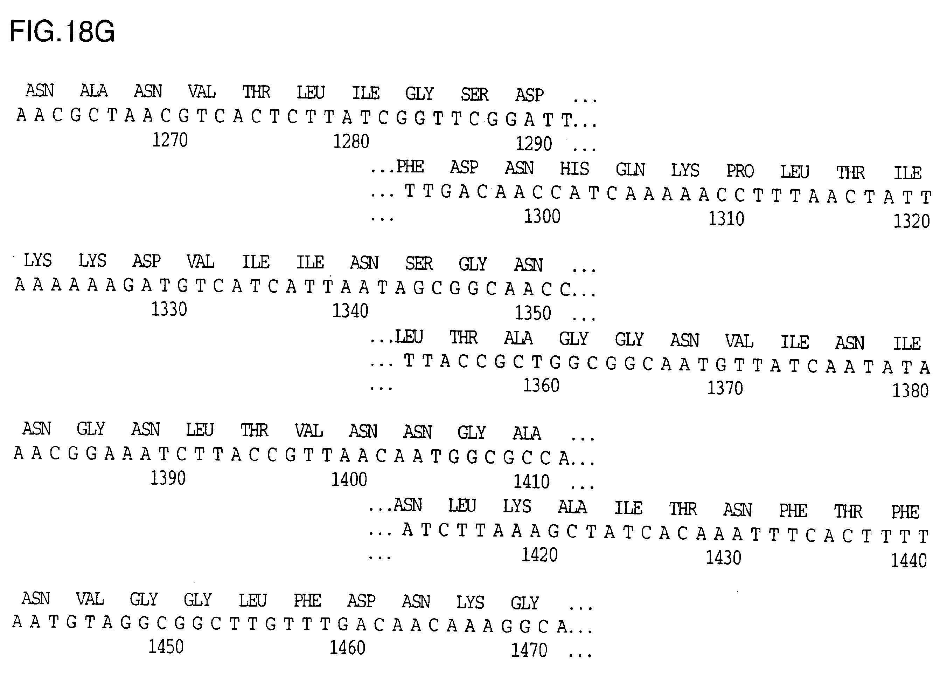

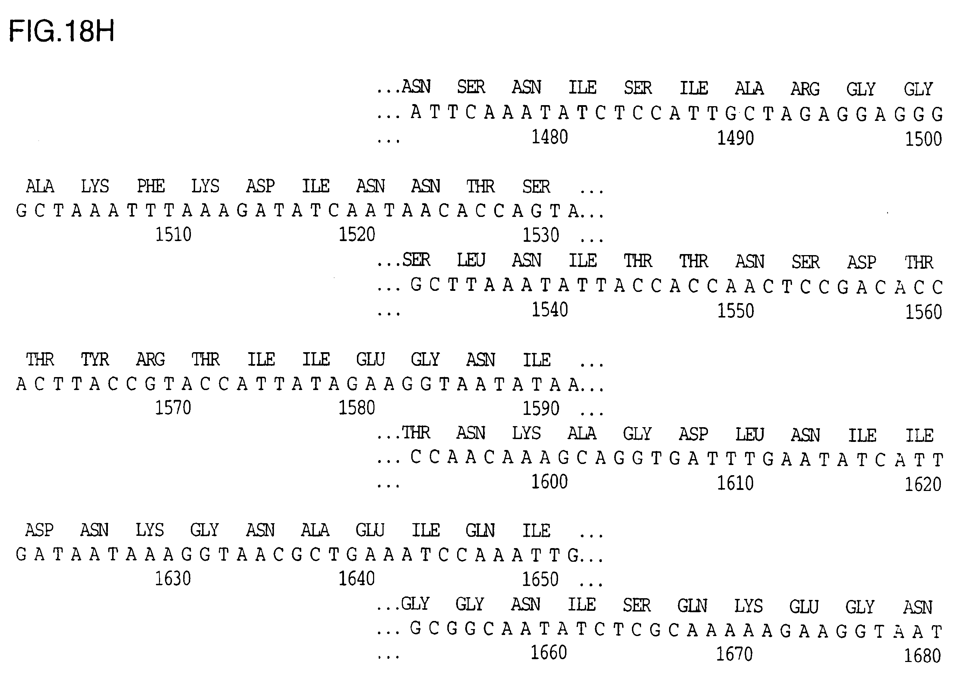

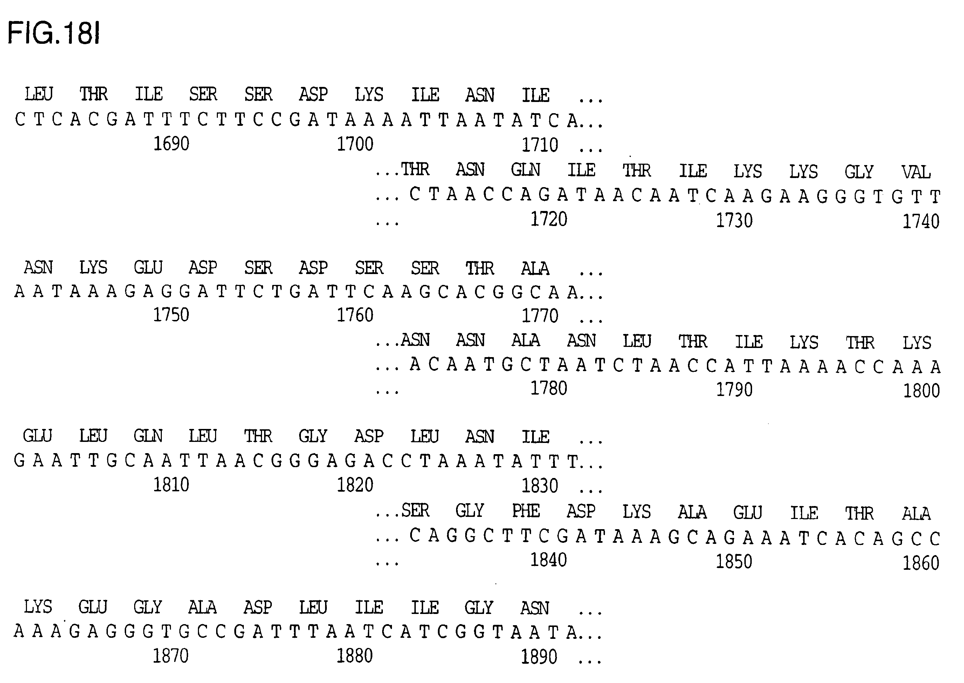

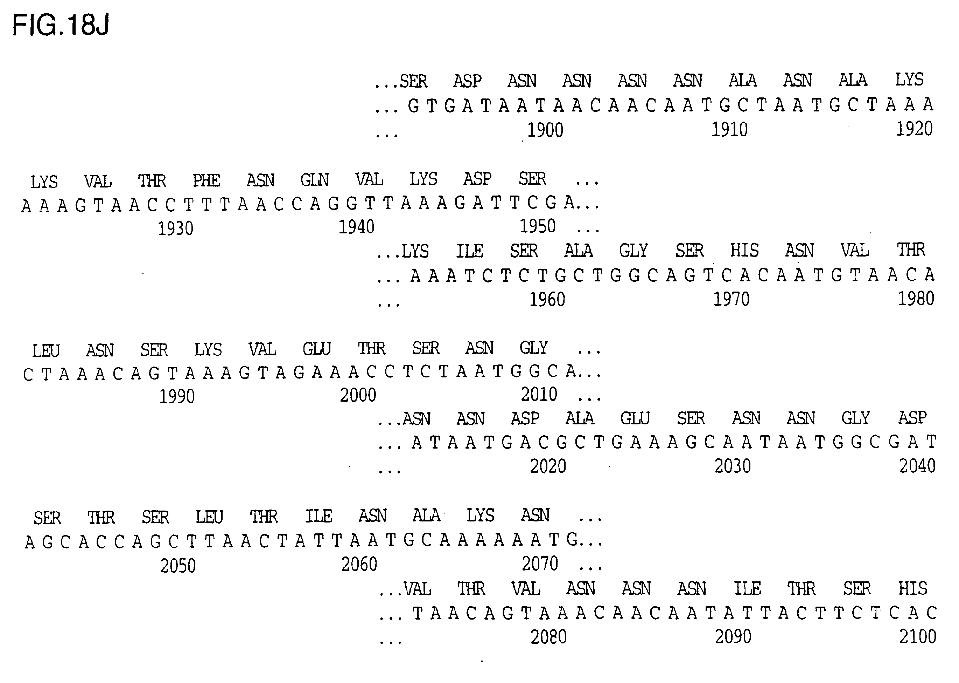

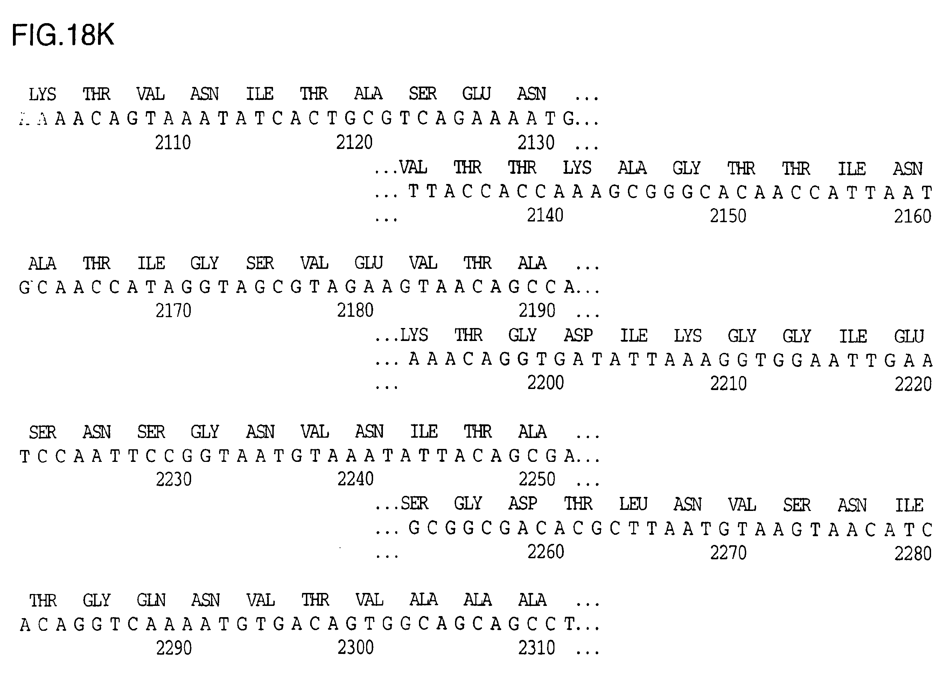

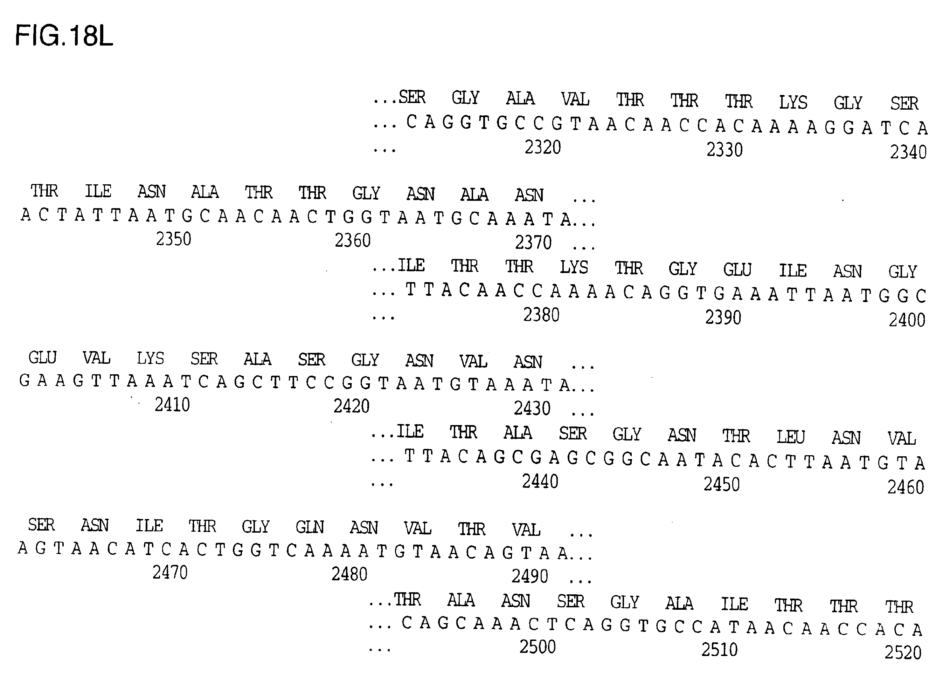

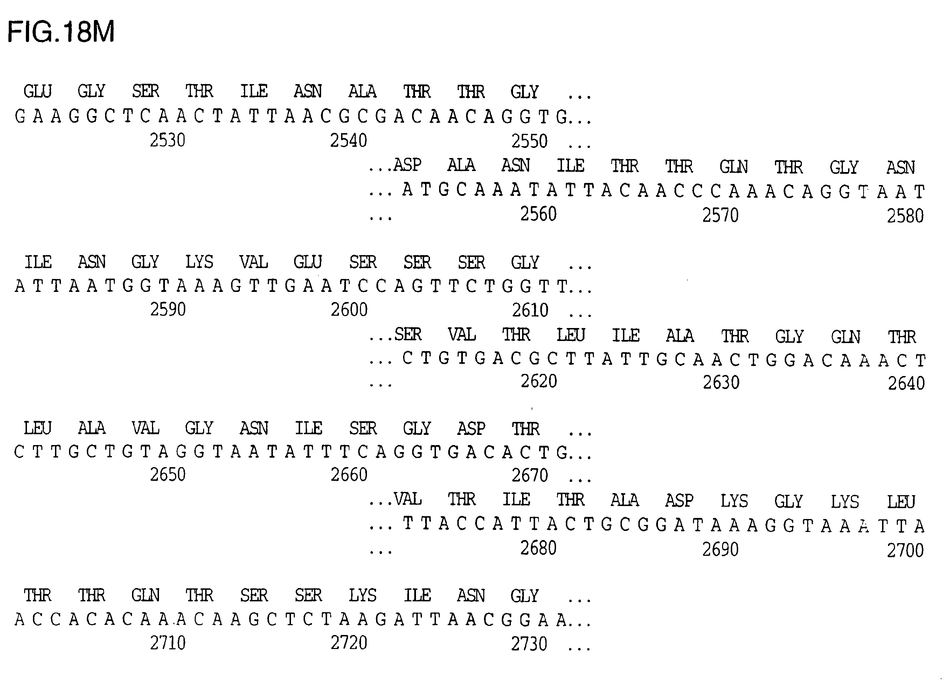

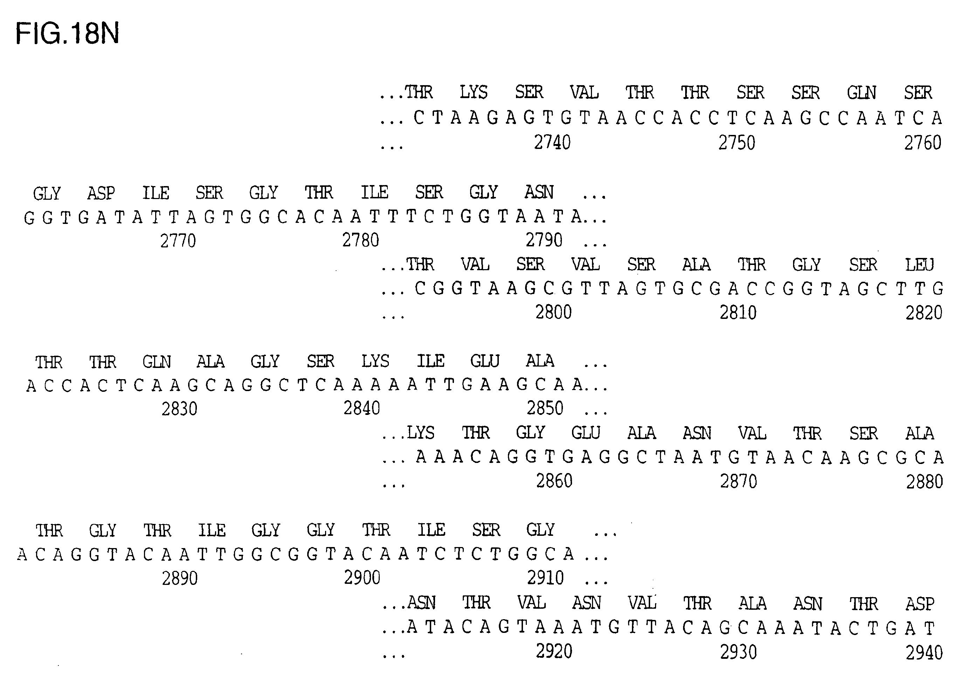

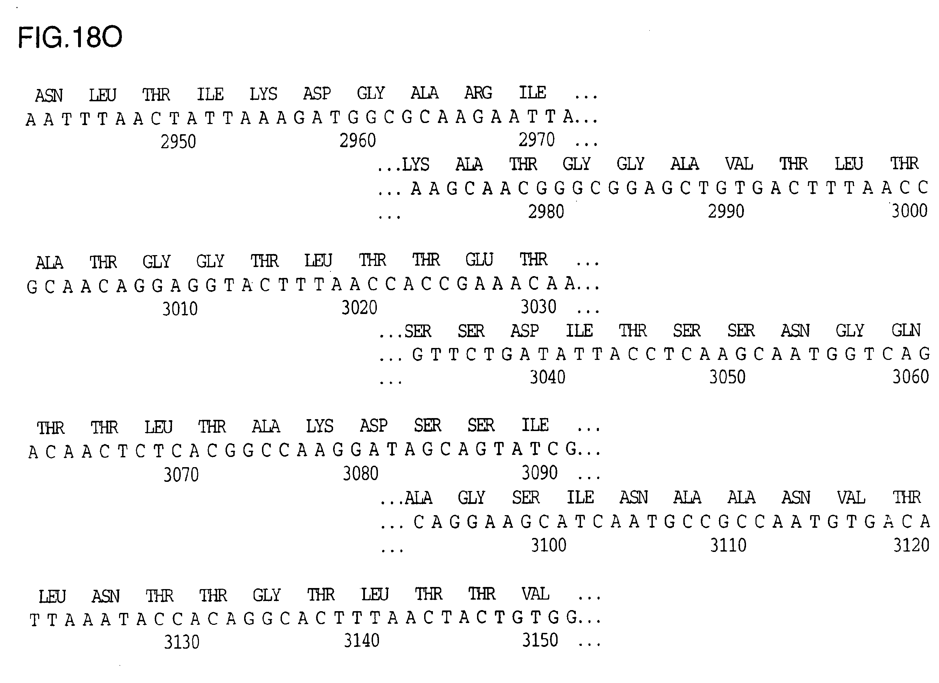

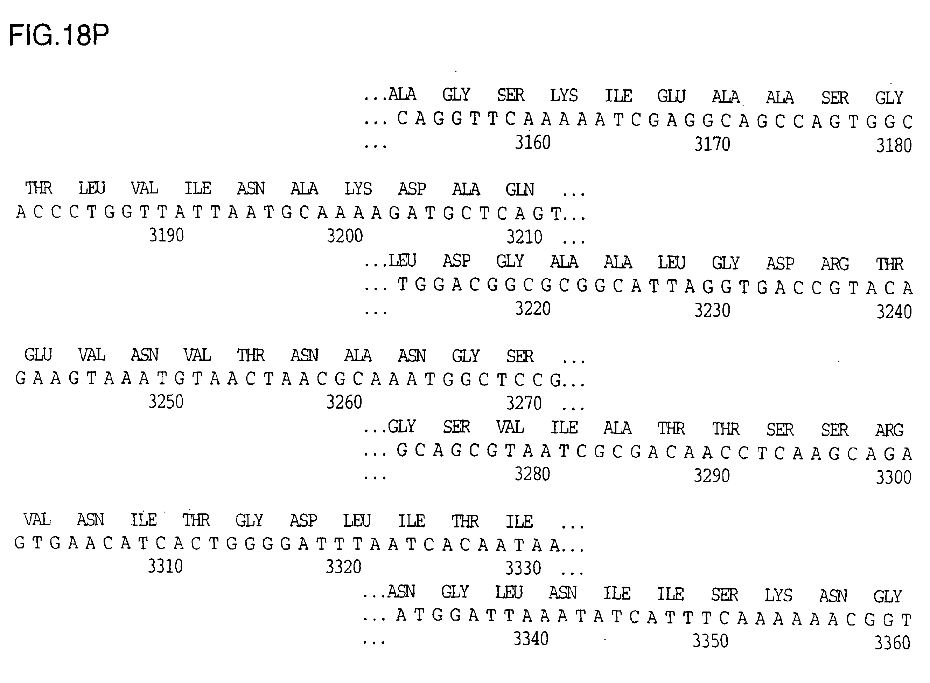

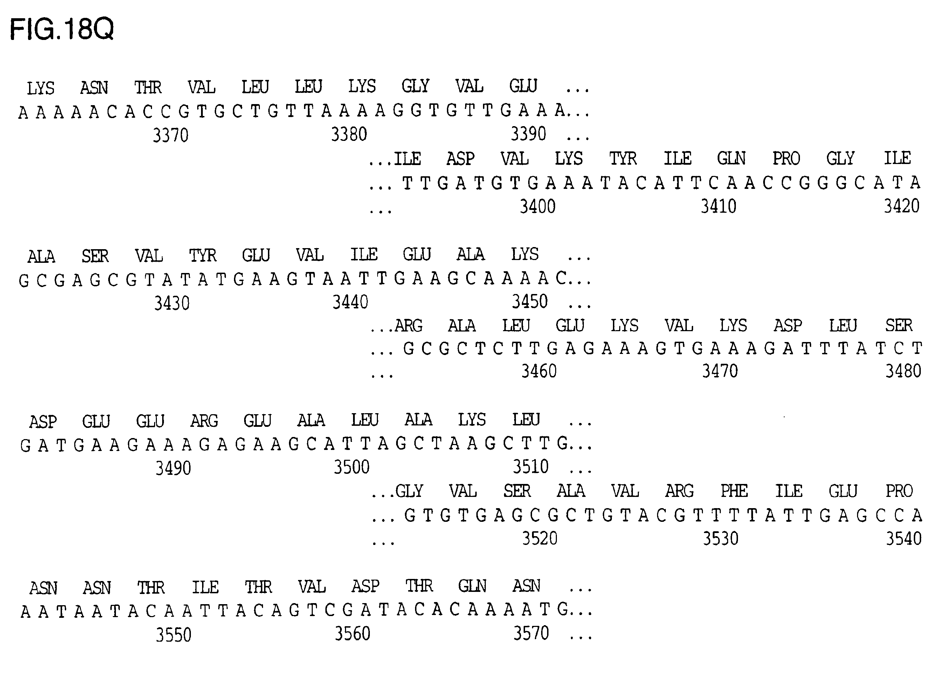

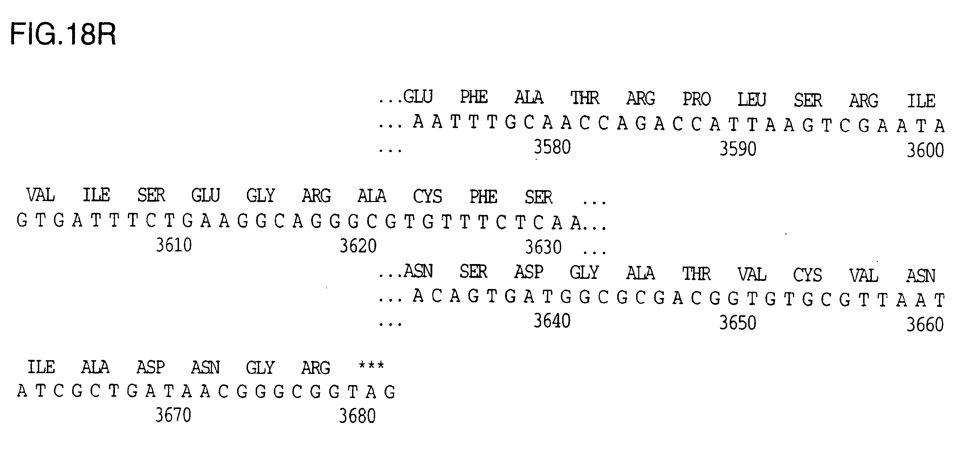

Accordingly, there is described an isolated and purified nucleic acid molecule encoding a high molecular weight (HMW) protein of a non-typeable strain of *Haemophilus influenzae* having:
(a) a DNA sequence selected from the group consisting of those shown in Figures 18, 19, 20, 21, 22, 23, 24, 25, 26 and 27 (SEQ ID NOS: 25, 27, 29, 32, 33, 36, 38, 40, 42, 44, 46, 48, 50, 52, 54, 56, 58, 60 62, 64), or a sequence complementary thereto; or
(b) a DNA sequence encoding a high molecular weight protein having an amino acid sequence selected from the group consisting of those shown in Figures 18, 19, 20, 21, 22, 23, 24, 25, 26 and 27 (SEQ ID NOS: 26, 28, 30, 32, 34, 35, 37, 39, 41, 43, 45, 47, 49, 51, 53, 55, 57, 59, 61, 63, 65), or a sequence complementary thereto.

The modified operon in the first aspect of the invention may include the mature protein encoding sequences (SEQ ID NOS: 27, 31, 36, 40, 44, 48, 52, 56, 60, 64, 68,72) or a DNA molecule encoding the mature protein having the amino acid sequences (SEQ ID NOS: 28, 32, 37, 41, 45, 49, 53, 57, 61, 65, 69,73) shown in Figures 18, 19, 20, 21, 22, 23, 24, 25, 26, 27, 28 and 29.

The nucleic acid molecule provided in accordance with the first aspect of the invention may further comprise a sequence containing at least one additional copy of the mature encoding region only of the operon of a non-typeable strain of *Haemophilus,* the cer gene of *E. coli* or both such segments.

The nucleic acid molecules provided in accordance with the first aspect of the invention may be incorporated into a vector, usually a plasmid vector, for transformation of *E. coli* for the purpose of expression of the mature protective high molecular protein of a non-typeable strain of *Haemophilus*.

Plasmid vectors for the latter purpose may have the identifying characteristics of a plasmid which is selected from the group consisting of:
DS-1046-1-1
JB-2507-7
BK-86-1-1
BK-35-4
BK-76-1-1
DS-2334-5
DS-2400-13

Details of the structures and preparation of such plasmids is provided in the Figures and Examples.

There is described a strain of *E. coli* transformed by the vectors provided herein and expressing a protective high molecular weight protein of a non-typeable strain of *Haemophilus*. The present invention further includes an isolated and purified recombinant protective high molecular weight protein of a non-typeable strain of *Haemophilus*, immunogenic segment or analog thereof, producible by the transformed *E. coli*.

There is described a recombinant method for a production of a protective high molecular weight protein of a non-typeable strain of *Haemophilus*, which comprises:
transforming *E. coli* with a vector comprising the nucleic acid molecule provided in the first aspect of the invention,
growing *E. coli* to express the encoded mature high molecular weight (HMW) protein, and
isolating and purifying the expressed HMW protein.

The non-typeable strain of *Haemophilus* may be any of the strains referred to above and the high molecular weight protein may be the HMW1 protein or HMW2 protein, which is provided in a form free from contamination by the other protein. The purification steps may include separating the HMW A protein from the B and C protein.

The present invention, in its various aspects, permits the production of protective high molecular weight proteins of non-typeable *Haemophilus* which are useful in providing immunogenic compositions to confer protection against disease caused by infection by non-typeable *Haemophilus* strains.

### BRIEF DESCRIPTION OF DRAWINGS

The present invention will be further understood from the following description with reference to the drawings, in which:
Figure 1A shows the construction scheme to generate plasmid DS-1091-2 that expresses the *hmw1ABC* genes encoding the full-length 160 kDa HMW1A protein. Restriction enzyme sites are: B, *BamH* I; Bg, *Bgl* II; Bs, *Bsm* I; H, *Hind* III; R, *EcoR* I; Xb, *Xba* I. Other abbreviations are: T7p, T7 promoter; HMW1p, *hmw1* promoter; ApR, ampicillin resistance gene; CAP, calf alkaline phosphatase.
Figure 1B shows the sequence of the oligonucleotides used in the construction scheme of Figure 1A (SEQ ID NOS: 1, 2 and 3).
Figure 2 shows the construction scheme to generate plasmid DS-1094-2 that expresses the *hmw2ABC* genes encoding the full-length 155 kDa HMW2A protein. Restriction enzyme sites are: Bg, *Bgl* II; Bs, *Bsm* I; H, *Hind* III; R, *EcoR* I; Xb, *Xba* I. Other abbreviations are: T7p, T7 promoter; HMW2p, *hmw2* promoter; ApR, ampicillin resistance gene; CAP, calf alkaline phosphatase.
Figure 3A shows the construction scheme to generate plasmid DS-1046-1-1 that expresses the *hmw1ABC* genes encoding the mature 125 kDa HMW1A protein. Restriction enzyme sites are: B, *BamH* I; Bg, *Bgl* II; H, *Hind* III; R, *EcoR* I; Xb, *Xba* I. Other abbreviations are: T7p, T7 promoter; HMW1p, *hmw1* promoter; ApR, ampicillin resistance gene.
Figure 3B shows the sequence of the oligonucleotides used in the construction scheme of Figure 3A (SEQ ID NOS: 4, 5 and 6).
Figure 4A shows the construction scheme to generate plasmid DS-1200-3 that expresses the *hmw2AB* genes encoding the mature 120 kDa HMW2A protein. Restriction enzyme sites are: Bg, *Bgl* II; H, *Hind* III; R, *EcoR* I; S, *Sal* I; Xb, *Xba* I; Xho, *Xho* I. Other abbreviations are: T7p, T7 promoter; HMW2p, *hmw2* promoter; ApR, ampicillin resistance gene; CAP, calf alkaline phosphatase.
Figure 4B shows the oligonucleotides used in the construction scheme of Figure 4A (SEQ ID NOS: 7, 8 and 9).
Figure 5 shows the construction scheme to generate plasmid DS-1122-2 that contains the *hmw1A* gene encoding the mature 125 kDa HMW1A protein, and part of the *hmw1B* gene. Restriction enzyme sites are: B, *BamH* I; Bg, *Bgl* II; H, *Hind* III; R, *EcoR* I; Xb, *Xba* I. Other abbreviations are: T7p, T7 promoter; ApR, ampicillin resistance gene.
Figure 6A shows the construction scheme to generate plasmid JB-2330-7 that expresses the *hmw1A* gene encoding the mature 125 kDa HMW1A protein. Restriction enzyme sites are: B, *BamH* I; Bg, *Bgl* II; H, *Hind* III; K, *Kpn* I; R, *EcoR* I; Xho, *Xho* I. Other abbreviations are: T7p, T7 promoter; ApR, ampicillin resistance gene; KanR, kanamycin resistance gene, CAP, calf alkaline phosphatase.
Figure 6B shows the oligonucleotides used to PCR amplify the 3'-end of *hmw1A* in the construction scheme of Figure 6A (SEQ ID NOS: 10, 11, 12, 13 and 14).
Figure 7 shows the construction scheme to generate plasmid JB-2369-6 that expresses tandem copies of the T7 hmw1a gene cassette encoding the mature 125 kDa HMW1A protein. Restriction enzyme sites are: B, *BamH* I; Bg, *Bgl* II; H, *Hind* III; K, *Kpn* I; R, *EcoR* I; S, *Sal* I; Xb, *Xba* I; Xho, *Xho* I. Other abbreviations are: T7p, T7 promoter; ApR, ampicillin resistance gene; TcR, tetracycline resistance gene; CAP, calf alkaline phosphatase; tt1, transcription terminator 1; tt2 transcription terminator 2; MCS, multiple cloning site.
Figure 8A shows the construction scheme to generate plasmids DS-2084-3 and DS-2084-1 that contain one or two copies, respectively, of the T7 *hmw2A* gene cassette encoding the mature 120 kDa HMW2A protein. Restriction enzyme sites are: B, *BamH* I; Bg, *Bgl* II; H, *Hind* III; M, *Mlu* I; R, *EcoR* I; Xb, *Xba* I. Other abbreviations are: T7p, T7 promoter; ApR, ampicillin resistance gene; TcR, tetracycline resistance gene; CAP, calf alkaline phosphatase; tt1, transcription terminator 1; tt2, transcription terminator 2; MCS, multiple cloning site.
Figure 8B shows the oligonucleotides used to PCR amplify the 3'-end of *hmw2A* in the construction scheme of Figure 8A (SEQ ID NOS: 15, 16, 17, 18 and 19).
Figure 9 shows the construction scheme to generate plasmids JB-2507-7 and BK-86-1-1 that contain tandem T7 *hmw1A*/*T7 hmw1ABC* genes encoding the mature 125 kDa HMW1A protein, with ampicillin or kanamycin selection, respectively. Restriction enzyme sites are: B, *BamH I;* Bg, *Bgl* II ; H, *Hind* III; K, *Kpn* I ; R, *EcoR I ;* S, *Sal* I; Xb, *Xba* I; Xho, *Xho* I. Other abbreviations are: T7p, T7 promoter; ApR, ampicillin resistance gene; KanR, kanamycin resistance gene; TcR, tetracycline resistance gene; CAP, calf alkaline phosphatase;
Figure 10 shows the construction scheme to generate plasmids BK-35-4 and BK-76-1-1 that contain T7 *hmw1ABC*/*cer* genes encoding the mature 125 kDa HMW1A protein, utilizing ampicillin or kanamycin selection, respectively. Restriction enzyme sites are: B, *BamH I;* Bg, *Bgl* II; H, *Hind* III; K, *Kpn* I; R, *EcoR I;* S, *Sal* I; Xb, *Xba* I. Other abbreviations are: T7p, T7 promoter; ApR, ampicillin resistance gene; KanR, kanamycin resistance gene; CAP, calf alkaline phosphatase.

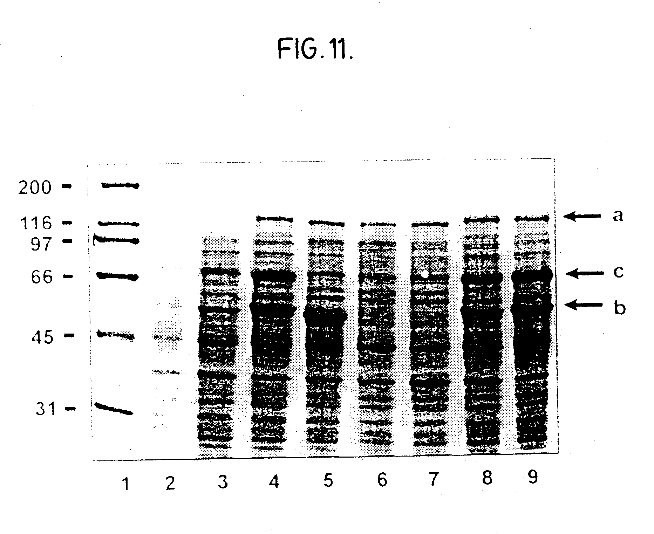

Figure 11 shows SDS-PAGE analysis of the expression of recombinant HMW1 proteins from various constructs. Lane 1, broad range molecular weight markers; lane 2, DS-1046-1-1 [*pT7 hmw1ABC (125)*]*,* no induction; lane 3, DS-1091-2 [*pT7 hmw1ABC (160)*] ; lane 4, DS-1046-1-1 [*pT7 hmw1ABC (125)*] ; lane 5, DS-1122-2 [*pT7 hmw1a partial B (125)*] *;* lane 6, JB-2330-7 [*pT7 hmw1A (125)];* lane 7, JB-2369-6 [pBr328 *T7 hmw1A (125)*/*T7 hmw1A (125)*] *;* lane 8, BK-86-1-1 [*pT7 hmw1A (125)*/*T7 hmw1ABC (125)*/*kanR*] ; lane 9, BK-76-1-1 [*pT7 hmw1ABC (125)*/*cer*/*kanR*]*;* lane 10, broad range molecular weight markers.
Figure 12 shows a purification scheme for recombinant HMW1 and HMW2 proteins. Abbreviations are: PPT, pellet; SUP, supernatant; OG, octylglucoside; PEG, polyethylene glycol.

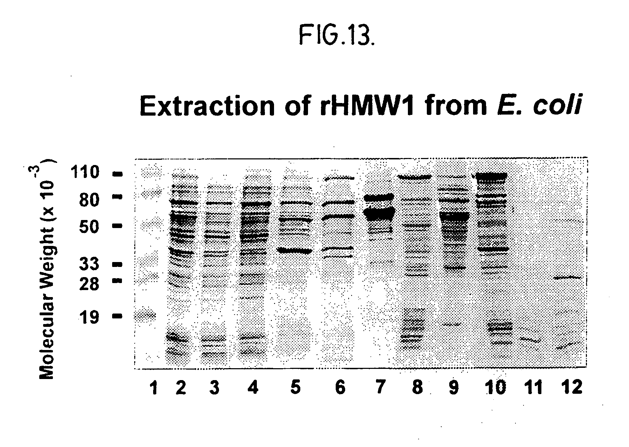

Figure 13 shows the SDS-PAGE analysis of rHMW1 extractions. Lane 1, prestained protein molecular weight markers; lane 2, *E. coli* whole cell lysates; lane 3, soluble proteins in the Tris-HCl/NaCl extraction; lane 4, soluble proteins in the Tris-HCl/Triton X-100/EDTA extraction; lane 5, soluble proteins in the Tris-HCl/octylglucoside extraction; lane 6, pellet after Tris-HC1/octylglucoside extraction; lane 7, insoluble proteins in 2M guanidine HCl; lane 8, supernatant of 7% PEG precipitation; lane 9, pellet of 7% PEG precipitation; lane 10, interphase pellet of 50% ammonium sulfate precipitation; lane 11, proteins recovered in the lower phase; lane 12, proteins recovered in the upper phase.
Figure 14, comprising panels A and B, shows an SDS-PAGE analysis of the purified rHMW1 and rHMW2.
Figure 15 contains an SDS-PAGE analysis showing the stability of rHMW1 from construct T7 *hmwABC*/*cer*/*kanR* stored at -20°C in the presence of 20% glycerol.
Figure 16, comprising panels A and B, shows the immunogenicity of rHMW1A protein produced from various constructs.
Figure 17 shows the sequences of oligonucleotides used to PCR amplify additional *hmw* genes from non-typable *H. influenzae* chromosomal DNA (SEQ ID NOS: 20, 21, 22, 23 and 24).
Figure 18 shows the nucleotide sequence (SEQ ID NO: 25) and deduced amino acid sequence (SEQ ID NO: 26) of the *hmw1A* gene from NTHi strain Joyc. The arrow marks the predicted start of the mature protein (mature protein: encoding sequence SEQ ID NO: 27; amino acid sequence SEQ ID NO: 28).
Figure 19 shows the nucleotide sequence (SEQ ID NO: 29) and deduced amino acid sequence (SEQ ID NO: 30) of the *hmw2A* gene from NTHi strain Joyc. The arrow marks the predicted start of the mature protein (mature protein: encoding sequence SEQ ID NO: 31; amino acid sequence SEQ ID NO: 32).
Figure 20 shows the nucleotide sequence (SEQ ID NO: 33) and deduced amino acid sequences (SEQ ID NO: 34, 35) of the defective *hmw1A* gene from NTHi strain K1. The arrow marks the predicted start of the mature protein (mature protein: encoding sequence SEQ ID NO: 36; amino acid sequence SEQ ID NOS: 37, 35).
Figure 21 shows the nucleotide sequence (SEQ ID NO: 38) and deduced amino acid sequence (SEQ ID NO: 39) of the *hmw2A* gene from NTHi strain K21. The arrow marks the predicted start of the mature protein (mature protein: encoding sequence SEQ ID NO: 40; amino acid sequence SEQ ID NO: 41).
   Figure 22 shows the nucleotide sequence (SEQ ID NO: 42) and deduced amino acid sequence (SEQ ID NO: 43) of the *hmw1A* gene from NTHi strain LCDC2. The arrow marks the predicted start of the mature protein (mature protein: encoding sequence SEQ ID NO: 44; amino acid sequence SEQ ID NO: 45).
   Figure 23 shows the nucleotide sequence (SEQ ID NO: 46) and deduced amino acid sequence (SEQ ID NO: 47) of the *hmw2A* gene from NTHi strain LCDC2. The arrow marks the predicted start of the mature protein (mature protein: encoding sequence SEQ ID NO: 48; amino acid sequence SEQ ID NO: 49).
   Figure 24 shows the nucleotide sequence (SEQ ID NO: 50) and deduced amino acid sequence (SEQ ID NO: 51) of the *hmw1A* gene from NTHi strain PMH1. The arrow marks the predicted start of the mature protein (mature protein: encoding sequence SEQ ID NO: 52; amino acid sequence SEQ ID NO: 53).
   Figure 25 shows the nucleotide sequence (SEQ ID NO: 54) and deduced amino acid sequence (SEQ ID NO: 55) of the *hmw2A* gene from NTHi strain PMH1. The arrow marks the predicted start of the mature protein (mature protein: encoding sequence SEQ ID NO: 56; amino acid sequence SEQ ID NO: 57).
   Figure 26 shows the nucleotide sequence (SEQ ID NO: 58) and deduced amino acid sequence (SEQ ID NO: 59) of the *hmw1A* gene from NTHi strain 15. The arrow marks the predicted start of the mature protein (mature protein: encoding sequence SEQ ID NO: 60; amino acid sequence SEQ ID NO: 61).
Figure 27 shows the nucleotide sequence (SEQ ID NO: 62) and deduced amino acid sequence (SEQ ID NO: 63) of the *hmw2A* gene from NTHi strain 15. The arrow marks the predicted start of the mature protein (mature protein: encoding sequence SEQ ID NO: 64; amino acid sequence SEQ ID NO: 65).
Figure 28 shows the nucleotide sequence (SEQ ID NO: 66) and deduced amino acid sequence (SEQ ID NO: 67 of the *hmw1A* gene from NTHi strain 12. The arrow marks the predicted start of the mature protein (mature protein: encoding sequence SEQ ID NO: 68; amino acid sequence SEQ ID NO: 69).
Figure 29 shows the nucleotide sequence (SEQ ID NO: 70) and deduced amino acid sequence (SEQ ID NO: 71 of the *hmw2A* gene from NTHi strain 12. The arrow marks the predicted start of the mature protein (mature protein: encoding sequence SEQ ID NO: 72; amino acid sequence SEQ ID NO: 73).
Figure 30 shows the alignment of the deduced HMW1A and HMW2A protein sequences (SEQ ID NOS: 26, 30, 34, 35, 39, 43, 47, 51, 55, 59, 63) with the published strain 12 HMW1A and HMW2A protein sequences (USP 5,603,938) (SEQ ID NOS: 67, 71).
Figure 31 shows the oligonucleotides (SEQ ID NOS: 74, 75, 76, 77, 78, 79, 80, 81) used to determine whether the PCR amplified *hmwA* genes were *hmw1* or *hmw2.*
Figure 32A shows the construction scheme to generate the generic *T7 hmwABC* expression plasmid JB-2646-1 into which can be inserted any *hmwA* gene. Restriction enzyme sites are: B, *BamH* I; Bg, *Bgl* II; H, *Hind* III; K, *Kpn* I; R, *EcoR* I; S, *Sal* I; Xb, *Xba* I. Other abbreviations are: T7p, T7 promoter; ApR, ampicillin resistance gene; KanR, kanamycin resistance gene; CAP, calf alkaline phosphatase.
Figure 32B illustrates the oligonucleotides (SEQ ID NOS: 82, 83, 84, 85, 86, 87, 88, 89, 90) used to PCR amplify the 3'-end of *hmw1A* and the 5'-end of *hmw1B* in the construction scheme of Figure 32A.
Figure 33A shows the construction of DS-2334-5 that contains a chimeric *T7 hmwABC* gene of the LCDC2 *hmw2A* gene and NTHI 12 *hmwBC* genes. Restriction enzyme sites are: B, *BamH* I; Bg, *Bgl* II; H, *Hind* III; K, *Kpn* I; N, *Nde* I; R, *EcoR* I; S, *Sal* I; Xb, *Xba* I, Xho, *Xho* I. Other abbreviations are: T7p, T7 promoter; ApR, ampicillin resistance gene; KanR, kanamycin resistance gene; CAP, calf alkaline phosphatase.
Figure 33B shows the oligonucleotides (SEQ ID NOS: 91, 92, 93, 94, 95) used to PCR amplify the LCDC2 *hmw2A* gene for expression in the generic expression vector constructed as shown in Figure 33A.
Figure 34 shows the construction of DS-2400-13 that contains the *T7 hmwA*/*T7 hmwABC* genes and the *E. coli* cer gene. Restriction enzyme sites are: B, *BamH* I; Bg, *Bgl* II; H, *Hind* III; R, *EcoR* I; S, *Sal* I; Xb, *Xba* I, Xho, *Xho I.* Other abbreviations are: T7p, T7 promoter; ApR, ampicillin resistance gene; KanR, kanamycin resistance gene; TetR, tetracycline resistance gene; CAP, calf alkaline phosphatase.

### GENERAL DESCRIPTION OF THE INVENTION

Any *Haemophilus* strain that has *hmw* genes may be conveniently used to provide the purified and isolated nucleic acid molecules (which may be in the form of DNA molecules), comprising at least a portion coding for a HMW1A, HMW1B, HMW1C, HMW2A, HMW2B, or HMW2C protein as described herein. Such strains are generally available from clinical sources and from bacterial culture collections, such as the American Type Culture Collection. Appropriate strains of non-typeable *Haemophilus* include:
Non-typeable *H. influenzae* strain 12
Non-typeable *H. influenzae* strain Joyc;
Non-typeable *H. influenzae* strain K1;
Non-typeable *H. influenzae* strain K21;
Non-typeable *H. influenzae* strain LCDC2;
Non-typeable *H. influenzae* strain PMH1; or
Non-typeable *H. influenzae* strain 15.

In this application, the term "HMW protein" is used to define a family of HMW proteins which includes those having naturally occurring variations in their amino acid sequences as found in various strains of non-typeable *Haemophilus* and characterized by an apparent molecular weight of about 100 to about 150.

Reference will now be made in detail to the presently preferred embodiments of the invention, which together with the following Examples, serve to explain the principle of the invention. For clarity of disclosure, and not by way of limitation, the detailed description of the invention is divided into the following sections:

### 1. Improved production of recombinant HMW proteins from E. coli.

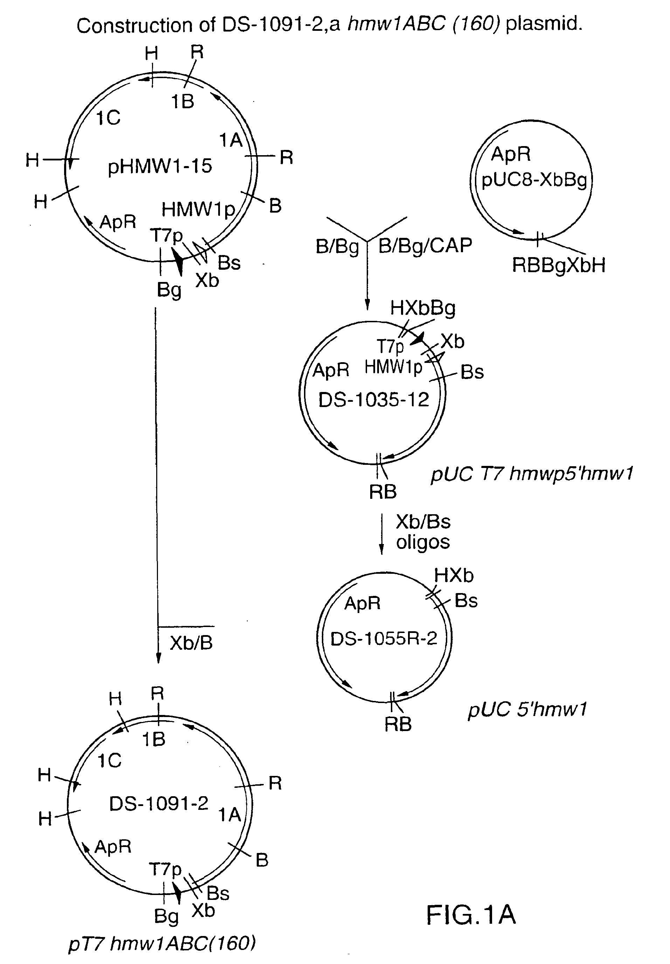

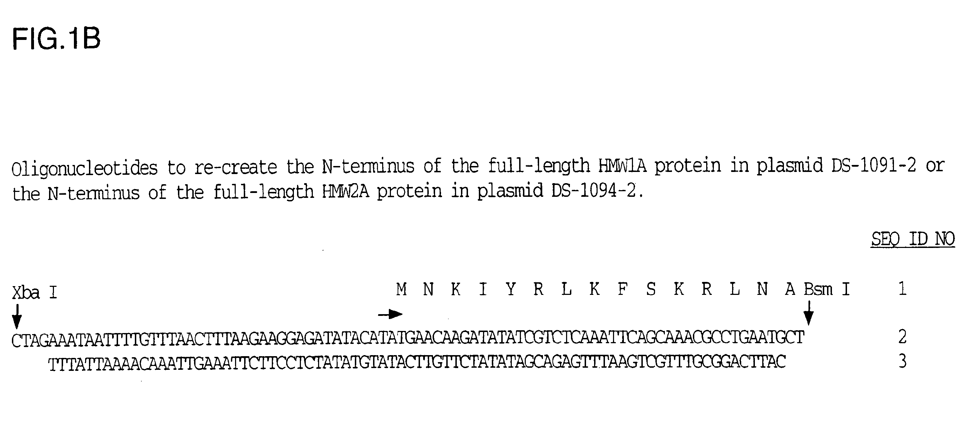

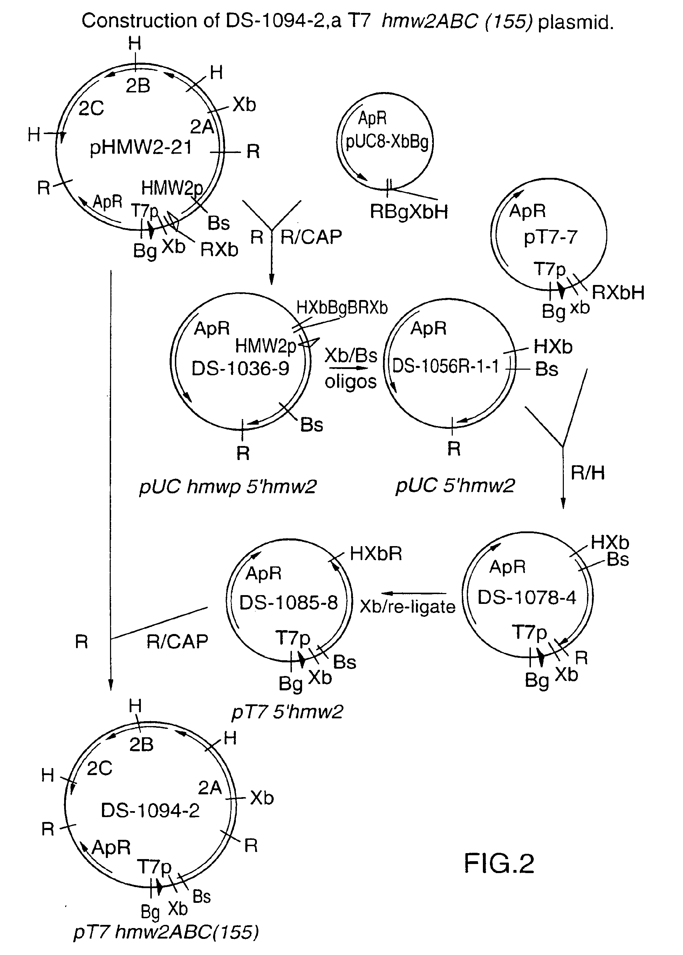

The production of native HMW1A or HMW2A proteins in *H. influenzae* is very low. The plasmids pHMW1-15 and pHMW2-21 (refs. 8, 10) contain the complete *hmw1ABC* and *hmw2ABC* operons from NTHi strain 12 cloned into the expression vector pT7-7. The production of the recombinant rHMW1A or rHMW2A proteins is low from these plasmids, possibly due to the 5'-flanking and *hmw* promoter sequences inserted between the T7 promoter and the start codon of the *hmwA* genes. By removal of the 5'-flanking and *hmw* promoter sequences, the yield of rHMW1A and rHMW2A proteins produced from plasmids DS-1091-2 and DS-1094-2 (Figures 1 and 2), was marginally improved.

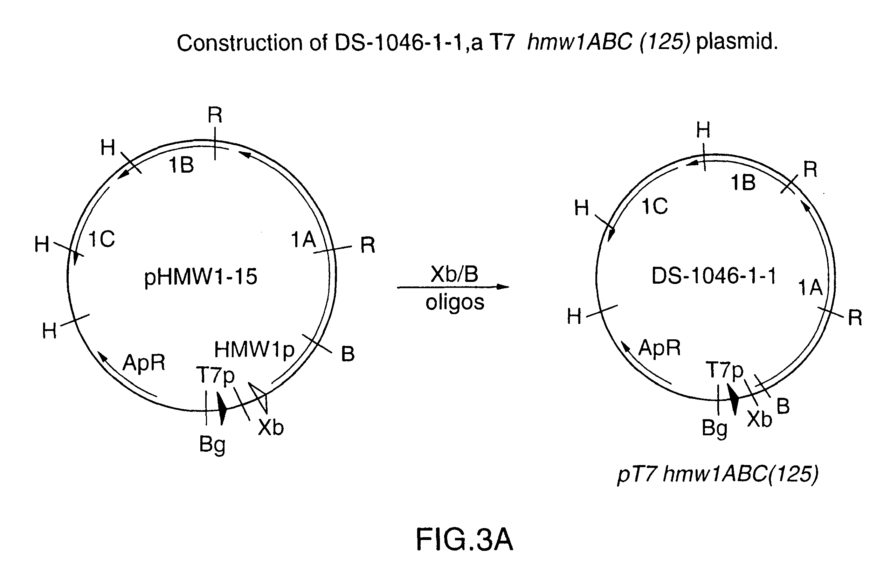

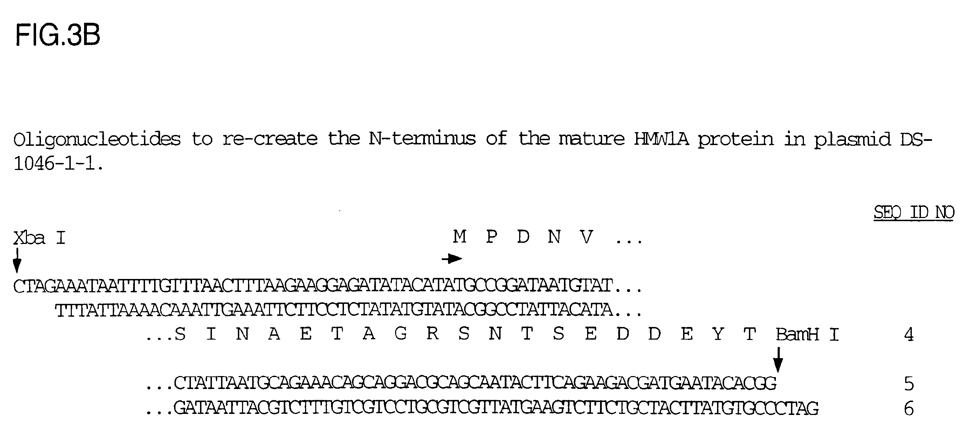

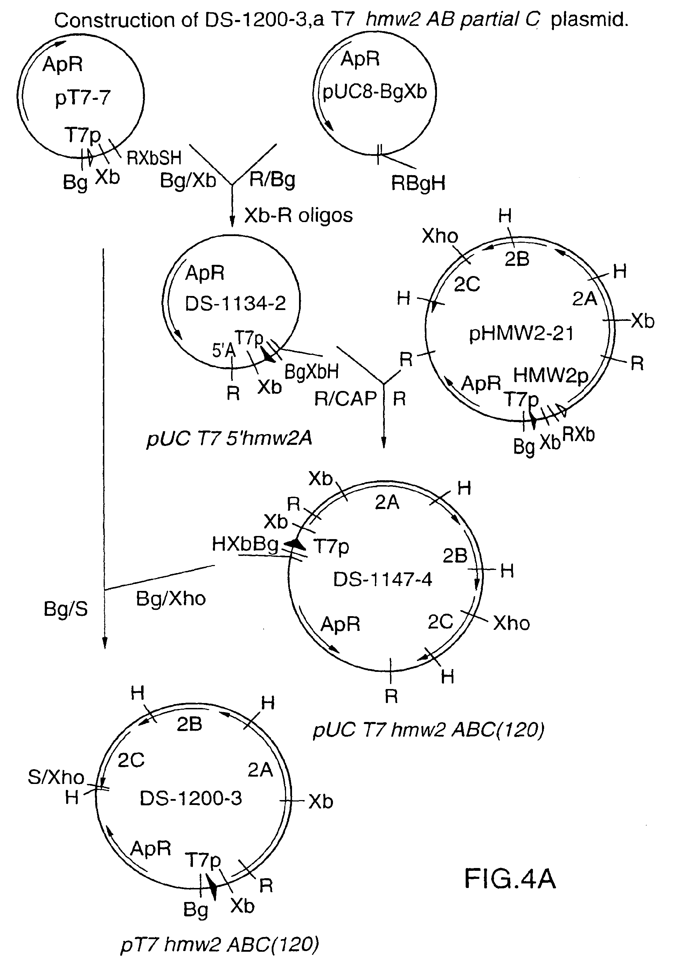

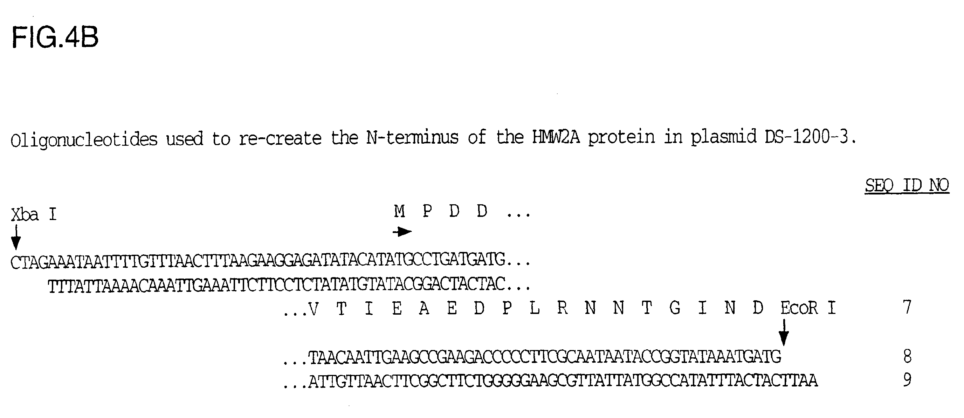

When produced in *H. influenzae*, the native HMWA proteins are processed and secreted, with a 35 kDa N-terminal fragment removed. Rather than relying upon the correct processing and secretion of the rHMWA proteins by *E. coli,* the gene sequences encoding the N-terminal 35 kDa fragments were removed genetically from the *hmw1ABC* and *hmw2ABC* genes. The production of the mature rHMW1A and rHMW2A proteins was enhanced in the resulting new constructs, DS-1046-1-1 and DS-1174-4 (Figures 3 and 4). The rHMW BC proteins were also overproduced. The *hmw1ABC* and *hmw2ABC* gene inserts in the pT7-7 vector were still approximately 8.6kb and approximately 8.3kb, respectively. Since the HMWA proteins are the structural and protective proteins, it was thought that the size of the gene insert could be reduced by deleting part or all of the *hmwBC* genes. Expression vectors with smaller inserts are generally more efficient at producing recombinant proteins and the overproduction of the rHMWBC proteins was thought to be undesirable.

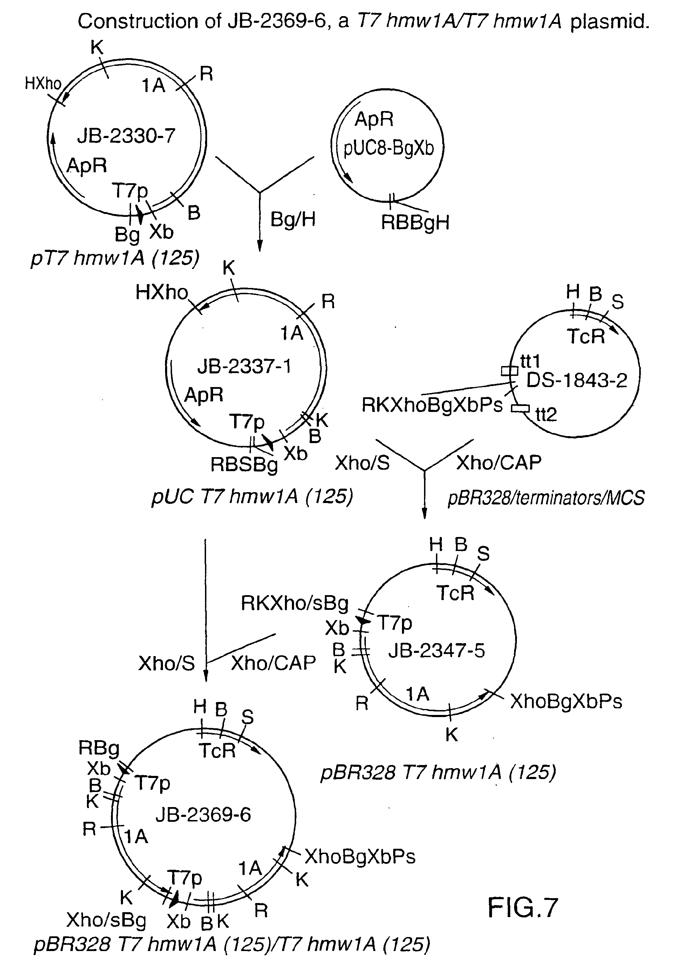

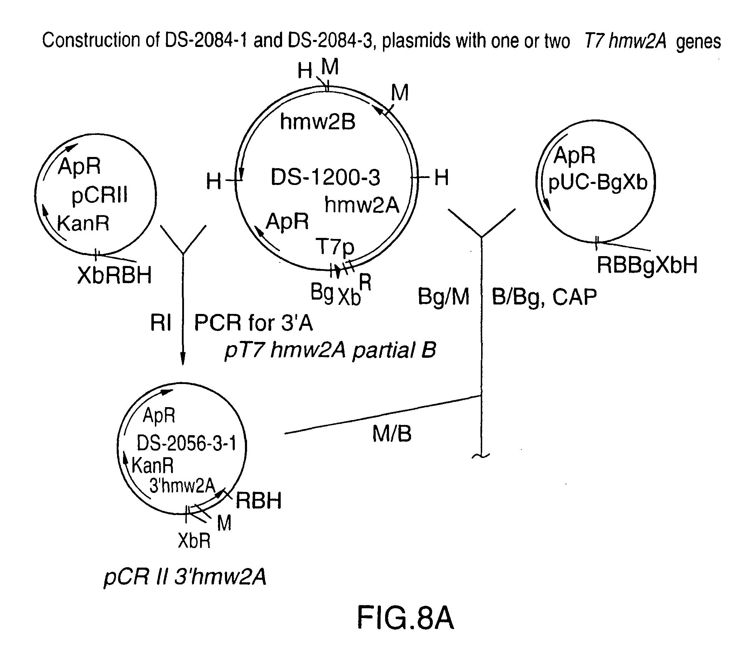

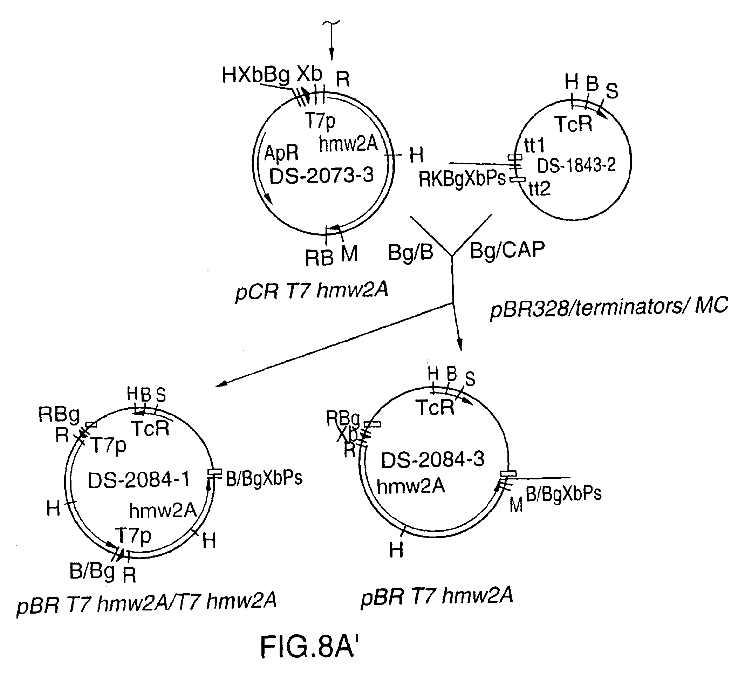

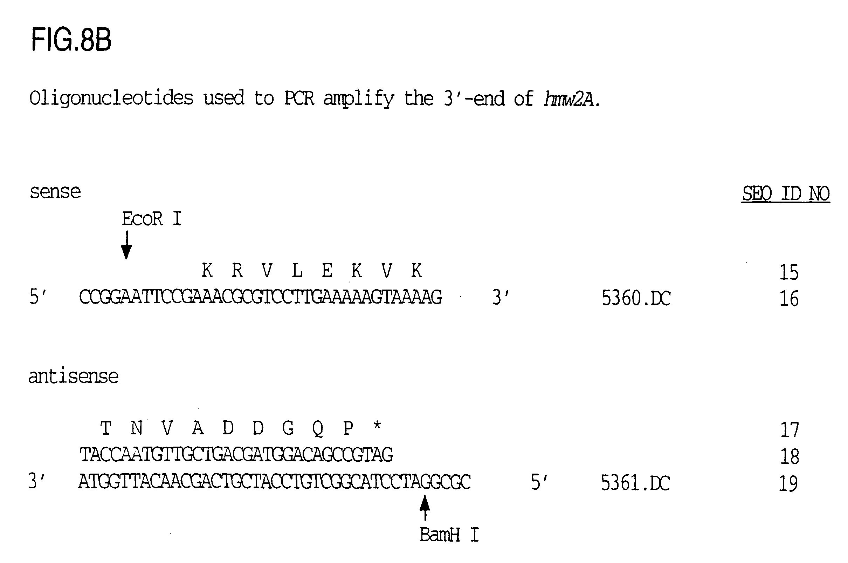

The production of rHMWA proteins was marginally improved when the *hmwBC* genes were deleted in vectors DS-1200-3, DS-1122-2, JB-2330-7 and DS-2084-3 (Figures 4, 5, 6 and 8). However, the production of rHMWB and rHMWC proteins was eliminated, which simplified the protein purification process. When tandem copies of T7 *hmwA* gene cassettes were used to express rHMWA proteins from vectors JB-2369-6 and DS-2084-1 (Figures 7 and 8), the production was marginally improved.

The construction of this series of expression vectors demonstrated that it was possible to increase the production of rHMW1 and rHMW2 proteins from *E. coli*. However, when tested in a nasopharyngeal colonization model for protection, the rHMWA proteins produced from the improved vectors were not protective. Only mixtures of native HMW1A + HMW2A proteins or rHMWA proteins produced from the lower yield vectors containing complete *hmwABC* genes were protective.

### 2. Modification of expression vectors to produce protective recombinant HMWA proteins.

The expression vectors that contained *hmwABC* genes encoding full-length HMW1A (DS-1091-2) or HMW2A (DS-1094-2) proteins and which relied upon *E. coli* to process them, did not produce enough protein to test in animal models. The expression vectors that contained *hmwABC* genes encoding mature HMW1A (DS-1046-1-1) or HMW2A (DS-1174-4) proteins, expressed protective rHMWA proteins in moderate yield. The vectors that overproduced rHMWA proteins alone did not yield protective antigens.

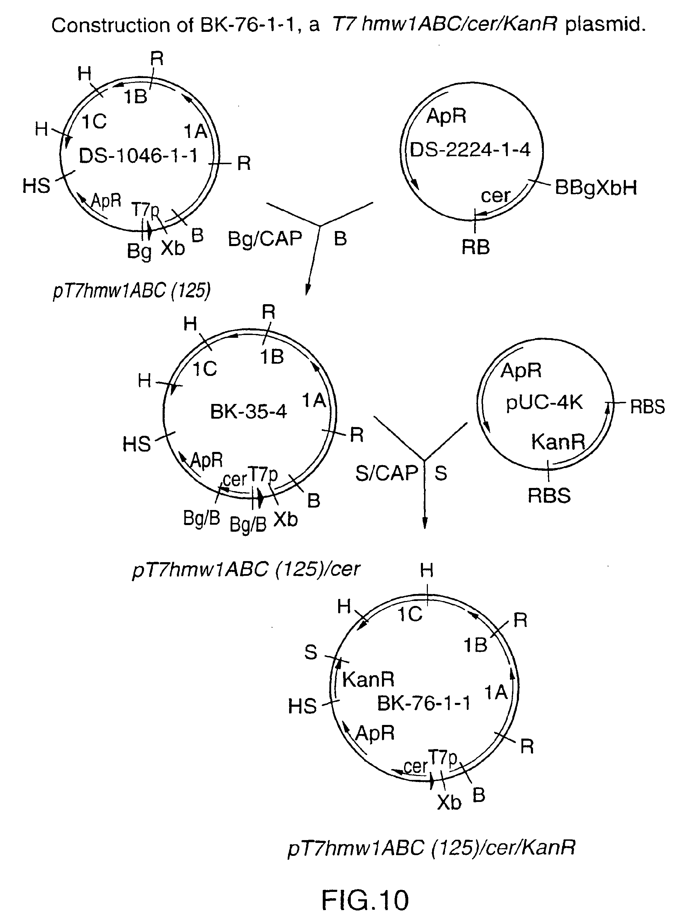

Two approaches were tried to enhance the yield of protective rHMWA protein. To the vector that contained the T7 *hmwABC* gene cassette expressing mature rHMW1A protein, was introduced the *E. coli cer* gene. The cer gene is thought to stabilize plasmids by preventing multimerization and its presence may stabilize expression vectors containing the large *hmwABC* gene cassettes. We had also found that sometimes the presence of cer also increased the production of recombinant proteins. The rHMW1A antigen that was overproduced from *T7 hmw1ABC*/*cer* constructs (Figure 10) was protective in the nasopharyngeal colonization model (Table 2).

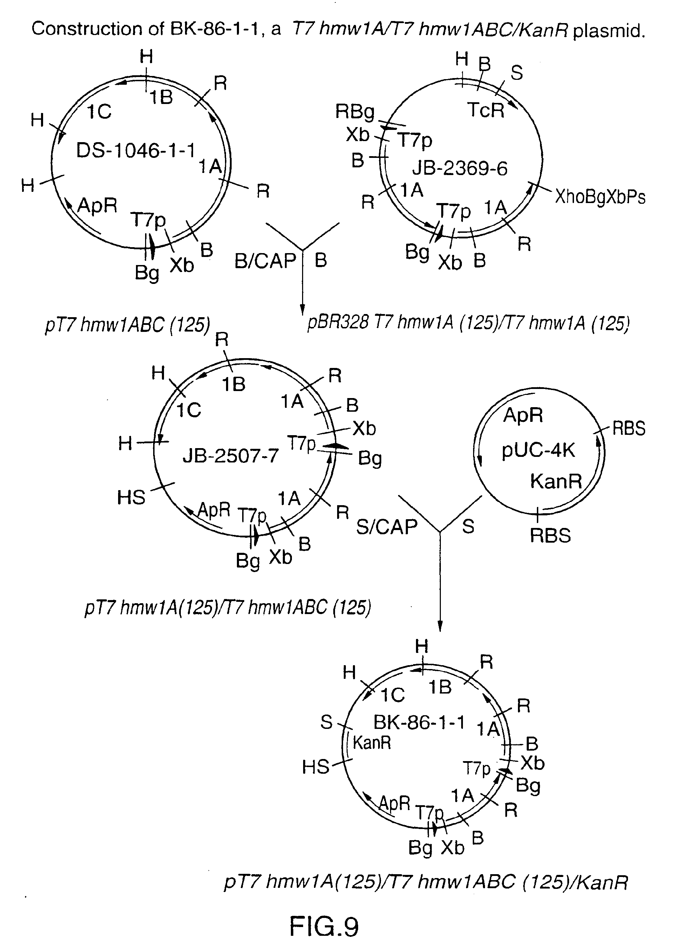

The second approach to overproduce protective rHMWA protein was to construct a vector in which the rHMWA protein was overproduced in the presence of rHMWBC proteins. To the vector that contained the *T7 hmwABC* gene cassette expressing mature rHMW1A protein, was added an additional *T7 hmwA* gene cassette. The rHMW1A antigen that was overproduced from *T7 hmw1A*/*T7 hmw1ABC* constructs (Figure 9) was protective in the nasopharyngeal colonization model (Table 2).

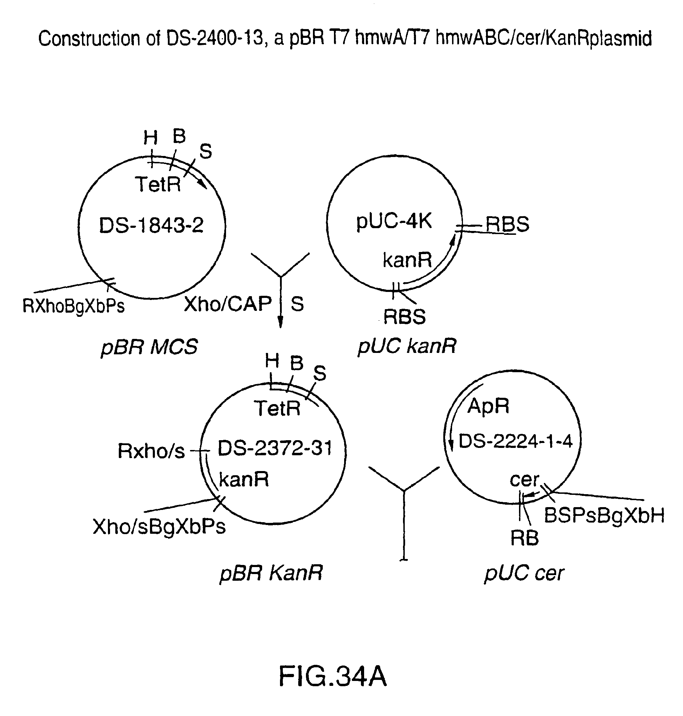

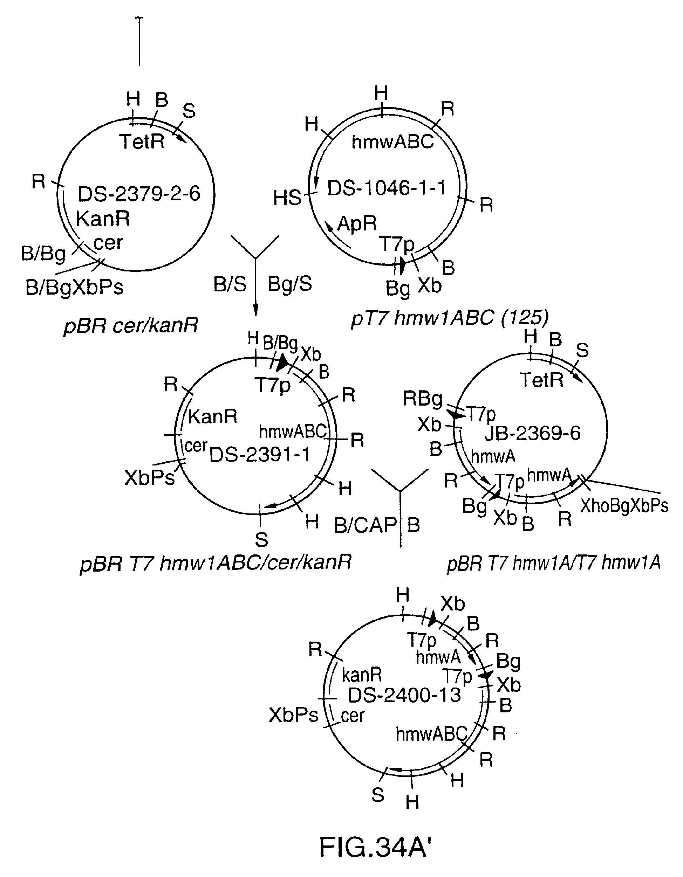

The two approaches can be combined so that tandem copies of the *T7 hmwA*/*T7 hmwABC* genes are co-expressed with the *E. coli* cer gene on the same plasmid, DS-2400-13 (Figure 34).

### 3. Cloning and sequence analysis of additional hmwA genes.

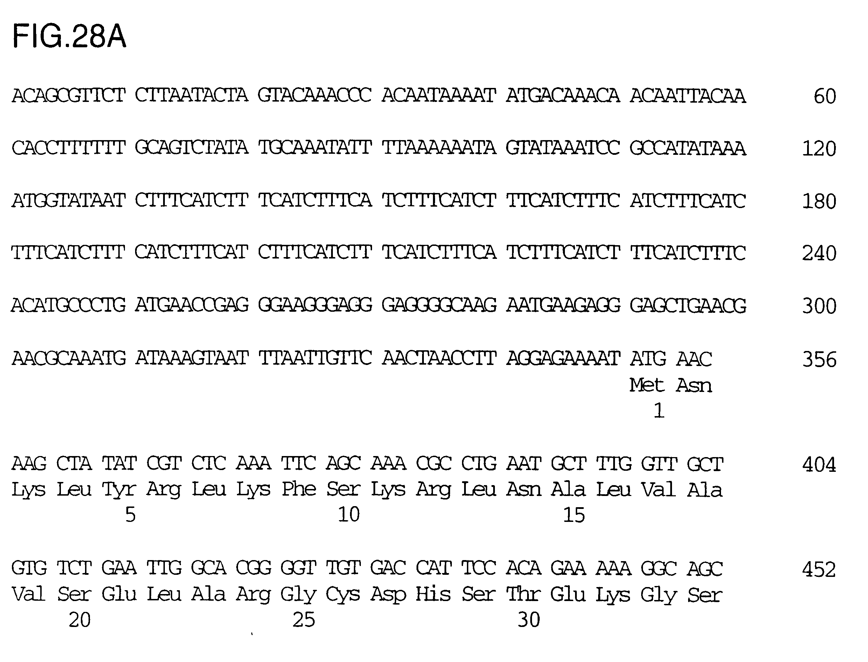

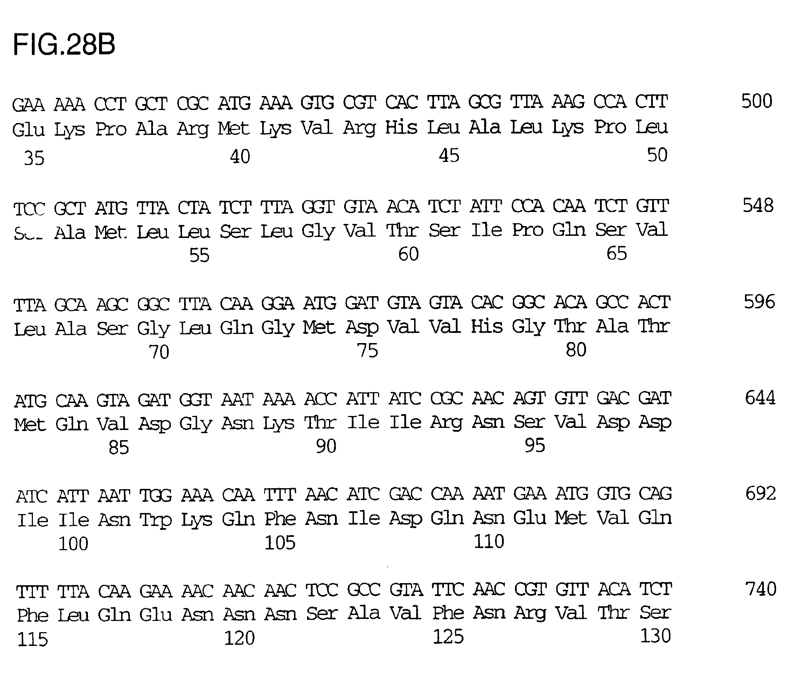

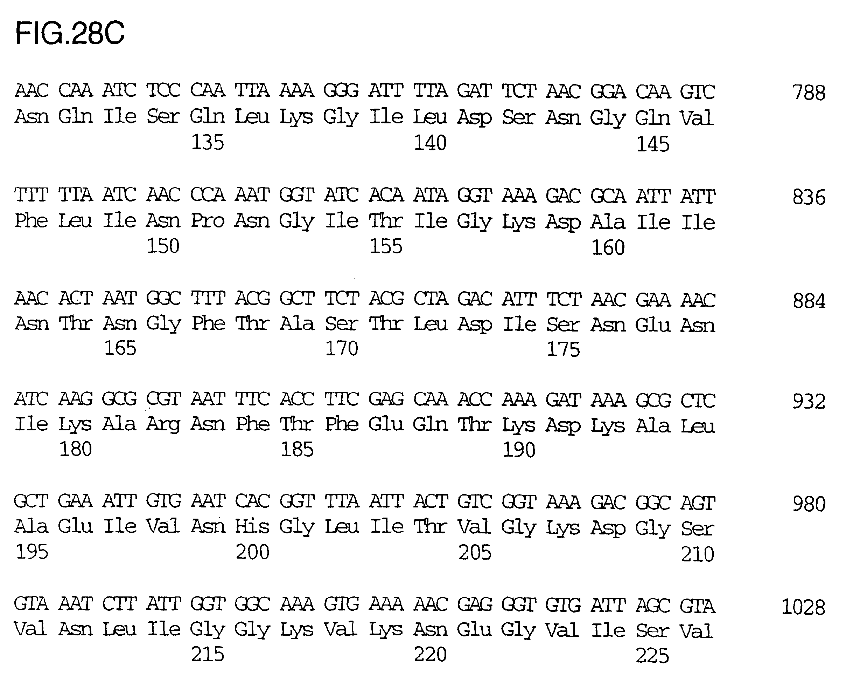

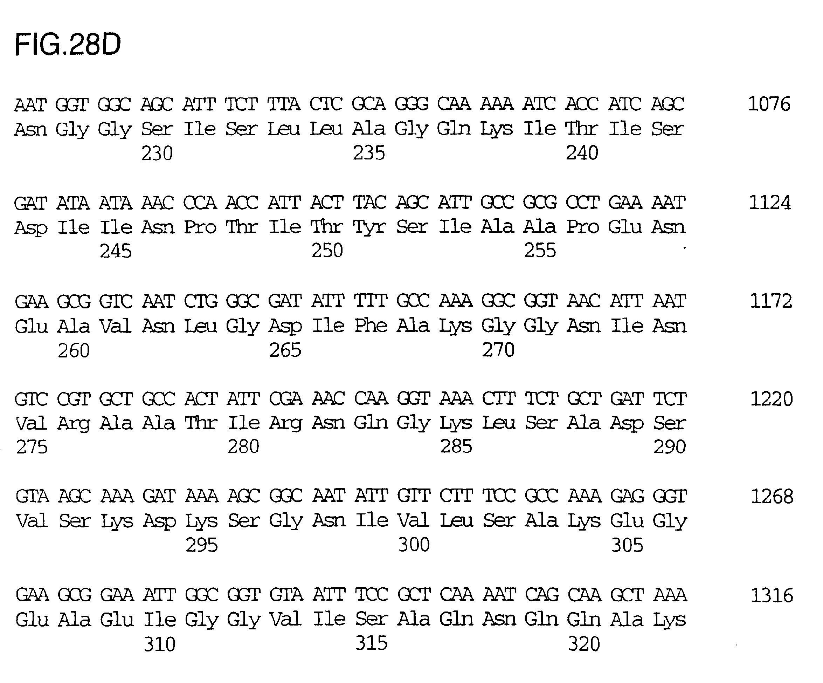

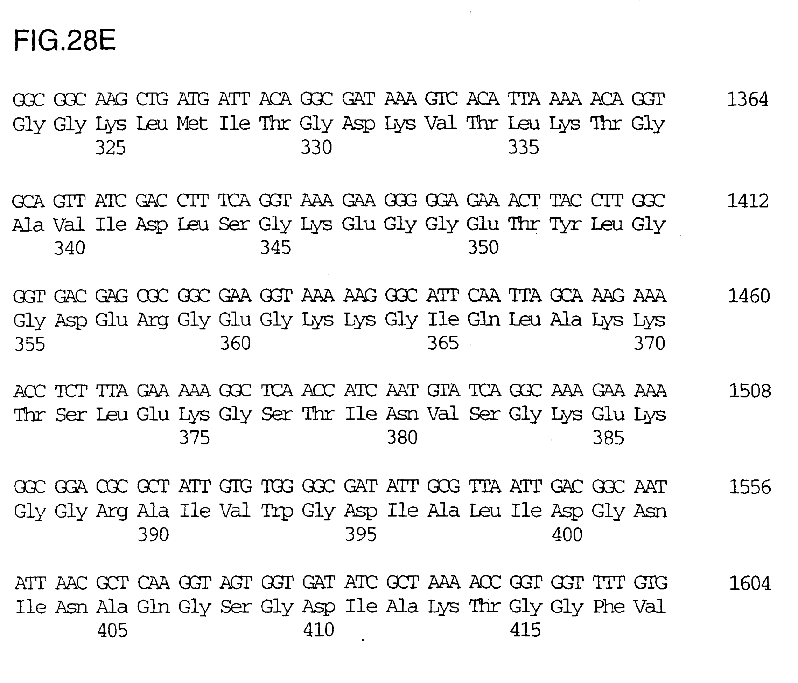

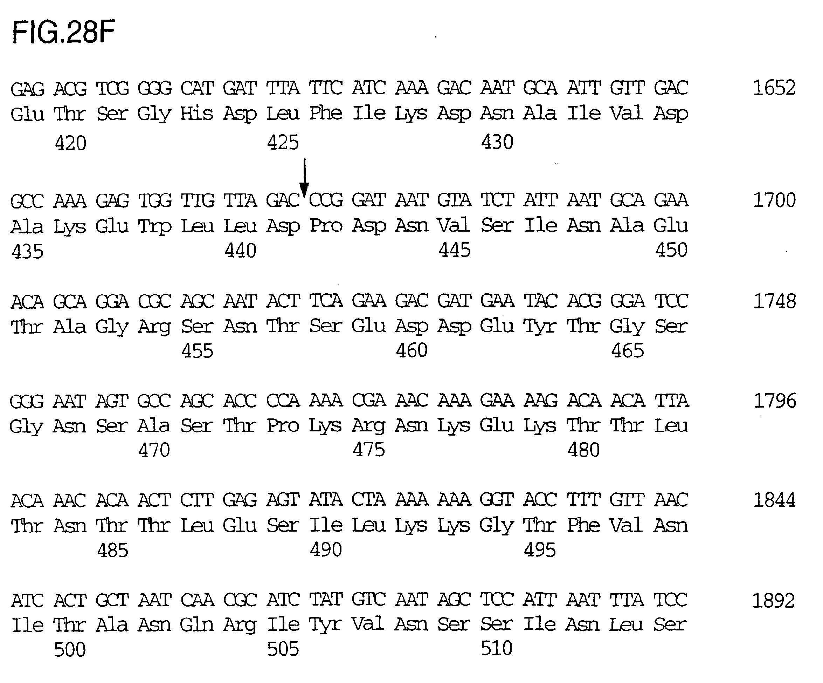

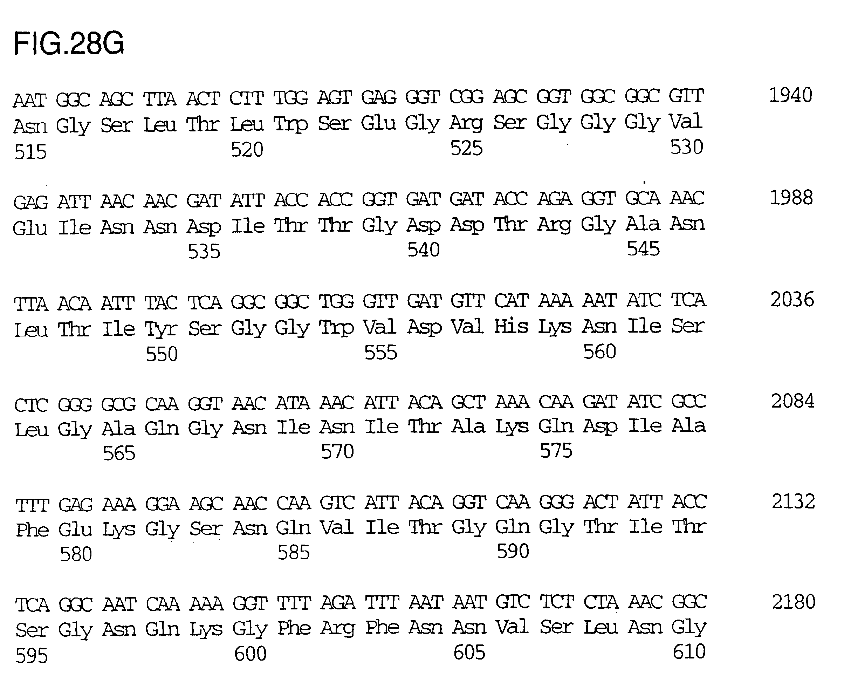

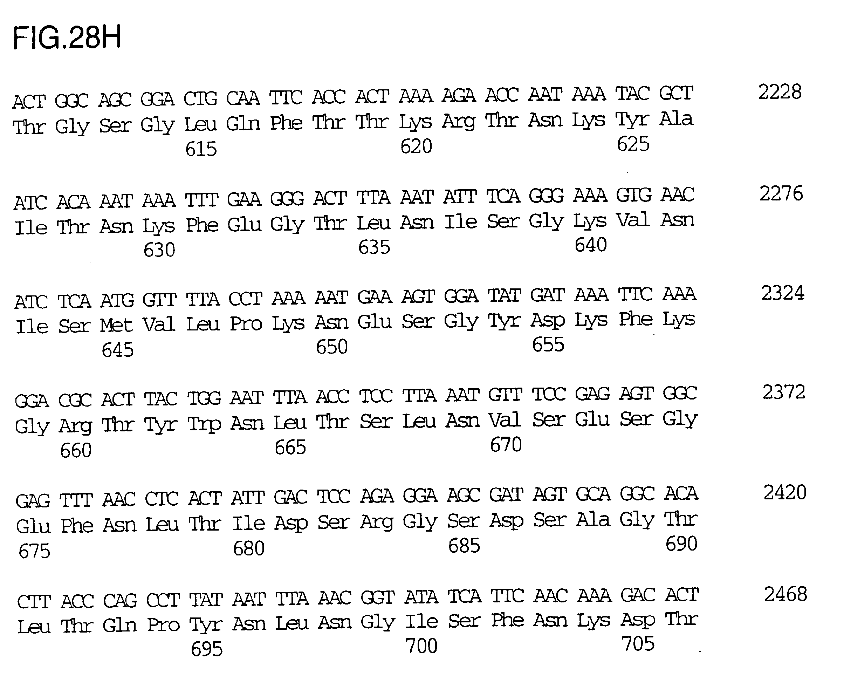

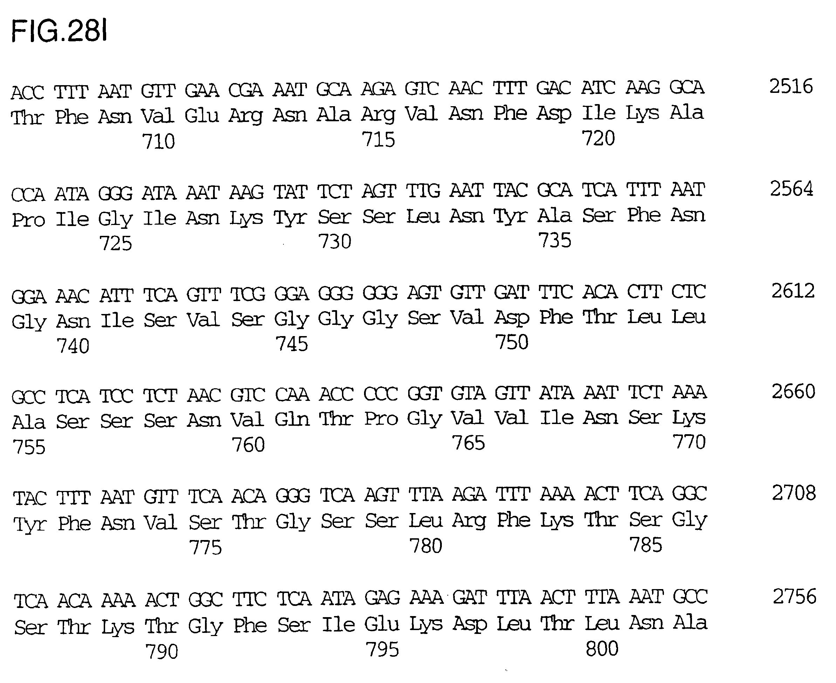

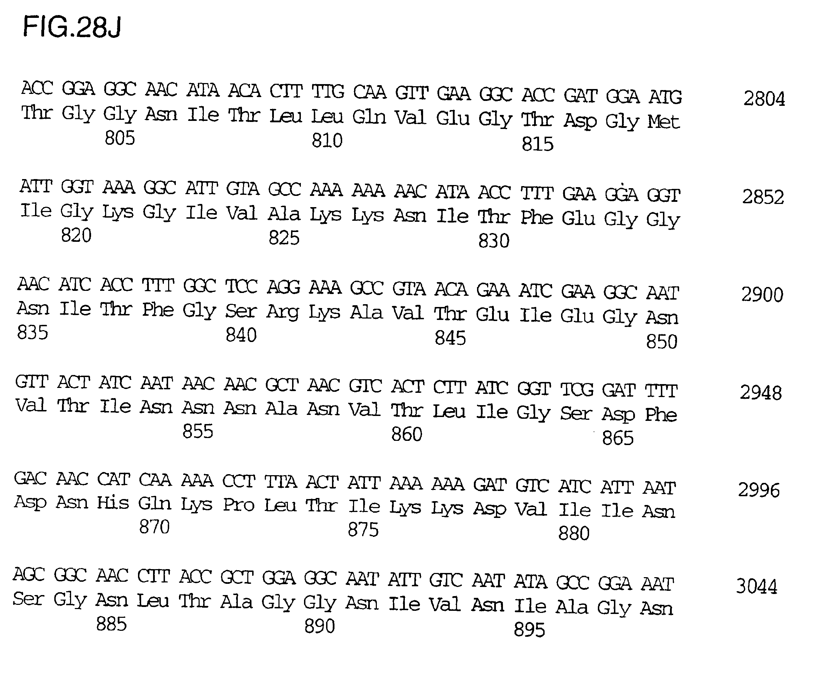

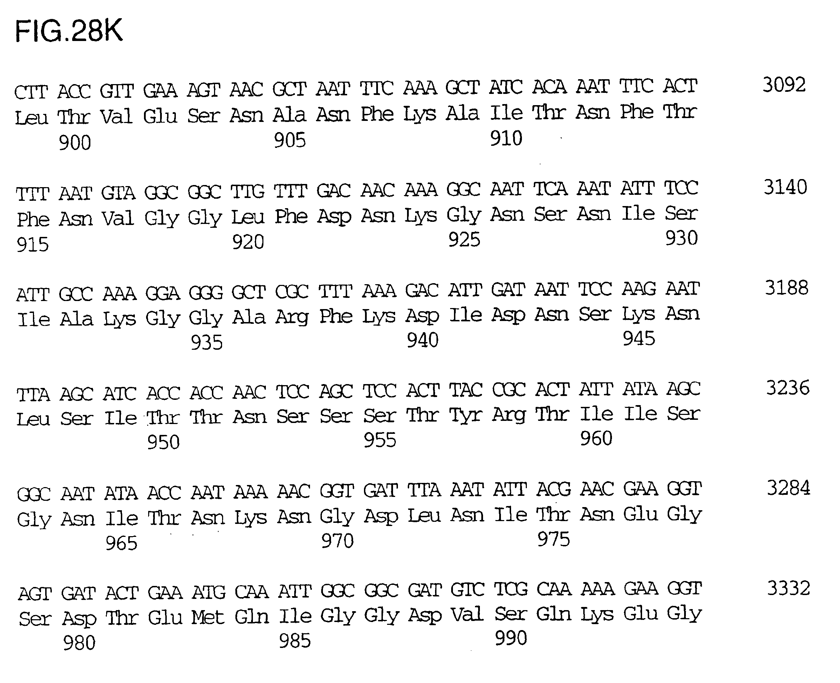

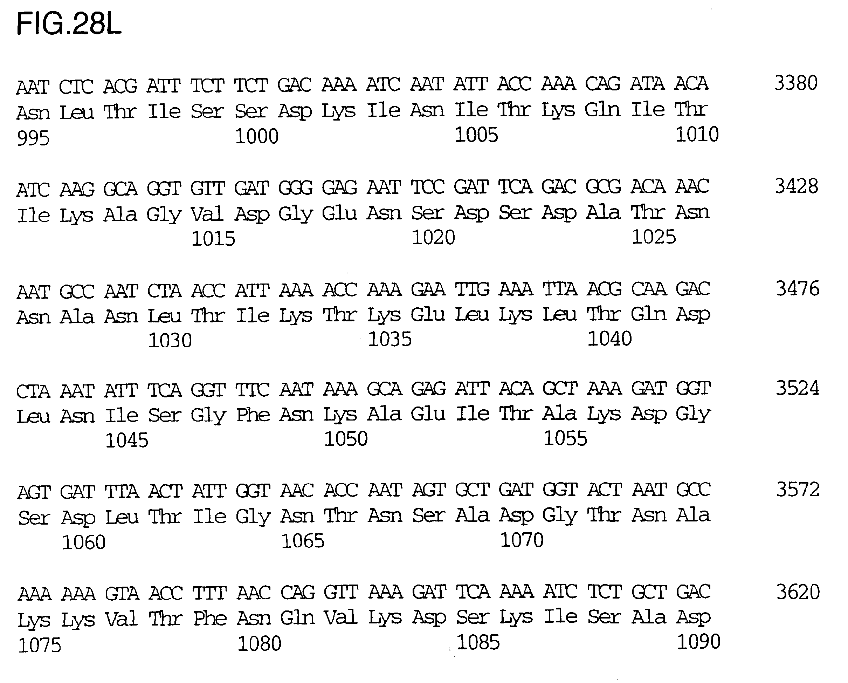

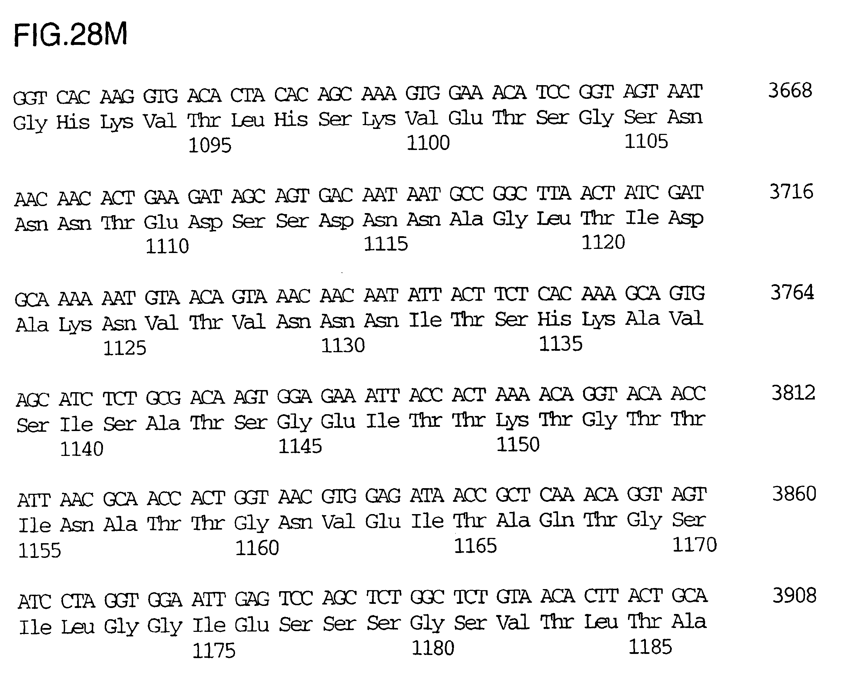

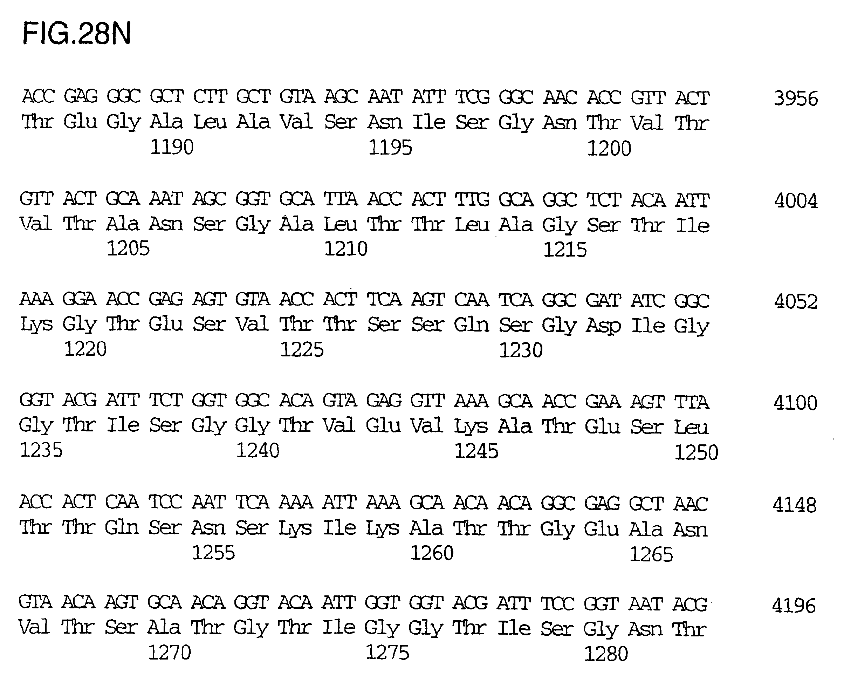

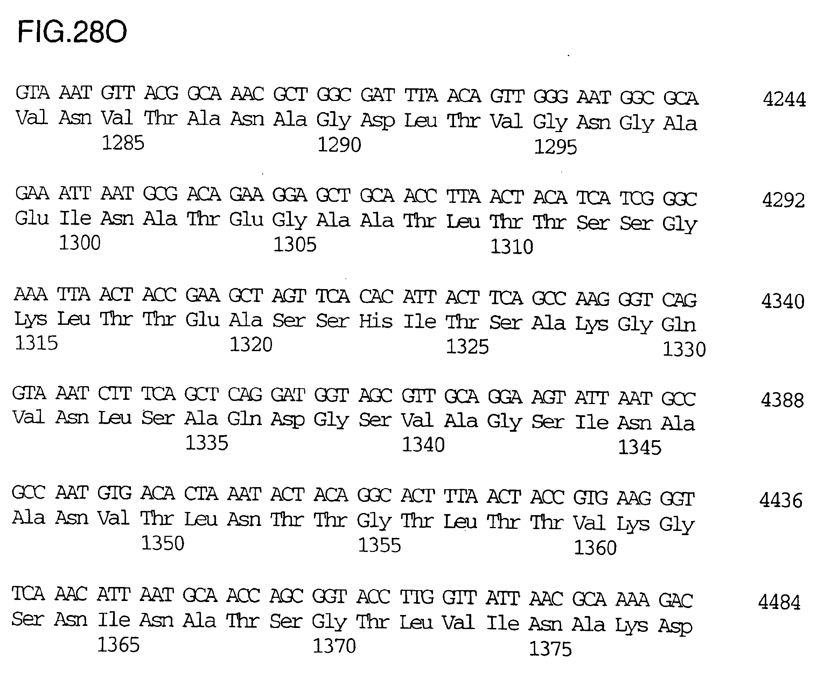

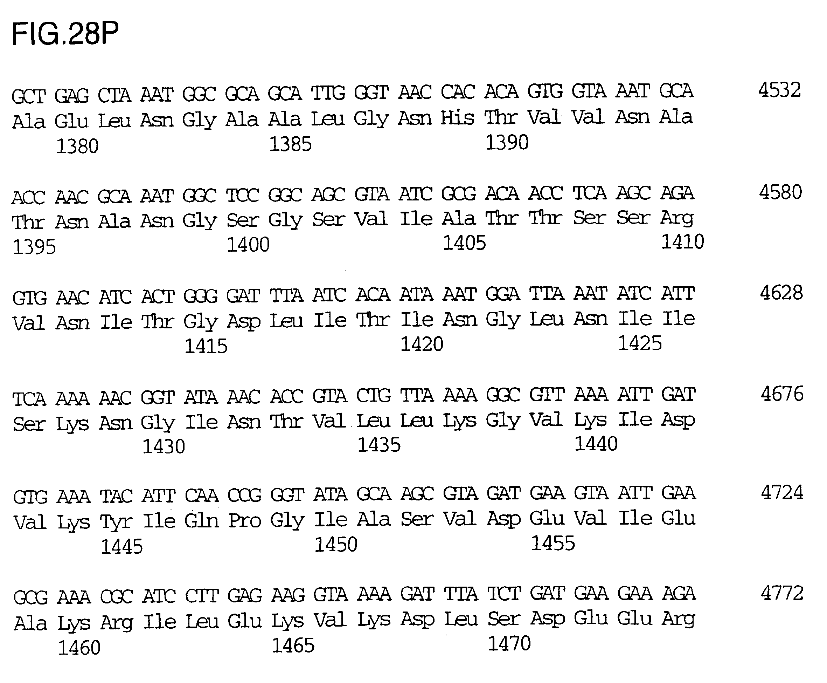

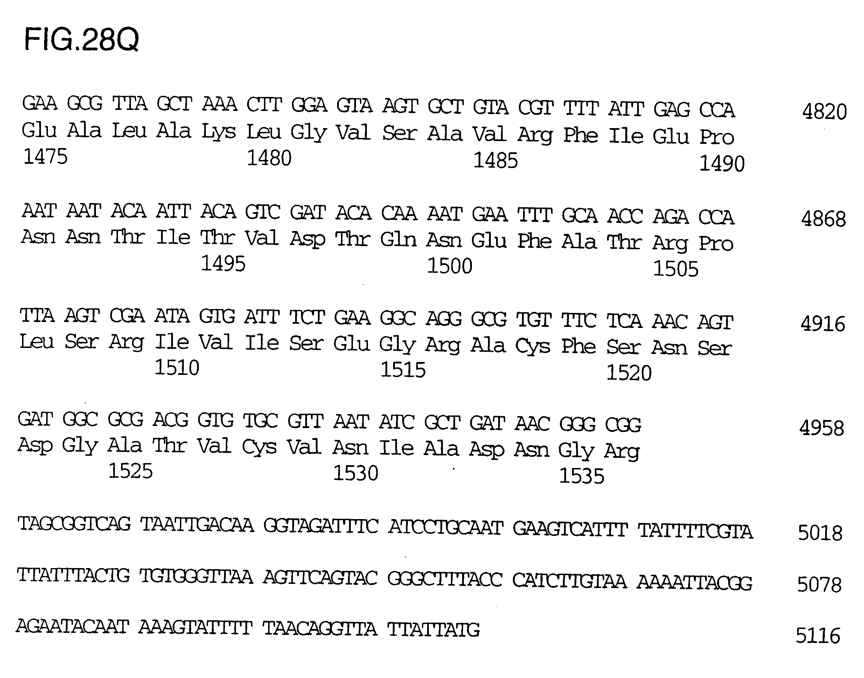

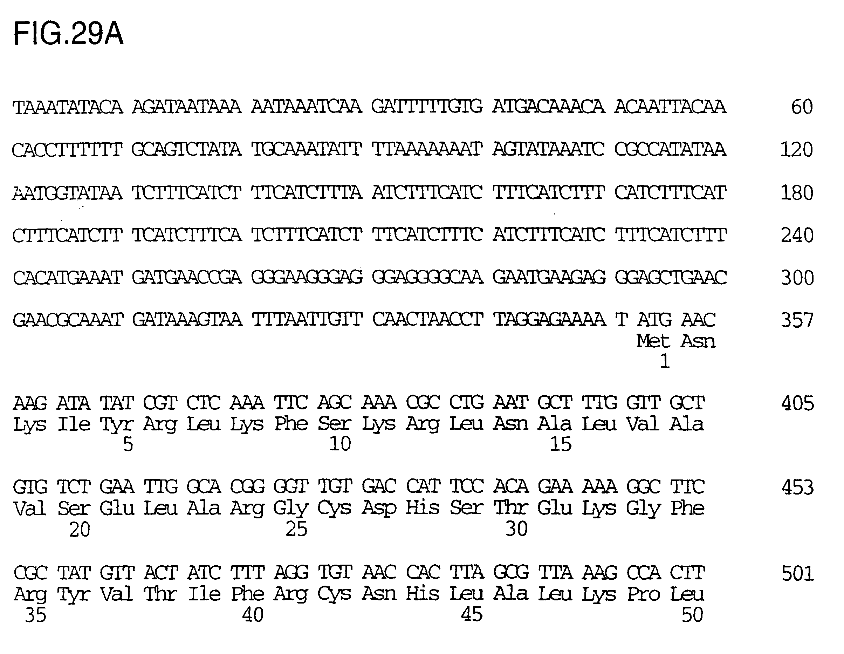

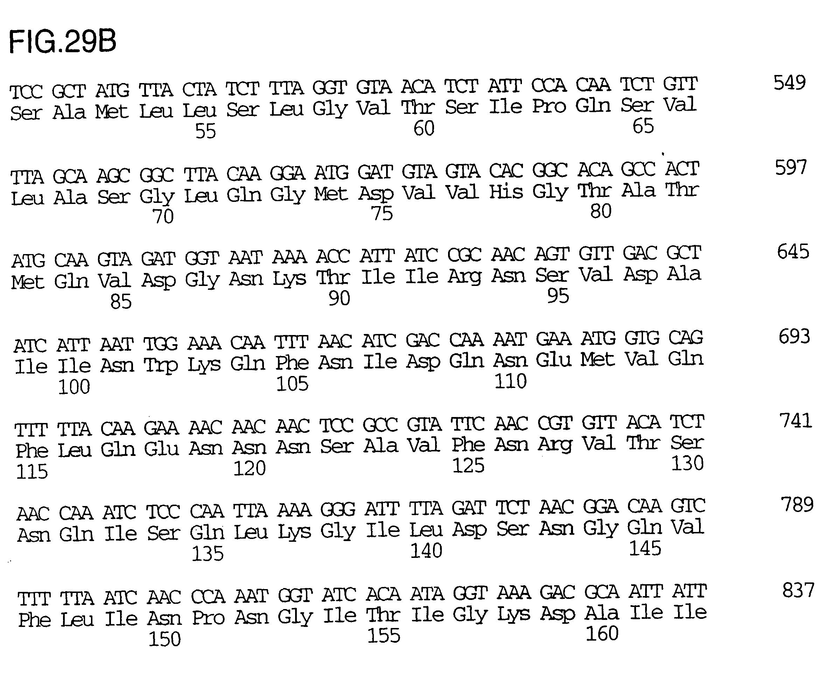

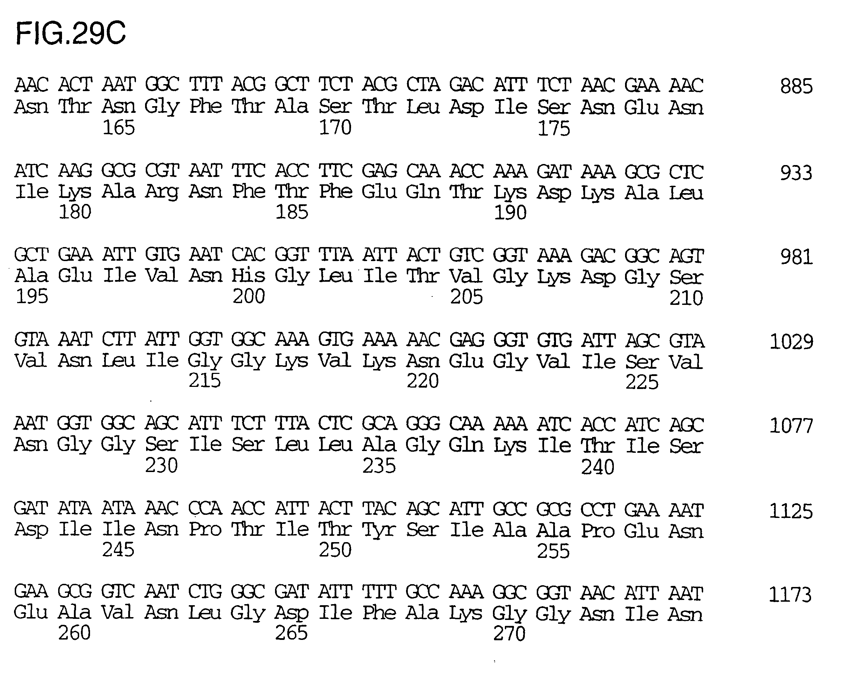

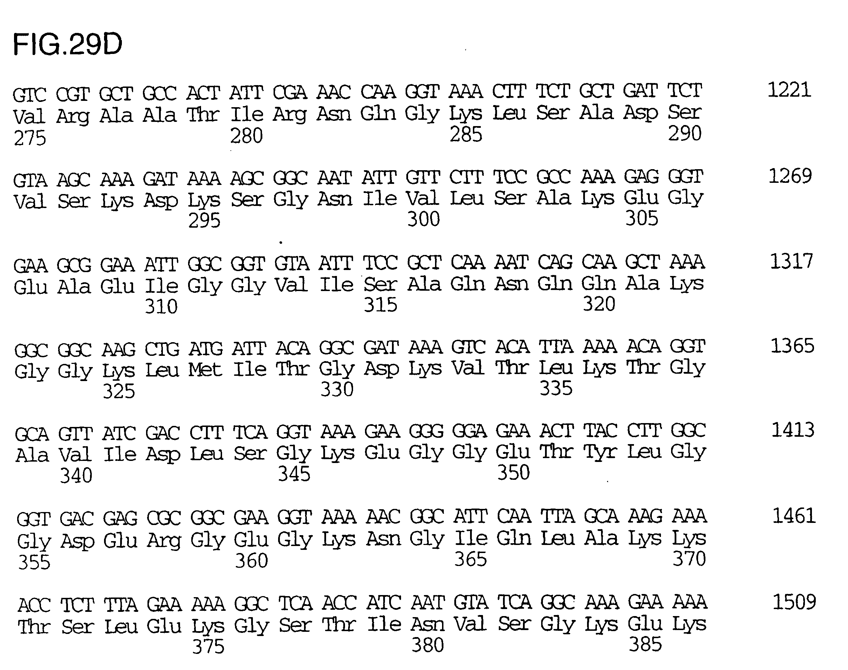

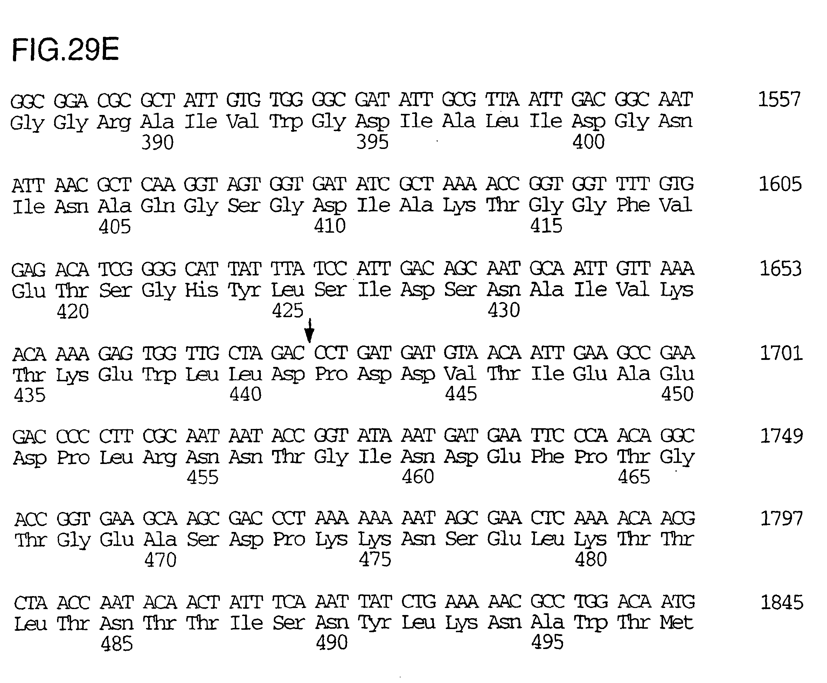

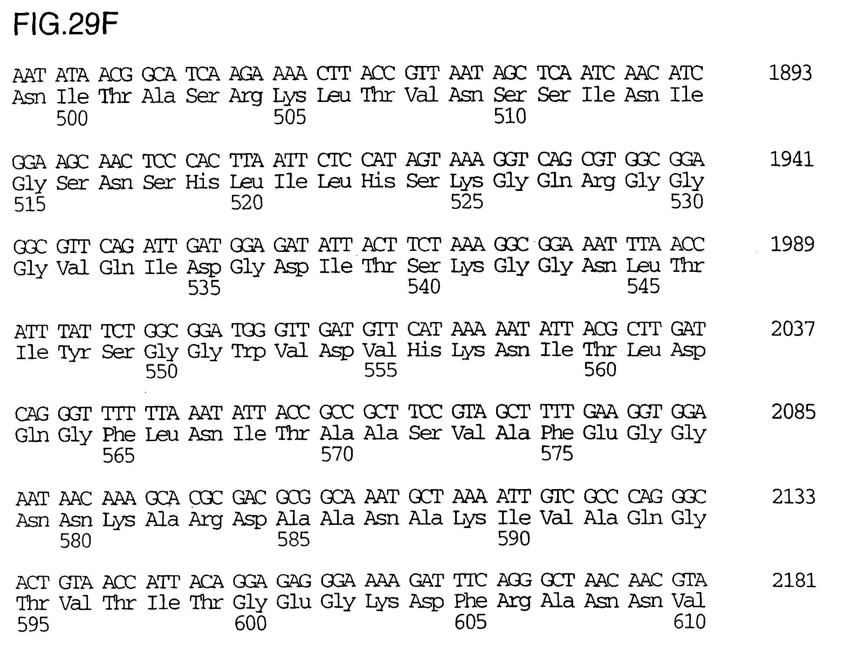

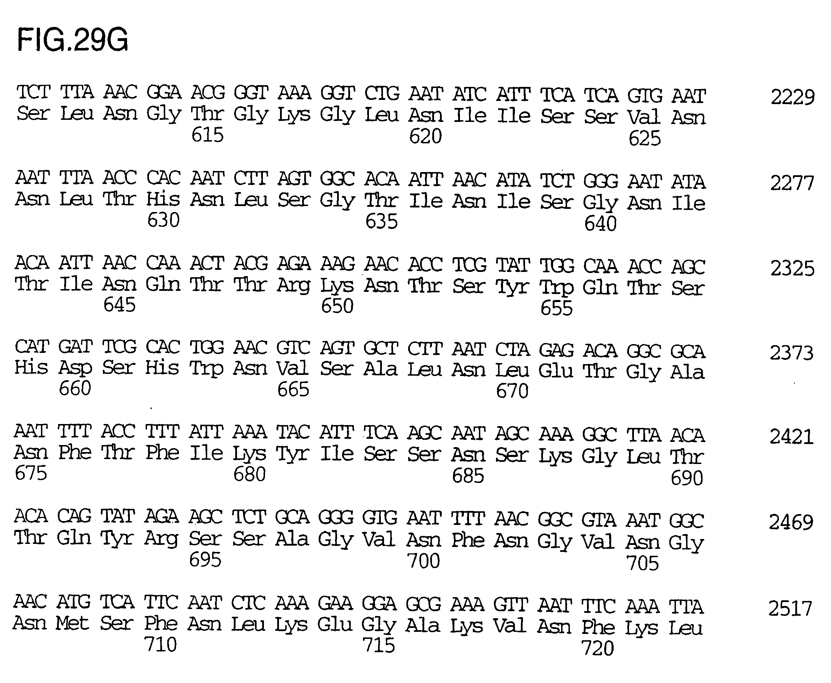

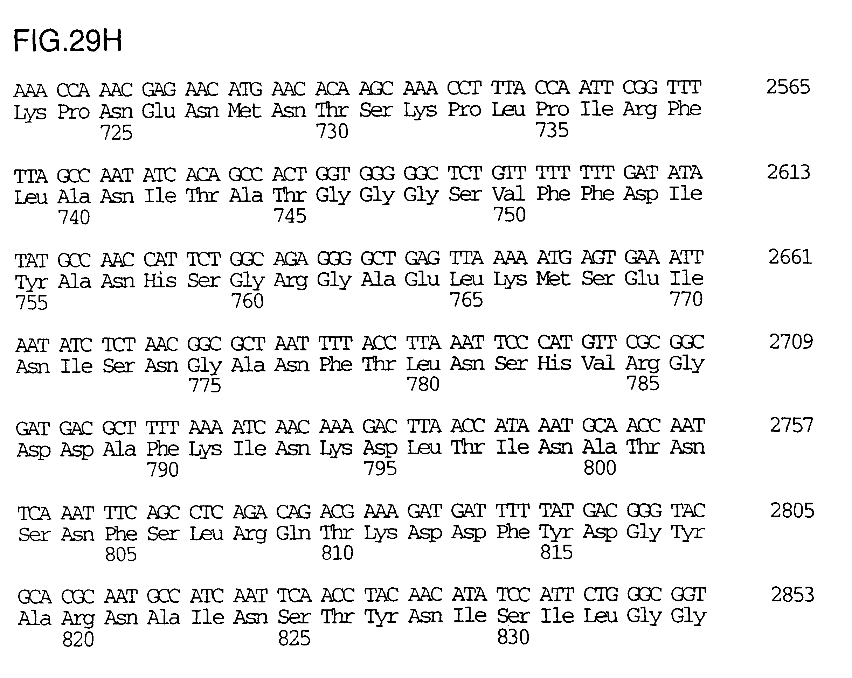

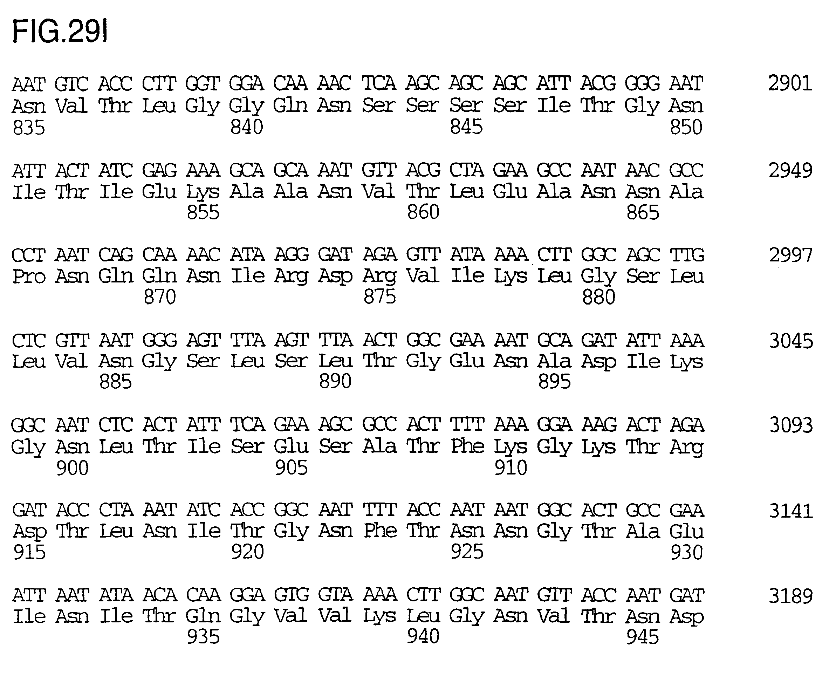

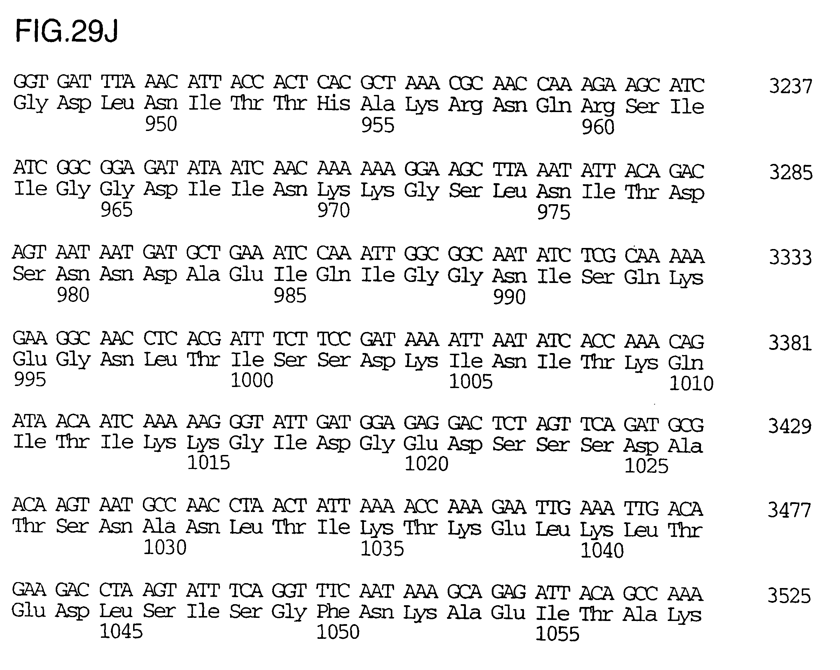

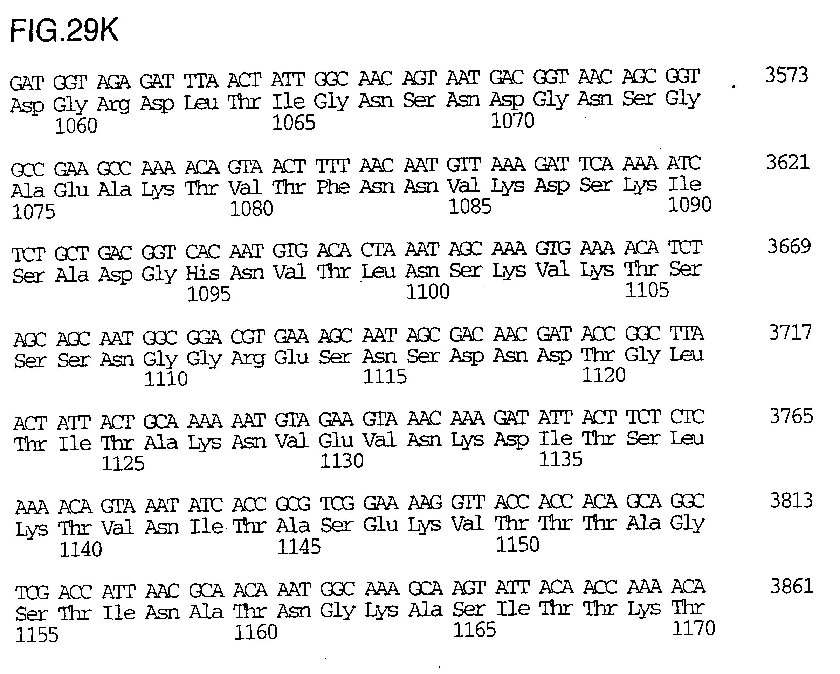

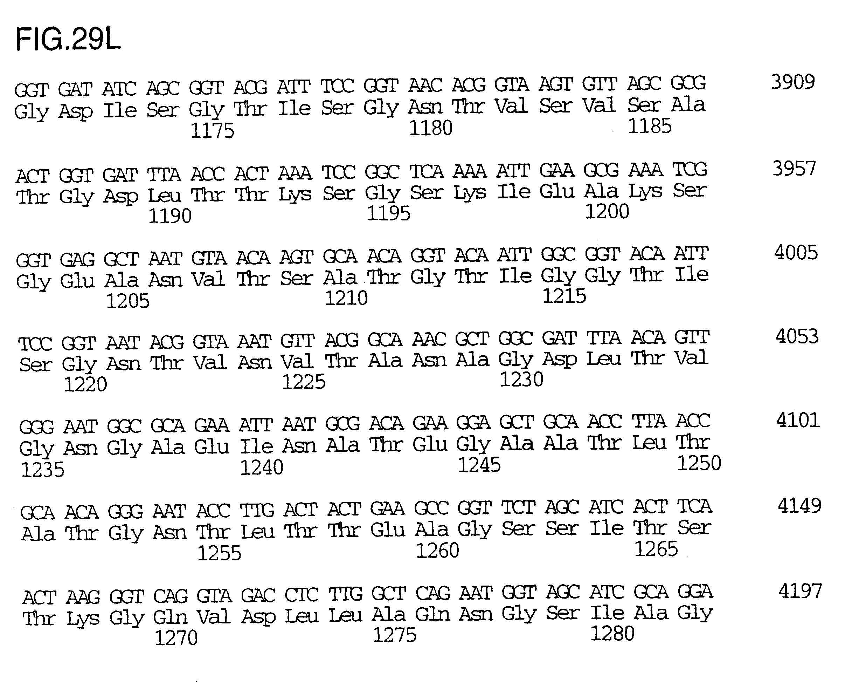

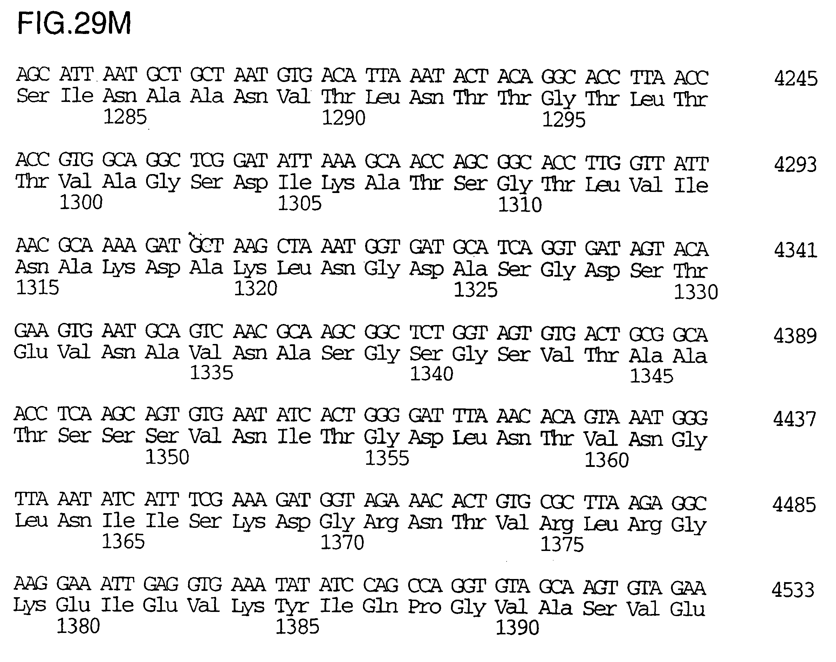

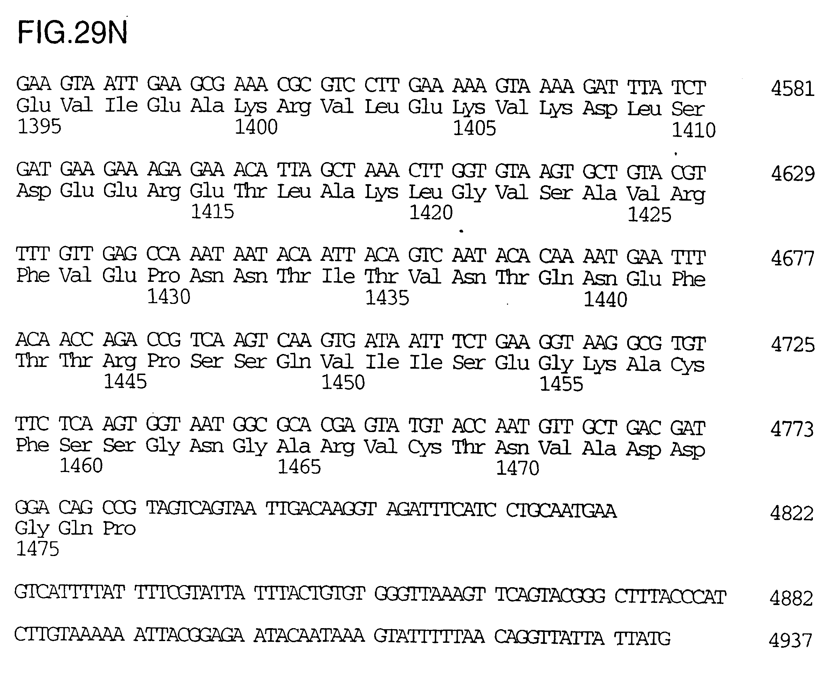

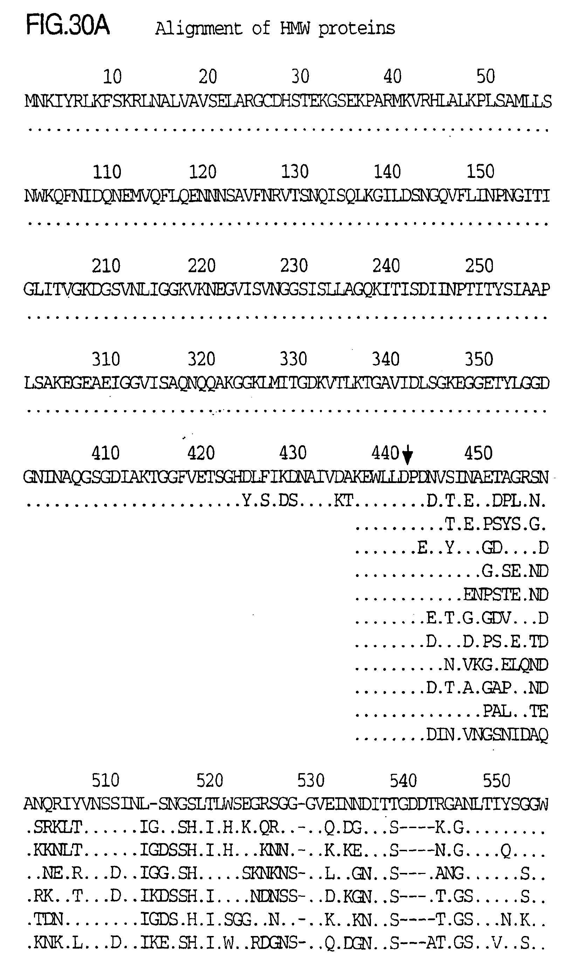

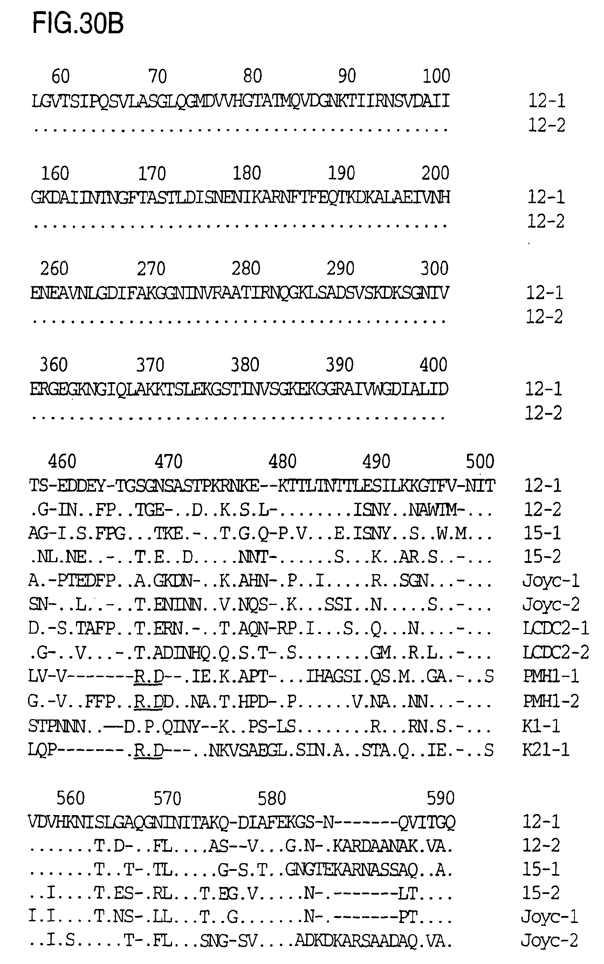

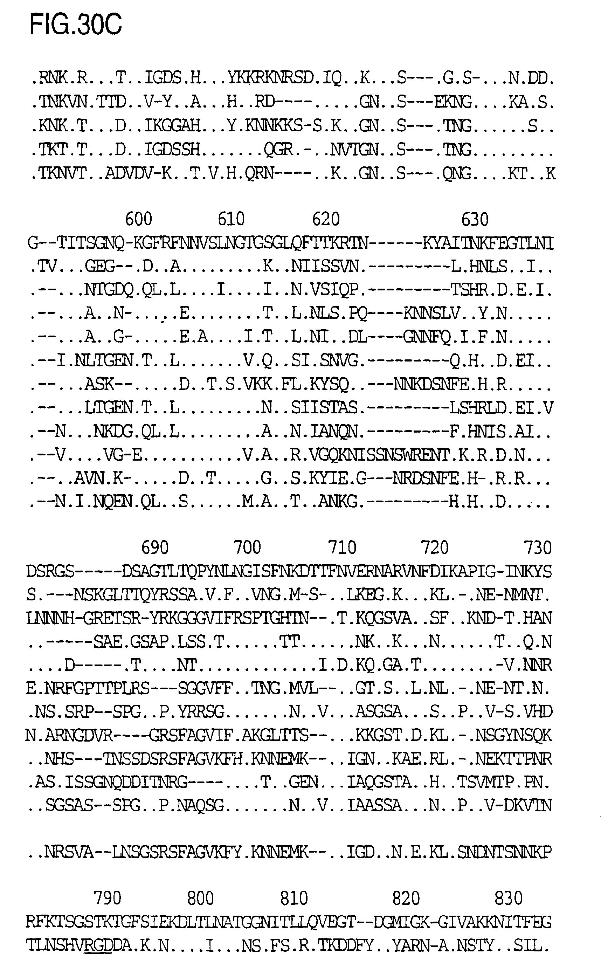

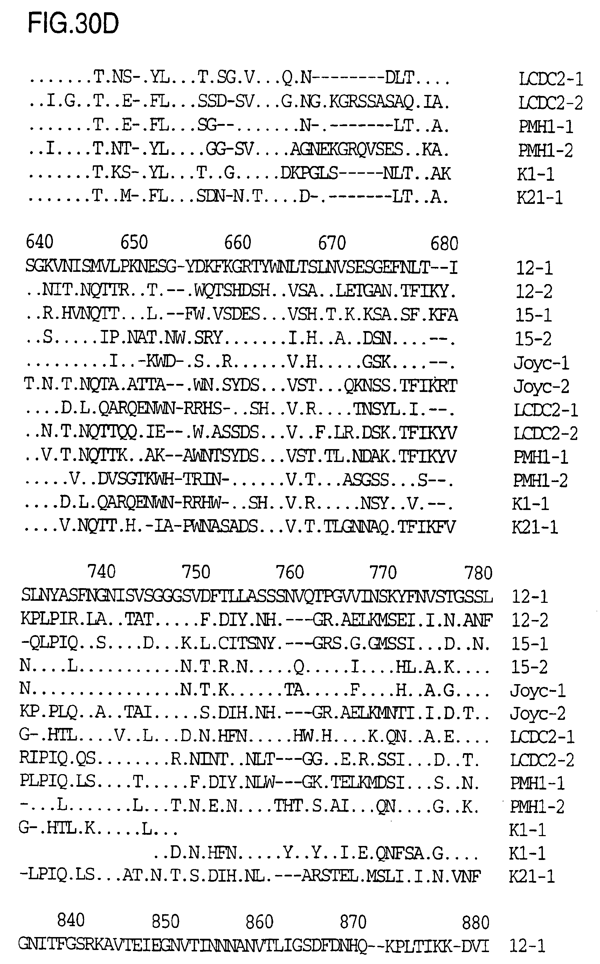

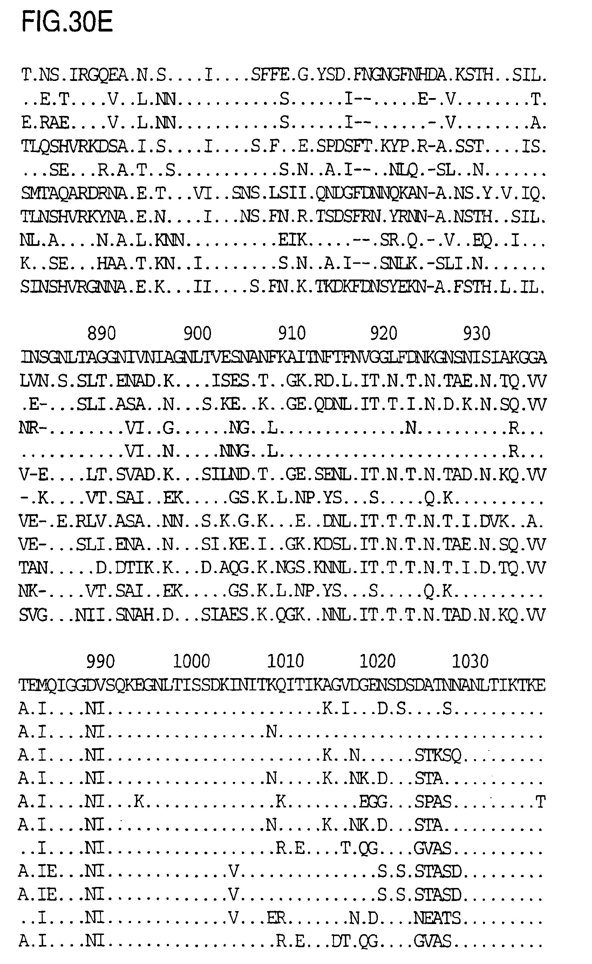

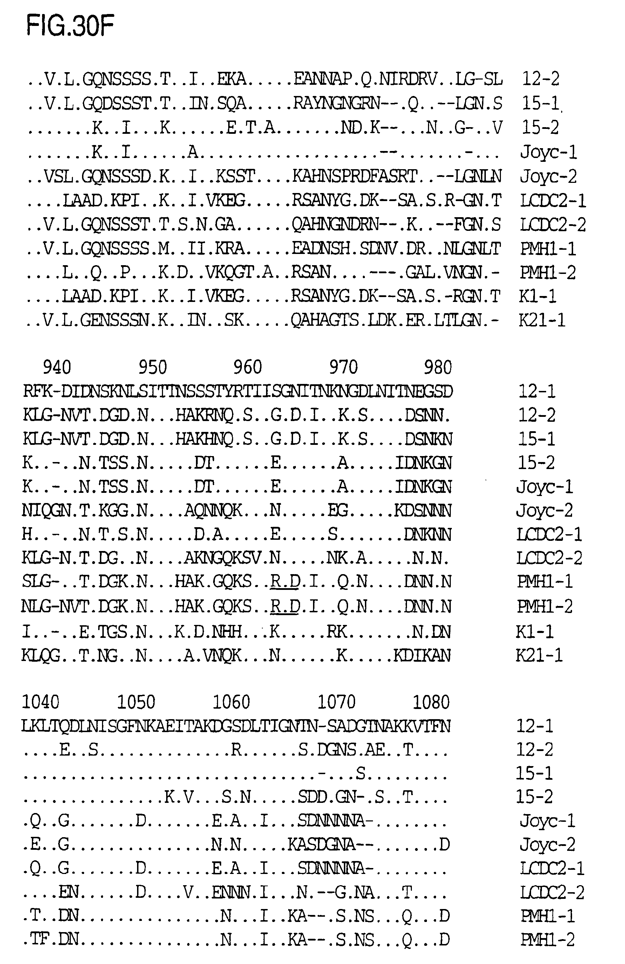

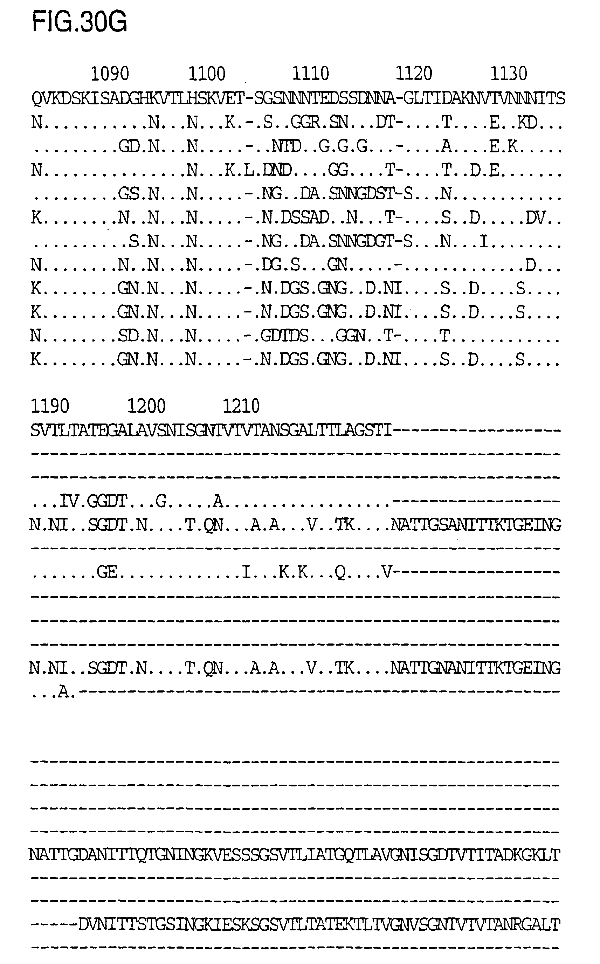

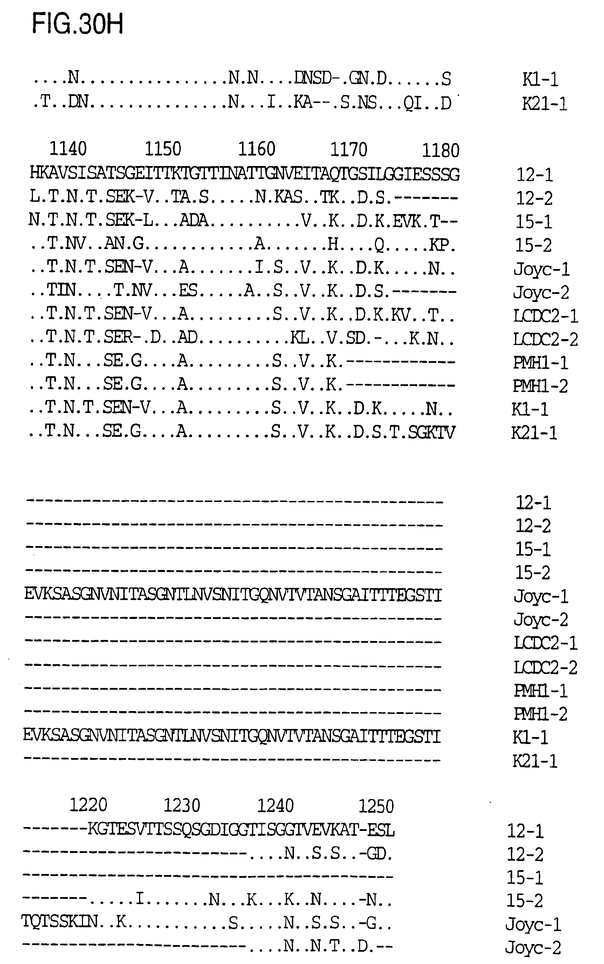

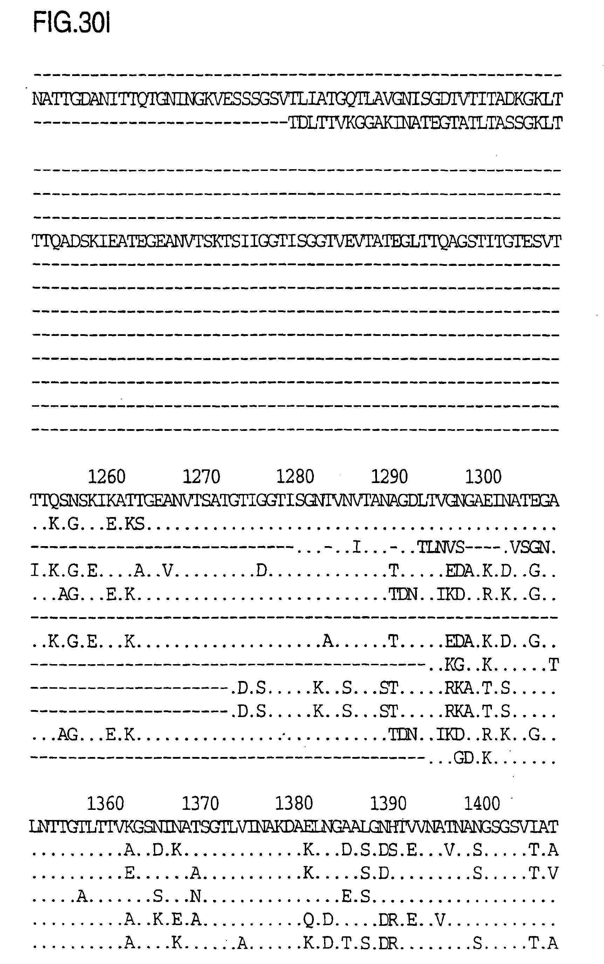

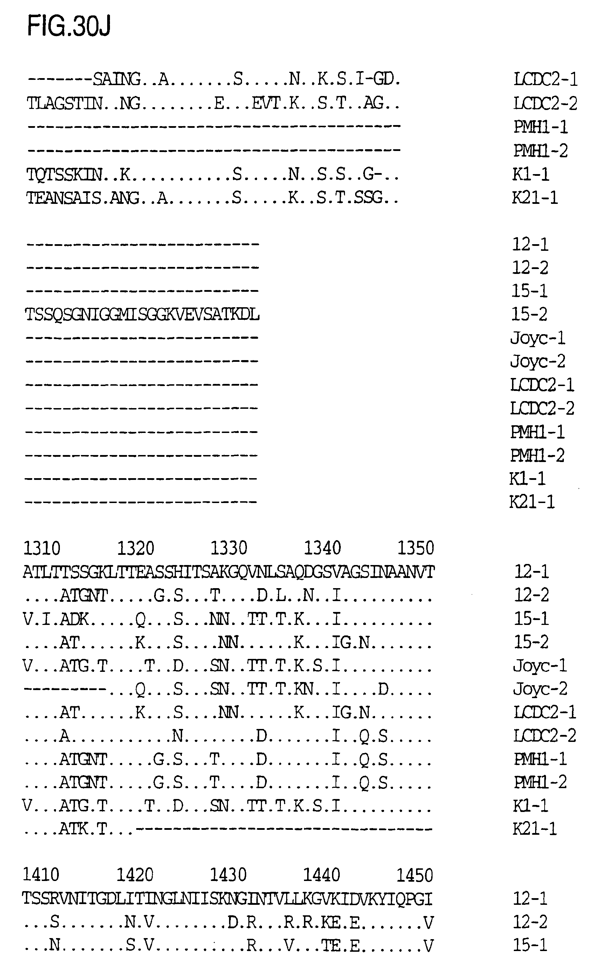

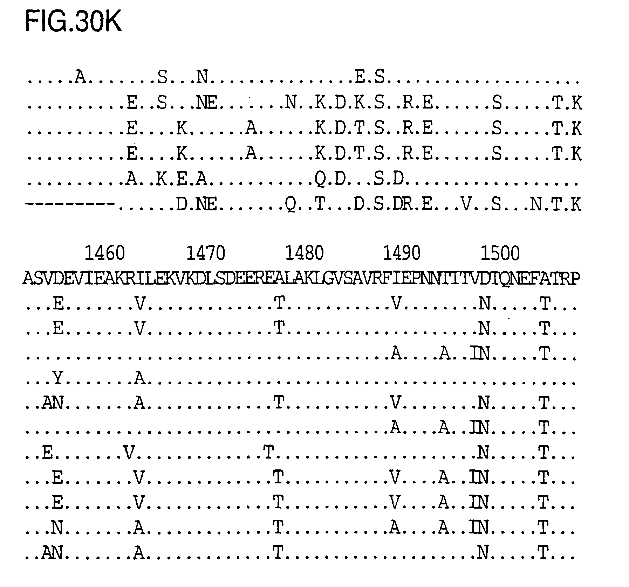

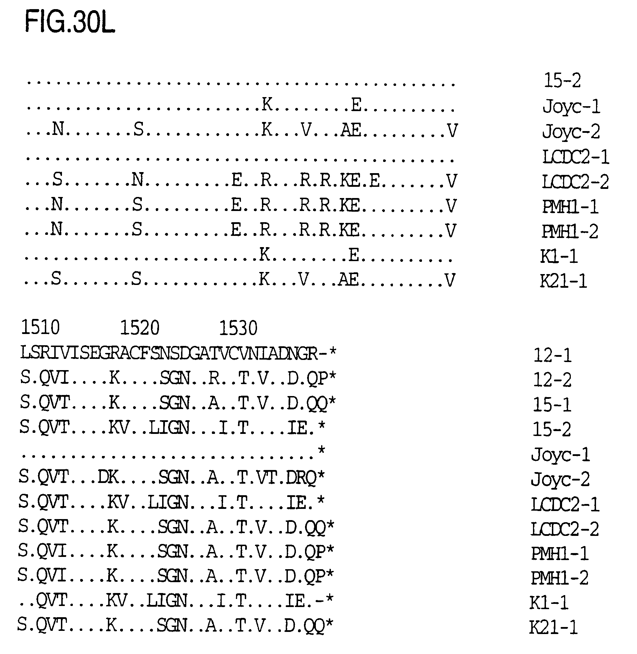

The *hmwA* genes and encoded proteins have variable sequences. In order to produce a completely effective vaccine, it may be necessary to use rHMWA proteins generated from multiple strains of non-typeable *Haemophilus.* The *hmw1A* and/or *hmw2A* genes were PCR amplified and sequenced from several strains of non-typeable *Haemophilus influenzae*. Figures 18 to 26 illustrate the nucleotide and deduced amino acid sequences for the *hmw1A* gene from strain Joyc, the *hmw2A* gene from strain Joyc, the defective *hmw1A* gene from strain K1, the *hmw2A* gene from strain K21, the *hmw1A* gene from strain LCDC2, the *hmw2A* gene from strain LCDC2, the *hmw1A* gene from strain PMH1, the *hmw2A* gene from strain PMH1, the *hmw1A* gene from strain 15, and the *hmw2A* gene from strain 15, respectively. The alignment of the deduced protein sequences with the previously described strain 12 HMW1A and HMW2A protein sequences (Figures 28 and 29) identifies both regions of sequence conservation and divergence (Figure 30). Such information may be useful in the identification of potential epitopes to generate peptides for vaccination or diagnostic purposes. The molecular weights of the mature HMW proteins from the various non-typeable *Haemophilus* strains is contained in Table 3.

### 4. Construction of a generic expression vector for production of protective recombinant HMW proteins.

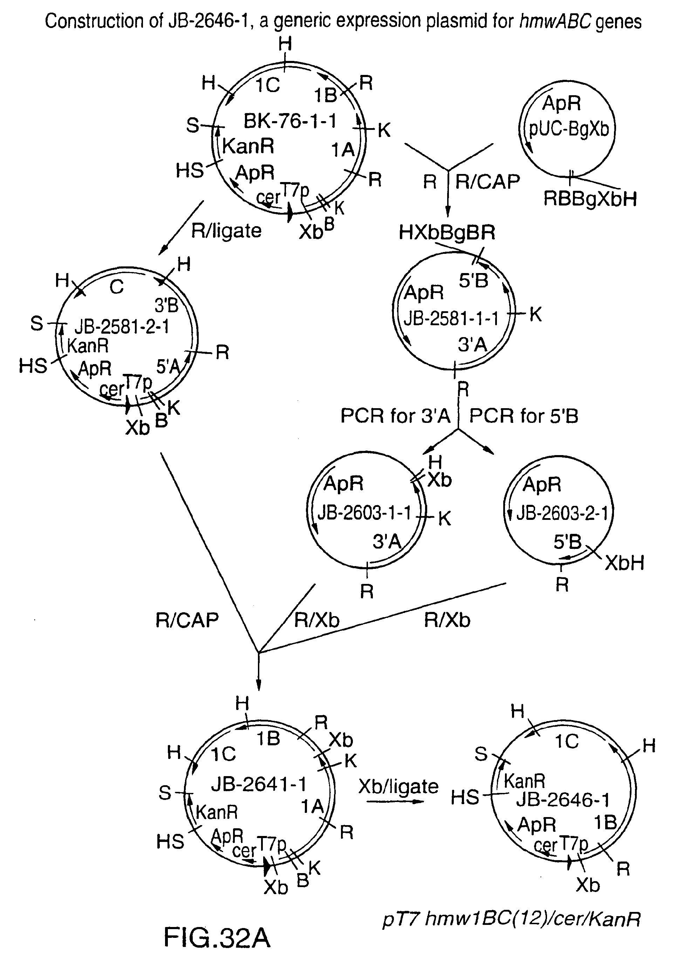

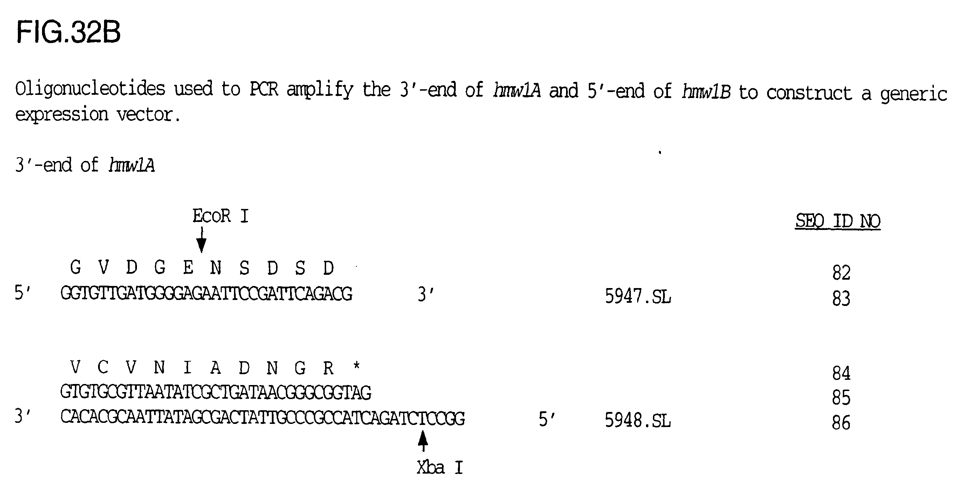

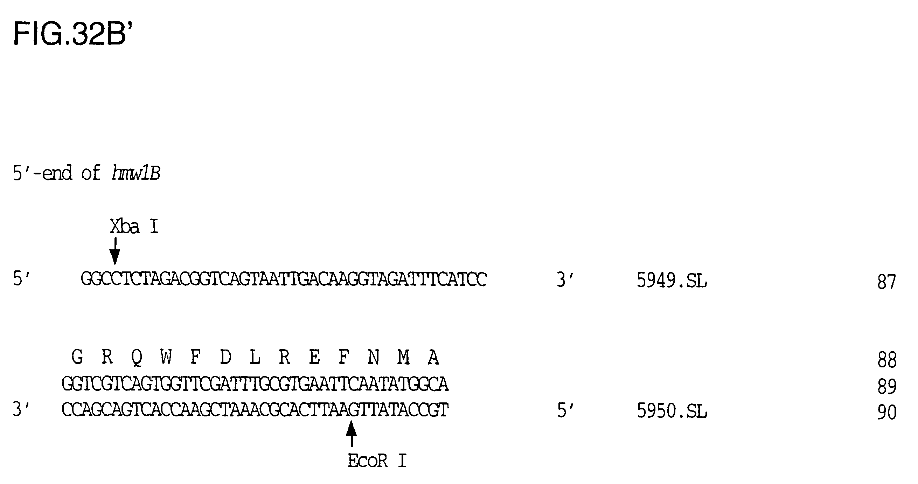

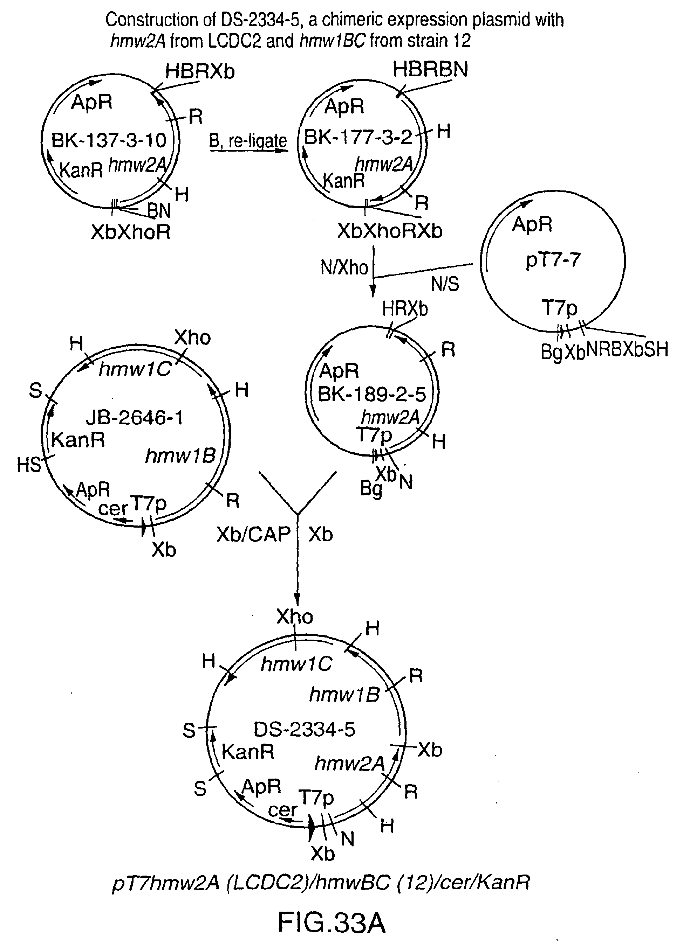

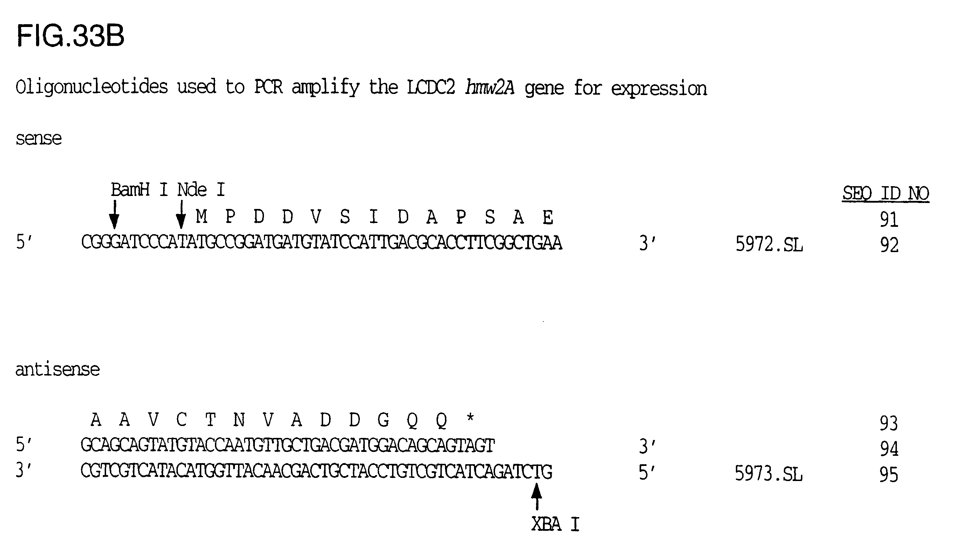

New *hmwA* genes can be PCR amplified from strains of non-typeable *Haemophilus* and sequenced as described above. However, in order to produce protective rHMWA antigens, the *hmwA* genes must be expressed in the presence of *hmwBC* genes. The deduced sequences of the accessory HMW1B and HMW2B proteins from the prototype strain 12 were found to be 99% identical, while the deduced HMW1C and HMW2C proteins from the same strain were 96% identical (ref. 8, USP 5,603,938). The highly conserved nature of the *hmwBC* genes lead to the possibility of constructing a generic expression vector using the *hmwBC* genes from a prototype strain, and introducing any *hmwA* gene to the vector for expression therein. Figure 32 illustrates the construction of a generic expression vector (JB-2646-1) that contains the T7 promoter, an Xba I cloning site for introduction of *hmwA* genes, the strain 12 *hmw1BC* genes, and the *E. coli* cer gene. Figure 33 illustrates the construction of a chimeric T7 *hmwABC* gene cassette in the generic expression vector, wherein a PCR amplified LCDC2 *hmw2A* gene is combined with the strain 12 *hmw1BC* genes to produce plasmid DS-2334-5. The expression of the genes from the chimeric construct was as seen for T7 *hmw1ABC* or T7 *hmw2ABC* constructs based on NTHi strain 12 *hmw1A* and *hmw2A* genes.

It is clearly apparent to one skilled in the art, that the various embodiments of the present invention have use in applications in the fields of vaccination, diagnosis, treatment of *Haemophilus* infection and the generation of immunological agents. A further nonlimiting discussion of such uses is further presented below.

### 5. Vaccine Preparation and Use

Immunogenic compositions, suitable to be used as vaccines, may be prepared from immunogenic high molecular weight (HMW) proteins of non-typeable *Haemophilus* strains, immunogenic analogs and fragments thereof and/or immunogenic peptides as disclosed herein. The vaccine elicits an immune response which produces antibodies, including anti-HMW antibodies and antibodies that are opsonizing or bactericidal. Immunogenic compositions, including vaccines, may be prepared as injectables, as liquid solutions or emulsions. The HMW protein, immunogenic analogs and fragments thereof and/or immunogenic peptides may be mixed with pharmaceutically acceptable excipients which are compatible with the HMW protein, immunogenic fragments, analogs or immunogenic peptides. Such excipients may include, water, saline, dextrose, glycerol, ethanol and combinations thereof. The immunogenic compositions and vaccines may further contain auxiliary substances such as wetting or emulsifying agents, pH buffering agents, or adjuvants to enhance the effectiveness of the vaccines. Immunogenic compositions and vaccines may be administered parenterally, by injection subcutaneously or intramuscularly. Alternatively, the immunogenic compositions formed according to the present invention, may be formulated and delivered in a manner to evoke an immune response at mucosal surfaces. Thus, the immunogenic composition may be administered to mucosal surfaces by, for example, the nasal or oral (intragastric) routes. The immunogenic composition may be provided in combination with a targeting molecule for delivery to specific cells of the immune system or to mucosal surfaces. Some such targeting molecules include vitamin B12 and fragments of bacterial toxins, as described in WO 92/17167 (Biotech Australia Pty. Ltd.), and monoclonal antibodies, as described in U.S. Patent No. 5,194,254 (Barber et al). Alternatively, other modes of administration including suppositories and oral formulations may be desirable. For suppositories, binders and carriers may include, for example polyalkylene glycols or triglycerides. Oral formulations may include normally employed incipients such as, for example pharmaceutical grades of saccharine, cellulose and magnesium carbonate. These compositions take the form of solutions, suspensions, tablets, pills, capsules, sustained release formulations or powders and contain about 1 to 95% of the HMW protein, fragments, analogs and/or peptides.

The vaccines are administered in a manner compatible with the dosage formulation, and in such amount as will be therapeutically effective, protective and immunogenic. The quantity to be administered depends on the subject to be treated, including, for example, the capacity of the individual's immune system to synthesize antibodies, and if needed, to produce a cell-mediated immune response. Precise amounts of active ingredient required to be administered depend on the judgment of the practitioner. However, suitable dosage ranges are readily determinable by one skilled in the art and may be of the order of micrograms of the high molecular weight protein, analogs and fragments thereof and/or peptides. Suitable regimes for initial administration and booster doses are also variable, but may include an initial administration followed by subsequent administrations. The dosage of the vaccine may also depend on the route of administration and will vary according to the size of the host.

The nucleic acid molecules encoding the HMW proteins of non-typeable *Haemophilus* may also be used directly for immunization by administration of the DNA directly, for example by injection for genetic immunization or by constructing a live vector, such as *Salmonella,* BCG, adenovirus, poxvirus, vaccinia or poliovirus, containing the nucleic acid molecule. A discussion of some live vectors that have been used to carry heterologous antigens to the immune system is contained in, for example, O'Hagan (1992) (ref. 17). Processes for the direct injection of DNA into test subjects for genetic immunization are described in, for example, Ulmer et al., 1993 (ref. 18).

Immunogenicity can be significantly improved if the antigens are co-administered with adjuvants, commonly used as an 0.05 to 1.0 percent solution in phosphate - buffered saline. Adjuvants enhance the immunogenicity of an antigen but are not necessarily immunogenic themselves. Adjuvants may act by retaining the antigen locally near the site of administration to produce a depot effect facilitating a slow, sustained release of antigen to cells of the immune system. Adjuvants can also attract cells of the immune system to an antigen depot and stimulate such cells to elicit immune responses.

Immunostimulatory agents or adjuvants have been used for many years to improve the host immune responses to, for example, vaccines. Intrinsic adjuvants, such as lipopolysaccharides, normally are the components of the killed or attenuated bacteria used as vaccines. Extrinsic adjuvants are immunomodulators which are typically non-covalently linked to antigens and are formulated to enhance the host immune responses. Thus, adjuvants have been identified that enhance the immune response to antigens delivered parenterally. Some of these adjuvants are toxic, however, and can cause undesirable side-effects, making them unsuitable for use in humans and many animals. Indeed, only aluminum hydroxide and aluminum phosphate (collectively commonly referred to as alum) are routinely used as adjuvants in human and veterinary vaccines. The efficacy of alum in increasing antibody responses to diphtheria and tetanus toxoids is well established.

A wide range of extrinsic adjuvants can provoke potent immune responses to antigens. These include saponins complexed to membrane protein antigens (immune stimulating complexes), pluronic polymers with mineral oil, killed mycobacteria and mineral oil, Freund's complete adjuvants, bacterial products, such as muramyl dipeptide (MDP) and lipopolysaccharide (LPS), as well as lipid A, and liposomes.

To efficiently induce humoral immune responses (HIR) and cell-mediated immunity (CMI), immunogens are emulsified in adjuvants. Many adjuvants are toxic, inducing granulomas, acute and chronic inflammations (Freund's complete adjuvant, FCA), cytolysis (saponins and pluronic polymers) and pyrogenicity, arthritis and anterior uveitis (LPS and MDP). Although FCA is an excellent adjuvant and widely used in research, it is not licensed for use in human or veterinary vaccines because of its toxicity.

Desirable characteristics of ideal adjuvants include:
(1) lack of toxicity;
(2) ability to stimulate a long-lasting immune response;
(3) simplicity of manufacture and stability in long-term storage;
(4) ability to elicit both CMI and HIR to antigens administered by various routes, if required;
(5) synergy with other adjuvants;
(6) capability of selectively interacting with populations of antigen presenting cells (APC);
(7) ability to specifically elicit appropriate T_{H}1 or T_{H}2 cell-specific immune responses; and
(8) ability to selectively increase appropriate antibody isotype levels (for example, IgA) against antigens.

US Patent No. 4,855,283 granted to Lockhoff et al on August 8, 1989 and which is incorporated herein by reference thereto, teaches glycolipid analogues including N-glycosylamides, N-glycosylureas and N-glycosylcarbamates, each of which is substituted in the sugar residue by an amino acid, as immuno-modulators or adjuvants. Thus, Lockhoff et al. 1991 (ref. 19) reported that N-glycolipid analogs displaying structural similarities to the naturally-occurring glycolipids, such as glycosphingolipids and glycoglycerolipids, are capable of eliciting strong immune responses in both herpes simplex virus vaccine and pseudorabies virus vaccine. Some glycolipids have been synthesized from long chain-alkylamines and fatty acids that are linked directly with the sugars through the anomeric carbon atom, to mimic the functions of the naturally occurring lipid residues.

U.S. Patent No. 4,258,029 granted to Moloney, assigned to the assignee hereof and incorporated herein by reference thereto, teaches that octadecyl tyrosine hydrochloride (OTH) functions as an adjuvant when complexed with tetanus toxoid and formalin inactivated type I, II and III poliomyelitis virus vaccine. Also, Nixon-George et al. 1990 (ref. 20), reported that octadecyl esters of aromatic amino acids complexed with a recombinant hepatitis B surface antigen, enhanced the host immune responses against hepatitis B virus.

### 6. Immunoassays

The HMW protein of a non-typeable strain of *Haemophilus,* analogs and fragments thereof and/or peptides of the present invention are useful as immunogens, as antigens in immunoassays including enzyme-linked immunosorbent assay (ELISA), RIAs and other non-enzyme linked antibody binding assays or procedures known in the art for the detection of antibacterial, *Haemophilus,* HMW and/or peptide antibodies. In ELISA assays, the HMW protein, analogs, fragments and/or peptides corresponding to portions of HMW protein are immobilized onto a selected surface, for example a surface capable of binding proteins or peptides, such as the wells of a polystyrene microtiter plate. After washing to remove incompletely adsorbed HMW protein, analogs fragments and/or peptides, a nonspecific protein such as a solution of bovine serum albumin (BSA) or casein that is known to be antigenically neutral with regard to the test sample may be bound to the selected surface. This allows for blocking of nonspecific adsorption sites on the immobilizing surface and thus reduces the background caused by nonspecific bindings of antisera onto the surface.

The immobilizing surface is then contacted with a sample, such as clinical or biological materials, to be tested in a manner conducive to immune complex (antigen/antibody) formation. This may include diluting the sample with diluents, such as BSA, bovine gamma globulin (BGG) and/or phosphate buffered saline (PBS)/Tween. The sample is then allowed to incubate for from about 2 to about 4 hours, at temperature such as of the order of about 25° to about 37°C. Following incubation, the sample-contacted surface is washed to remove non-immunocomplexed material. The washing procedure may include washing with a solution, such as PBS/Tween, or a borate buffer.

Following formation of specific immunocomplexes between the test sample and the bound HMW protein, analogs, fragments and/or peptides, and subsequent washing, the occurrence, and even amount, of immunocomplex formation may be determined by subjecting the immunocomplex to a second antibody having specificity for the first antibody. If the test sample is of human origin, the second antibody is an antibody having specificity for human immunoglobulins and in general IgG. To provide detecting means, the second antibody may have an associated activity, such as an enzymatic activity, that will generate, for example, a color development, upon incubating with an appropriate chromogenic substrate. Quantification may then achieved by measuring the degree of color generation using, for example, a visible spectra spectrophotometer.

### 7. Use of Sequences as Hybridization Probes

The nucleotide sequences of the present invention, comprising the newly-isolated and characterized sequences of the *hmw* genes, allow for the identification and cloning of the *hmw* genes from other non-typeable strains of *Haemophilus.*

The nucleotide sequences comprising the sequence of *hmw* genes of the present invention are useful for their ability to selectively form duplex molecules with complementary stretches of other *hmw* genes. Depending on the application, a variety of hybridization conditions may be employed to achieve varying degrees of selectivity of the probe toward the other *hmw* genes. For a high degree of selectivity, relatively stringent conditions are used to form the duplexes, such as low salt and/or high temperature conditions, such as provided by 0.02 M to 0.15 M NaCl at temperatures of between about 50°C to 70°C. For some applications, less stringent hybridization conditions are required such as 0.15 M to 0.9 M salt, at temperatures ranging from between 20°C to 55°C. Hybridization conditions can also be rendered more stringent by the addition of increasing amounts of formamide, to destabilize the hybrid duplex. Thus, particular hybridization conditions can be readily manipulated, and will generally be a method of choice depending on the desired results. In general, convenient hybridization temperatures in the presence of 50% formamide and 0.15 M NaCl are: 42°C for an *hmw* gene which is about 95 to 100% homologous to the target nucleic acid fragment, 37°C for about 90 to 95% homology and 32°C for about 85 to 90% homology.

In a clinical diagnostic embodiment, the nucleic acid sequences of the hmw genes of the present invention may be used in combination with an appropriate means, such as a label, for determining hybridization. A wide variety of appropriate indicator means are known in the art, including radioactive, enzymatic or other ligands, such as avidin/biotin, which are capable of providing a detectable signal. In some diagnostic embodiments, an enzyme tag, such as urease, alkaline phosphatase or peroxidase, instead of a radioactive tag may be used. In the case of enzyme tags, colorimetric indicator substrates are known which can be employed to provide a means visible to the human eye or spectrophotometrically, to identify specific hybridization with samples containing *hmw* genes sequences.

The nucleic acid sequences of *hmw* genes of the present invention are useful as hybridization probes in solution hybridizations and in embodiments employing solid-phase procedures. In embodiments involving solid-phase procedures, the test DNA (or RNA) from samples, such as clinical samples, including exudates, body fluids (e.g., serum, amniotic fluid, middle ear effusion, sputum, bronchoalveolar lavage fluid) or even tissues, is adsorbed or otherwise affixed to a selected matrix or surface. The fixed, single-stranded nucleic acid is then subjected to specific hybridization with selected probes comprising the nucleic acid sequences of the *hmw* genes or fragments thereof of the present invention under desired conditions. The selected conditions will depend on the particular circumstances based on the particular criteria required depending on, for example, the G+C contents, type of target nucleic acid, source of nucleic acid, size of hybridization probe, etc. Following washing of the hybridization surface so as to remove non-specifically bound probe molecules, specific hybridization is detected, or even quantified, by means of the label. It is preferred to select nucleic acid sequence portions which are conserved among species of *Haemophilus.* The selected probe may be at least 18 bp in length and may be in the range of 30 bp to 90 bp long.

### 8. Expression of the High Molecular Weight Protein Genes

Plasmid vectors containing replicon and control sequences which are derived from species compatible with the host cell may be used for the expression of the high molecular weight protein genes in expression systems. The vector ordinarily carries a replication site, as well as marking sequences which are capable of providing phenotypic selection in transformed cells. For example, *E. coli* may be transformed using pBR322 which contains genes for ampicillin and tetracycline resistance and thus provides easy means for identifying transformed cells. The pBR322 plasmid, or other microbial plasmid or phage, must also contain, or be modified to contain, promoters which can be used by the host cell for expression of its own proteins.

In addition, phage vectors containing replicon and control sequences that are compatible with the host can be used as a transforming vector in connection with these hosts. For example, the phage in lambda GEM™-11 may be utilized in making recombinant phage vectors which can be used to transform host cells, such as *E. coli* LE392.

Promoters commonly used in recombinant DNA construction include the β-lactamase (penicillinase) and lactose promoter systems and other microbial promoters, such as the T7 promoter system employed herein in preferred embodiments (U.S. Patent 4,952,496). Details concerning the nucleotide sequences of promoters are known, enabling a skilled worker to ligate them functionally with genes. The particular promoter used will generally be a matter of choice depending upon the desired results. Hosts that are appropriate for expression of the HMW protein and immunological fragments or analogs thereof include *E. coli*, *Bordetella* species, *Bacillus* species, *Haemophilus,* fungi, yeast or the baculovirus expression system may be used. *E. coli* is the preferred host used herein.

In accordance with this invention, it is preferred to produce the HMW proteins by recombinant methods, particularly when the naturally-occurring HMW protein as purified from a culture of a species of *Haemophilus* may include trace amounts of toxic materials or other contaminants. This problem can be avoided by using recombinantly-produced HMW protein in heterologous systems which can be isolated from the host in a manner to minimize contaminants in the purified materials, specifically employing the constructs described herein. Furthermore, recombinant methods of production permit the manufacture of HMW1 or HMW2, or immunogenic fragments and analogs thereof, separate from one another and in highly-purified form, which is distinct from the normal combined proteins present in *Haemophilus* strains.

### Biological Deposits

Certain vectors that contain nucleic acid coding for a high molecular weight protein of a non-typeable strain of *Haemophilus* that are described and referred to herein have been deposited with the America Type Culture Collection (ATCC) located at 10801 University Boulevard, Manassas, Virginia 20110-2209, USA, pursuant to the Budapest Treaty and prior to the filing of this application. Samples of the deposited vectors will become available to the public and all restrictions imposed on access to the deposits will be removed upon grant of a patent based on this United States patent application. In addition, the deposits will be replaced if viable samples cannot be dispensed by the Depository. The invention described and claimed herein is not limited in scope by the biological materials deposited, since the deposited embodiment is intended only as an illustration of the invention. Any equivalent or similar vectors that contain nucleic acid which encodes equivalent or similar antigens as described in this application are within the scope of the invention.

### Deposit Summary

| Plasmid | | ATCC | Date Deposited |
|---|---|---|---|
| DS-1046-1-1 | (pT7 hmw1ABC(125)) | 203263 | 25-Sept-1998 |
| JB-2507-7 | (pT7 hmw1A(125)/T7 hmw1ABC(125)) | 203262 | 25-Sept-1998 |
| BK-86-1-1 | (pT7 hmw1A(125)/T7 hmw1ABC(125)/Kan^{R}) | 203258 | 25-Sept-1998 |
| BK-35-4 | (pT7 hmw1ABC(125)/cer) | 203259 | 25-Sept-1998 |
| BK-76-1-1 | (pT7 hmw1ABC(125)/cer/Kan^{R}) | 203261 | 25-Sept-1998 |
| DS-2334-5 | (pT7 hmw2A (LCDC2)/hmw1BC(12)/cer/Kan^{R}) | 203260 | 25-Sept-1998 |
| JB-2646-1 | (pT7 hmw1BC(12)/cer/Kan^{R}) | 203256 | 25-Sept-1998 |
| DS-2400-13 | (pBRT7 hmw1A/T7 hmw1ABC/cer/Kan^{R}) | 203257 | 25-Sept-1998 |

### EXAMPLES

The above disclosure generally describes the present invention. A more complete understanding can be obtained by reference to the following specific Examples. These Examples are described solely for the purposes of illustration and are not intended to limit the scope of the invention. Changes in form and substitution of equivalents are contemplated as circumstances may suggest or render expedient. Although specific terms have been employed herein, such terms are intended in a descriptive sense and not for the purposes of limitations.

Methods of molecular genetics, protein biochemistry, immunology and fermentation technology used, but not explicitly described in this disclosure and these Examples, are amply reported in the scientific literature and are well within the ability of those skilled in the art.

### Example 1

This Example describes the construction of plasmid DS-1091-2 that expresses the *hmw1ABC* genes encoding the full-length 160 kDa HMW1A protein.

Plasmid pHMW1-15 (ref. 8) contains about 400 bp of 5'-flanking region, including the *hmw1* promoter, inserted between the T7 promoter and the start of the hmw1ABc coding region (Figure 1). There is a unique *Bgl* II site in the multiple cloning site of pHMW1-15 and a unique *BamH* I site in the coding region of *hmw1A.* The 2.2 kb *Bgl* II-*BamH* I fragment was subcloned for further manipulation, generating plasmid DS-1035-12. A 400 bp *Xba* I-*Bsm* I fragment containing the 5'-flanking region was replaced by approximately 86 bp oligonucleotides (Figure 1B) that joined the T7 promoter directly to the ATG start codon of the *hmw1A* gene in plasmid DS-1055R-2. The 1.5 kb *Xba* I-*BamH* I fragment from DS-1055R-2 was inserted into pHMW1-15 that had been digested with the same enzymes to generate plasmid DS-1091-2.

### Example 2

This Example describes the construction of plasmid DS-1094-2 that expresses the *hmw2ABC* genes encoding the full-length 155 kDa HMW2A protein.

Plasmid pHMW2-21 (ref. 10) contains about 800 bp of 5'-flanking sequence, including the *hmw2* promoter, between the T7 promoter and the start of the *hmw2ABC* coding sequence (Figure 2). Plasmid pHMW2-21 has two *EcoR* I sites, one in the multiple cloning site, and one within the coding sequence of the *hmw2A* gene. The 2.5 kb *EcoR* I fragment was subcloned for further manipulation, generating plasmid DS-1036-9. The approximately 800 bp *Xba* I-*Bsm* I fragment containing the 5'-flanking sequences, was replaced by the same approximately 86 bp oligonucleotides that were used for *hmw1* (Figure 1B), to join the T7 promoter directly to the ATG start codon of *hmw2A*, generating plasmid DS-1056R-1-1. An intermediate plasmid (DS-1078-4) was necessary to introduce convenient restriction enzyme sites, and the *Xba* I insert was excised, then re-ligated to change orientation for plasmid DS-1085-8. Plasmid DS-1085-8 was linearized with *EcoR* I, dephosphorylated, and ligated with the 8 kb *EcoR* I fragment from pHMW2-21, to generate plasmid DS-1094-2 that contains the T7 promoter joined directly to the coding sequence of *hmw2ABC.*

### Example 3

This Example illustrates the construction of plasmid DS-1046-1-1 that expresses the *hmw1ABC* genes encoding the mature 125 kDa HMW1A protein.

Plasmid pHMW1-15 (ref. 8) contains a *Xba* I site within the T7 promoter sequence and a unique *BamH* I site within the coding sequence of the mature HMW1A protein (Figure 3A). The 1.8 kb *Xba* I*-BamH* I fragment of pHMW1-15 was deleted and replaced by an approximately 114 bp *Xba* I*-BamH* I fragment generated from oligonucleotides (Figure 3B). The resultant 11.3 kb plasmid, DS-1046-1-1, thus contains the T7 promoter joined in frame with the *hmw1ABC* operon that encodes the mature 125 kDa HMW1A protein.

### Example 4

This Example illustrates the construction of plasmid DS-1200-3 that expresses the *hmw2AB* partial C genes encoding the mature 120 kDa HMW2A protein.

Plasmid pHMW2-21 (ref. 10) contains an *EcoR* I site within the coding sequence of the mature HMW2A protein. However, it is not unique (Fig. 4A). A multi-step construction process involved first re-creating part of the T7 promoter and the start of the *hmw2A* gene encoding the mature HMW2A protein, from 105 bp oligonucleotides (Figure 4B). Plasmid DS-1134-2 contains the complete T7 promoter and the 5'-sequence encoding the mature HMW2A protein. Plasmid DS-1134-2 was linearized with *EcoR* I, dephosphorylated, and the 8 kb *EcoR* I fragment from pHMW2-21, containing most of the *hmw2A* gene and all of the *hmw2B* and *hmw2C* genes, was inserted. Plasmid DS-1147-4 is a pUC-based plasmid containing the *T7 hmw2ABC* gene cassette. The entire cassette was removed on a 6.5 kb *Bgl* II-*Xho* I fragment and inserted into pT7-7 that had been digested with *Bgl* II and *Sal* I, to create plasmid DS-1200-3. Part of the *hmw2C* gene was deleted in this construct.

### Example 5

This Example illustrates the construction of plasmid DS-1122-2 that contains the *hmw1A* gene encoding the mature 125 kDa HMW1A protein, and part of the *hmw1B* gene.

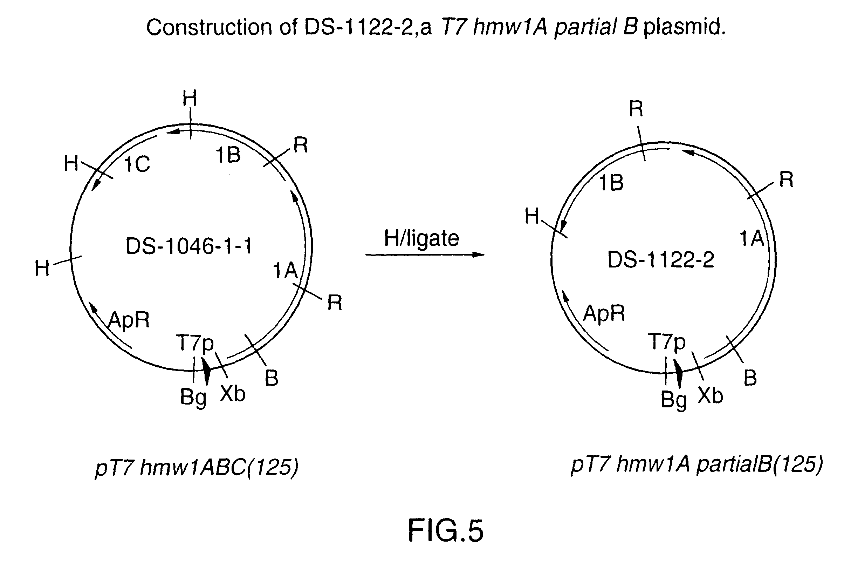

Plasmid DS-1046-1-1 (Figure 3A; Example 3) contains three *Hind* III sites, one within the *hmw1b* gene, one within the *hmw1C* gene and one in the 3'-region of the multiple cloning site (Fig 5). When DS-1046-1-1 was digested with *Hind* III, then re-ligated, plasmid DS-1122-2 was generated that contains a complete *hmw1A* gene encoding the mature 125 kDa HMW1A protein, part of the *hmw1B* gene, and no *hmw1C* gene.

### Example 6

This Example illustrates the construction of plasmid JB-2330-7 that contains the *hmw1A* gene encoding the mature 125 kDa HMW1A protein, with no other *hmw* genes.

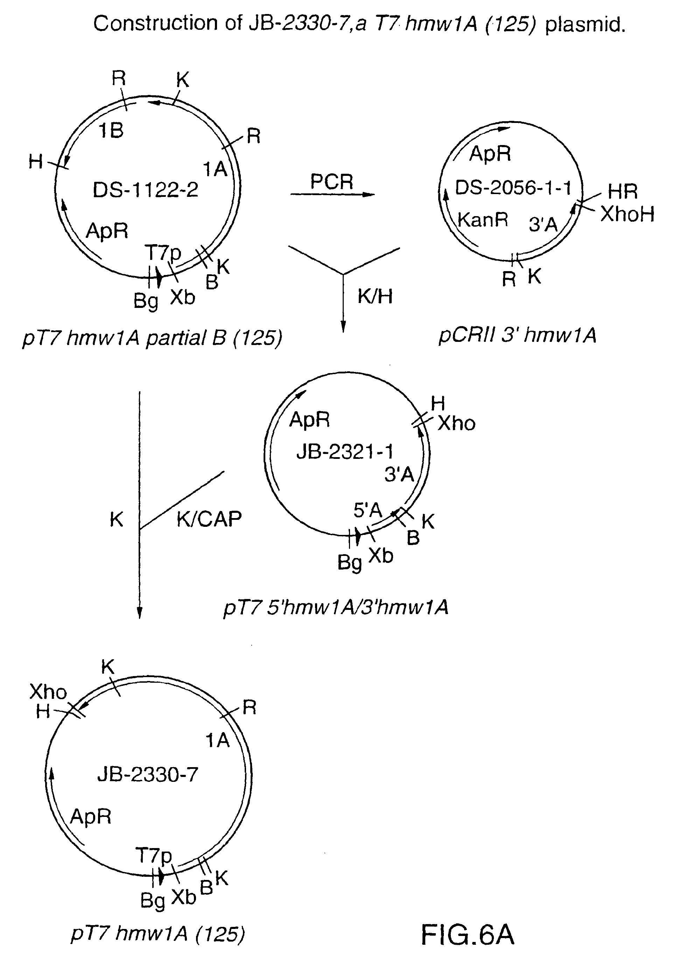

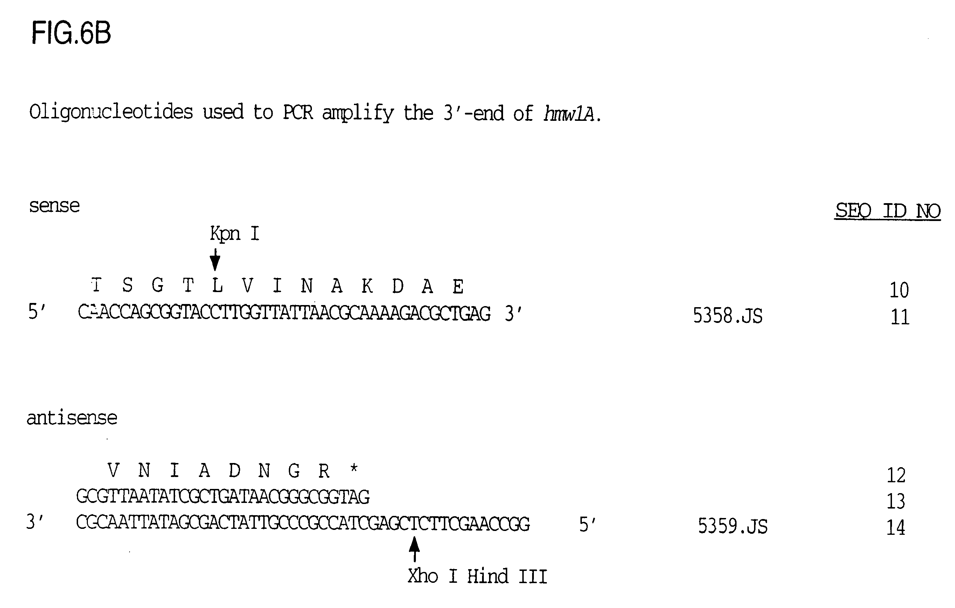

PCR amplification was performed on plasmid DS-1122-2 (Figure 5; Example 5) DNA to generate a 500 bp fragment from the *Kpn* I site near the 3'-end of *hmw1A,* through the terminator, and introducing restriction enzyme sites for *Xho* I and *Hind* III at the 3'-end. The fragment was cloned into pCR II, generating plasmid DS-2056-1-1 (Figure 6A) and the oligonucleotides used are shown in Figure 6B. Plasmid DS-1122-2 was digested with *Kpn* I and *Hind* III, which deletes most of the *hmw1A* gene and all of the *hmw1B* gene fragment. The 2.6 kb *Kpn I-Hind* III vector fragment from DS-1122-2 was ligated with the 0.5 kb *Kpn* I*-Hind* III fragment from DS-2056-1-1 to generate plasmid JB-2321-1, that contains approximately 0.2 kb of *5'-hmw1A* sequence and approximately 0.5 kb of *3'-hmw1A* sequence, joined at the *Kpn* I site. Plasmid JB-2321-1 was linearized with *Kpn* I, dephosphorylated, and the internal 2.7 kb *Kpn* I fragment from DS-1122-2 was inserted to create plasmid JB-2330-7. This plasmid contains a T7 *hmw1A* gene cassette with no additional *hmw1* gene sequences.

### Example 7

This Example illustrates the construction of plasmid JB-2369-6 that contains tandem copies of the T7 *hmw1A* gene cassette encoding the mature 125 kDa HMW1A protein.

Plasmid JB-2330-7 (Figure 6A; Example 6) was digested with *Bgl* II and *Hind* III and the T7 *hmw1A* gene cassette was subcloned into pUC-BgXb that had been digested with *Bgl* II and *Hind* III, creating plasmid JB-2337-1 (Figure 7). Plasmid JB-2337-1 was digested with *Sal* I and *Xho* I which released the T7 *hmw1A* cassette, on a fragment with compatible ends. Vector DS-1843-2 is a pBR328-based plasmid containing transcription terminators and a multiple cloning site with a unique *Xho* I site. Vector DS-1843-2 was digested with *Xho* I, dephosphorylated, and then ligated with the 3.5 kb *Sal I-Xho* I *T7 hmw1A* gene cassette to generate plasmid JB-2347-5. Because the *Sal* I and *Xho* I sites seal, this plasmid contains a unique *Xho* I site at the 3'-end of the *T7 hmw1A* gene cassette that can be used to insert additional *Sal* I-*Xho* I *T7 hmw1A* gene cassettes derived from JB-2337-1. Plasmid JB-2369-6 contains two tandem *T7 hmw1A* genes introduced in this way.

### Example 8

This Example illustrates the construction of plasmids DS-2084-3 and DS-2084-1 that contain one or two tandem copies of the *T7 hmw2A* gene cassette encoding the mature HMW2A protein.

Plasmid DS-1200-3 (Figure 4A, Example 4) contains the T7 *hmw2AB partial C* gene cassette. There are two *Mlu* I sites in DS-1200-3, one located near the 3' -end of *hmw2A* and the other located near the 5'-end of *hmw2B* (Figure 8A). Oligonucleotide primers were used to PCR amplify a 247 bp fragment of the 3'-end of the *hmw2A* gene from the *Mlu* I site, and to introduce a unique *BamH* I site following the termination codon of *hmw2A* (Figure 8B). The 247 bp PCR fragment was subcloned into pCRII generating plasmid DS-2056-3-1. Plasmid DS-1200-3 was digested with *Bgl* II and *Mlu* I and the 3.2 kb fragment containing the T7 promoter and most of the *hmw2A* gene was purified. Plasmid pUC-BgXb was digested with *Bgl* II and *BamH* I and dephosphorylated. The *Bgl* II-*Mlu* I *hmw2A* gene fragment and the *Mlu* I*-BamH* I PCR fragment from DS-2056-3-1 were ligated into the pUC vector to generate plasmid DS-2073-3, which thus contains a 3.4 kb *T7 hmw2A* gene cassette on a *Bgl* II-*BamH* I fragment, with no additional *hmw2* genes. Plasmid DS-1843-2 was linearized with *Bgl* II and the 3.4 kb *Bgl* II*-BamH* I cassette was inserted, generating plasmid DS-2084-3 that contains a single *T7 hmw2A* gene cassette and plasmid DS-2084-1 that contains two tandem *T7 hmw2A* gene cassettes.

### Example 9

This Example illustrates the construction of plasmids JB-2507-7 and BK-86-1-1 that contain tandem T7 *hmw1A*/*T7 hmw1ABC* genes encoding the mature 125 kDa HMW1A protein and are resistant to ampicillin or kanamycin, respectively.

Plasmid DS-1046-1-1 (Figure 3A; Example 3) contains the T7 *hmw1ABC* gene cassette and has a unique *BamH* I site within the coding region of the mature HMW1A protein. Plasmid JB-2369-6 (Figure 7; Example 7) contains tandem *T7 hmw1A* gene cassettes, each of which contains an internal *BamH* I site within the coding sequence for HMW1A. When plasmid JB-2369-6 was digested with *BamH* I, a 3.5 kb fragment was generated that contains the 3'-end of the first *hmw1A* gene and the T7 promoter and 5'-end of the second *hmw1A* gene. This fragment was ligated into the *BamH* I site of DS-1046-1-1 to create plasmid JB-2507-7 that contains tandem *T7 hmw1A*/*T7 hmw1ABC* gene cassettes (Figure 9). The unique *Sal* I site found in the multiple cloning site of the pT7-7 vector backbone, was used to linearize JB-2507-7. The kanamycin resistance cassette was excised from pUC-4K by *Sal* I digestion, and ligated with the JB-2507-7 vector to generate plasmid BK-86-1-1.

### Example 10

This Example illustrates the construction of plasmids BK-35-4 and BK-76-1-1 that contain the T7 *hmw1ABC* gene cassette and an *E. coli* cer gene and are ampicillin or kanamycin resistant, respectively.

Plasmid DS-2224-1-4 (Figure 10) contains an *E. coli* cer gene (ref. 13) that was created from approximately 290 bp oligonucleotides cloned into the *BamH* I site of pUC-BgXb. Plasmid DS-1046-1-1 (Figure 3; Example 3) contains a unique *Bgl* II site upstream of the T7 promoter. Plasmid DS-1046-1-1 was linearized with *Bgl* II, dephosphorylated, and ligated with the 290 bp *BamH* I fragment containing the cer gene from DS-2224-1-4, to create plasmid BK-35-4. The kanamycin resistance cassette was excised from pUC-4K by *Sal* I digestion and was inserted at the unique *Sal* I site of BK-35-4 to create plasmid BK-76-1-1.

### Example 11

This Example illustrates the analysis of the production of rHMW1 and rHMW2 proteins from the different constructs produced in the preceding Examples.

Plasmids were introduced into *E. coli* BL21(DE3) cells by electroporation using a BioRad apparatus. Strains were grown at 37°C in NZCYM medium to an optical density of A₅₇₈=0.3, then induced by the addition of lactose to 1.0% for 4 hours. Samples were adjusted to 0.2 OD/µl with SDS-PAGE lysis + loading buffer and the same amount of protein sample was loaded onto SDS-PAGE gels. Figure 11 illustrates the relative production of rHMW proteins from various constructs as analysed by SDS PAGE gels. The identification of the lanes in relation to the specific constructs is given in the description of the Figure above. "a" indicates the band for HMWA proteins, "b" indicates the band for HMWB proteins and "c" indicates the band for HMWC proteins.

As may be seen therein, the production of the HMW1A, B, and C proteins from the T7 hmw1ABC(160 construct (lane 3) is negligible. The production of all three proteins is improved in the T7 hmw1ABC(125) construct (lane 4). In lane 5, there is no production of the HMW1C protein and the HMW1B protein is slightly reduced in size due to the truncation of its gene in the T7 hmw1A partial B construct. In lane 6, there is no production of HMW1B or HMW1C protein from the T7 hmw1A(125) construct. Lane 7 shows that there was marginal, if any, improvement in the production of HMW1A from the T7 hmw1A/T7 hmw1A construct. In lane 8, the production of HMW1A, HMW1B and HMW1C proteins is evident when expressed from the T7 hmw1A/T7 hmw1ABC construct. In lane 9, the HMW1A, HMW1B and HMW1C proteins are all produced from the T7 hmw1ABC/cer construct.

### Example 12

This Example illustrates the purification of recombinant HMW1 and HMW2 proteins.

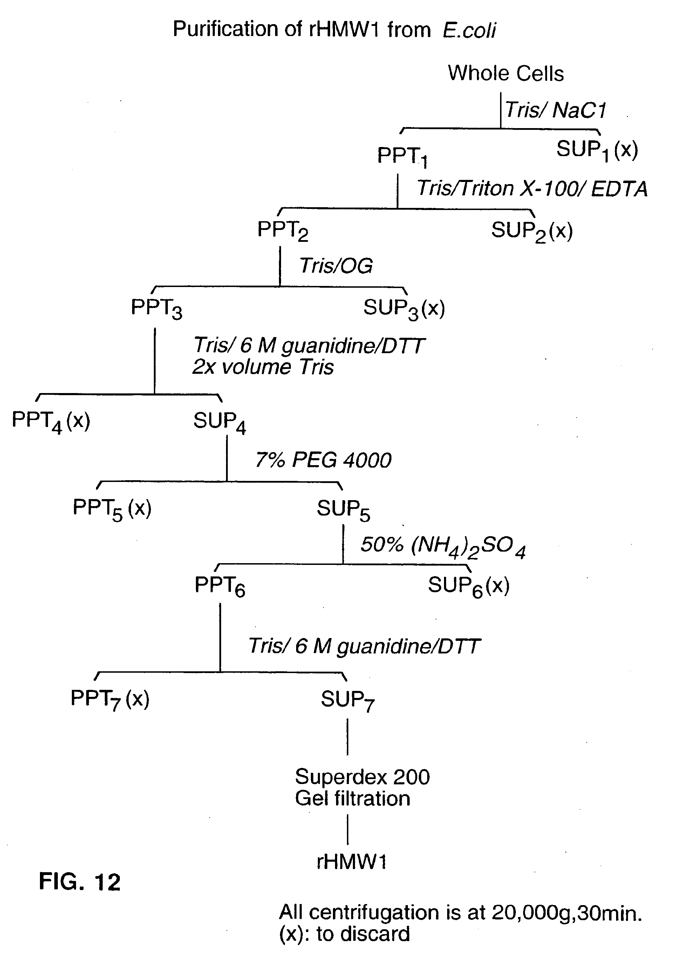

All the recombinant HMW proteins were expressed as inclusion bodies in *E. coli,* regardless of whether there were complete or partial deletions of the *B* and *C* genes in the various constructs, and were purified by the same procedure (Figure 12) *E. coli* cell pellets from 500 ml culture were resuspended in 50 ml of 50 mM Tris-HC1, pH 8.0, containing 0.1 M NaCl, and disrupted by sonication. The extract was centrifuged at 20,000 g for 30 min and the resultant supernatant was discarded. The pellet (PPT₁) was further extracted, in 50 ml of 50 mM Tris-HCl, pH 8.0 containing 0.5% Triton X-100 and 10 mM EDTA, then centrifuged at 20,000 g for 30 min, and the supernatant was discarded. The pellet (PPT₂) was further extracted in 50 ml of 50 mM Tris-HCl, pH 8.0, containing 1% octylglucoside, then centrifuged at 20,000 g for 30 min, and the supernatant was discarded.

The resultant pellet (PPT₃), obtained after the above extractions, contains the inclusion bodies. The pellet was solubilized in 6 ml of 50 mM Tris-HCl, pH 8.0, containing 6 M guanidine and 5 mM DTT. Twelve ml of 50 mM Tris-HCl, pH 8.0 was added to this solution and the mixture was centrifuged at 20,000 g for 30 min. The supernatant (SUP₄) was precipitated with polyethylene glycol (PEG) 4000 at a final concentration of 7%. The resultant pellet (PPT₅) was removed by centrifugation at 20,000 g for 30 min and the supernatant was precipitated by (NH₄) ₂SO₄ at 50% saturation. After the addition of (NH₄) ₂SO₄, the solution underwent phase separation with protein going to the upper phase, which was then subjected to centrifugation at 20,000 g for 30 min. The resultant pellet (PPT₆) was dissolved in 2 ml of 50 mM Tris-HCl, pH 8.0, containing 6 M guanidine HCl and 5 mM DTT and the clear solution was purified on a Superdex 200 gel filtration column equilibrated in 50 mM Tris-HCl, pH 8.0, containing 2 M guanidine HCl. The fractions were analysed by SDS-PAGE and those containing the purified rHMW1 were pooled and dialysed overnight at 4°C against PBS, then centrifuged at 20,000 g for 30 min. The protein remained soluble under these conditions and glycerol was added to the rHMW1 preparation at a final concentration of 20% for storage at -20°C.

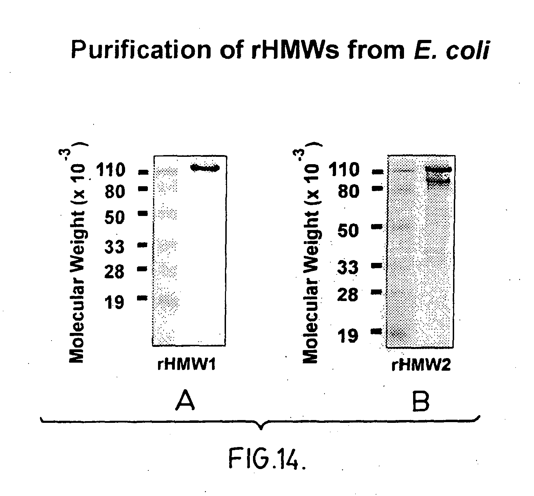

SDS-PAGE analysis of a representative rHMW1 protein (abc/cer) at various stages of purification is shown in Figure 13. The identification of the lanes is given above in the description of the Figures. Three major protein bands at approximately 110, 80, and 60 kDa, (lane 6) were evident after the initial three extractions with 50 mM Tris-HCl/0.1 M NaCl, pH 8.0 (lane 3); 50 mM Tris-HCl/0.5% Triton X-100, pH 8.0 (lane 4); and 50 mM Tris-HCl/1% octylglucoside, pH 8.0 (lane 5). These three proteins represent the products of hmw1A, C and B genes, respectively, as confirmed by N-terminal amino acid sequencing. The products of the B and C genes were less soluble in the guanidine hydrochloride solution (lane 7), and were easily separated from the gene A product (HMW1, lane 8) by diluting the guanidine HCl concentration from 6 M to 2 M. Precipitation with 7% polyethylene glycol (PEG) 4000 removed other contaminating proteins (lane 9) from the rHMW1 preparation. A final ammonium sulfate precipitation not only concentrated rHMW1 from the PEG soluble fraction (lane 10), but also effectively removed the residual PEG (lane 11) and (NH₄)₂SO₄ salt (lane 12) through a phase separation that resulted from the mixing of the PEG solution with a high concentration of (NH₄) ₂SO₄. The rHMW1 pellet was then dissolved in 50 mM Tris-HCl, pH 8.0, containing 6 M guanidine HCl and 5 mM DTT, and purified on a Superdex 200 gel filtration column pre-equilibrated in 50 mM Tris-HCl, pH 8.0, containing 2 M guandine HCl (Figure 14, panel A). The average yield of the purified rHMW1 is about 10 mg L⁻¹ culture. SDS-PAGE analysis of the purification of rHMW2A from construct *T7 hmw2A*/*T7 hmw2A* is shown in Figure 14, panel B.

### Example 13

This Example illustrates the stability of the purified rHMW1A protein.

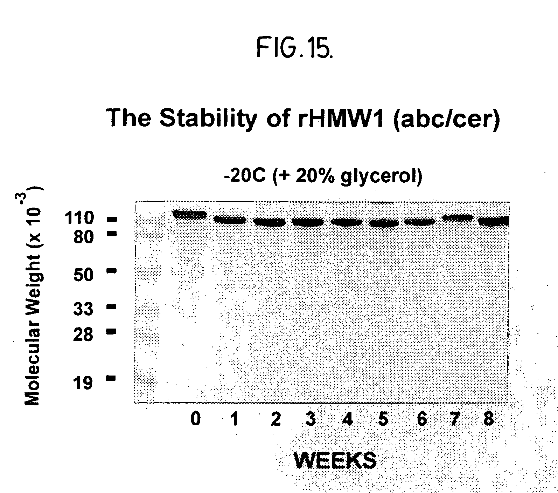

To study the stability of rHMW1A, the purified rHMW1A protein produced in accordance with Example 12 was stored at 4°C or -20°C, with or without glycerol. In the absence of glycerol, the protein was found to be degraded when stored at 4°C and tended to precipitate when stored at -20°C. The addition of glycerol to a final concentration of 20% not only significantly enhanced the solubility of rHMW1A, but also increased the stability of the protein when stored at -20°C. The protein remained intact for at least eight weeks even after repeated freezing and thawing (Figure 15).

### Example 14

This Example illustrates the immunogenicity of rHMW1A and rHMW2A proteins produced from different constructs.

To study the immunogenicity of the rHMW1 protein produced from *T7 hmw1ABC* (pDS-1046-1-1; Figure 3A, Example 3) or *T7 hmw1ABC*/*cer* (pBK-76-1-1; Figure 10, Example 10) constructs and purified by the procedure of Example 12, groups of five BALB/c mice (Charles River, Quebec) were immunized s.c. on days 1, 29, and 43 with 0.3, 1, and 3 µg of antigen, in the presence AlPO₄ (1.5 mg per dose). Blood samples were collected on days 0, 14, 28, 42 and 56.

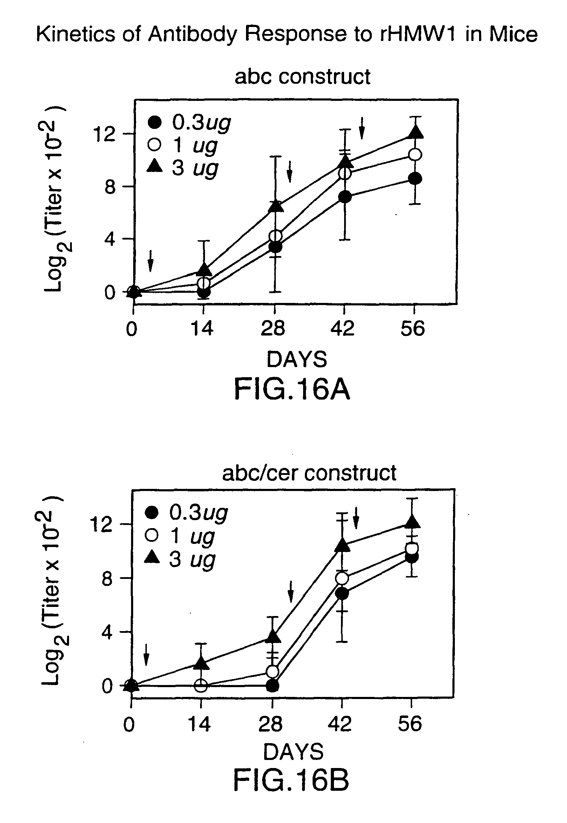

Mice immunized with purified rHMW1 derived from the T7 *hmw1ABC or T7 hmw1ABC*/*cer* constructs (0.3 to 3 µg per dose), generated dose-dependent anti-rHMW1 antibody responses (Figure 16), suggesting that both proteins had remained immunogenic after inclusion body extraction and solubilization. No statistically significant difference was found in the antibody titers induced by the protein from these two constructs in mice.

To compare the immunogenicity of rHMW1 and rHMW2 proteins produced from several different constructs and purified according to Example 12, groups of 9 chinchillas (Moulton Chinchilla Ranch) were immunized i.m. on days 1, 14, and 28 with 30 µg of rHMW protein in the presence AlPO₄ (1.5 mg per dose). Blood samples were collected on day 42. Chinchilla anti-HMW antibody responses induced by various forms of rHMW are summarized in Table 1.

It was found that the rHMW1 prepared from the T7 *hmw*/*ABC (abc)* (pDS-1046-1-1; Figure 3A, Example 3), T7 *hmw1A*/*T7 hmw1ABC* (*a*/*abc*) (pBK-86-1-1; Figure 9, Example *9), T7 hmw1ABC*/*cer (abc*/*cer)* (pBK76-1-1; Figure 10, Example 10), and *T7 hmw1A*/*T7 hmw1A (2xa)* (pJB-2369-6; Figure 7, Example 7) constructs, but not the T7 *hmw1AB(125) (abΔ)* (pDS-1122-2; Figure 5, Example 5) construct, induced significant antibody titers in chinchillas after three immunizations. Similarly, the rHMW2 prepared from *T7 hmw2ABC (abc)* (pDS-1147-4; Figure 4A, Example 4) or *T7 hmw2A*/*T7 hmw2A (2xa)* (pDS-2084-1; Figure 8A, Example 8) constructs and purified following the procedure of Example 12 elicited significant antibody titers in chinchillas after three immunizations.

Anti-rHMW IgG titers were determined by antigen-specific enzyme-linked immunosorbent assays (EIAs). Microtiter wells (Nunc-MAXISORP, Nunc, Denmark) were coated with 50 µl of protein antigen (0.5 µg ml⁻¹). The reagents used in the assays are as follows: affinity-purified F (ab')₂ fragments of goat anti-mouse IgG (Fc-specific) conjugated to horseradish peroxidase (Jackson ImmunoResearch Labs, Mississauga, Ontario); affinity-purified guinea pig anti-IgG antibody (1 µg ml⁻¹) (prepared by this laboratory); and affinity-purified F(ab')₂ fragment of goat anti-guinea pig IgG (H+L) antibodies conjugated to horseradish peroxidase (HRP) (Jackson ImmunoResearch Laboratories) used as a reporter. Chinchilla IgG was purified from chinchilla serum according to Barenkamp (ref. 14). Generation and purification of guinea pig anti-chinchilla IgG antibodies were described earlier (ref. 15). The reactions were developed using tetramethylbenzidine (TMB/H₂O₂, ADI, Mississauga, Ontario) and absorbancies were measured at 450 nm (using 540 nm as a reference wavelength) in a Flow Multiskan MCC microplate reader (ICN Biomedicals, Mississauga, Ontario). The reactive titer of an antiserum was defined as the reciprocal of the dilution consistently showing a two-fold increase in absorbance over that obtained with the pre-bleed serum sample.

### Example 15

This Example illustrates the protective ability of rHMW1A and rHMW2A proteins produced from different constructs.

The immunization and intranasal challenge with freshly grown streptomycin resistant NTHi strain 12 in chinchillas has been described (ref. 15). Briefly, groups of 8 to 9 animals were immunized three times i.m. with one of: 30 µg of purified rHMW1 or rHMW2, 2 x 10⁹ cfu of heat inactivated (56°C, 10 min) NTHi whole cells in alum, or alum alone on days 0, 14 and 28. Serum samples and nasal wash samples were taken on day 42 for measurement of anti-HMW1 or anti-rHMW2 antibody titers by EIAs.

On day 44, animals were lightly anesthetized using xylazine/ketamine HCl by intramuscular injection (0.06 mg xylazine and 0.3 mg ketamine HCl per kg body weight). Intranasal inoculations were performed via passive inhalation (50 µl per nares, total 0.1 ml per animal) of freshly cultured streptomycin-resistant NTHi strain 12 in BHI medium supplemented with hemin and NAD both at 2 µg ml⁻¹. The dose of bacterial challenge was 1 x 10⁸ cfu per animal. Nasopharyngeal lavages were performed 4 days post inoculation on anesthetized chinchillas (xylazine/ketamine HCl, same route and dose as on day 44). Secretions were obtained by irrigating the nasopharynx with 1 ml sterile saline and collecting fluid out of the contralateral nares. Normally, about 500 µl of fluid was collected from each animal and 25 µl of sample was plated on a chocolate agar plate in the presence of 50 µl of streptomycin (20 mg ml⁻¹).

The protective effect of parenteral immunization with various rHMW1 and rHMW2 preparations on NP colonization of chinchilla nasopharynx with NTHi strain 12 is summarized in Table 2. 67 to 88% of the control animals immunized with alum only, had culture-positive nasal lavage fluids. In contrast, 67 to 80% of animals immunized with the rHMW1 protein purified from the constructs *abc* (pDS-1046-1-1), *a*/*abc* (pBK86-1-1), or *abc*/*cer* (pBK-76-1-1) were largely protected. In animals immunized with the rHMW1 protein derived from either construct abΔ (pDS-1122-2) or construct 2x*a* (pJB-2369-6), 70 to 90% were infected. These results clearly indicated that, in order to achieve a significant protection against NTHi strain 12 colonization in the chinchilla model, the rHMW1 protein must be derived from a construct with intact ABC genes.

Similar results were also observed with rHMW2 protein. As shown in Table 2, animals immunized with the rHMW2 protein purified from the construct *abc* (pDS-1147-4), but not from the construct 2x*a* (pDS-2084-1), were protected against NTHi strain 12 colonization in the chinchilla model. In all cases, significant protection was observed in chinchillas immunized with the heat-inactivated NTHi 12 whole cell preparations, prepared in accordance with Example of USP 5,603,938.

### Example 16

This Example illustrates the cloning and sequence analysis of *hmwA* genes from additional NTHi strains.

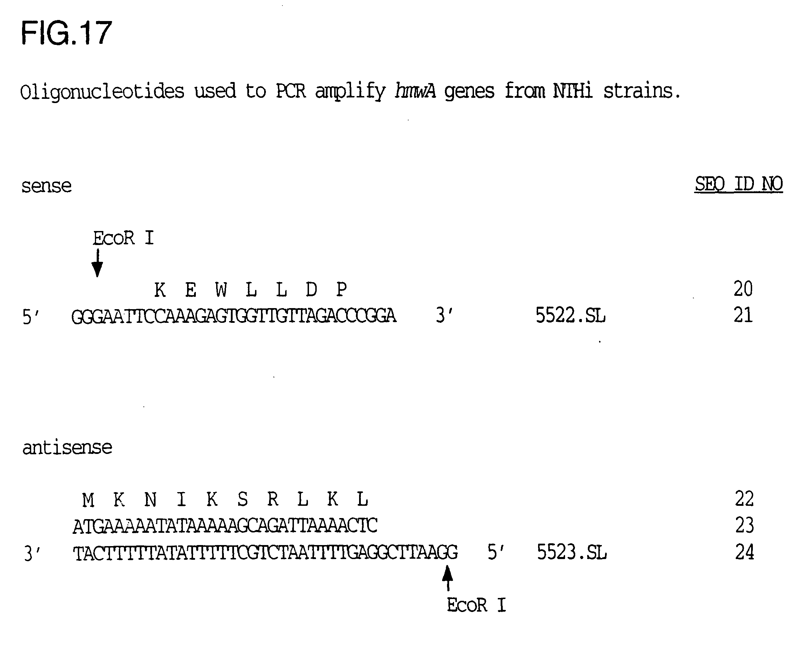

Chromosomal DNA was prepared from several NTHi strains and PCR was performed using the oligonucleotide primers shown in Figure 17. The sense primer (5522.SL, SEQ ID NO: 21) corresponds to the conserved region in the *hmwA* genes encoding the residues immediately upstream of the processing site for the mature HMW proteins. The antisense primer (5523.SL, SEQ ID NO: 24) corresponds to the start of the *hmwB* gene that is also conserved.

PCR amplification was performed as follows: each reaction mixture contained 5-100 ng of DNA, 1 µg of each primer, 5 units of taq+ or tsg+ (Sangon) or taq plus long (Stratagene), 2mM dNTPs, 20 mM Tris-HCl (pH 8.8) , 10 mM KCl, 10 mM (NH₄) ₂SO₄, 2 mM MgSO₄, 0.1% Triton X-100, BSA. Cycling conditions were: 95°C for 1 min, followed by 25 cycles of 95°C for 30 sec, 45°C for 1 min, 72°C for 2 min; then 72°C for 10 min.

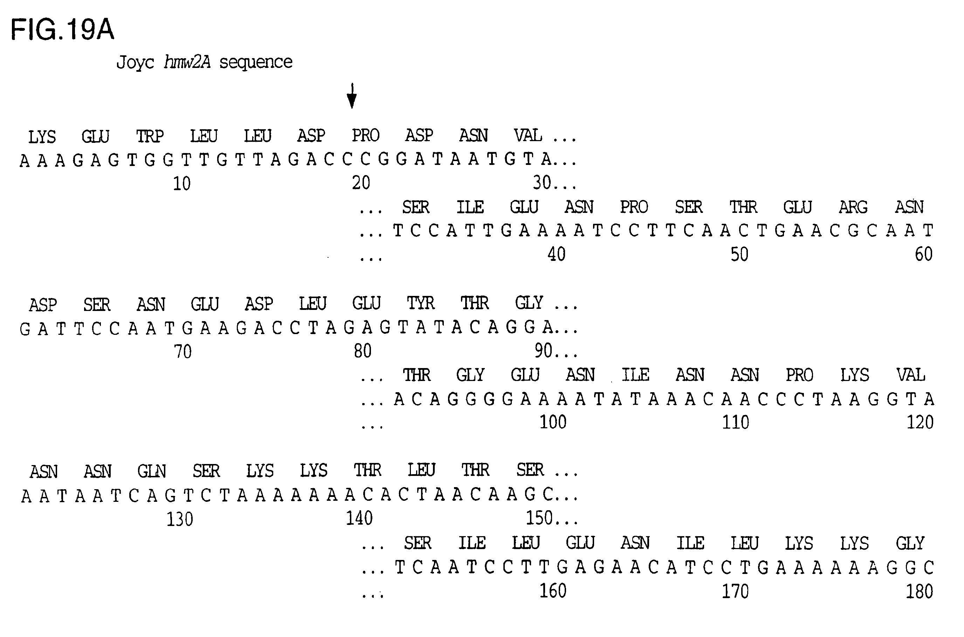

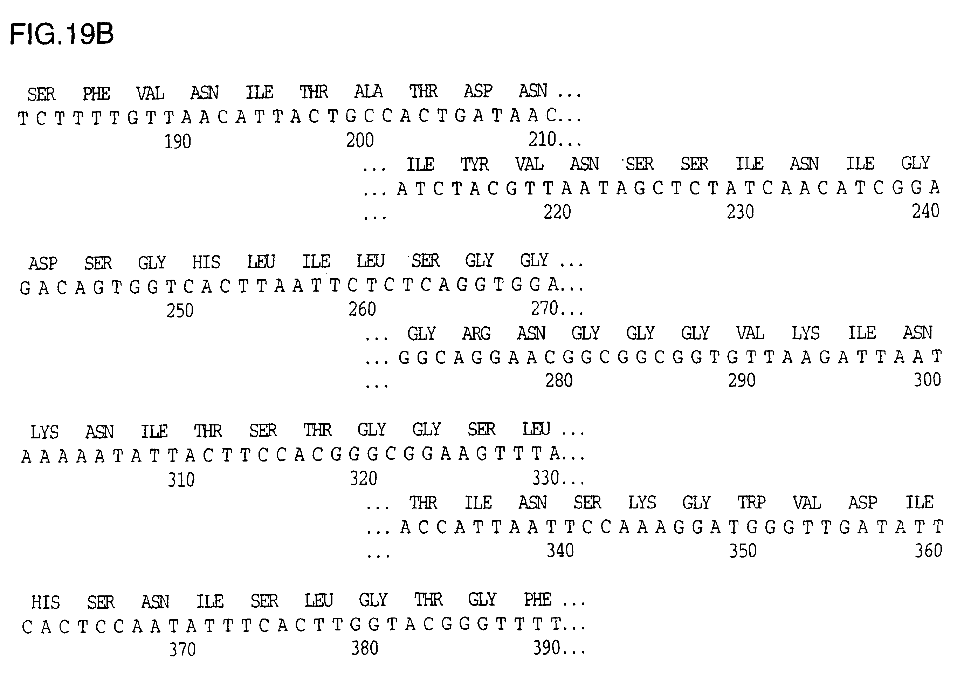

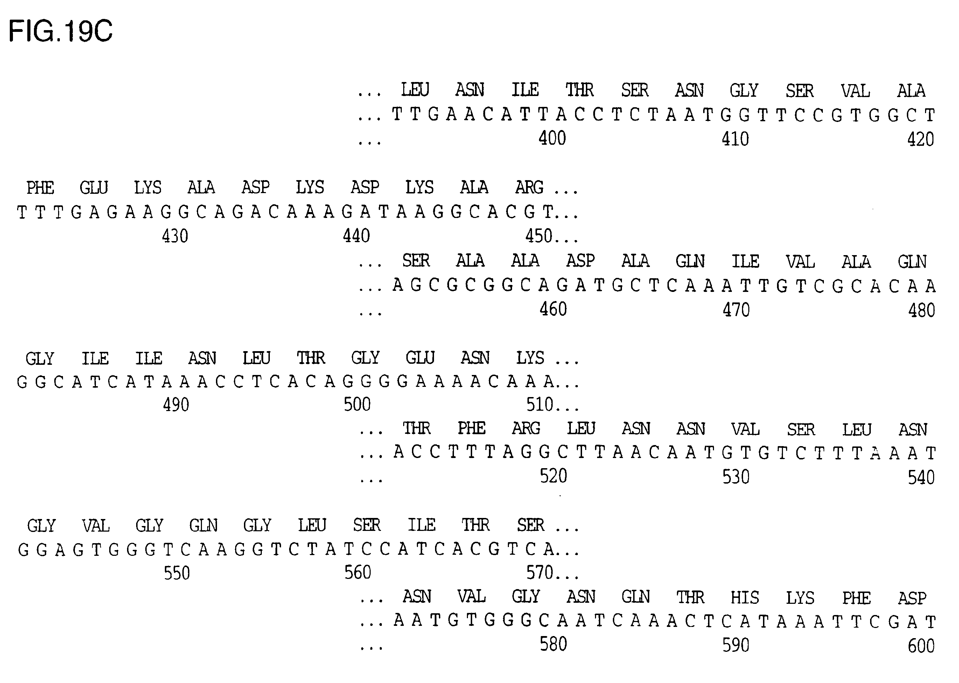

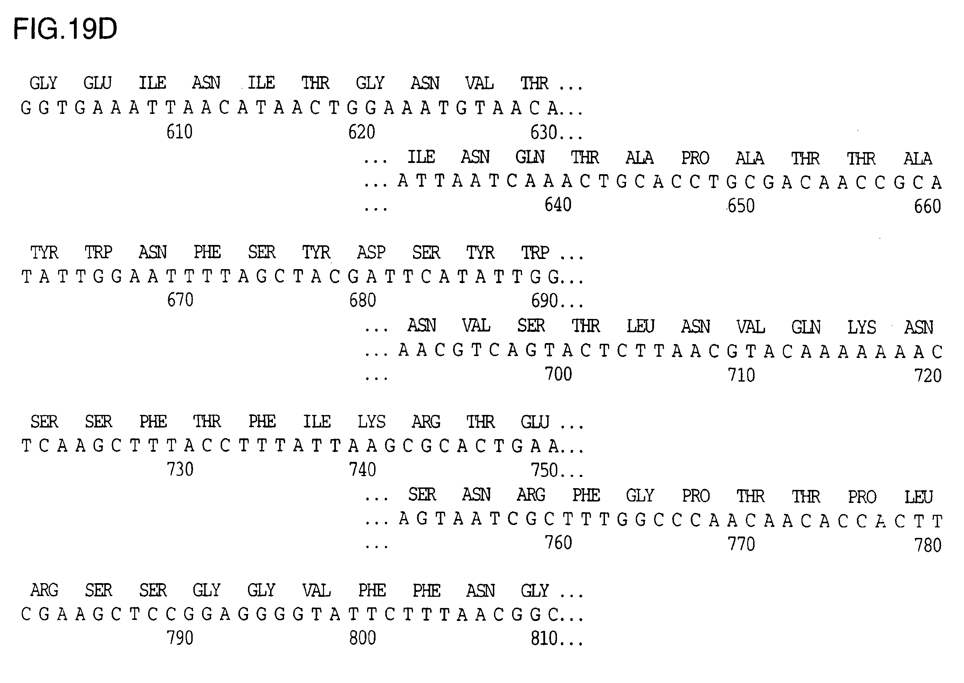

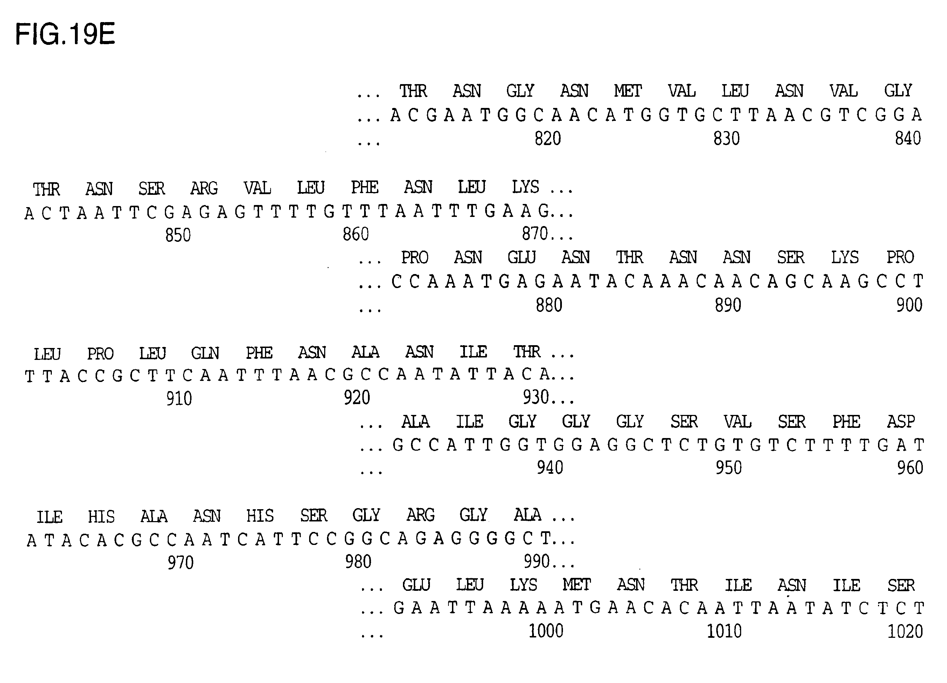

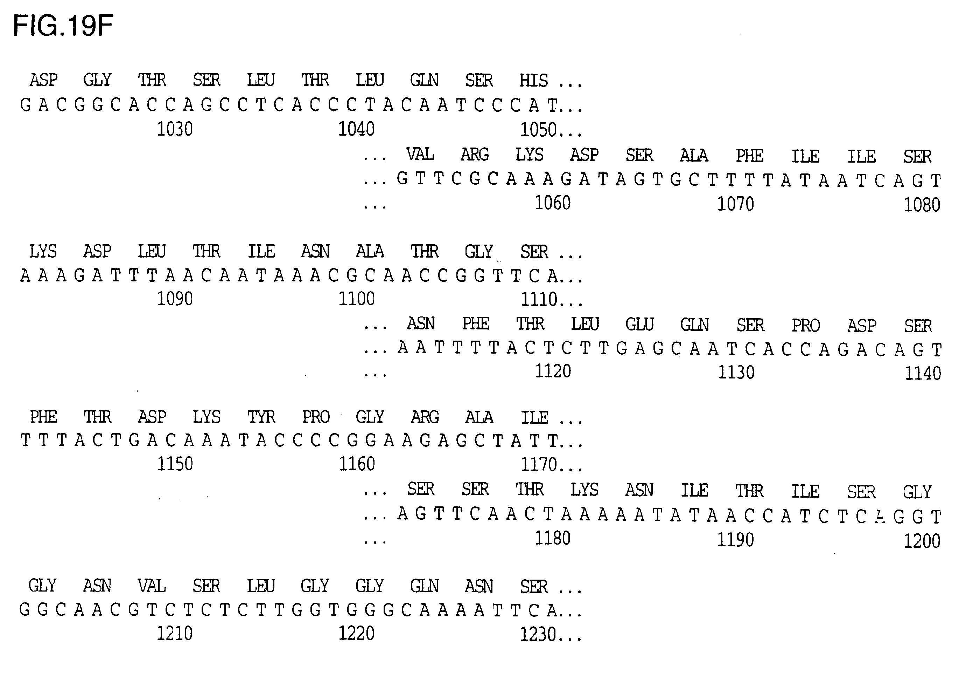

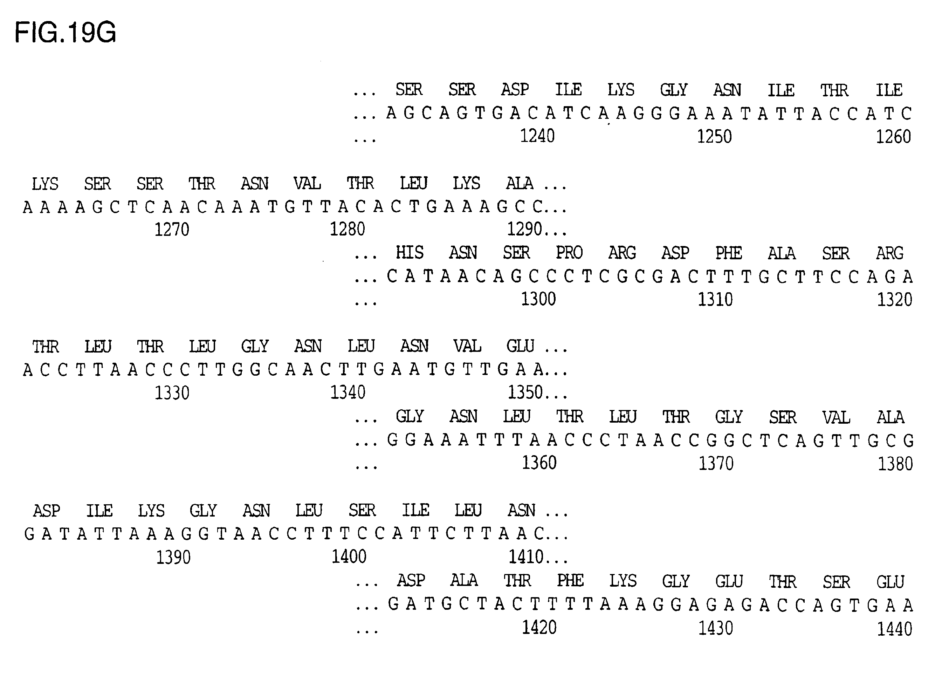

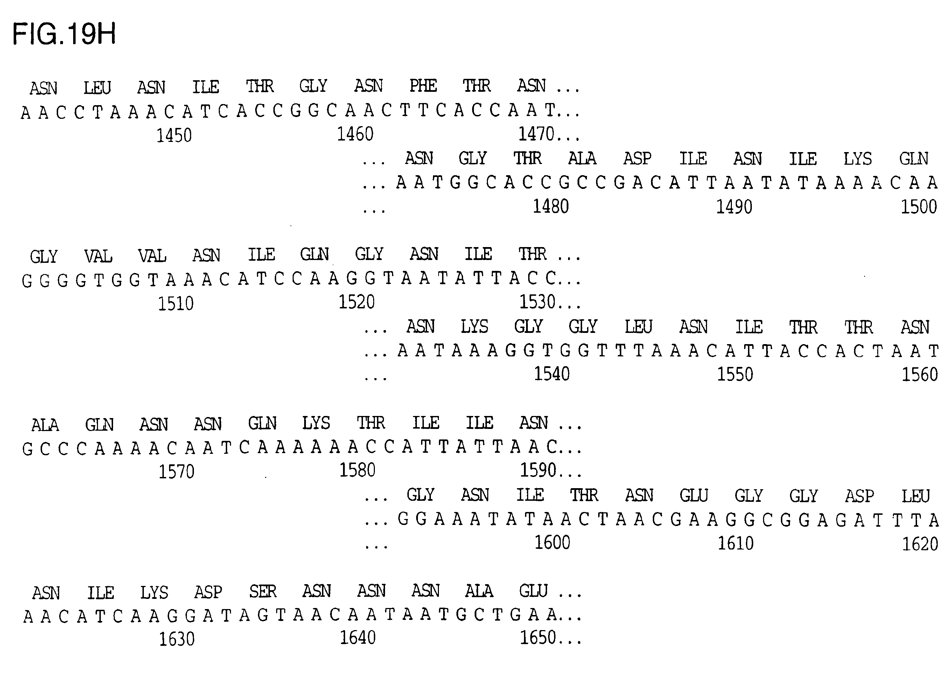

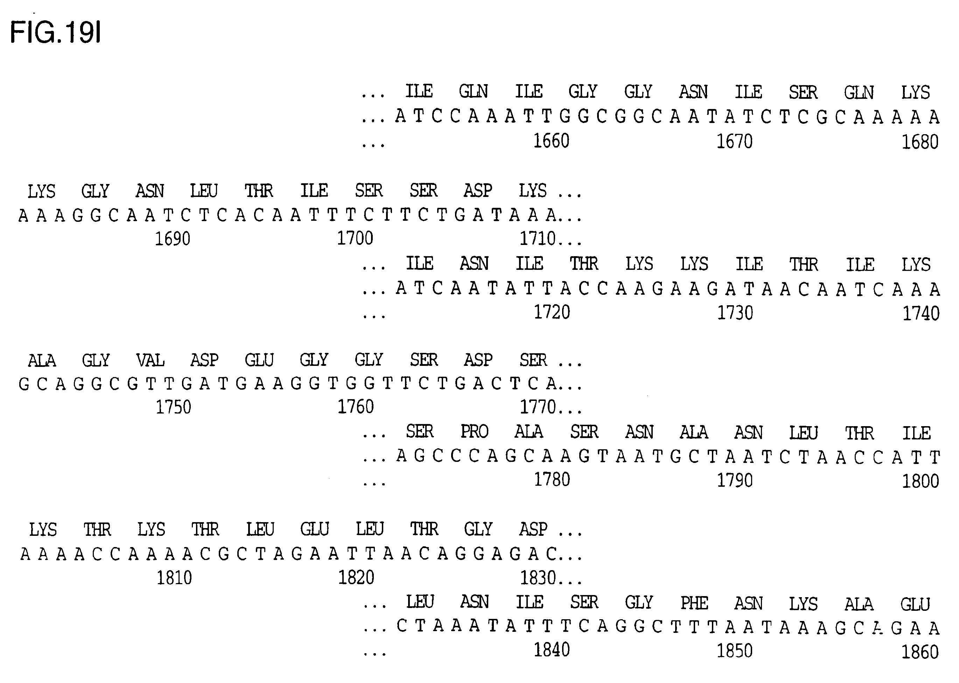

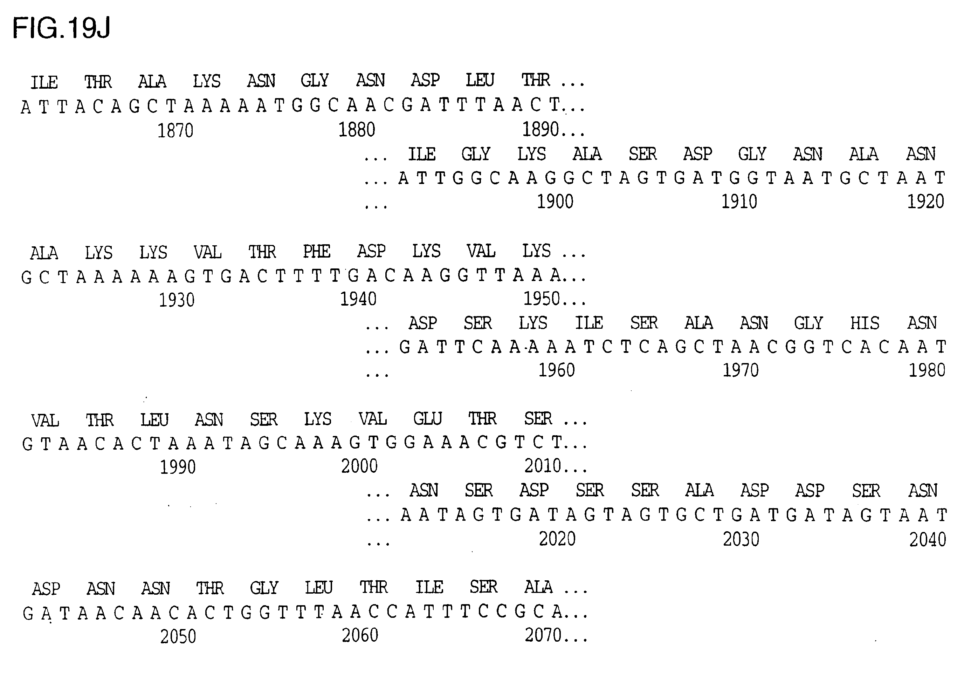

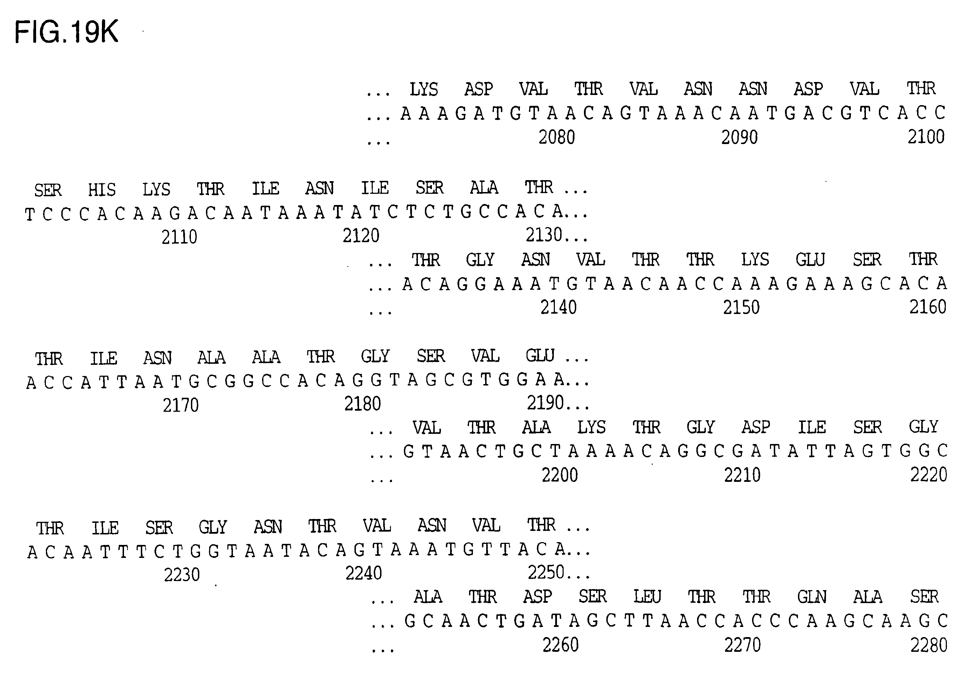

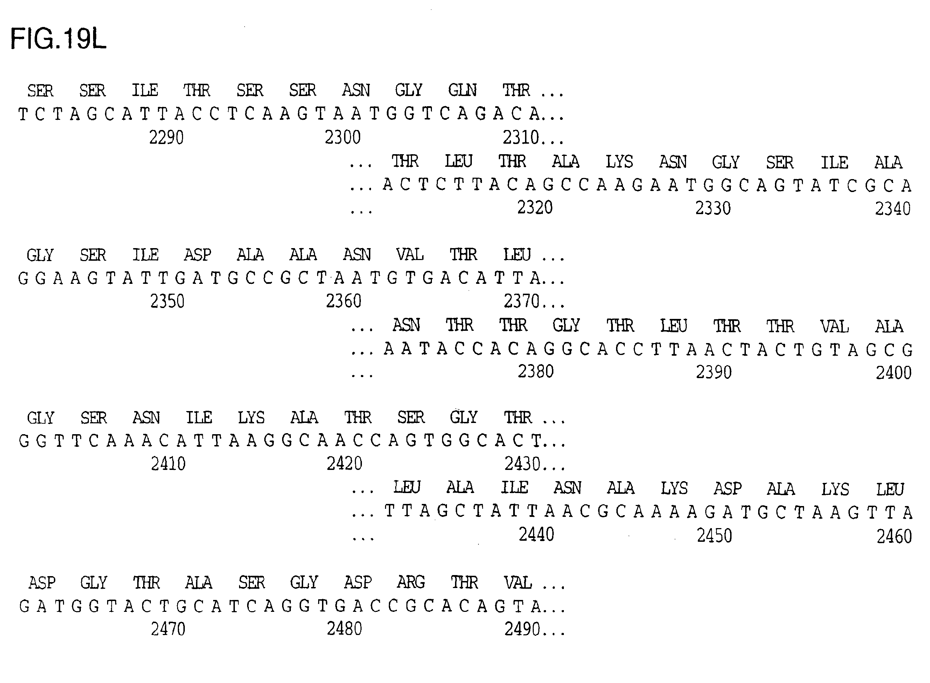

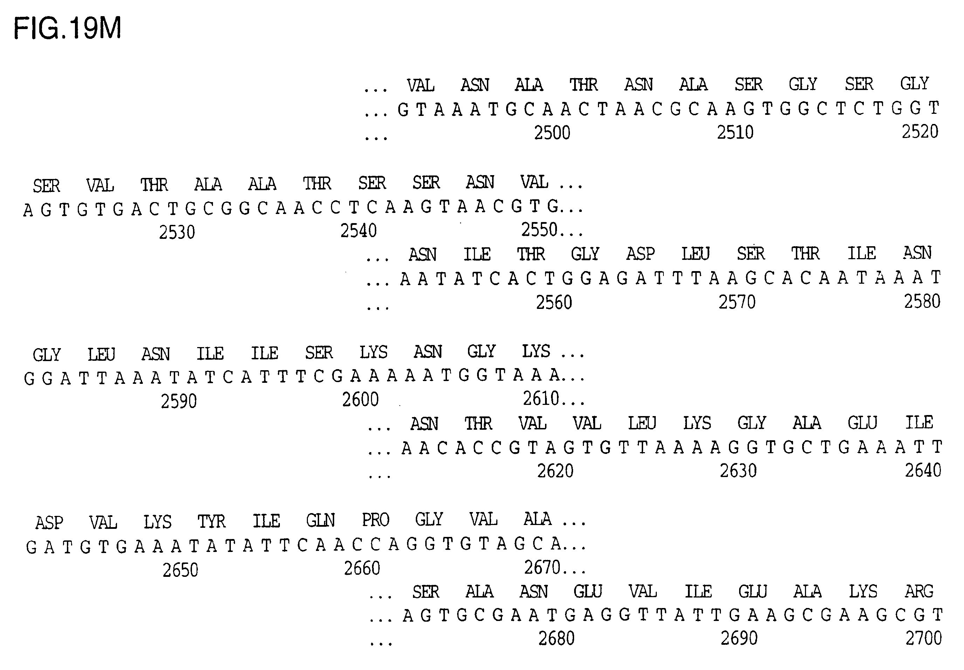

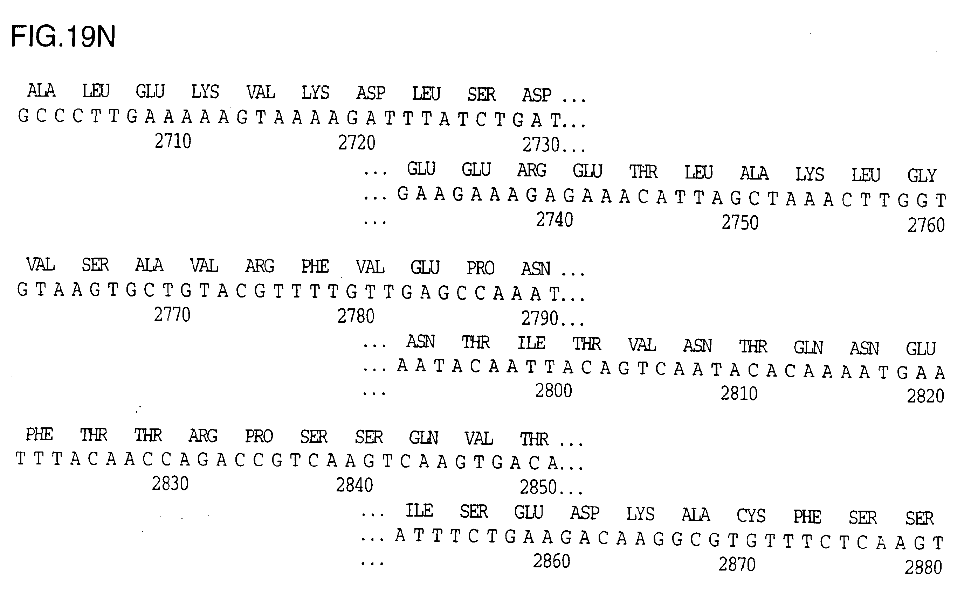

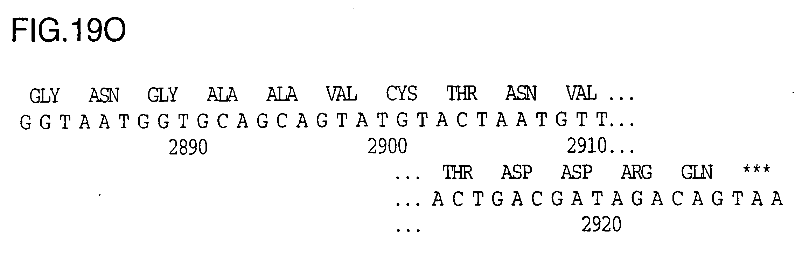

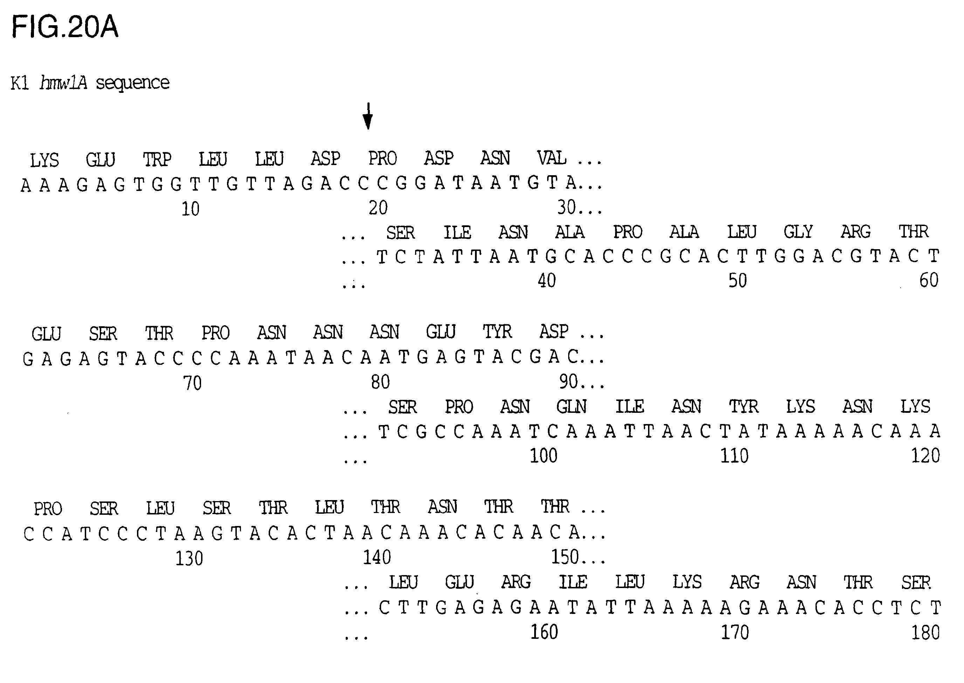

The nucleotide (SEQ ID NO: 25) and deduced amino acid (SEQ ID NO: 26) sequences of the *hmw1A* gene from strain Joyc are shown in Figure 18. The predicted mature HMW1A protein from strain Joyc (encoding sequence SEQ ID NO: 27, amino acid sequence SEQ ID NO: 28) has a molecular weight of 125.9 kDa and a pI of 8.21. There are no RGD motifs found in Joyc HMW1A. The nucleotide (SEQ ID NO: 29) and deduced amino acid (SEQ ID NO: 30) sequences of the *hmw2A* gene from strain Joyc are shown in Figure 19. The predicted mature HMW2A protein from strain Joyc (encoding sequence SEQ ID NO: 31, amino acid sequence SEQ ID NO: 32) has a molecular weight of 100.9 kDa and a pI of 6.91. There are no RGD motifs found in Joyc HMW2A.

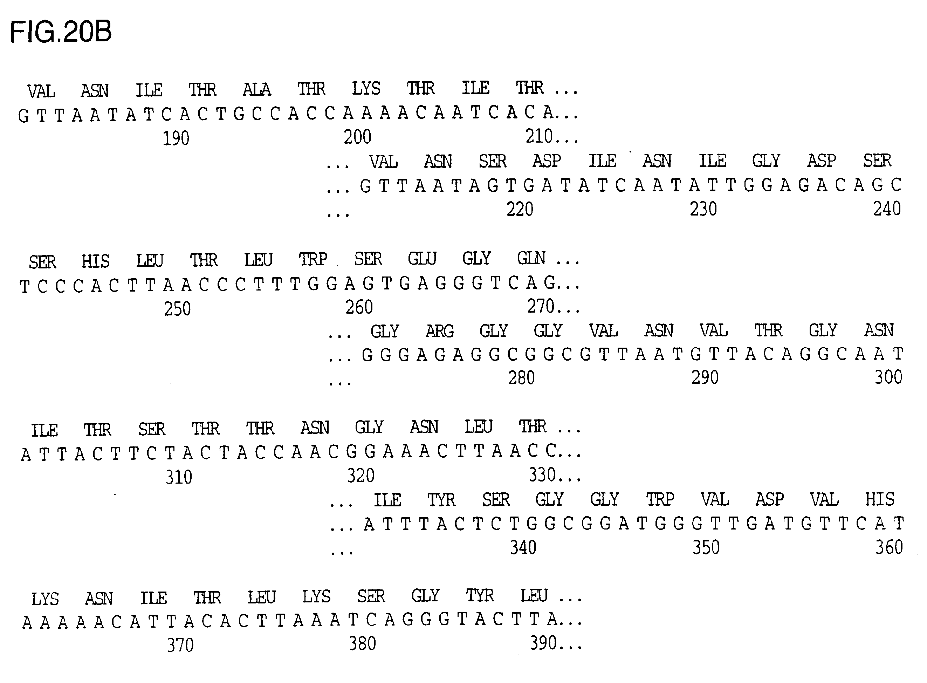

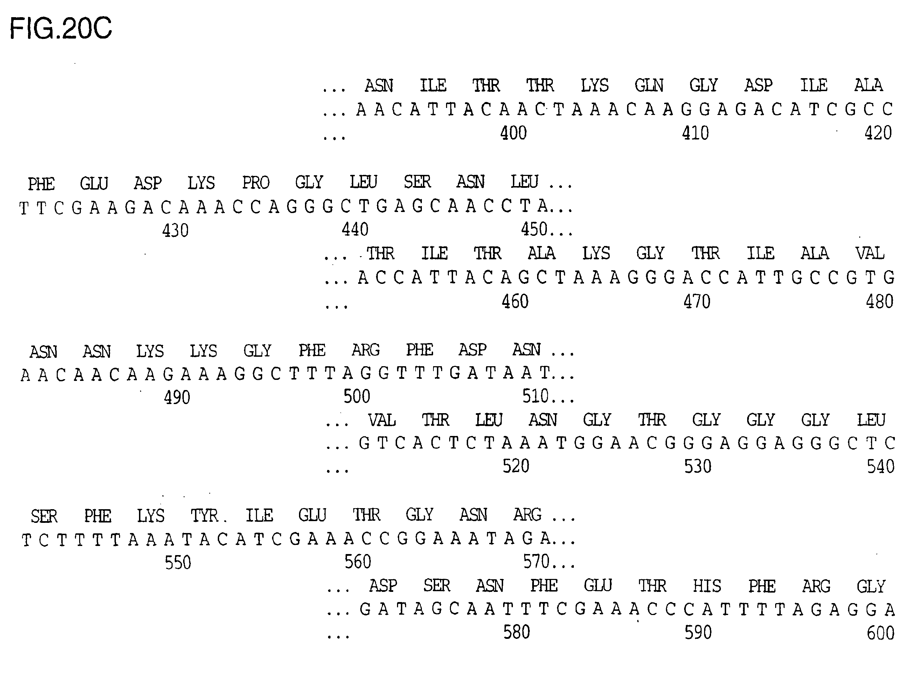

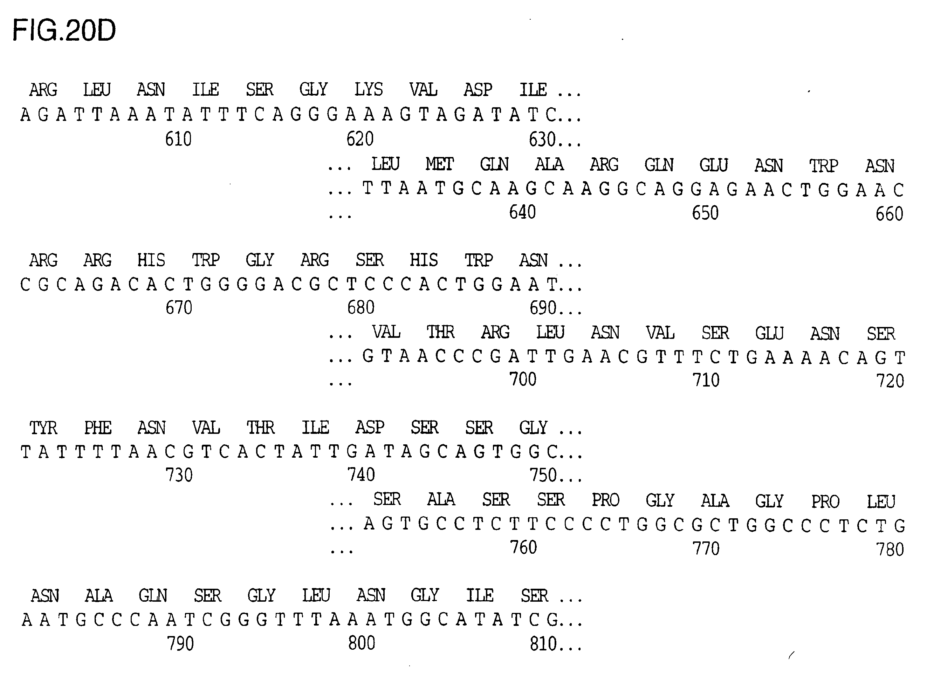

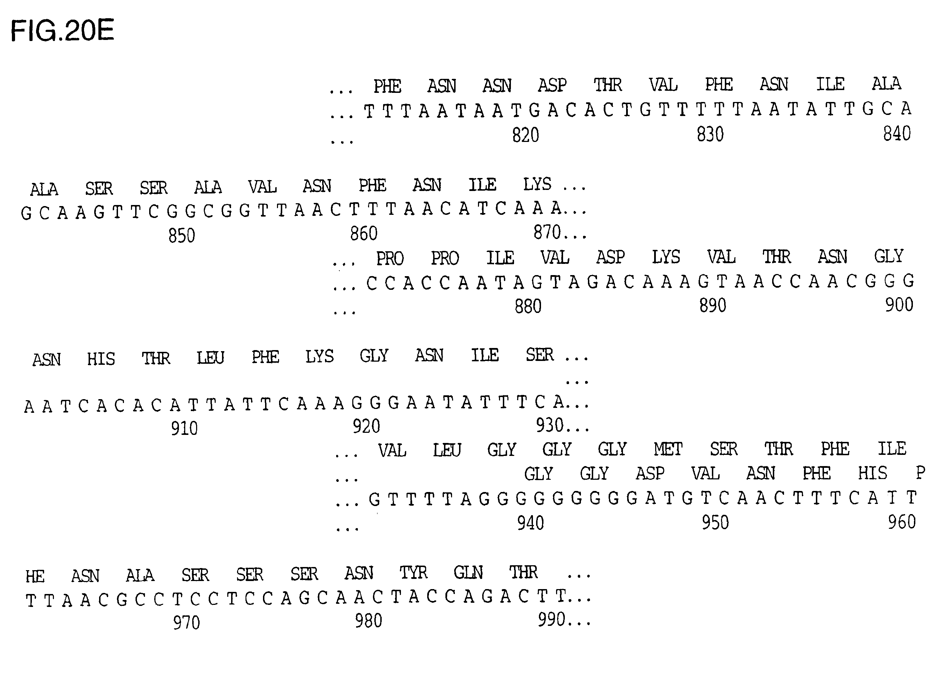

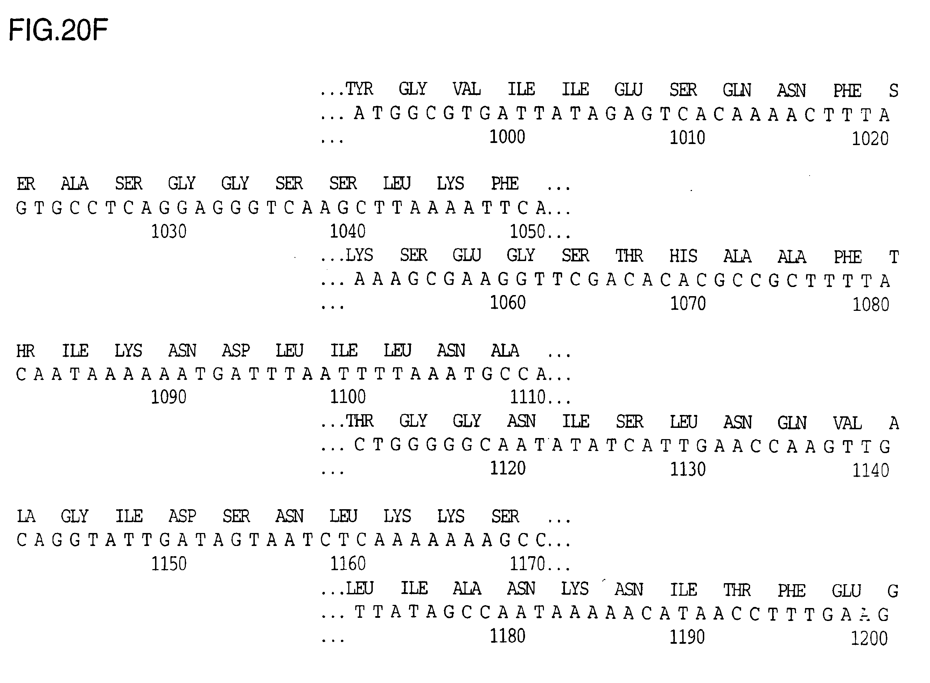

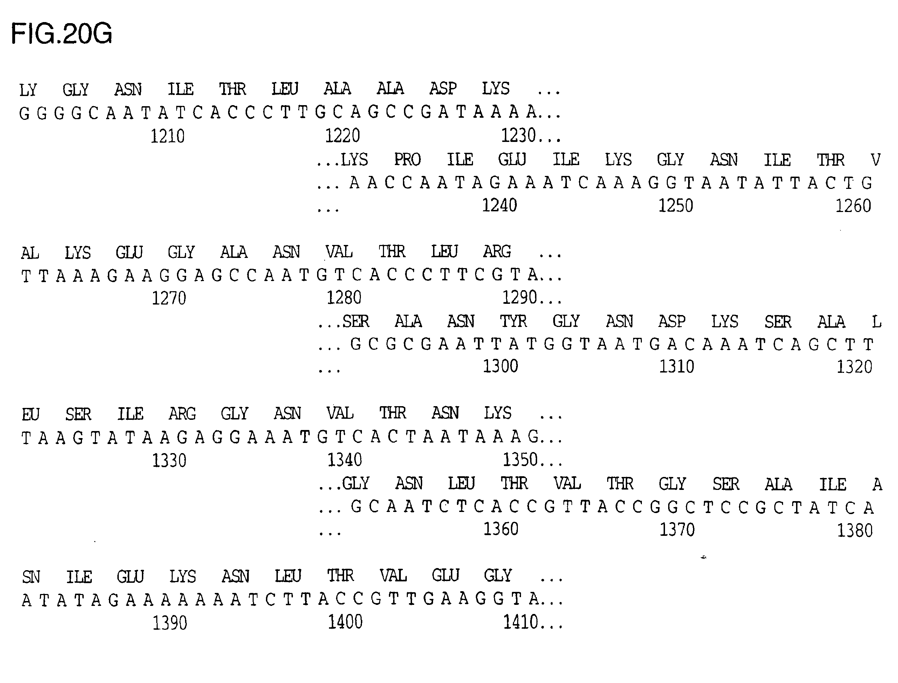

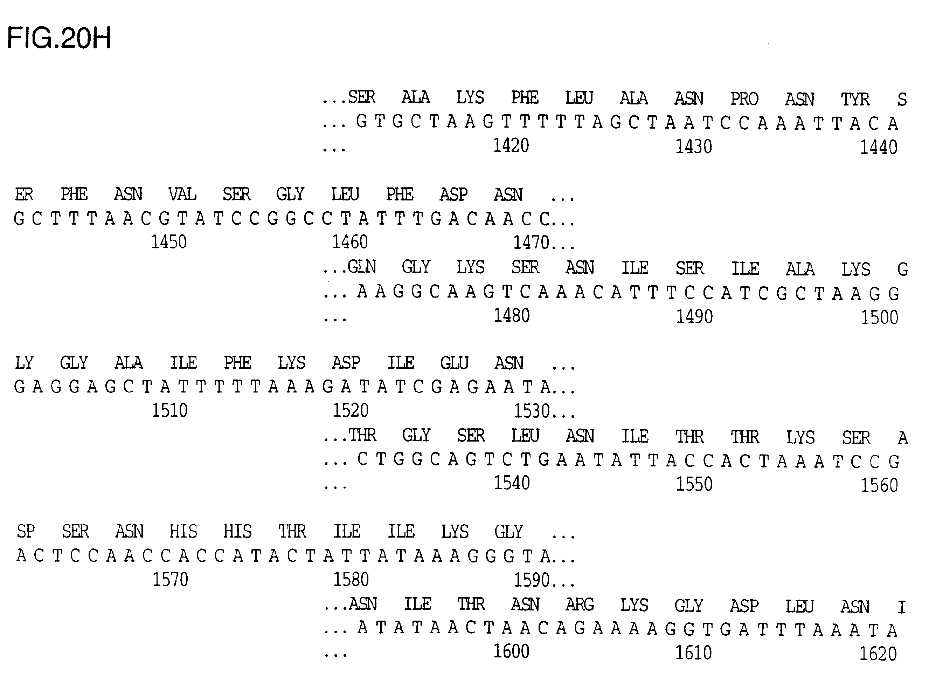

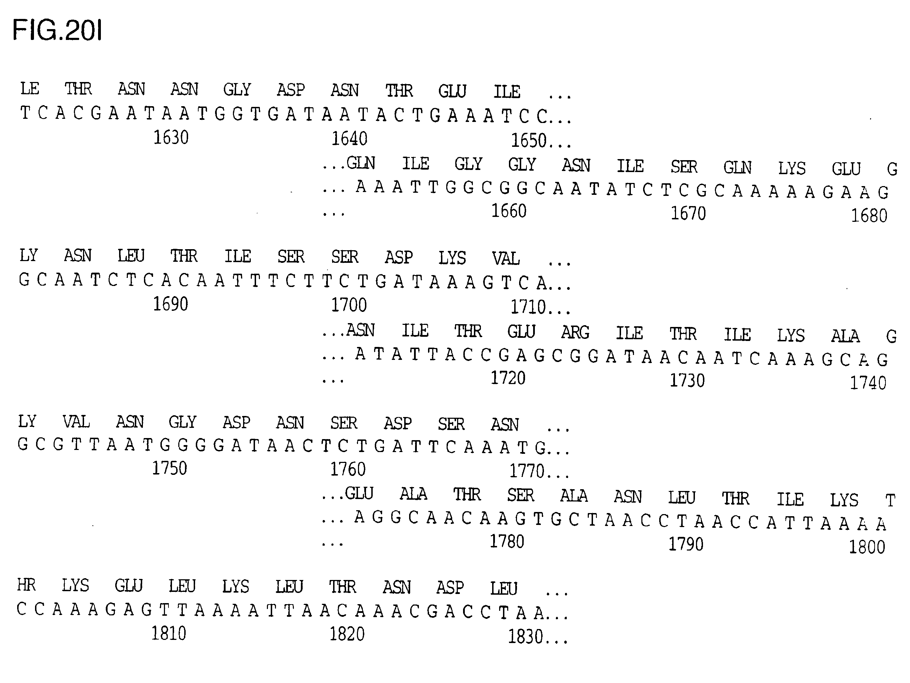

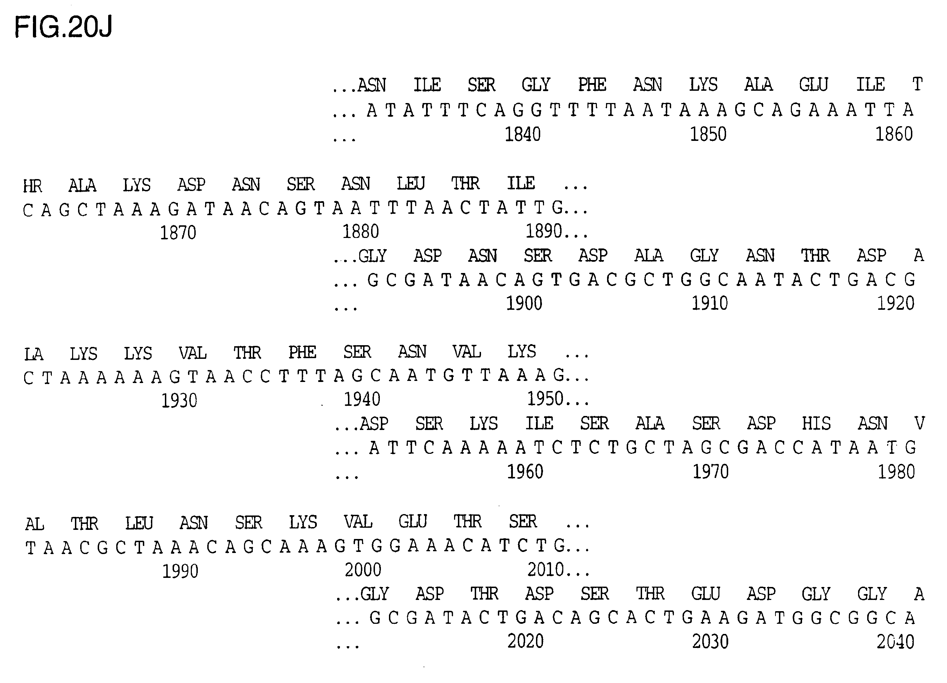

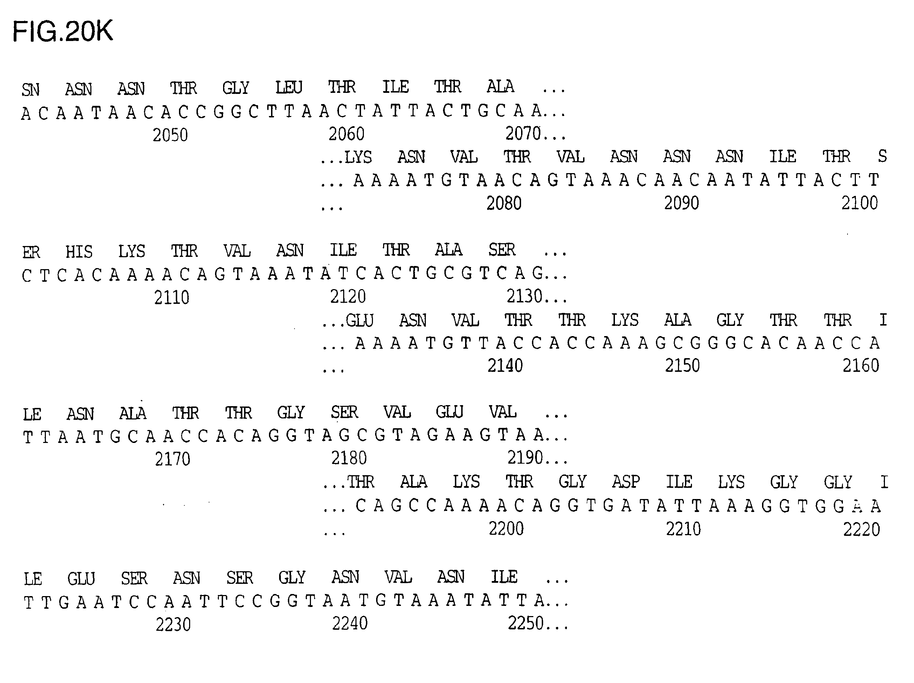

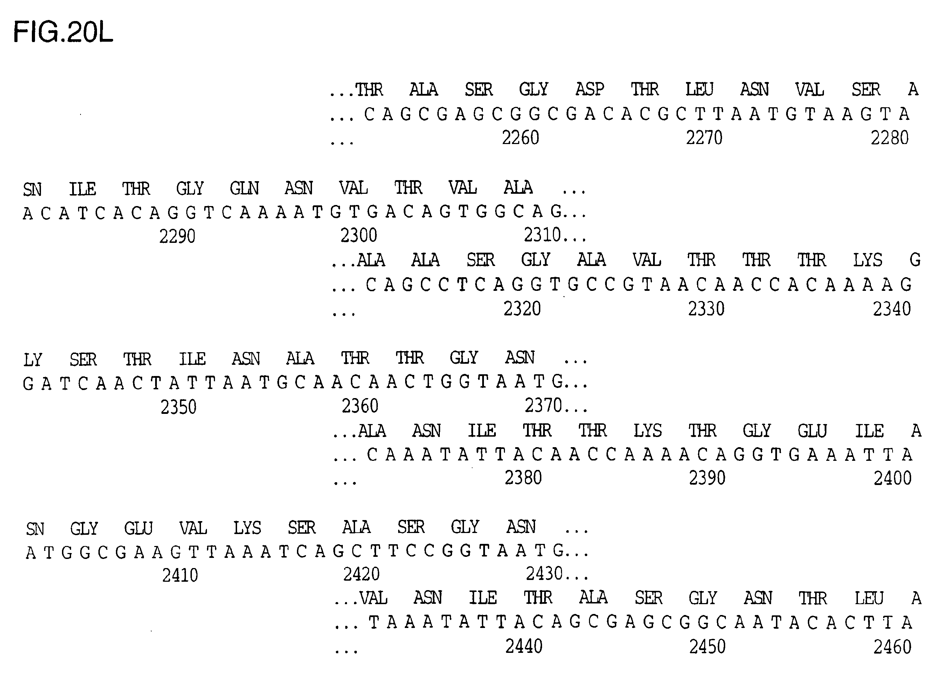

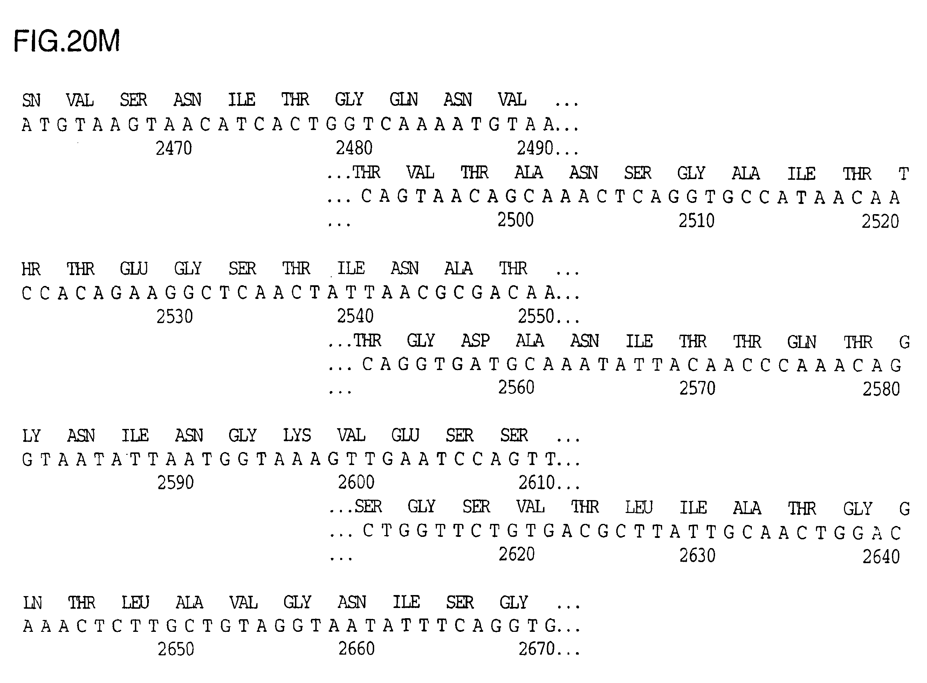

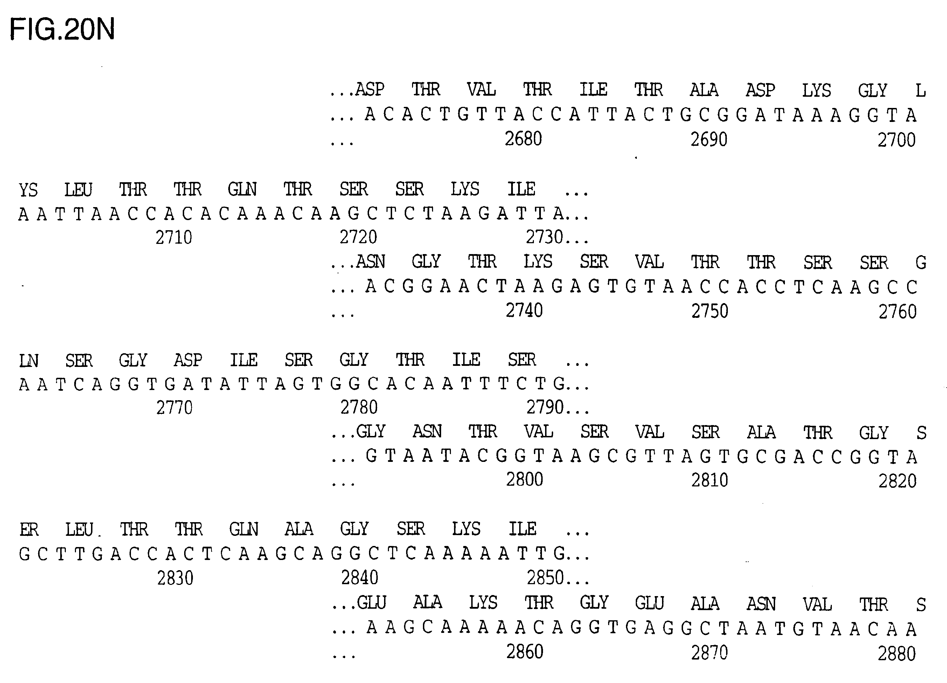

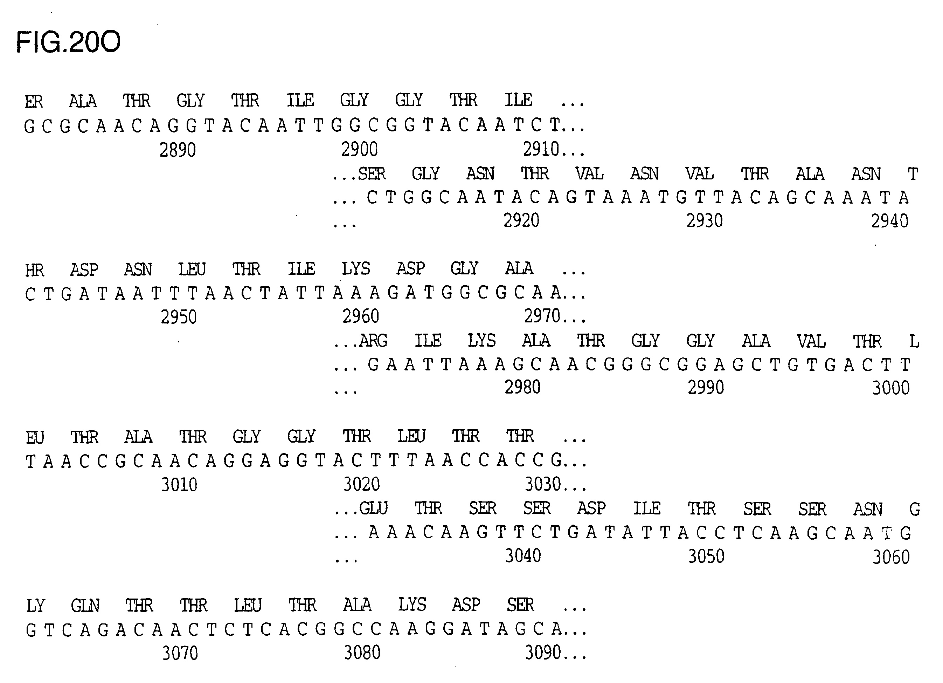

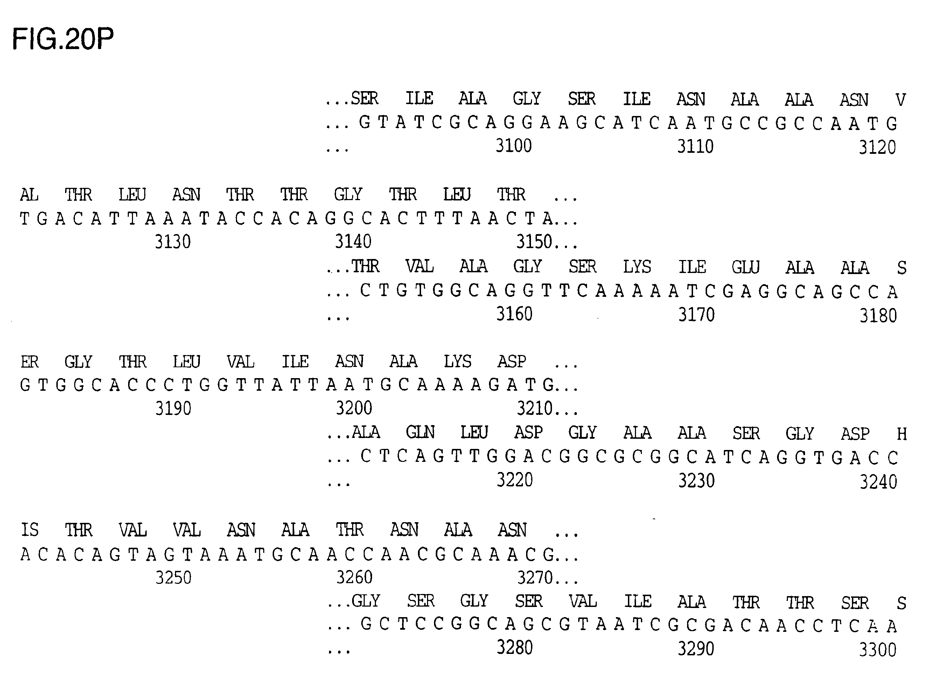

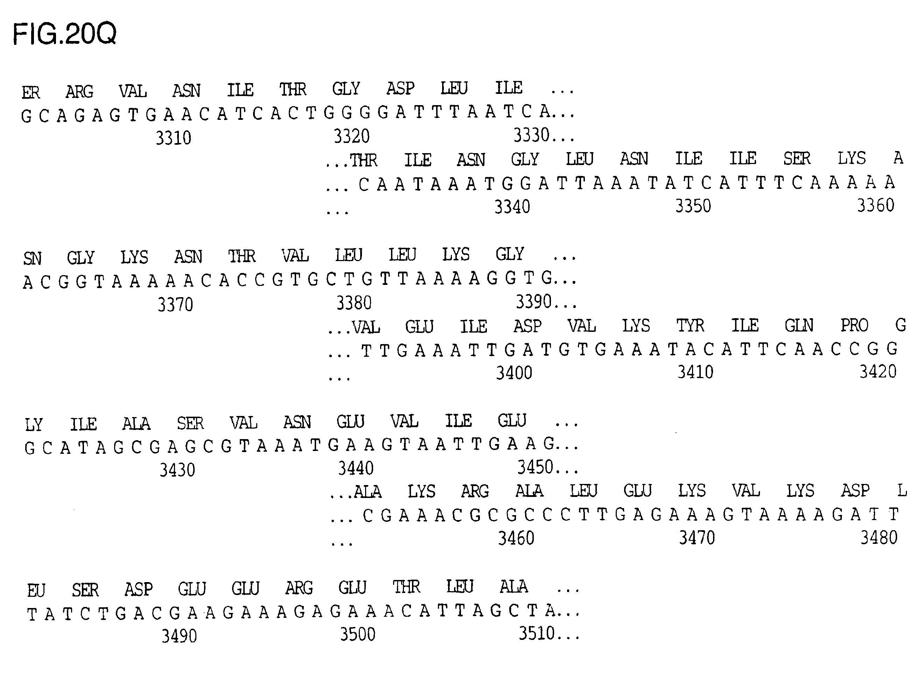

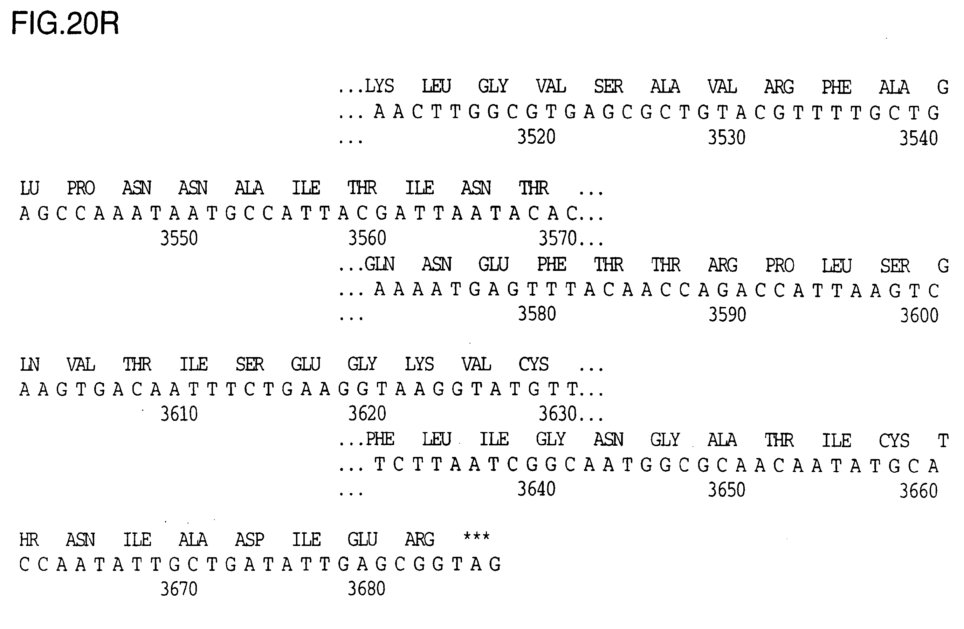

The nucleotide (SEQ ID NO: 33) and deduced amino acid (SEQ ID NOS: 34, 35) sequences of the defective *hmw1A* gene from strain K1 are shown in Figure 20. Although there is a complete *hmw1A* gene in strain K1, there is a frame-shift immediately following a poly G tract, that results in early termination of the HMW1A protein after 326 amino acids.

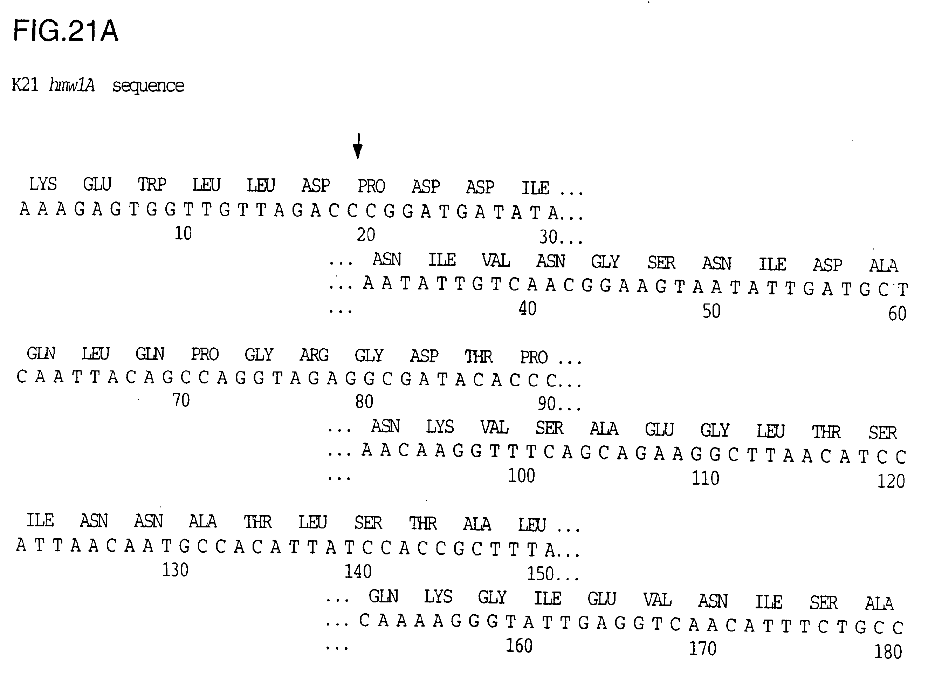

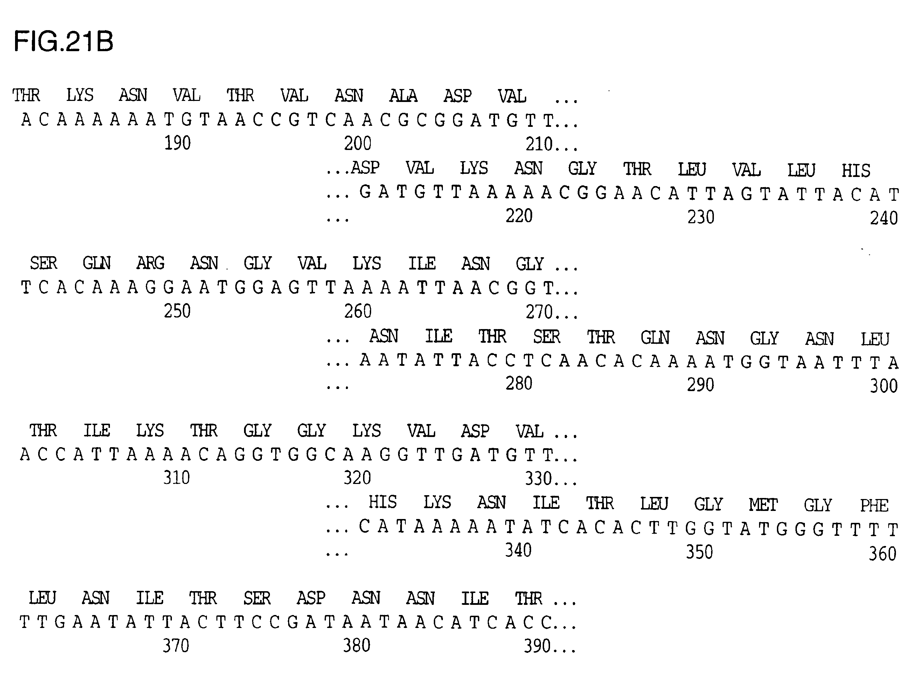

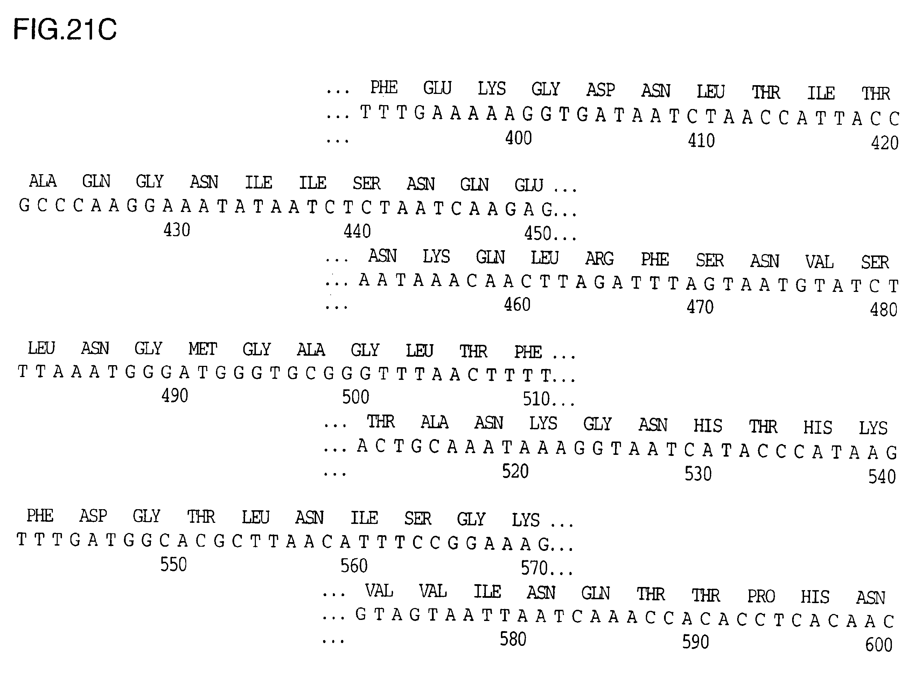

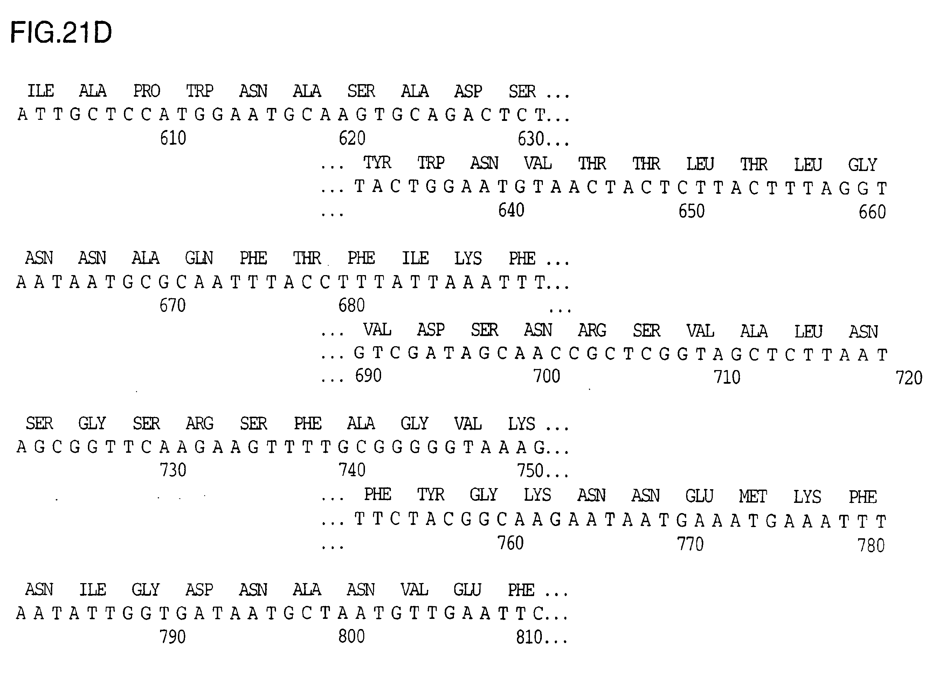

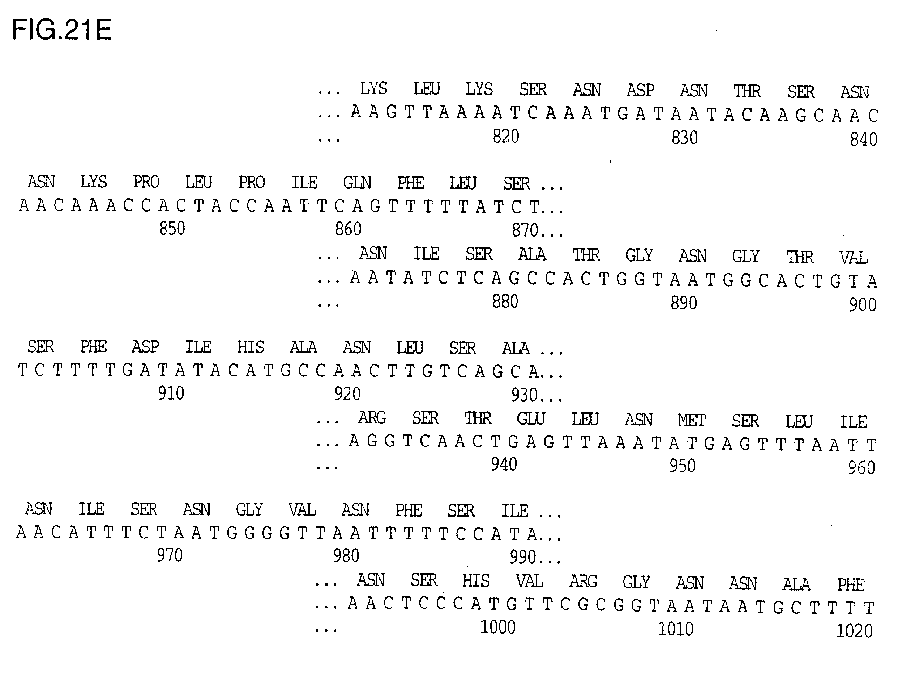

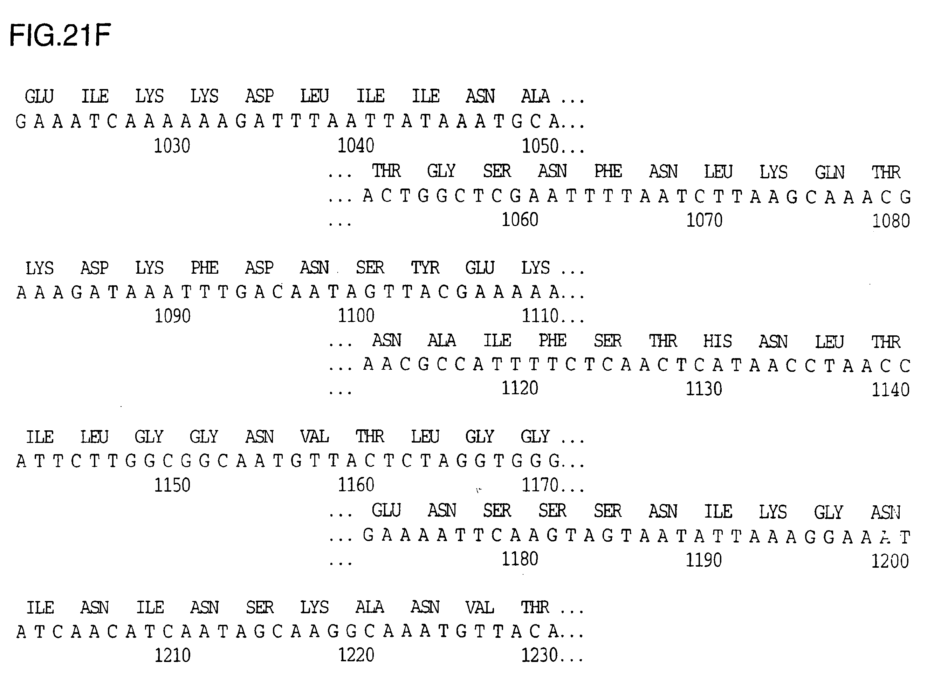

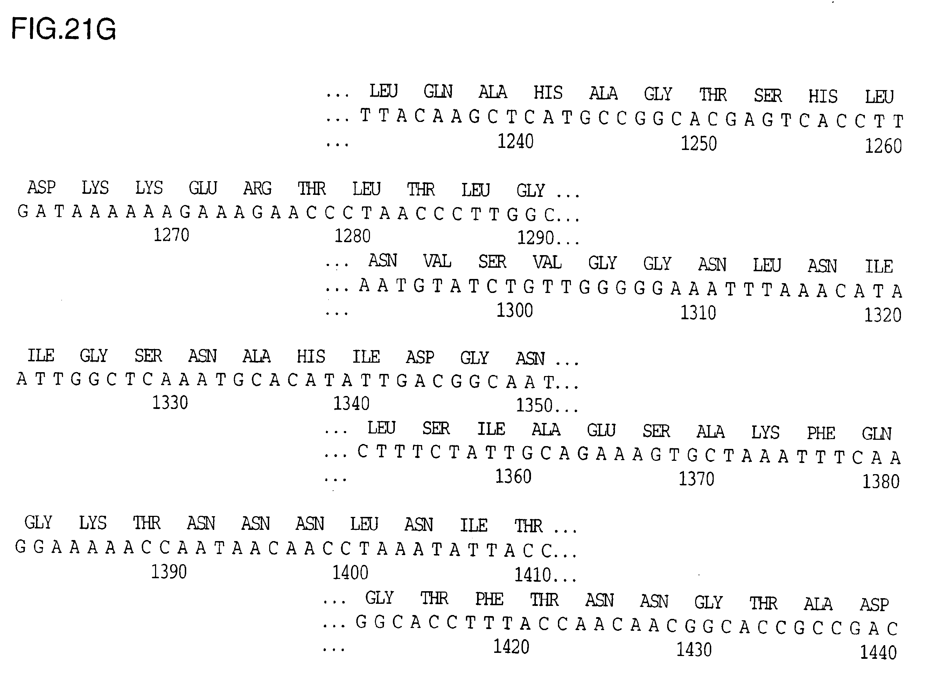

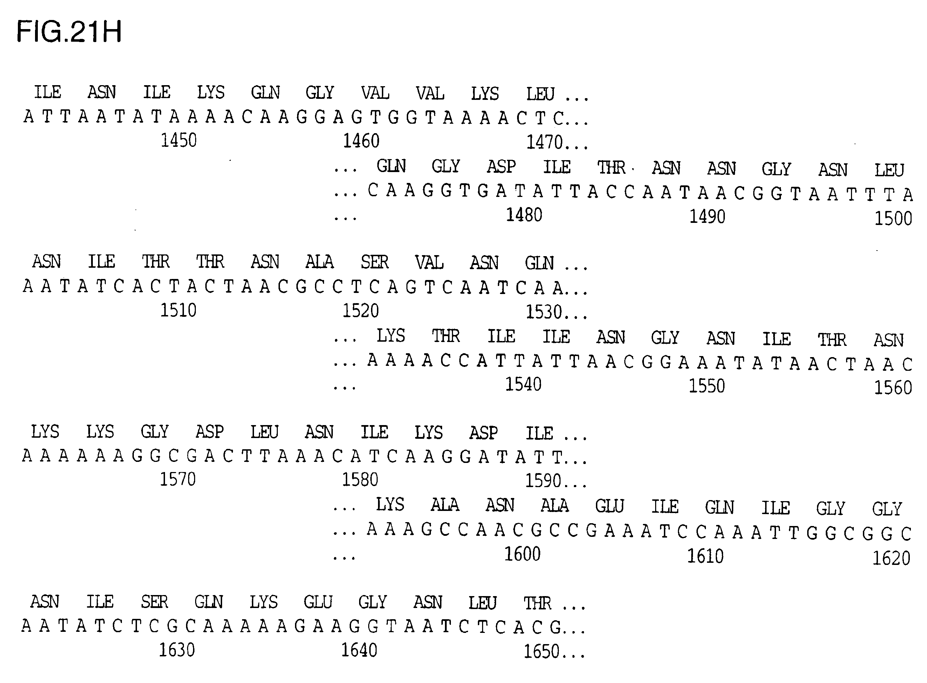

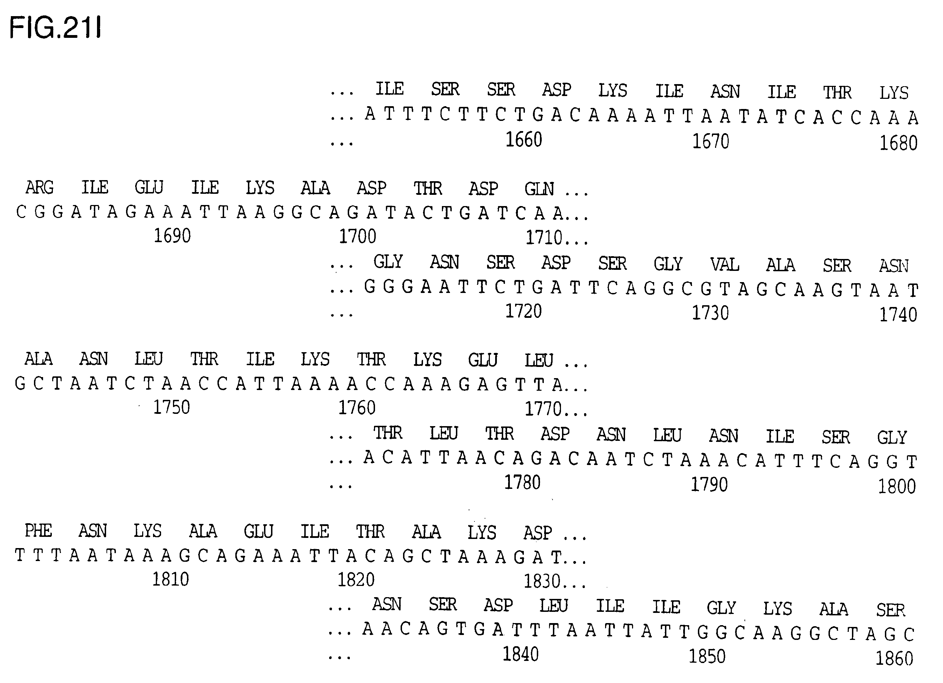

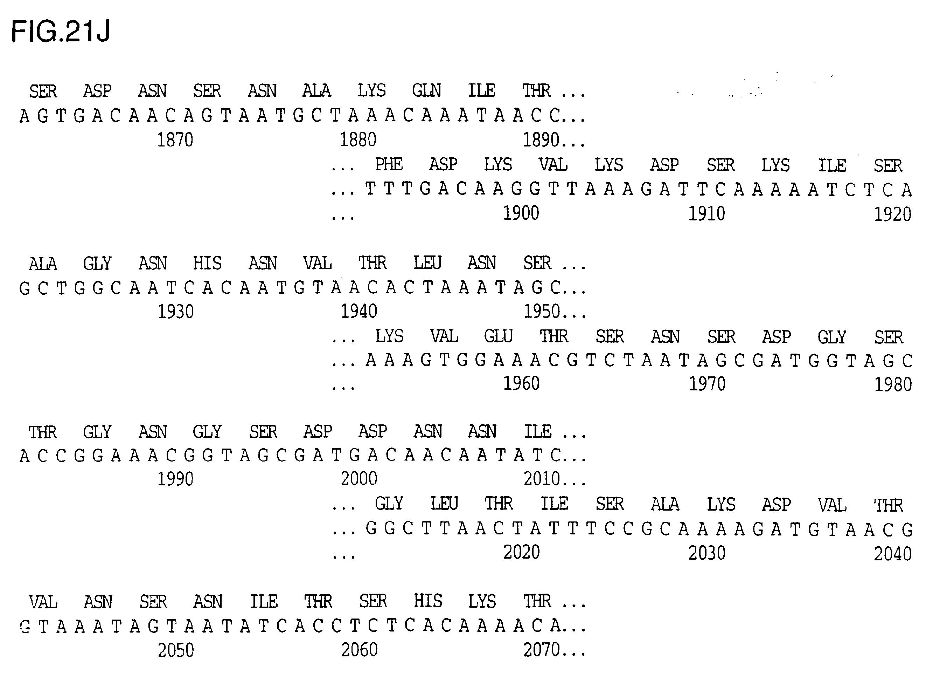

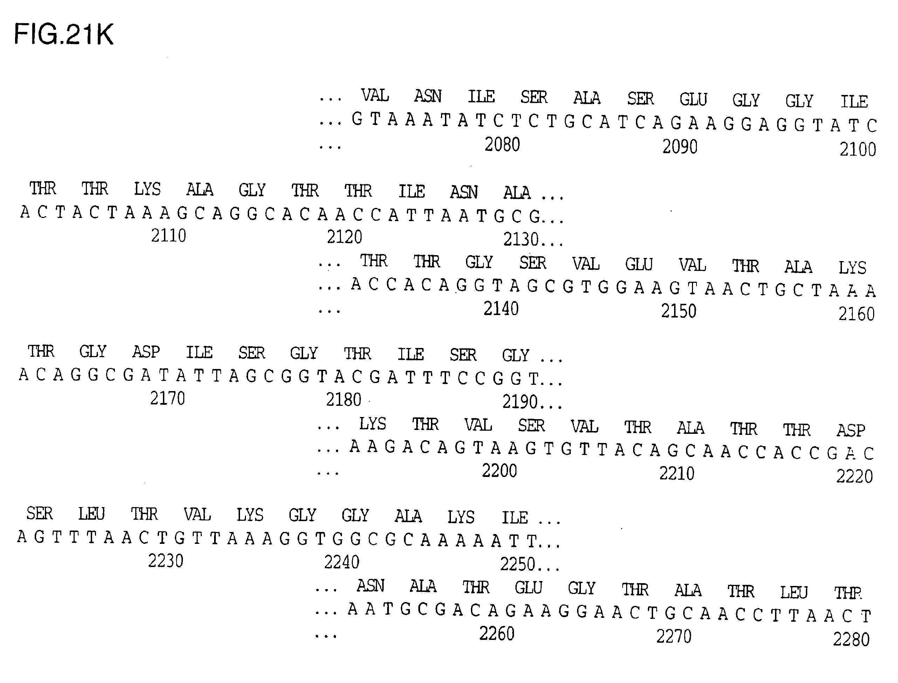

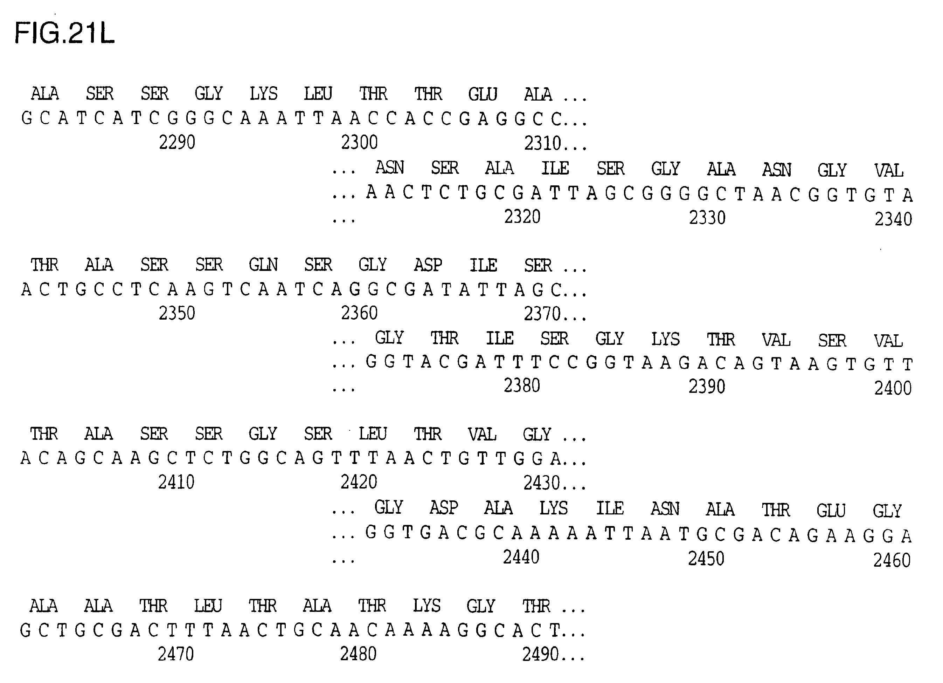

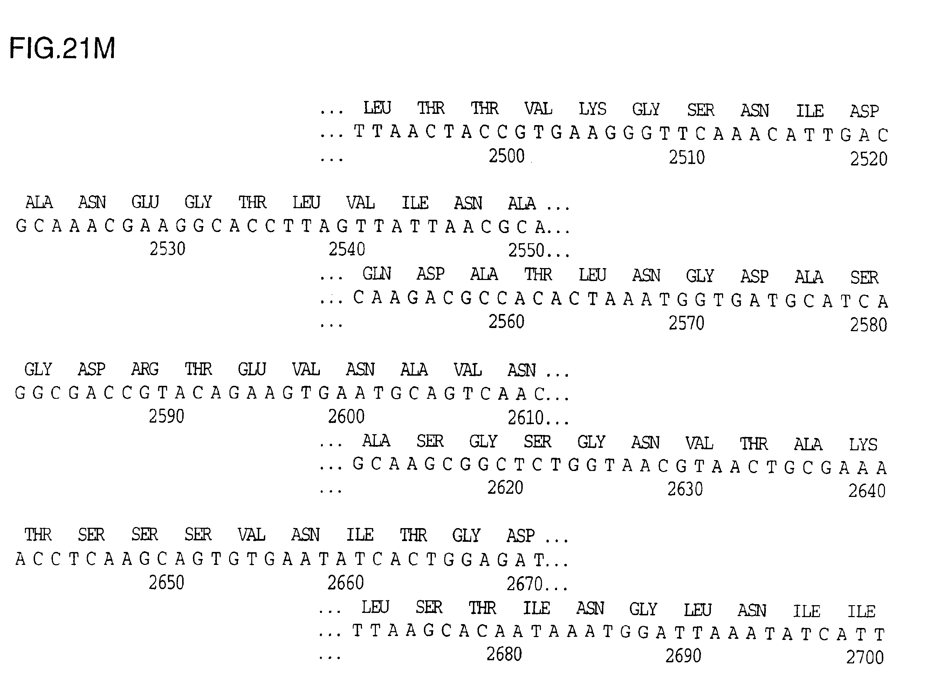

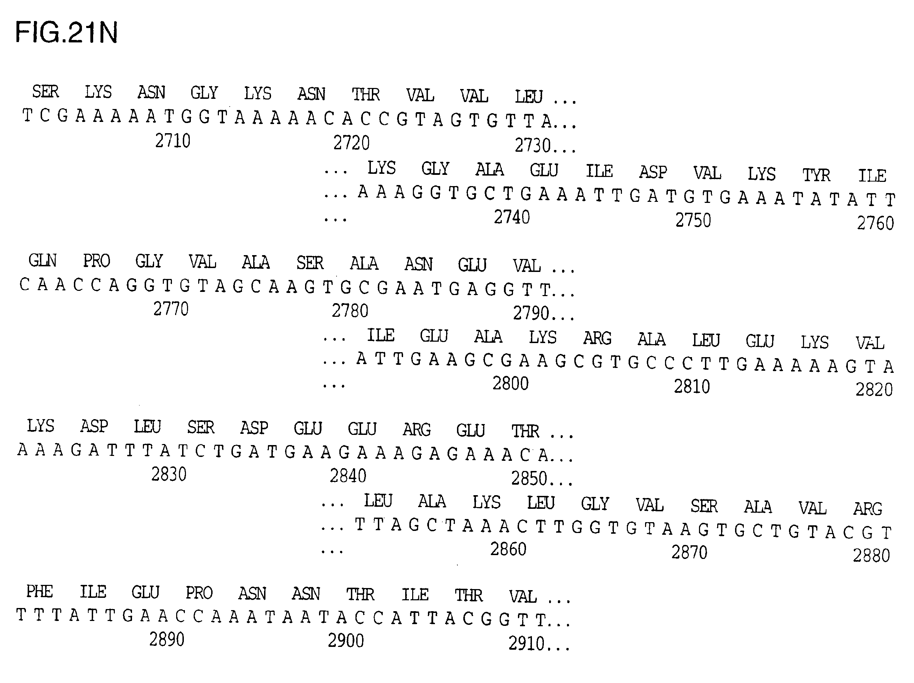

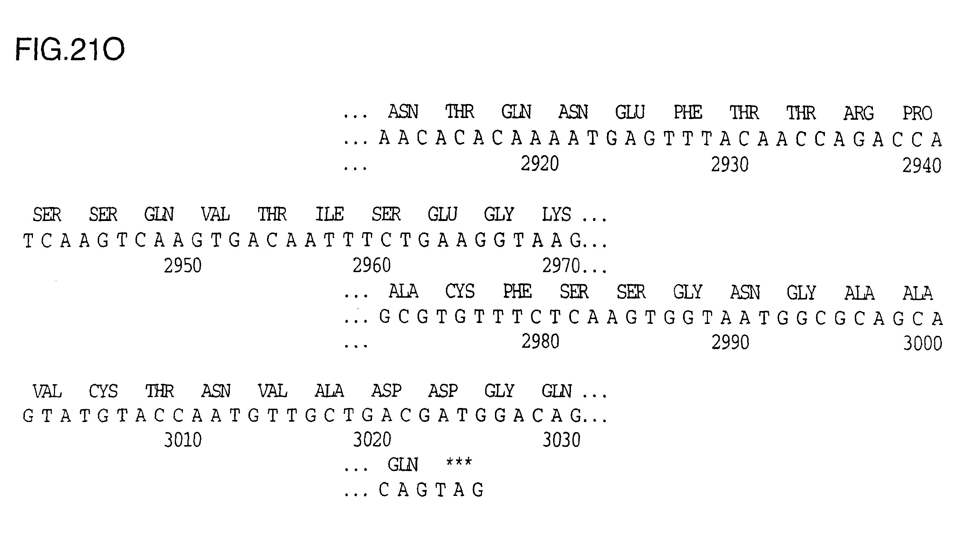

The nucleotide (SEQ ID NO: 38) and deduced amino acid (SEQ ID NO: 39) sequences of the *hmw1A* gene from strain K21 are shown in Figure 21. The predicted mature HMW1A protein from strain K21 (encoding sequence SEQ ID NO: 40, amino acid sequence SEQ ID NO: 41) has a molecular weight of 104.4 kDa and a pI of 8.71. There is a single RGD motif located at residues 20 to 22 in K21 HMW1A.

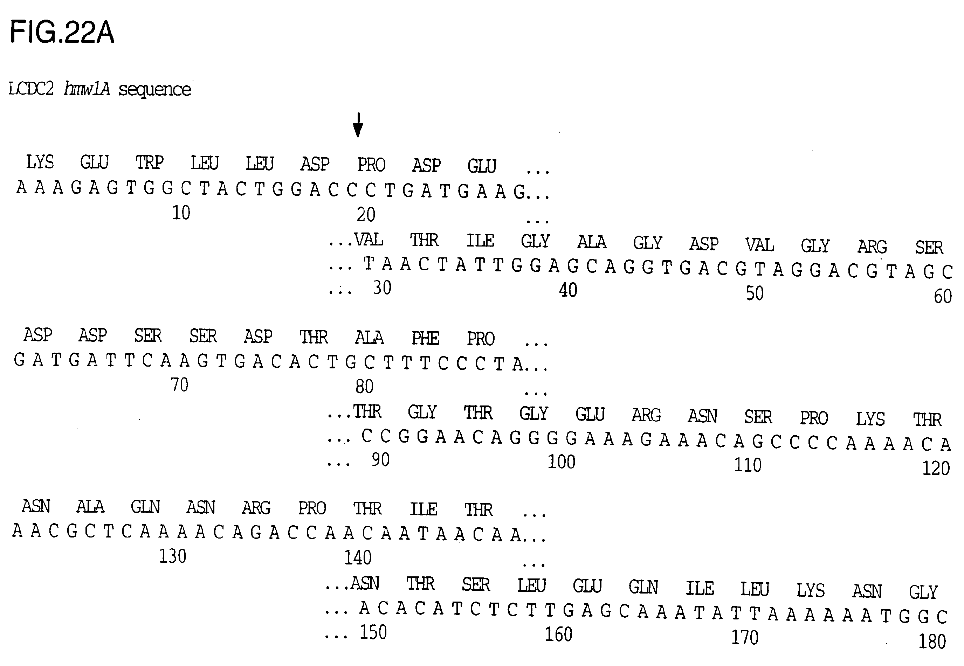

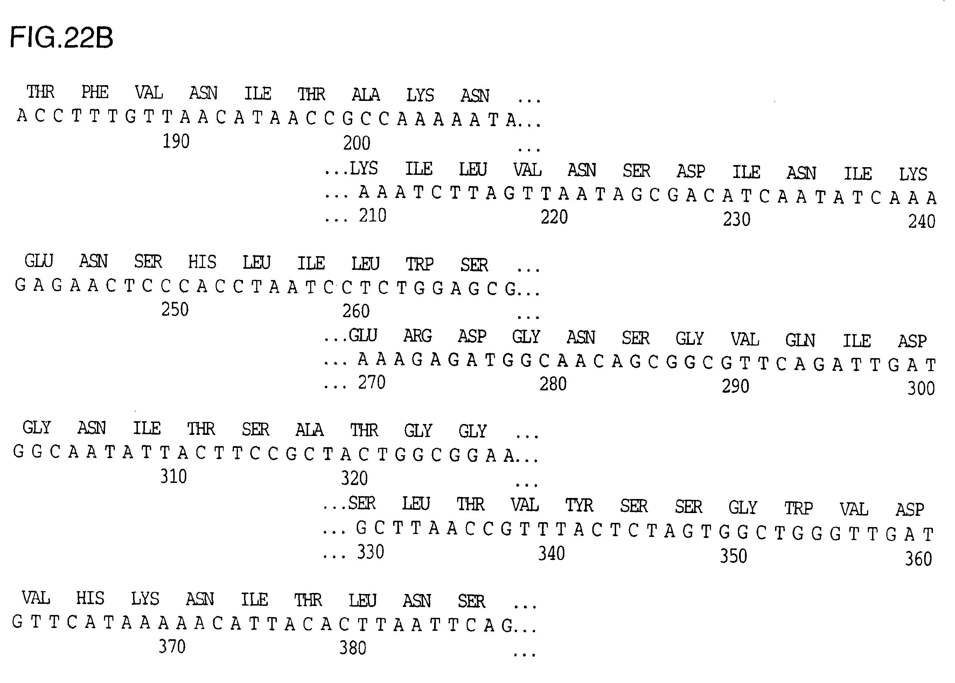

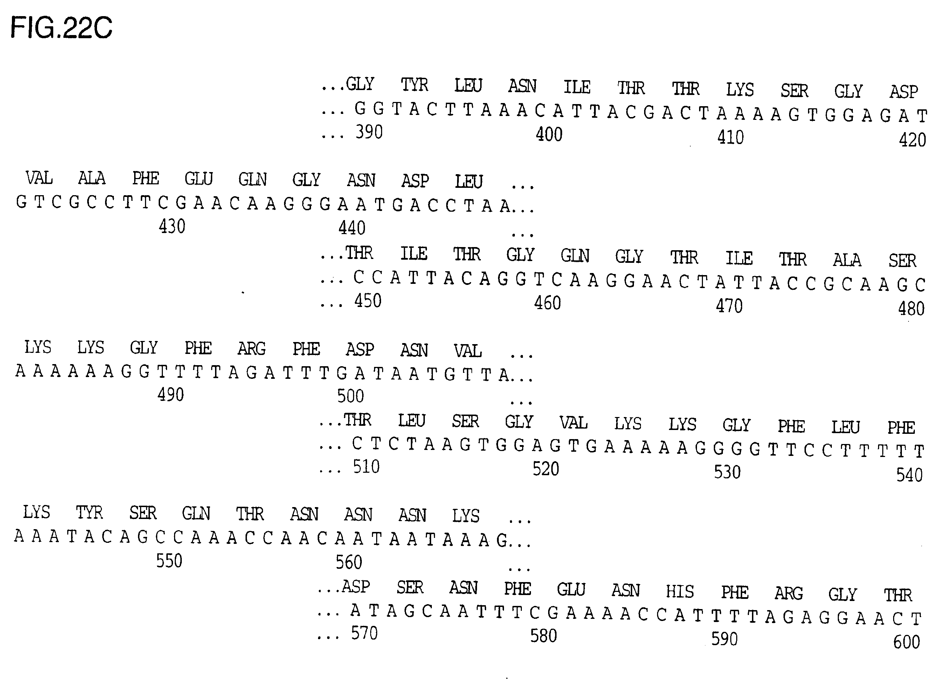

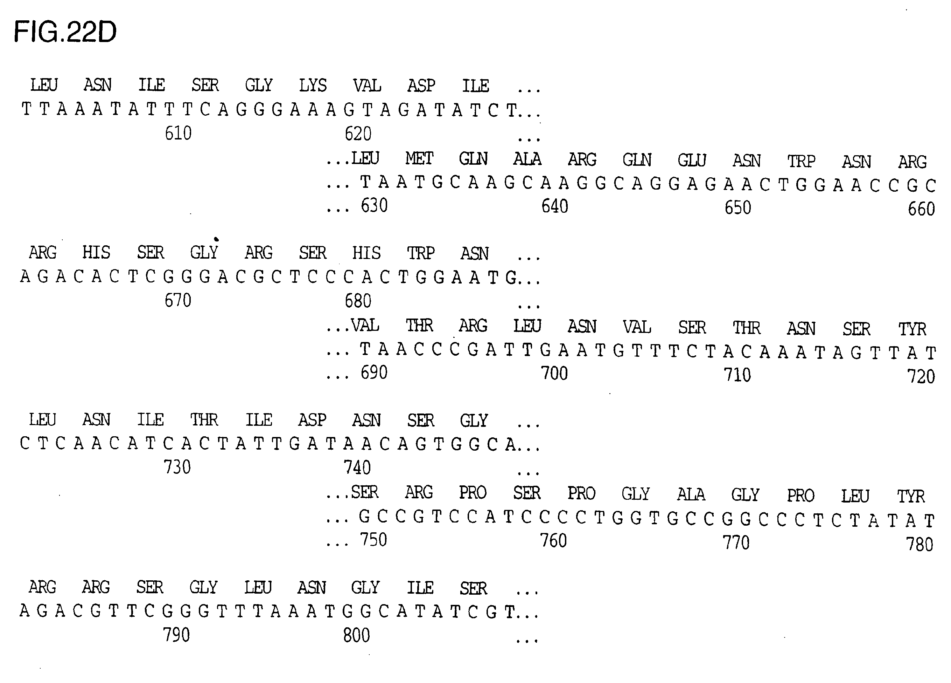

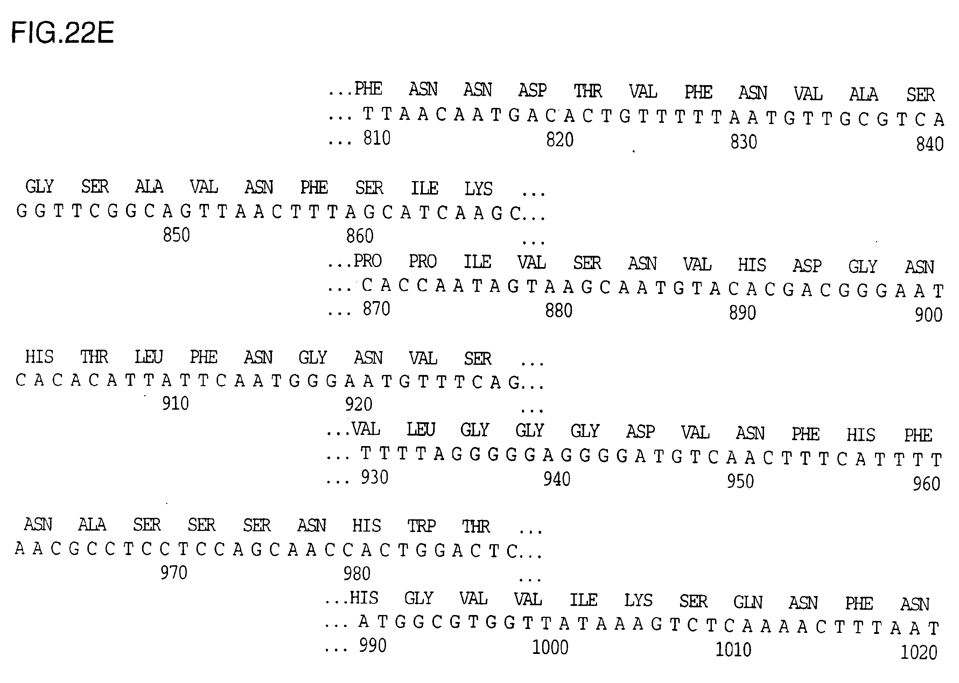

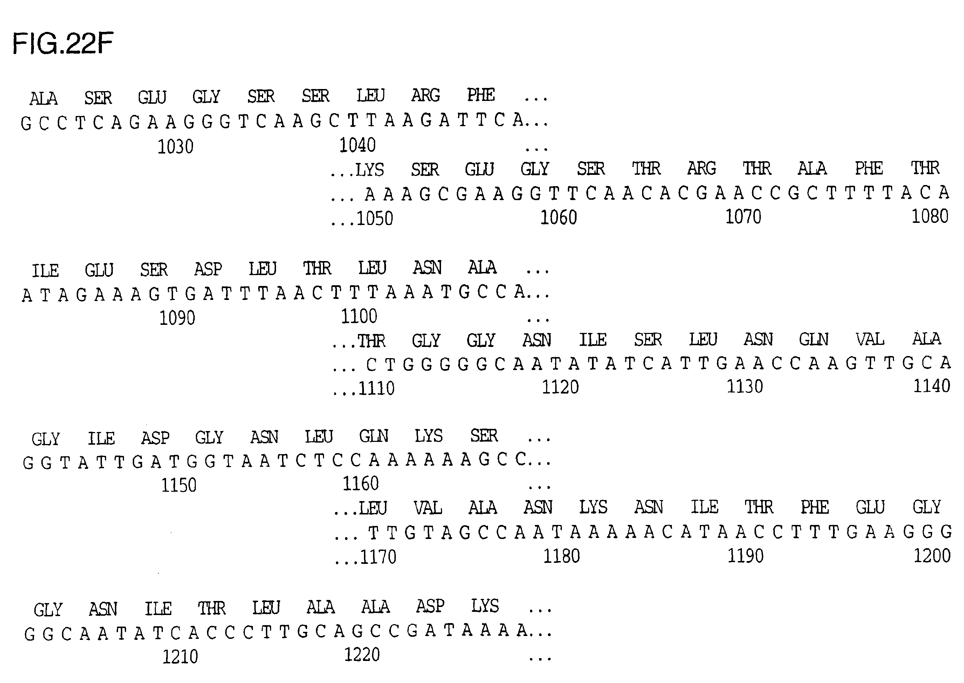

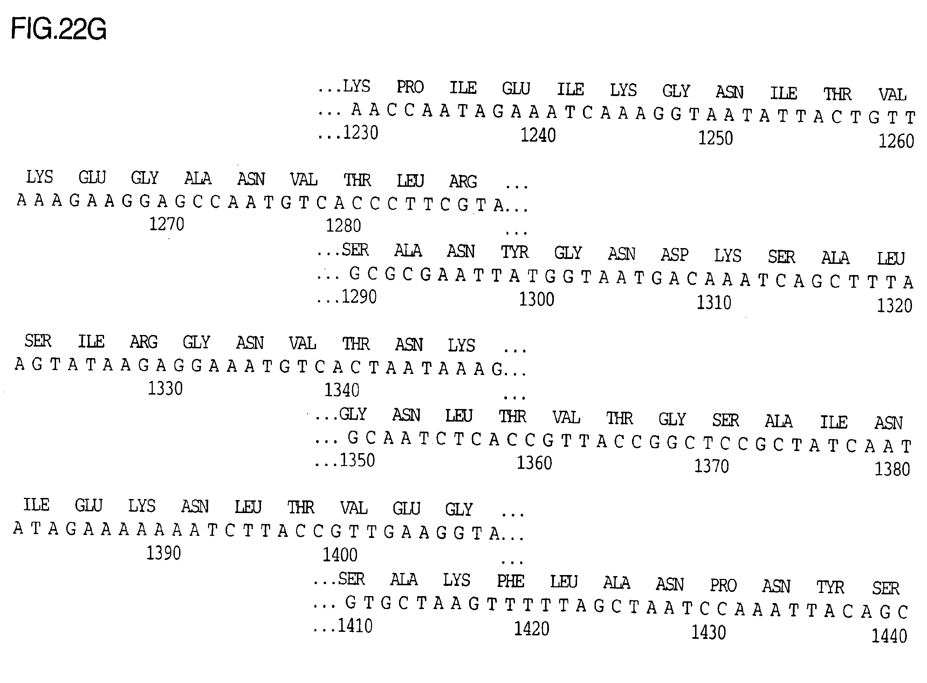

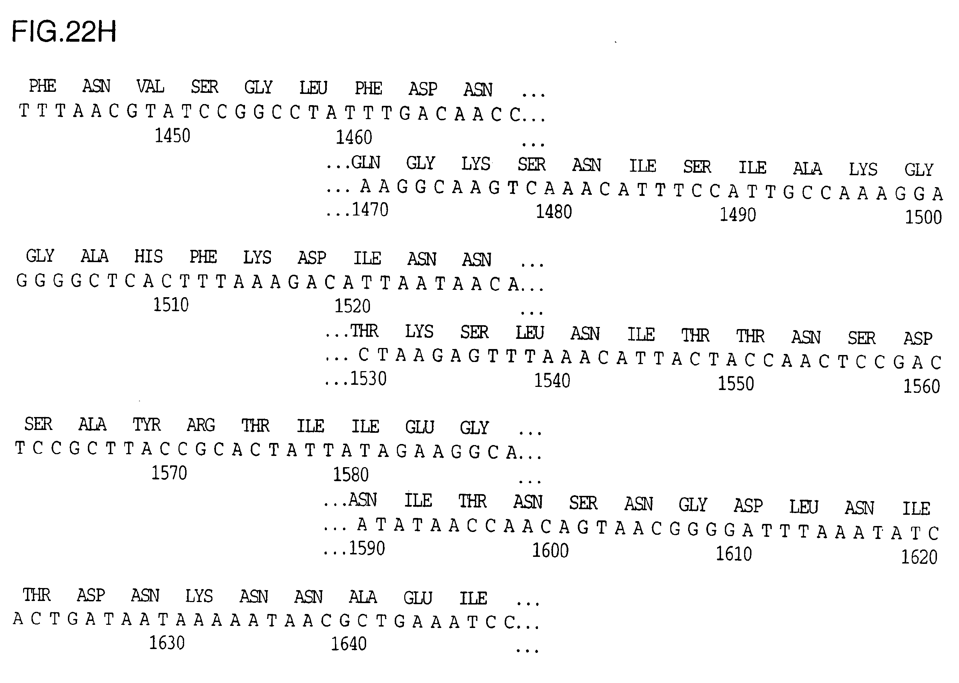

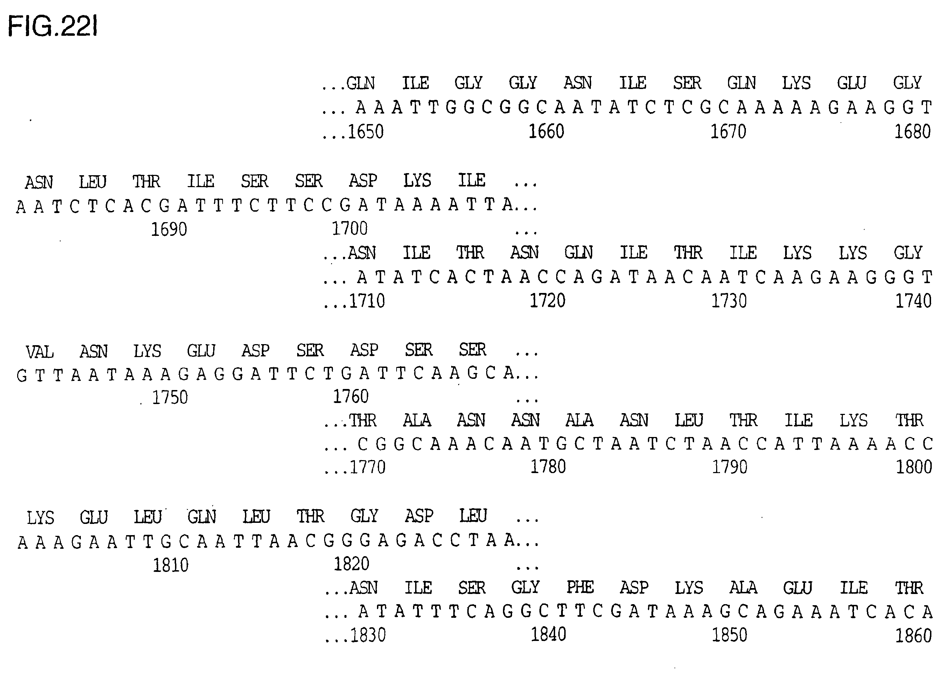

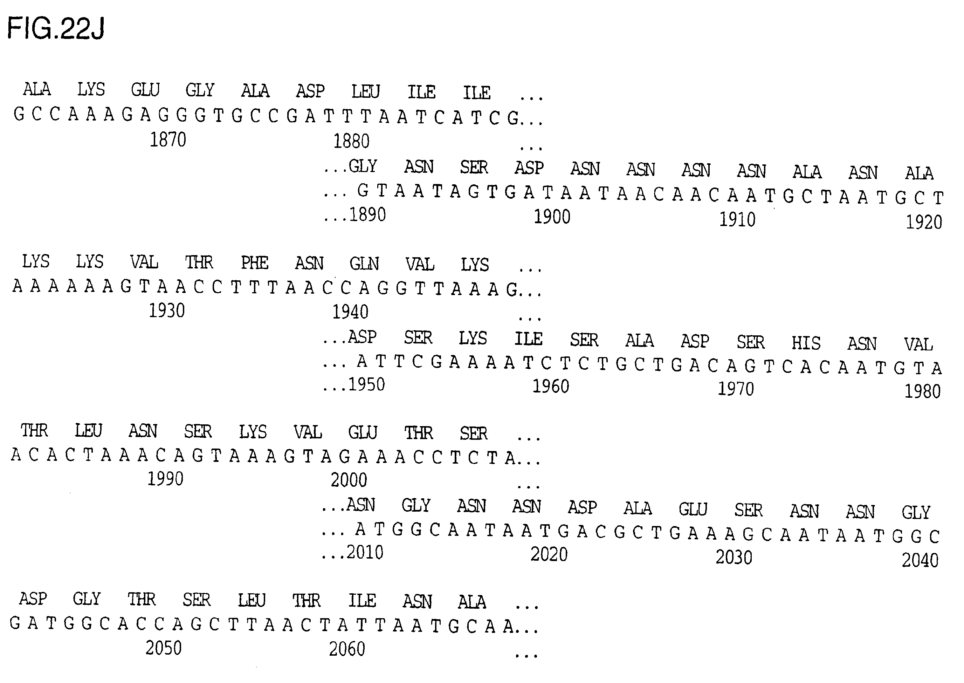

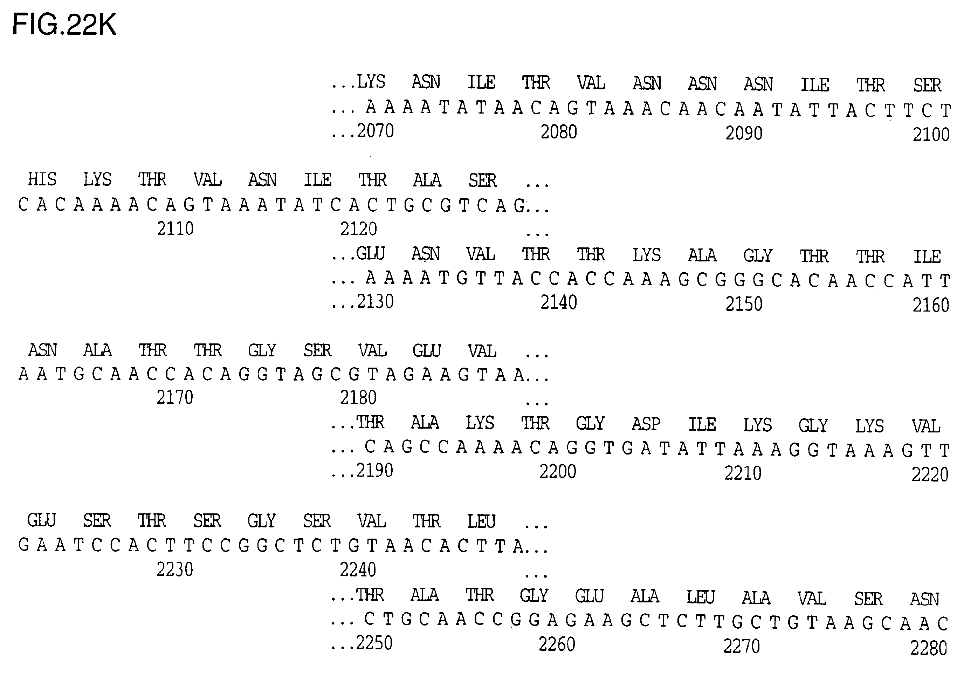

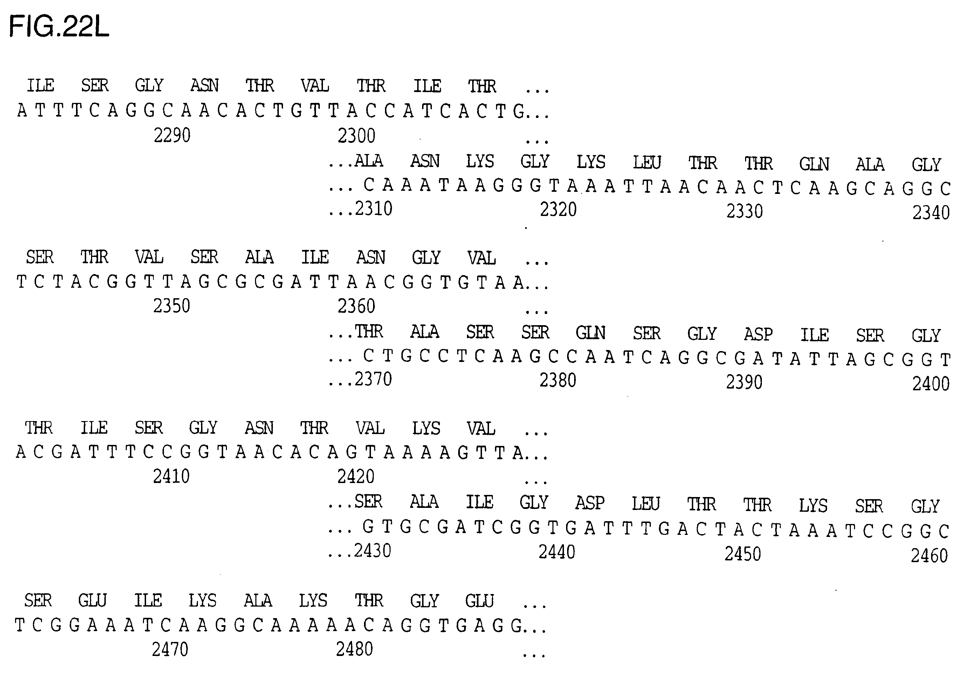

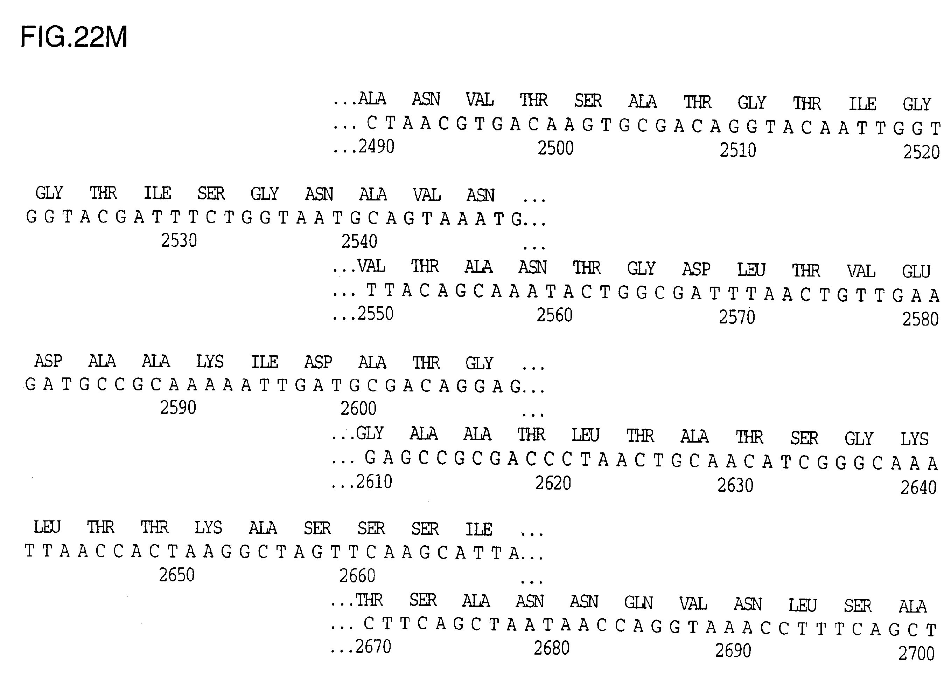

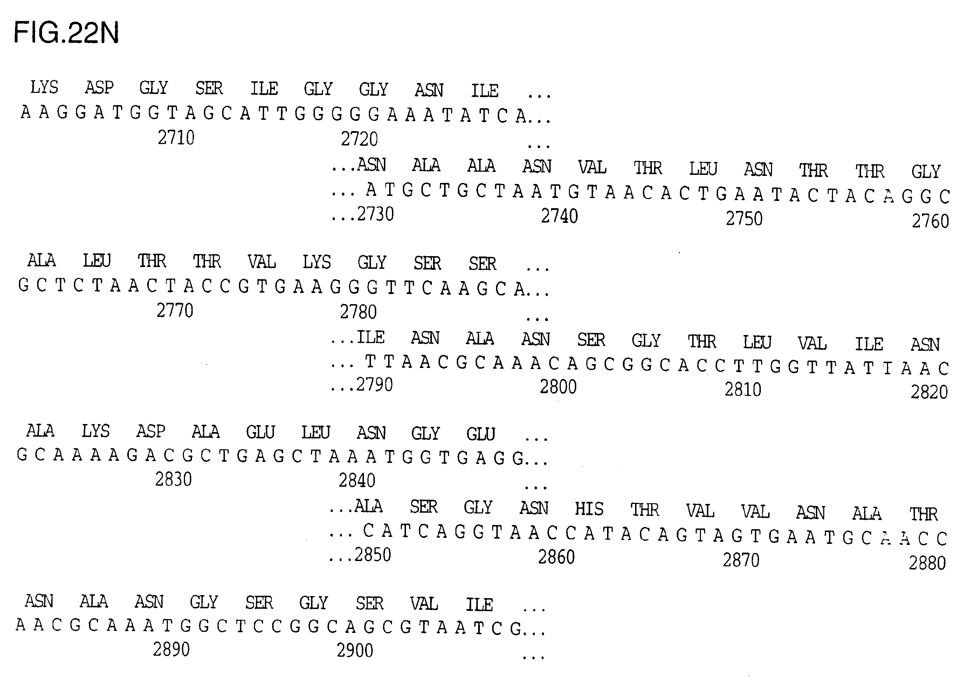

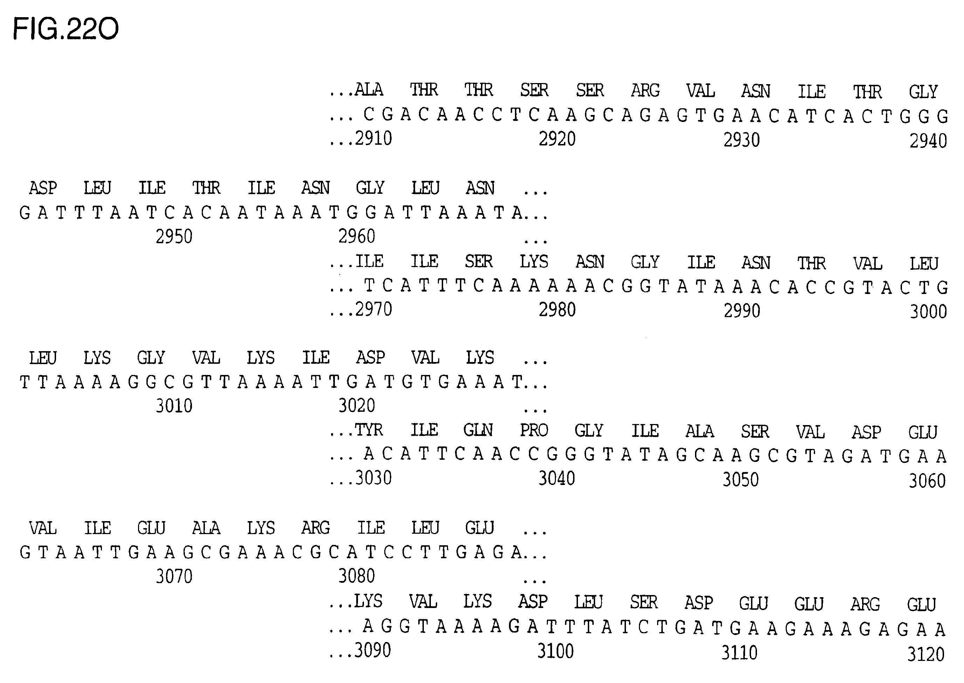

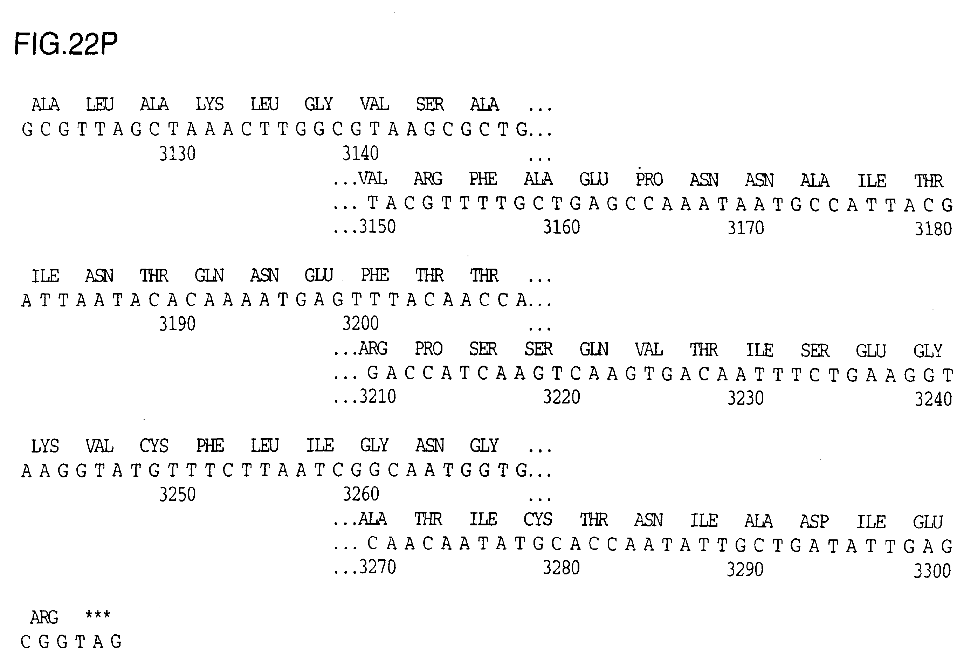

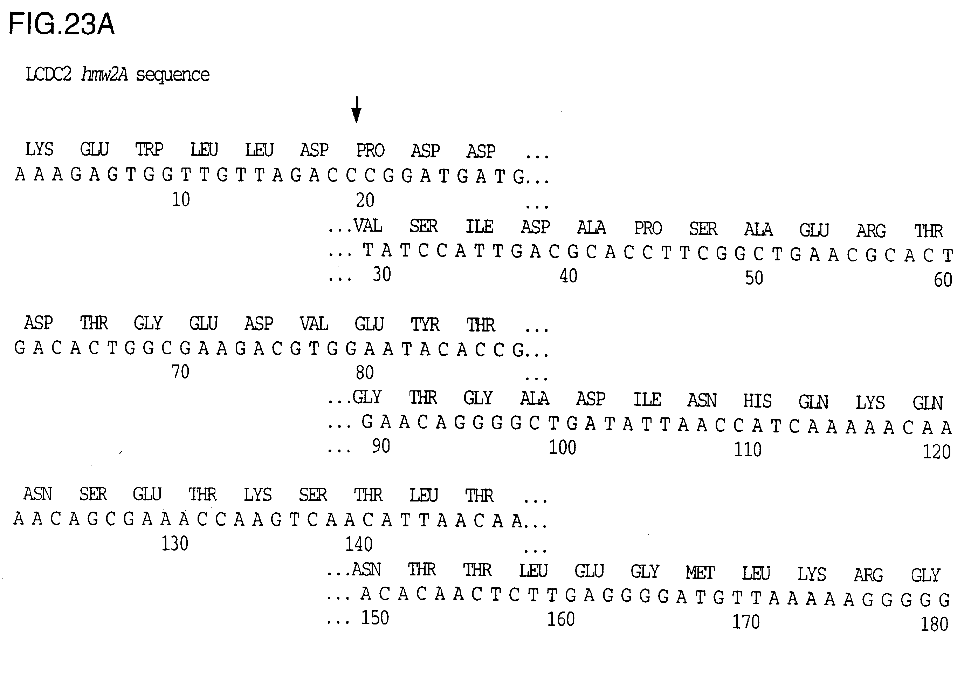

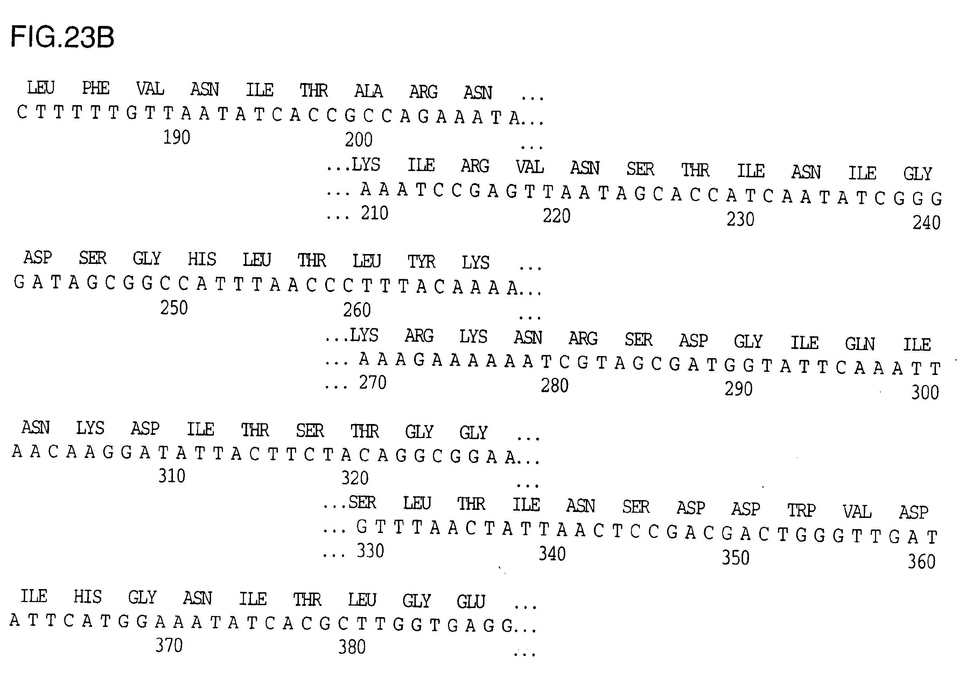

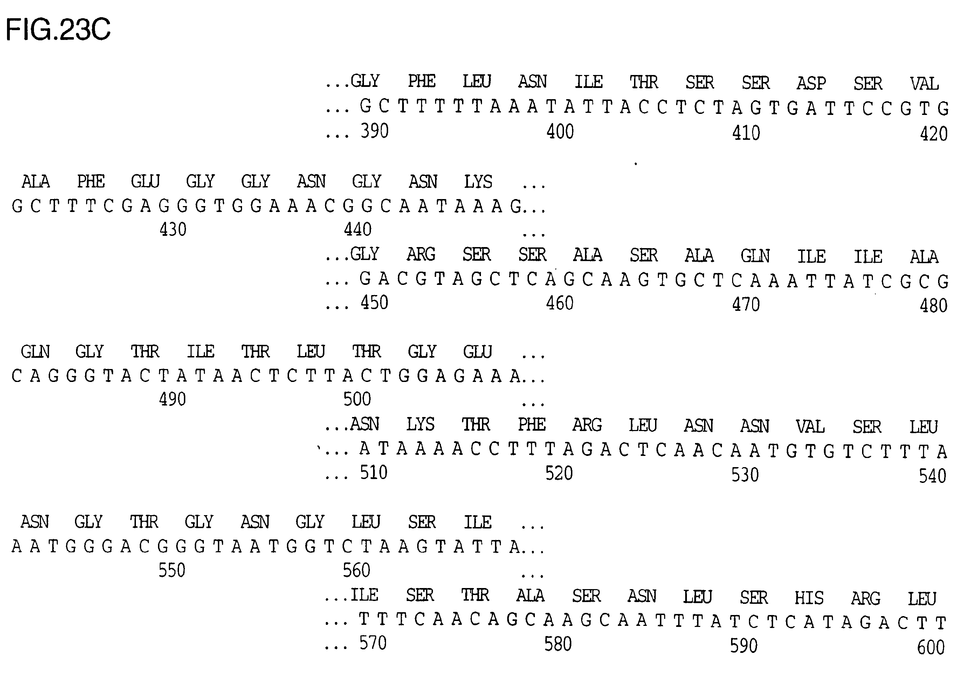

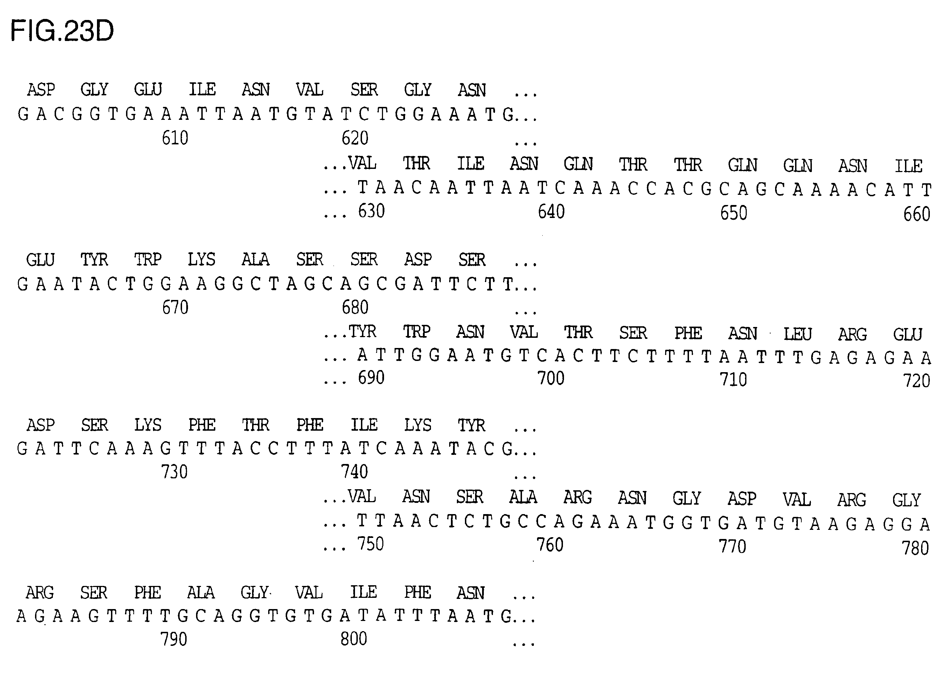

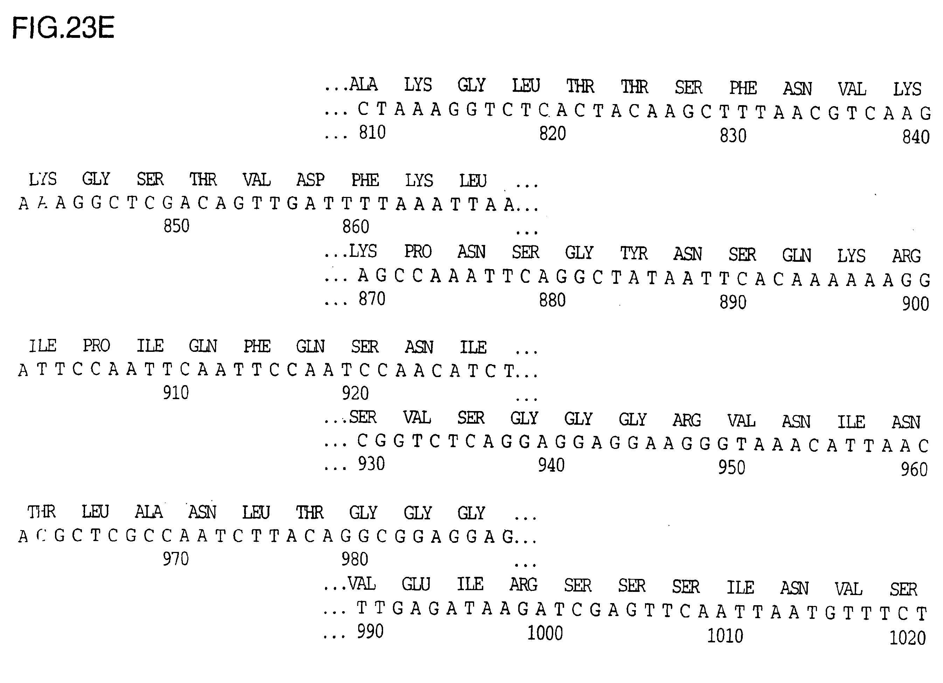

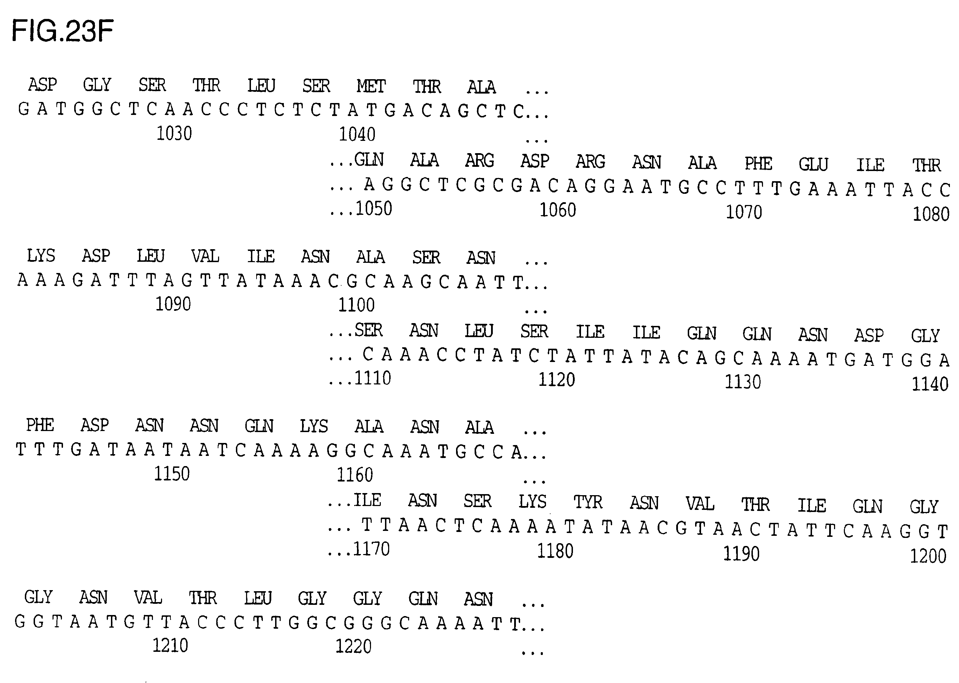

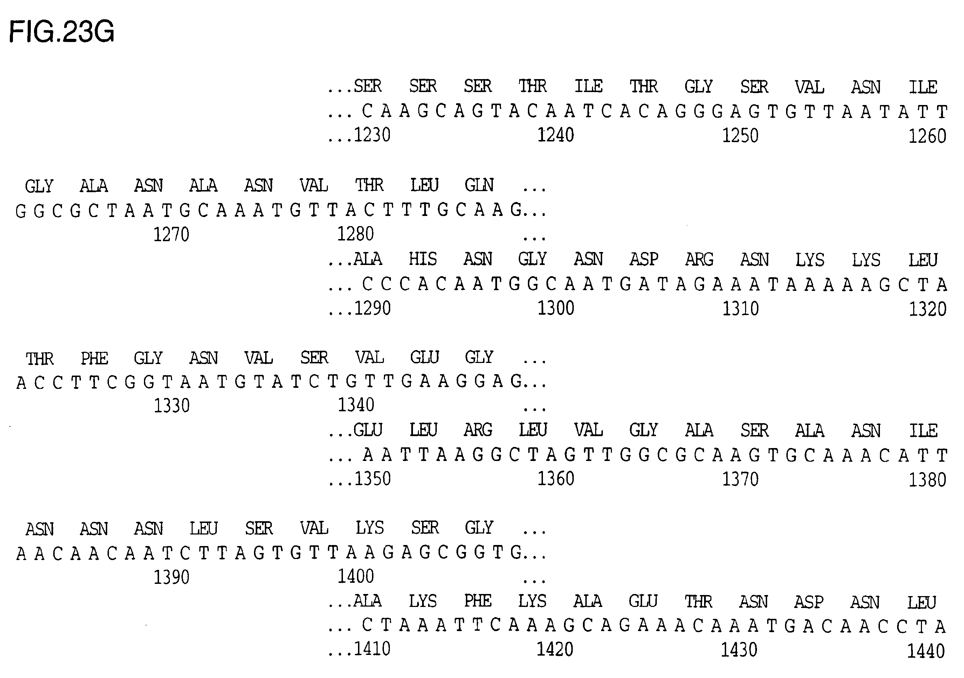

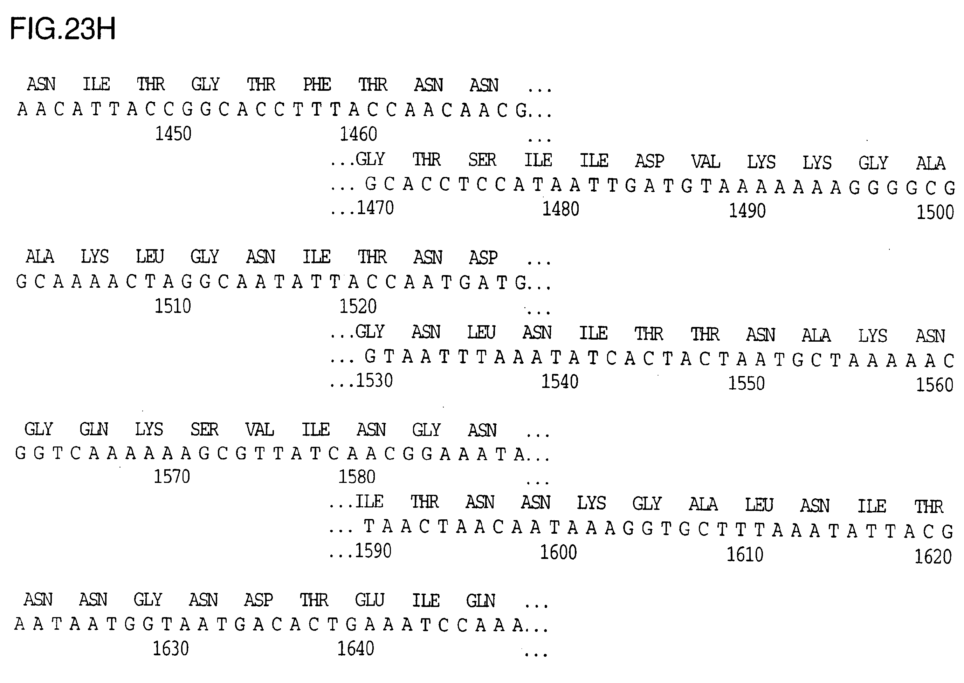

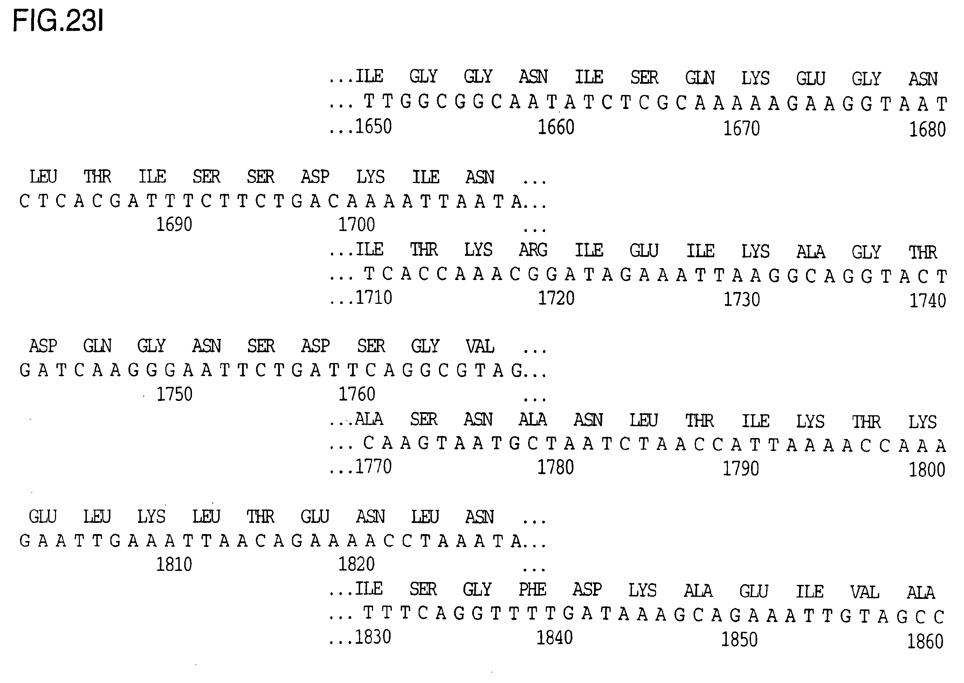

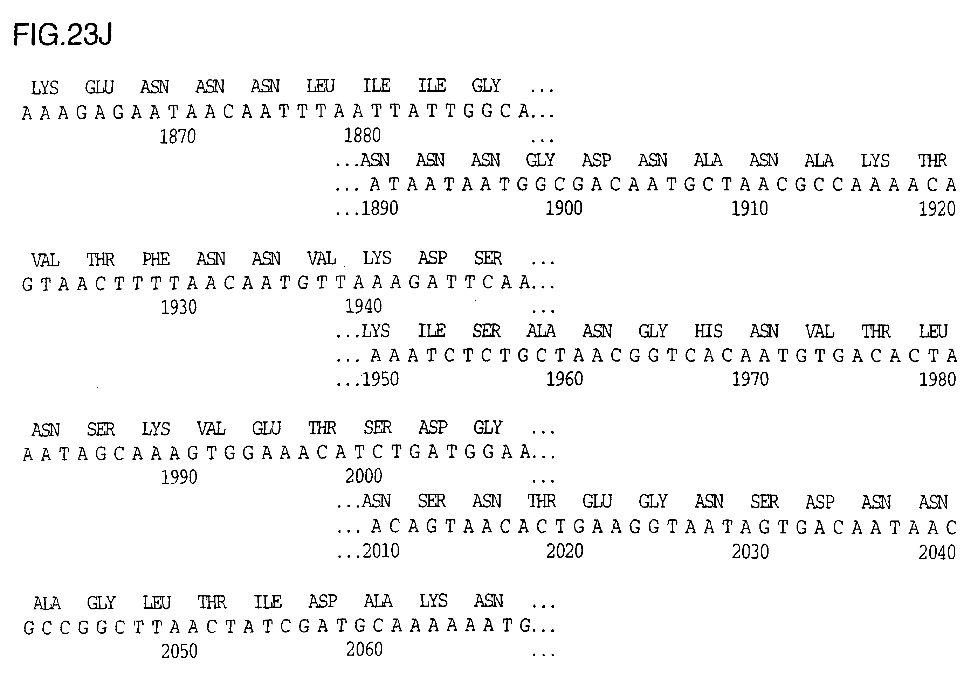

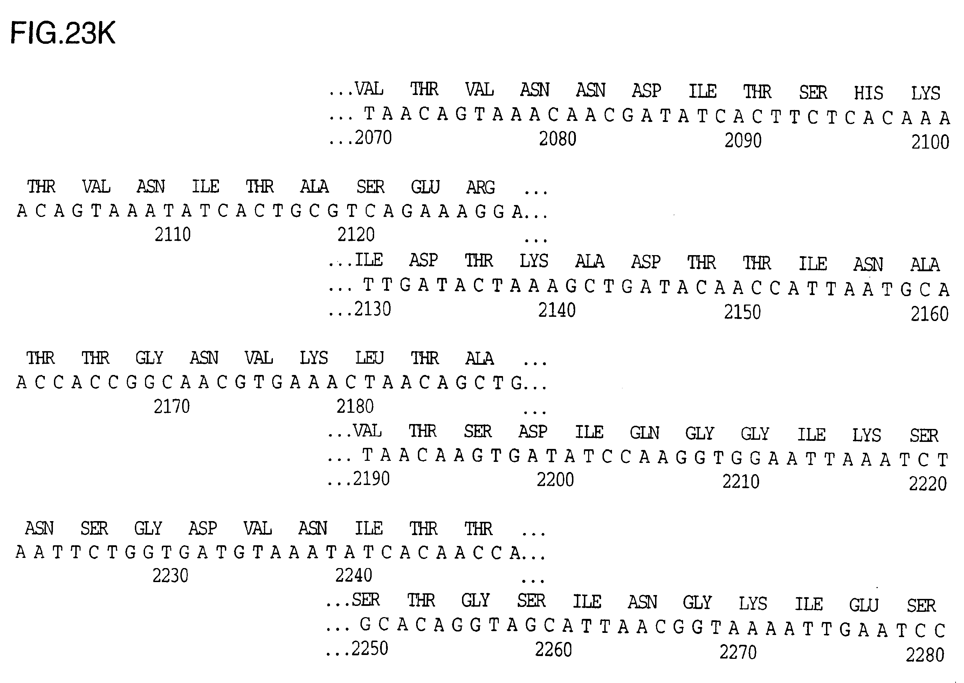

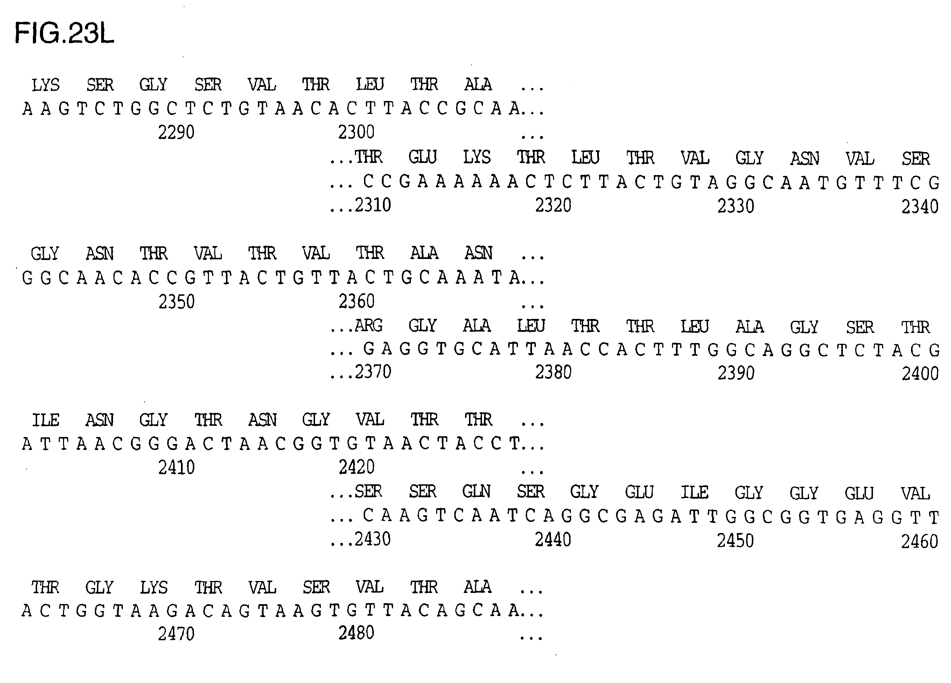

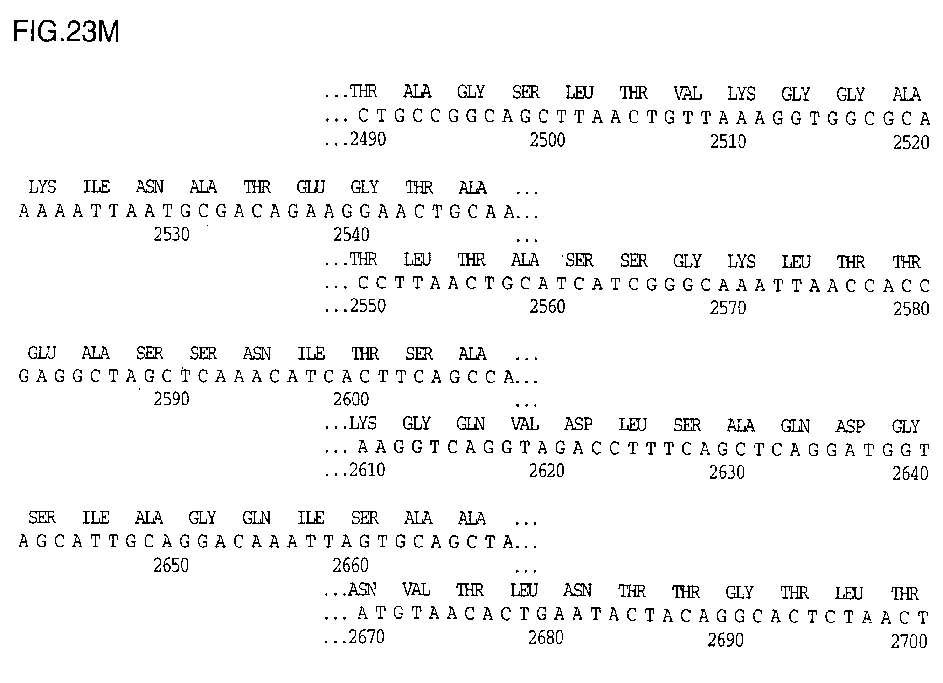

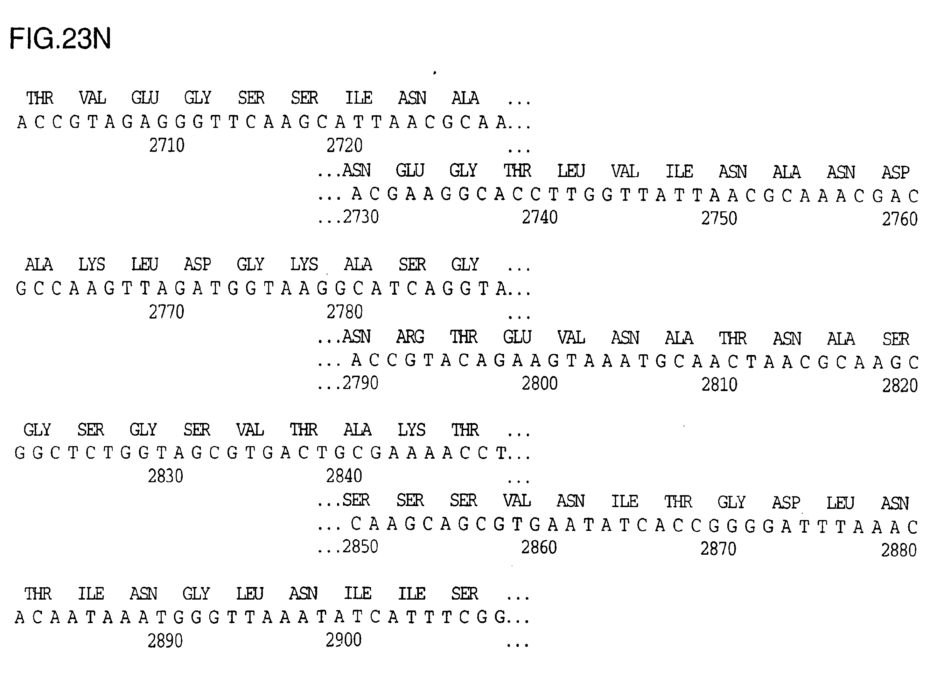

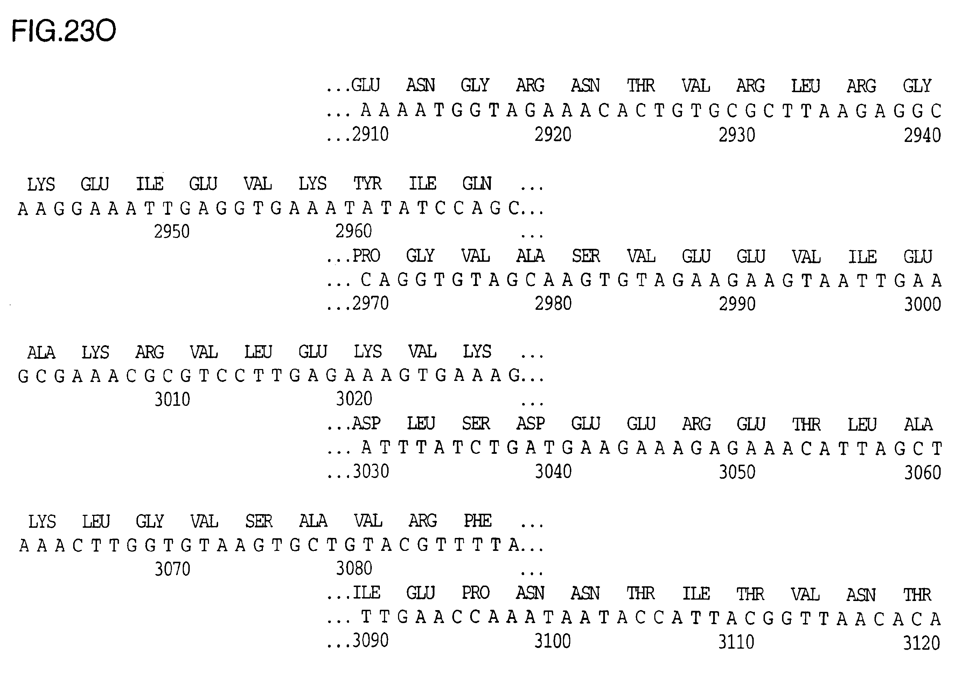

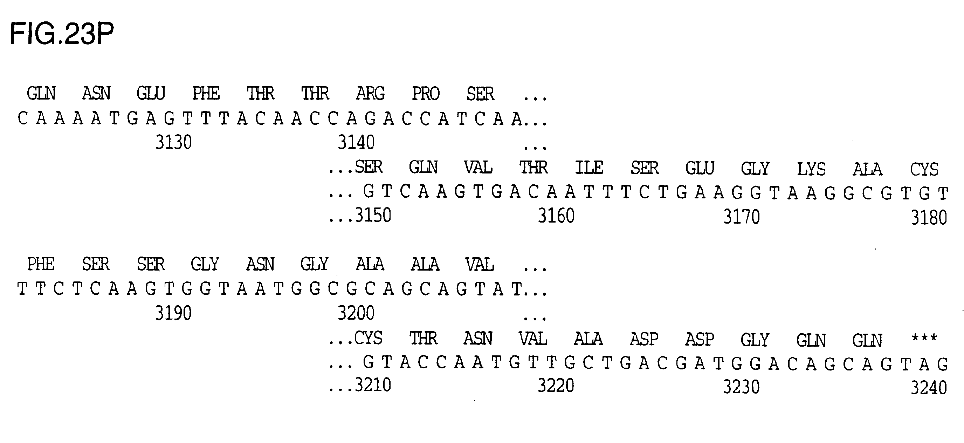

The nucleotide (SEQ ID No: 42) and deduced amino acid (SEQ ID NO: 43) sequence of the *hmw1A* gene from strain LCDC2 are shown in Figure 22. The predicted mature HMW1A protein from strain LCDC2 (encoding sequence SEQ ID NO: 44, amino acid sequence SEQ ID NO: 45) has a molecular weight of 114.0 and a pI of 8.72. There are no RGD motifs found in LCDC2 HMW1A. The nucleotide (SEQ ID NO: 46) and deduced amino acid (SEQ ID NO: 47) sequences of the *hmw2A* gene from strain LCDC2 are shown in Figure 23. The predicted mature HMW2A protein from strain LCDC2 (encoding sequence SEQ ID NO: 48, amino acid sequence SEQ ID NO: 49) has a molecular weight of 111.7 kDa and a pI of 8.22. There are no RGD motifs found in LCDC2 HMW2A.

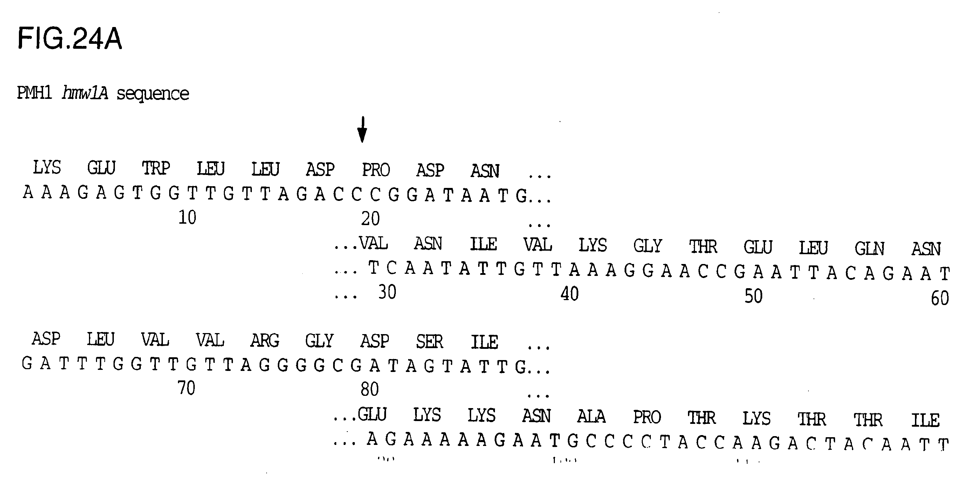

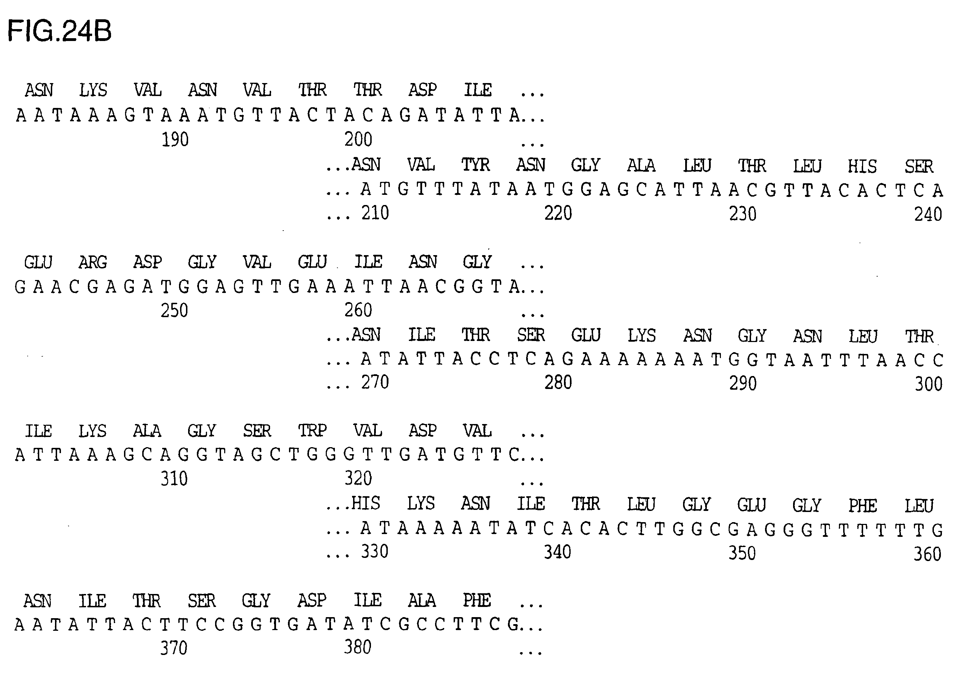

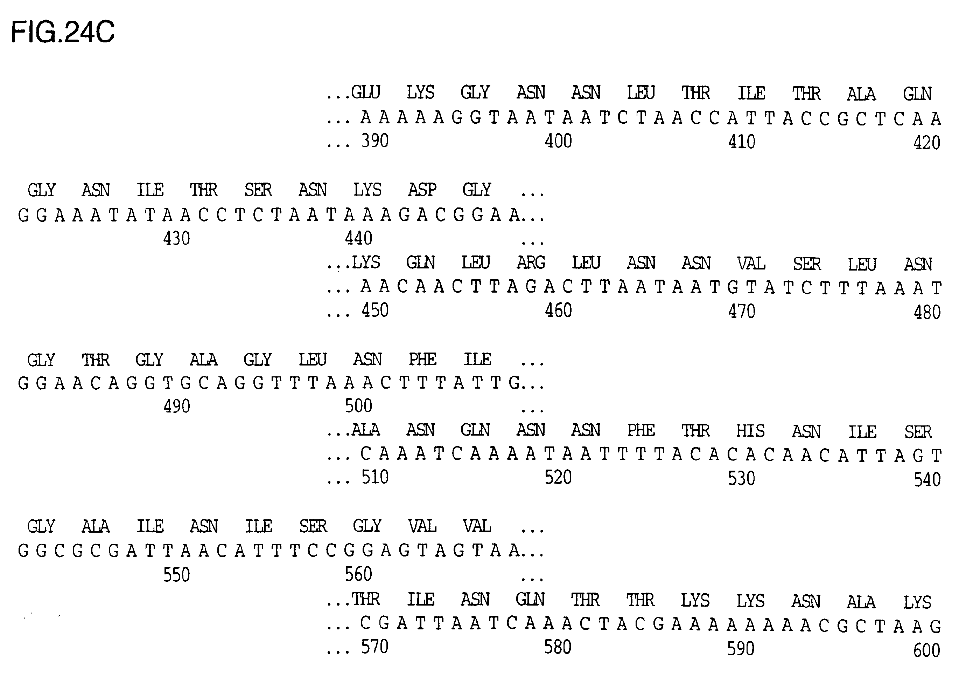

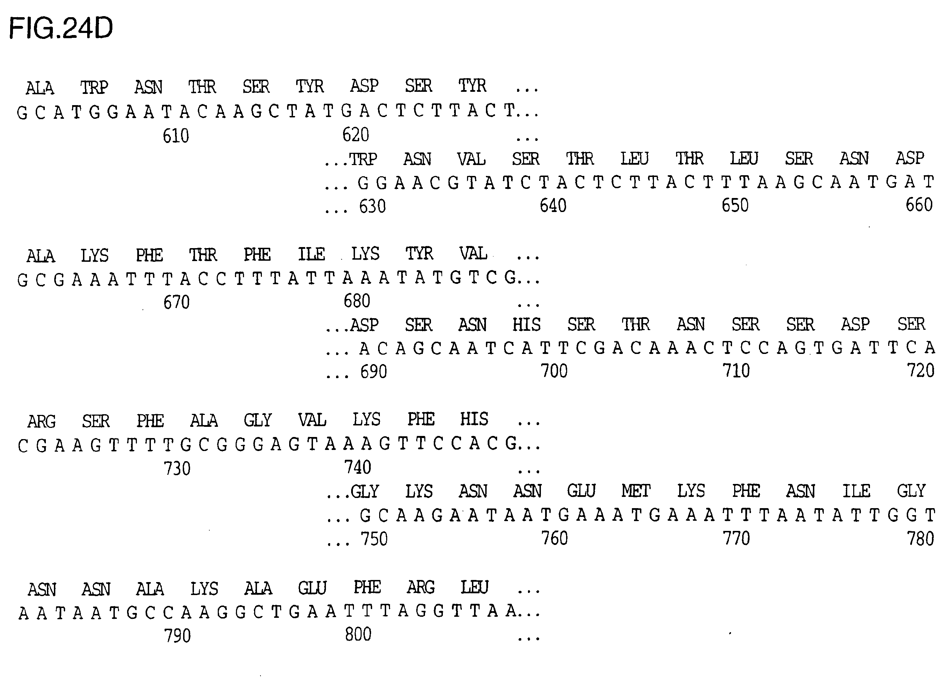

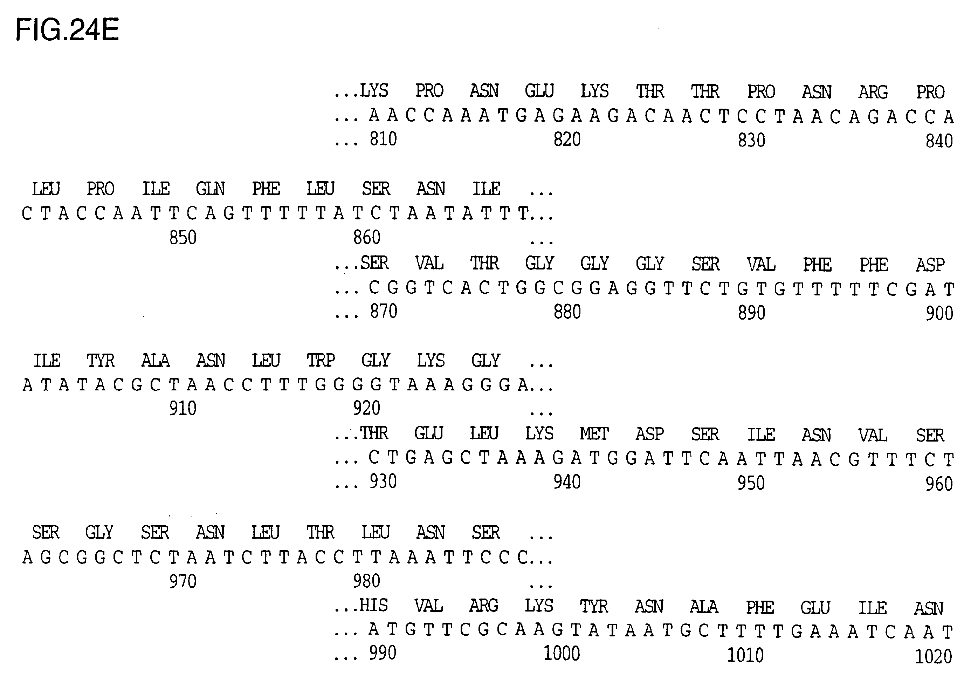

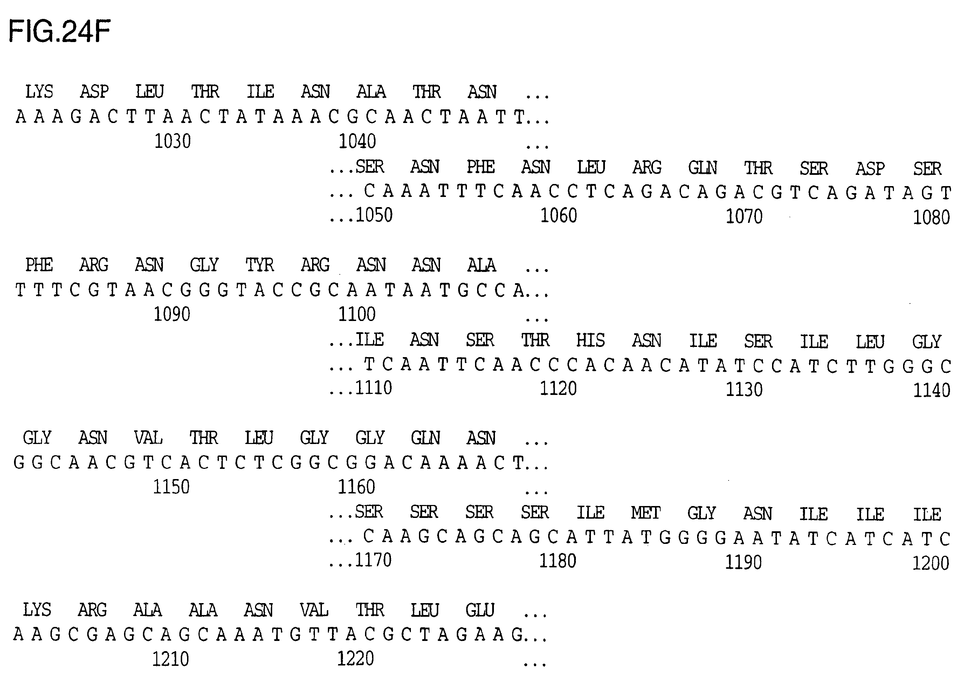

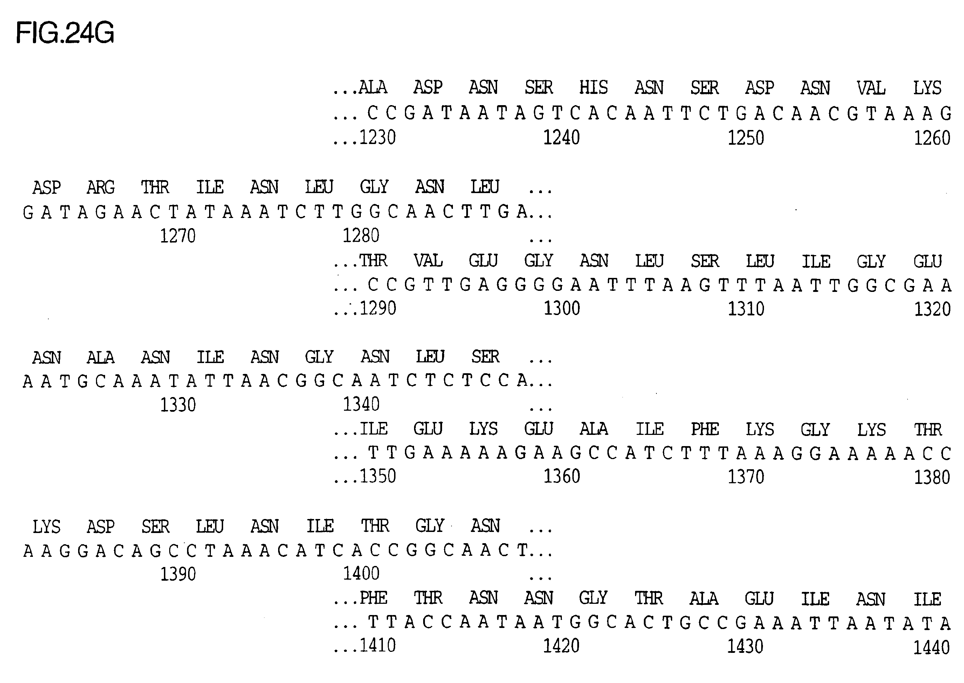

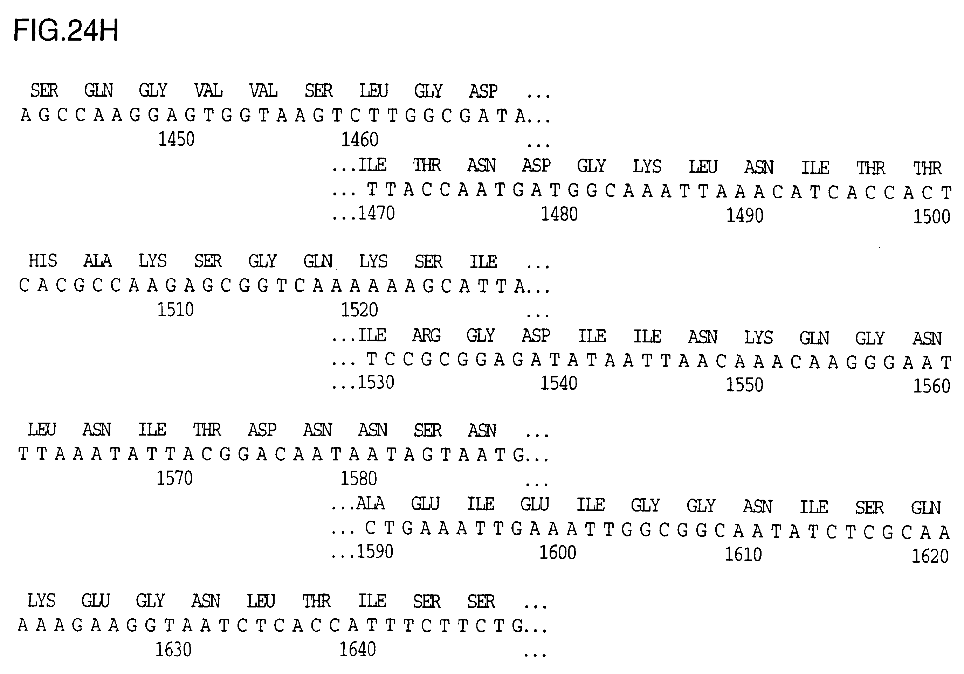

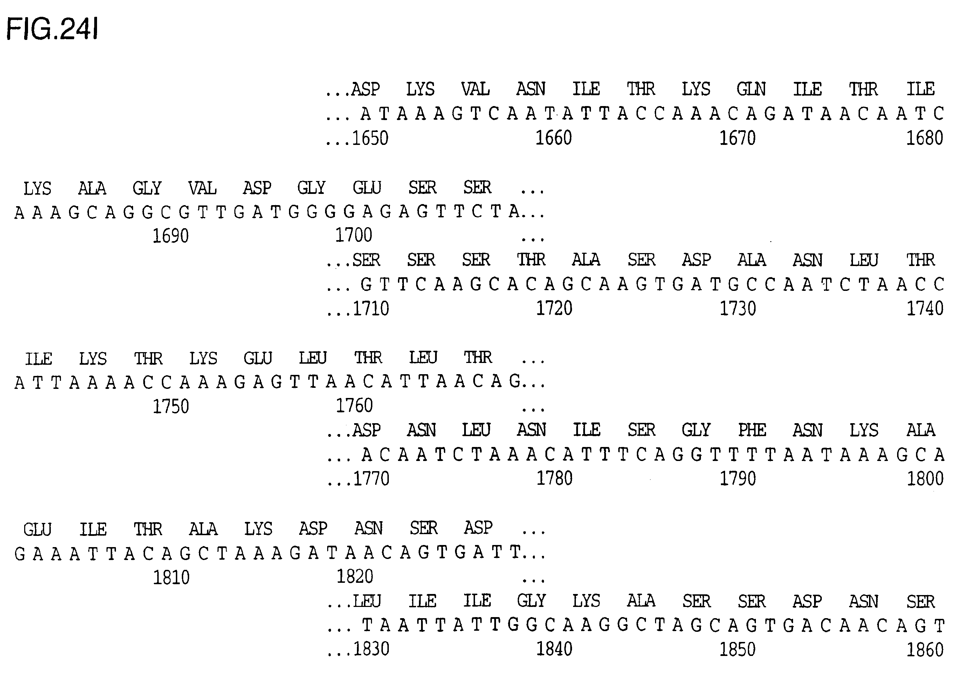

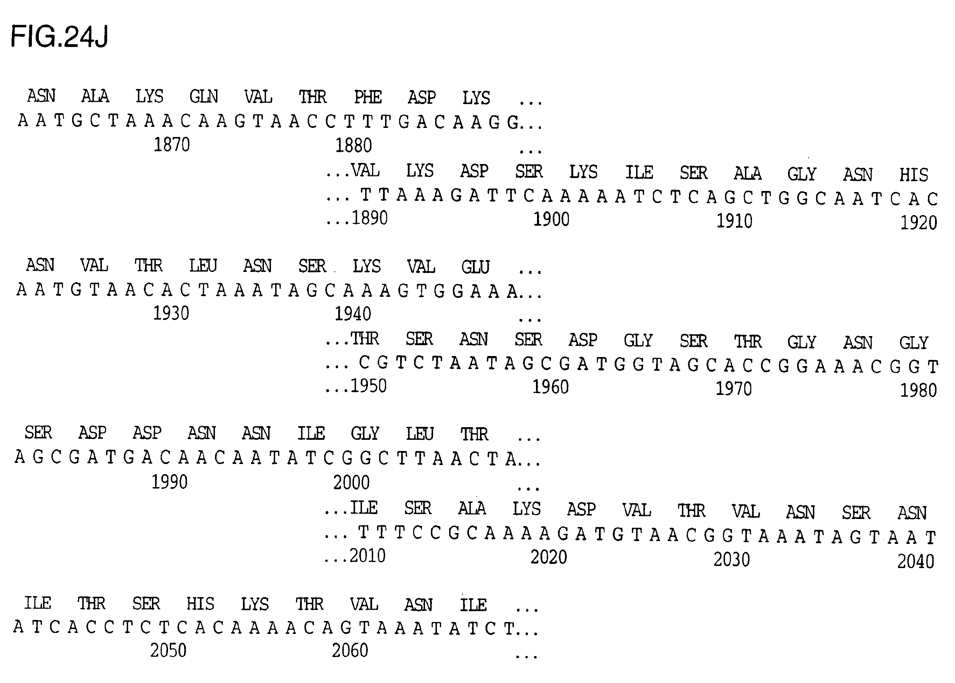

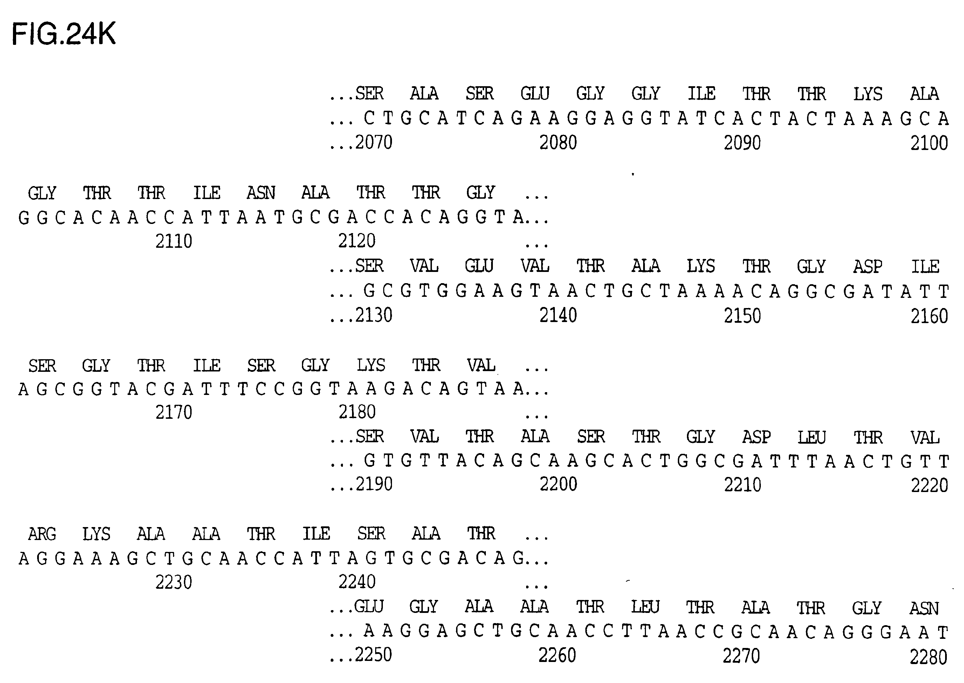

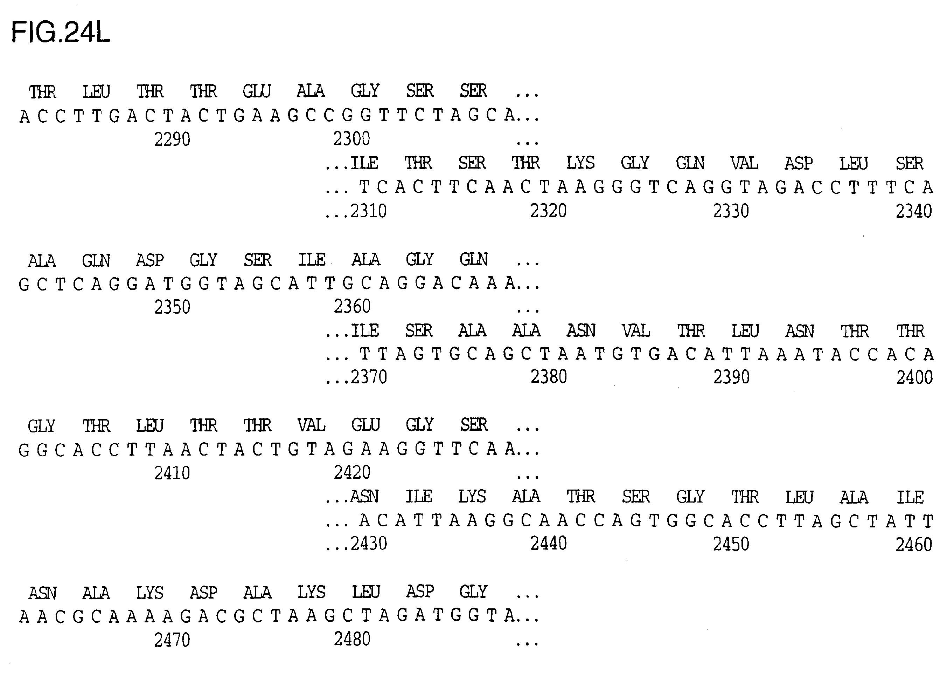

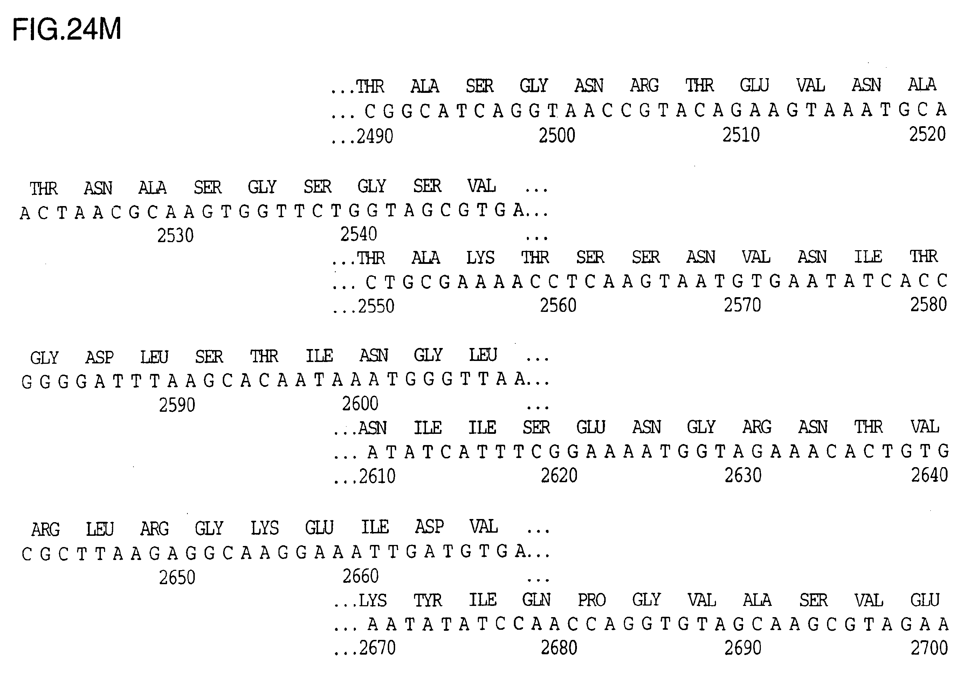

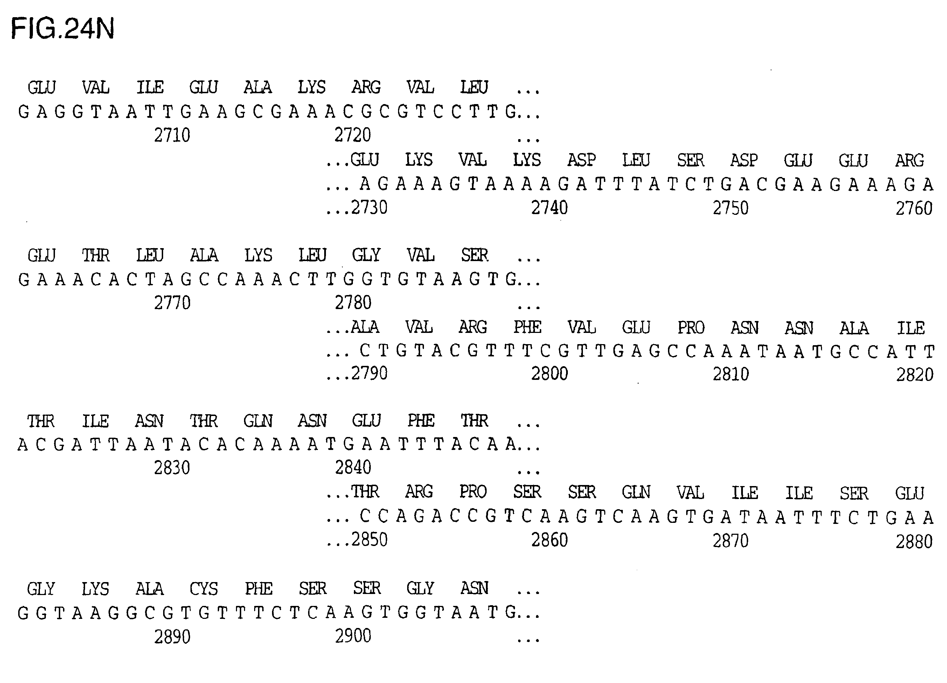

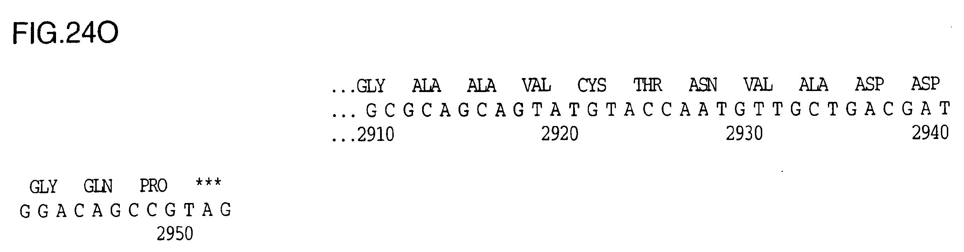

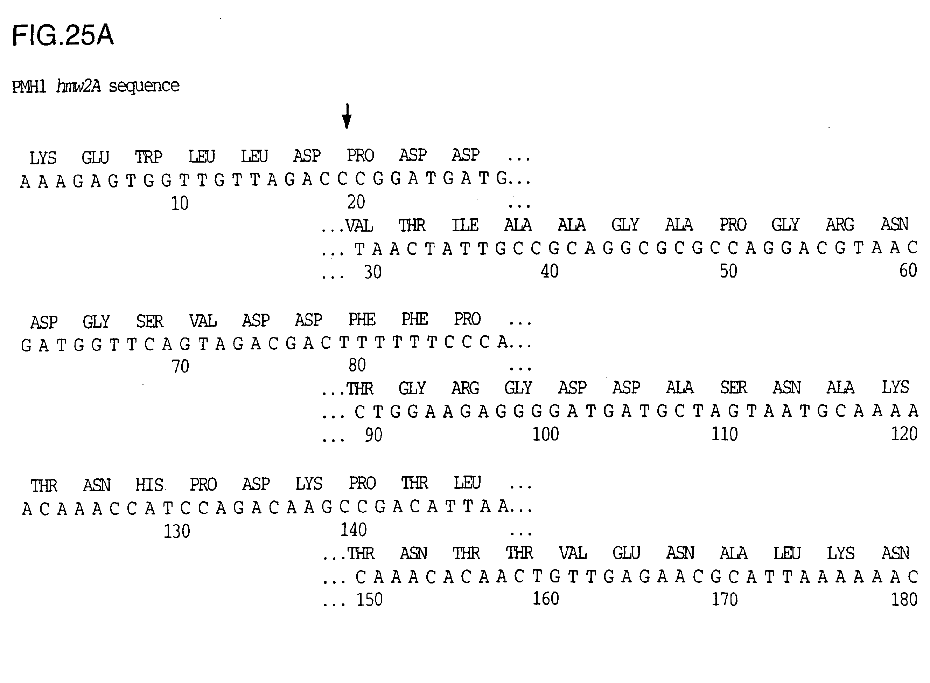

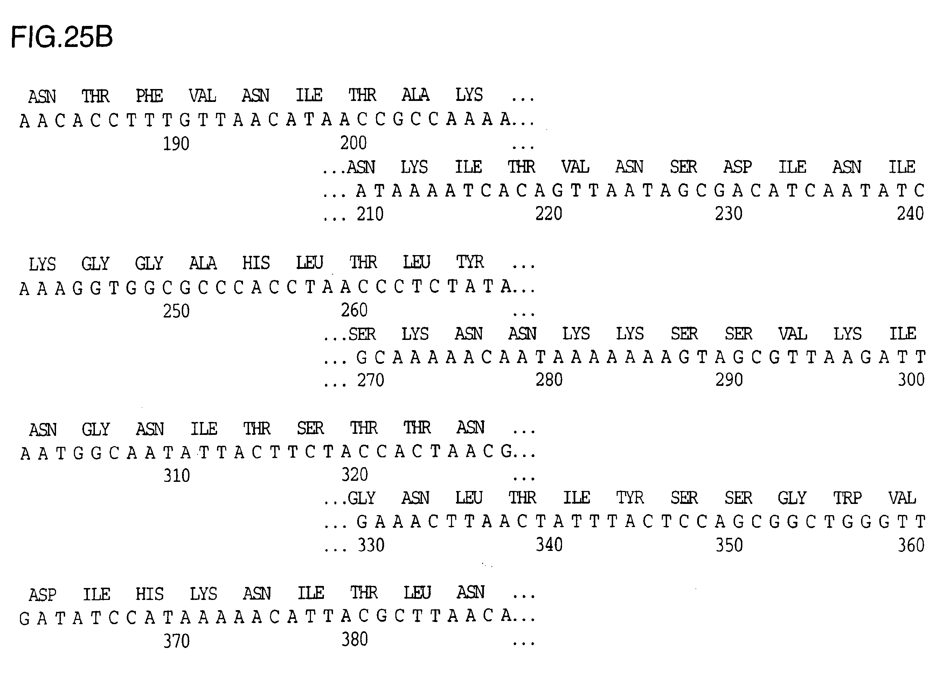

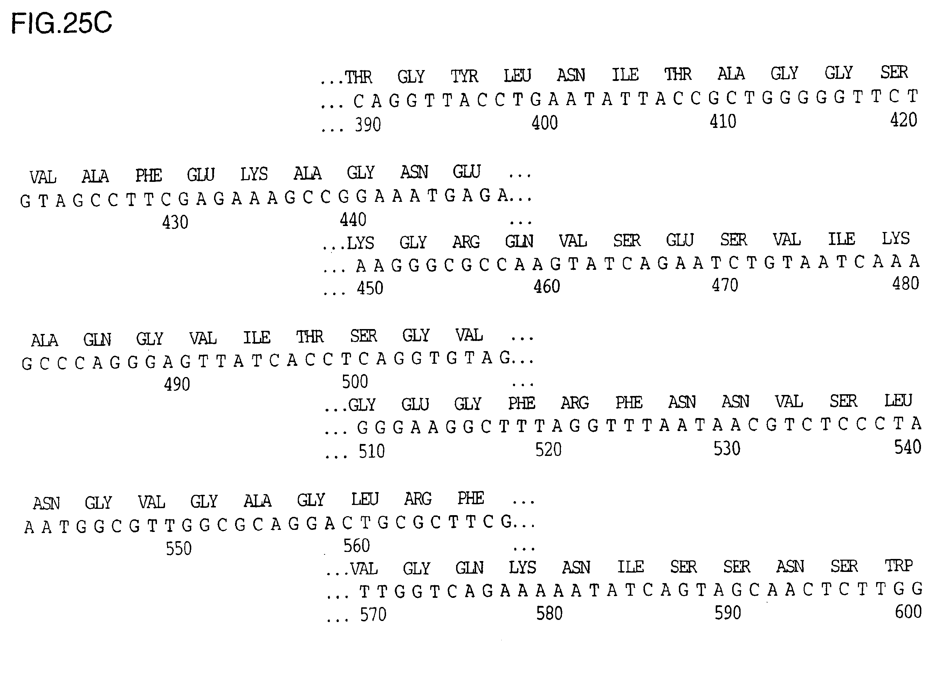

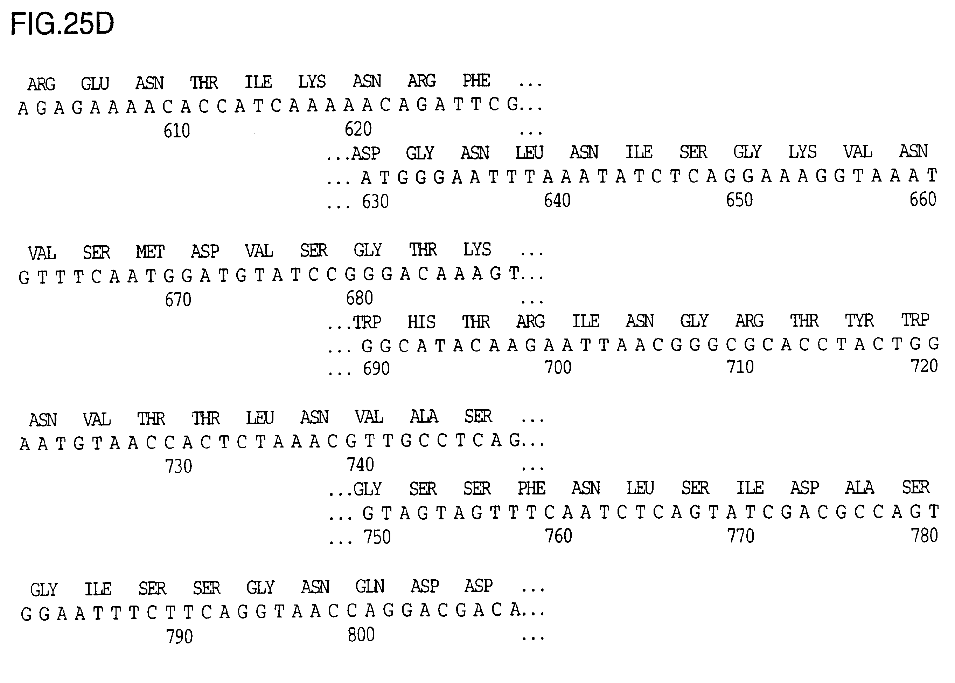

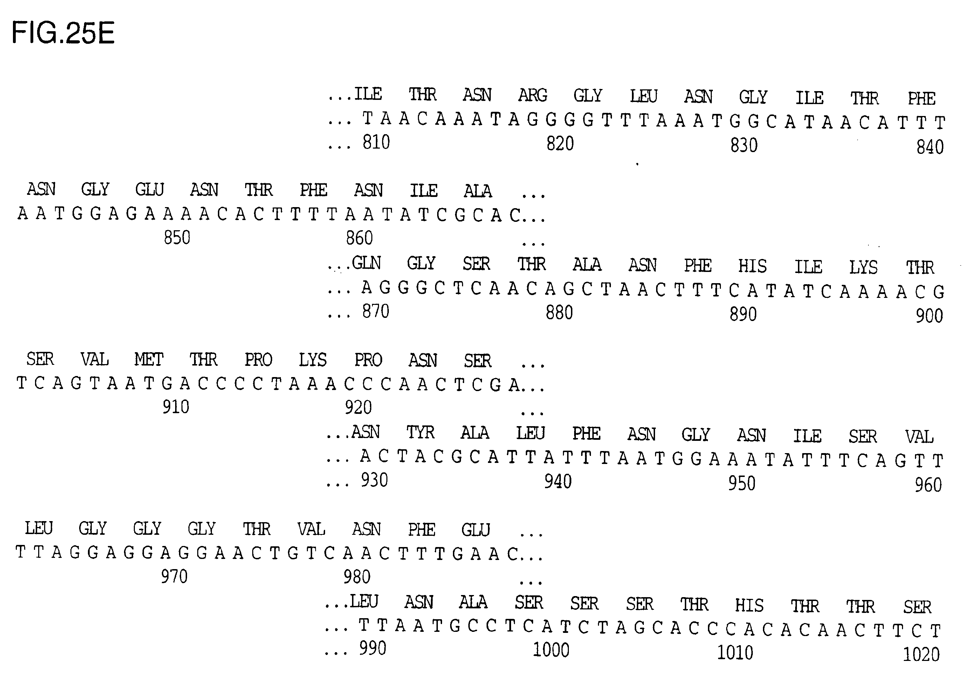

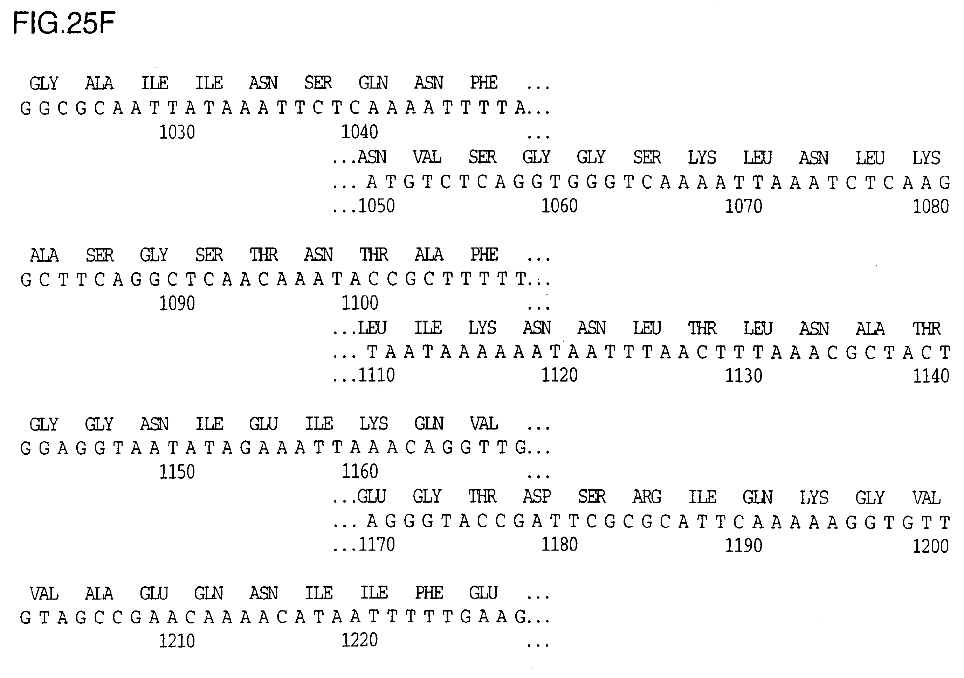

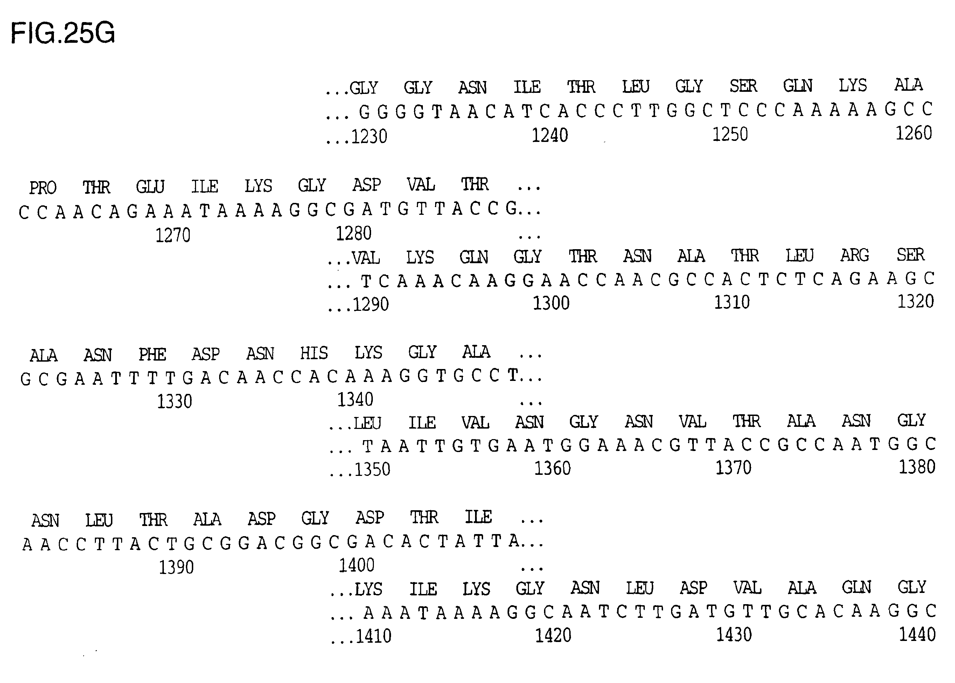

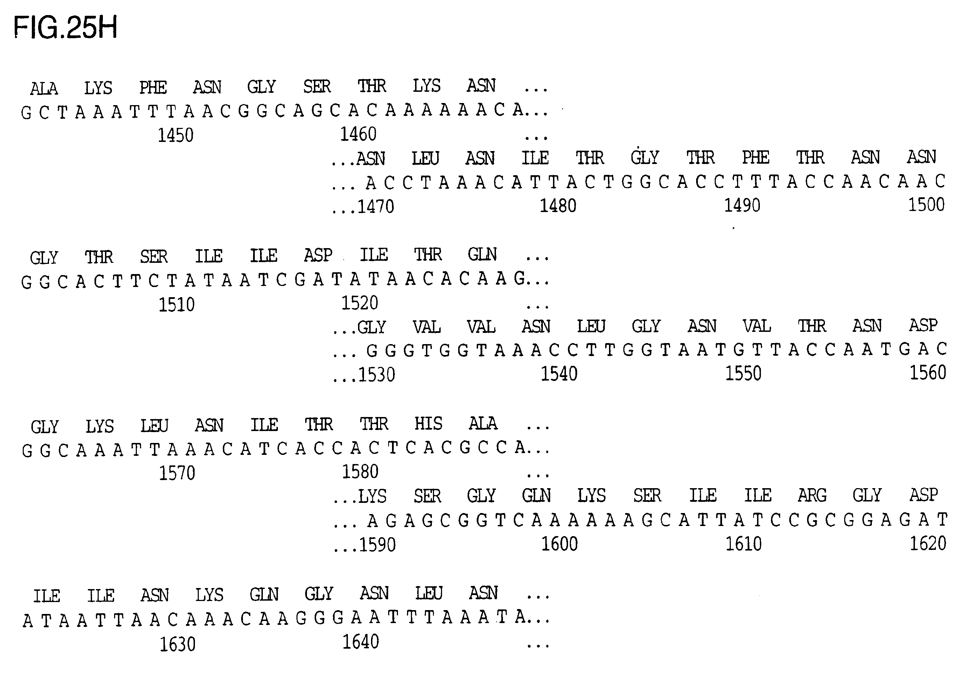

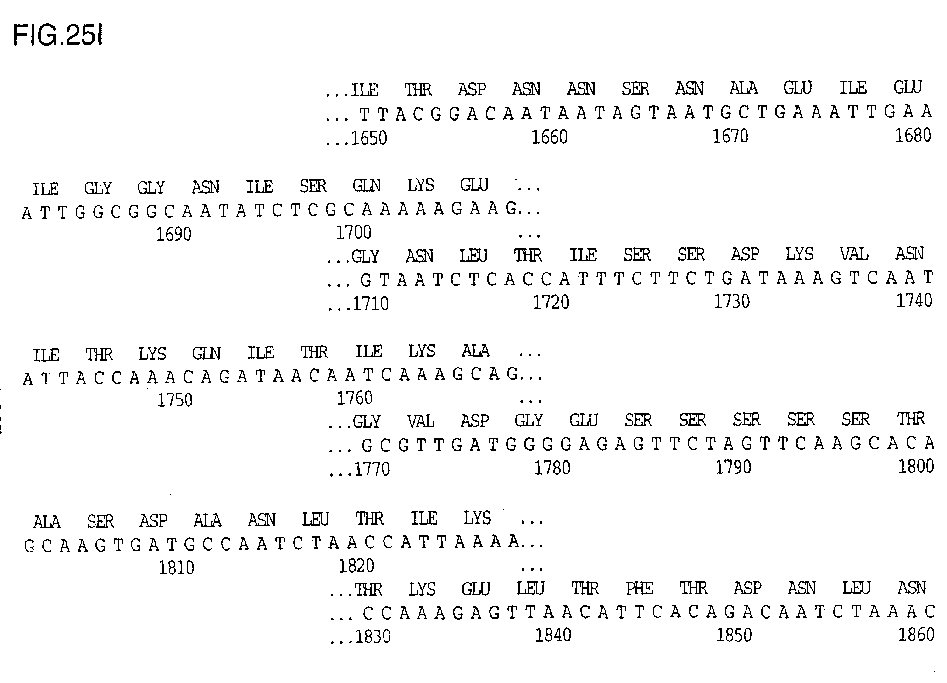

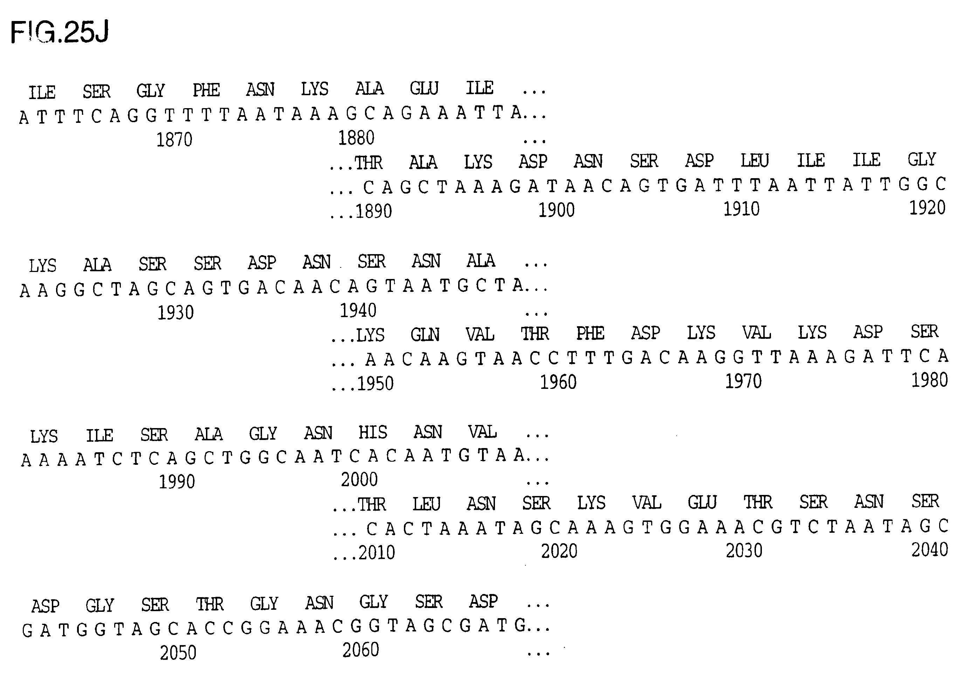

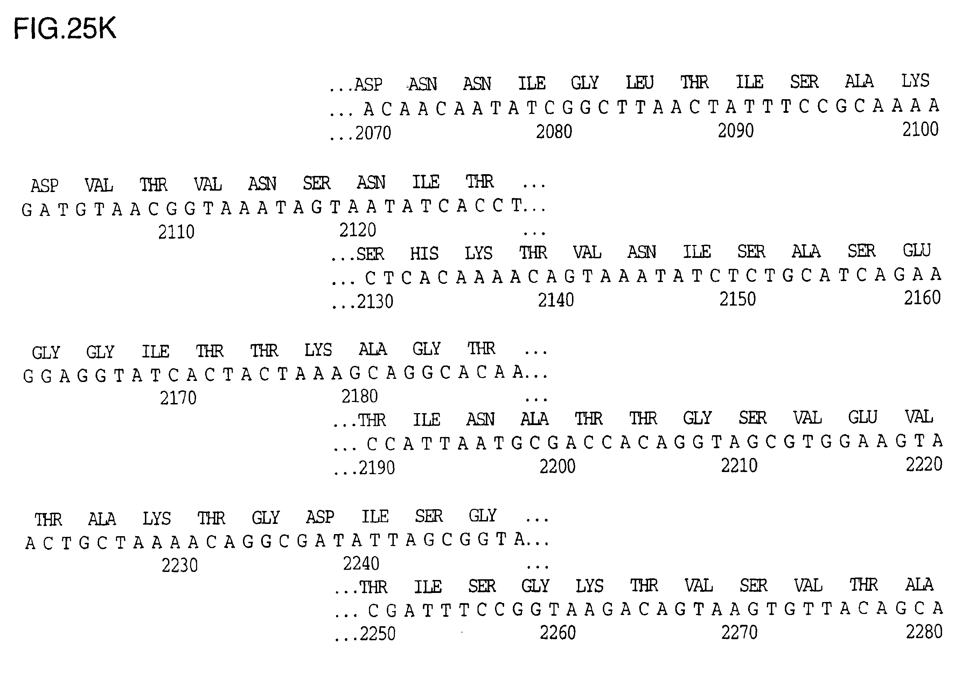

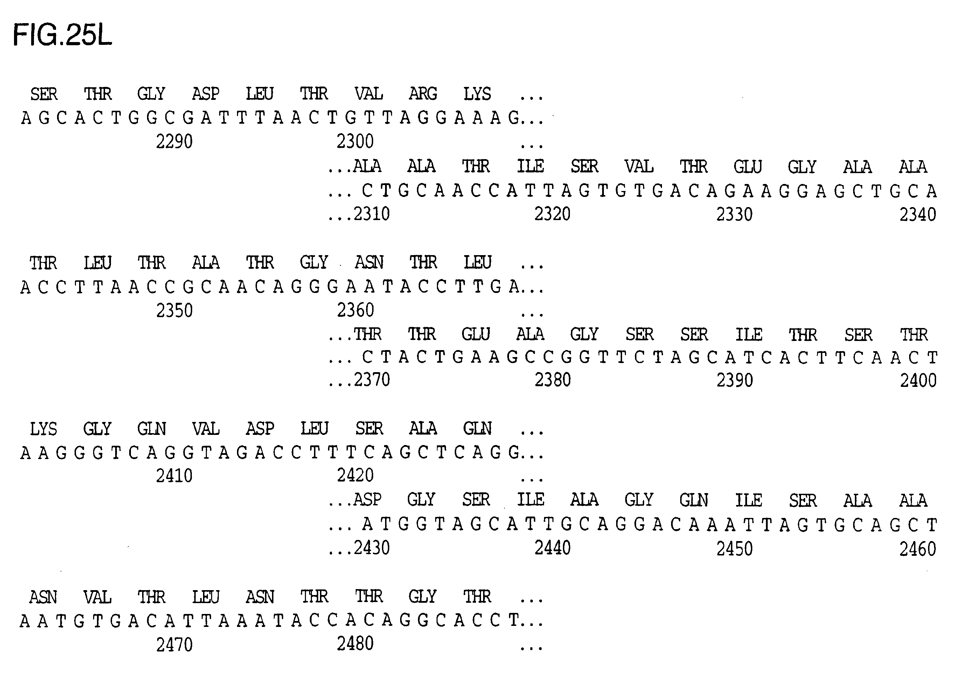

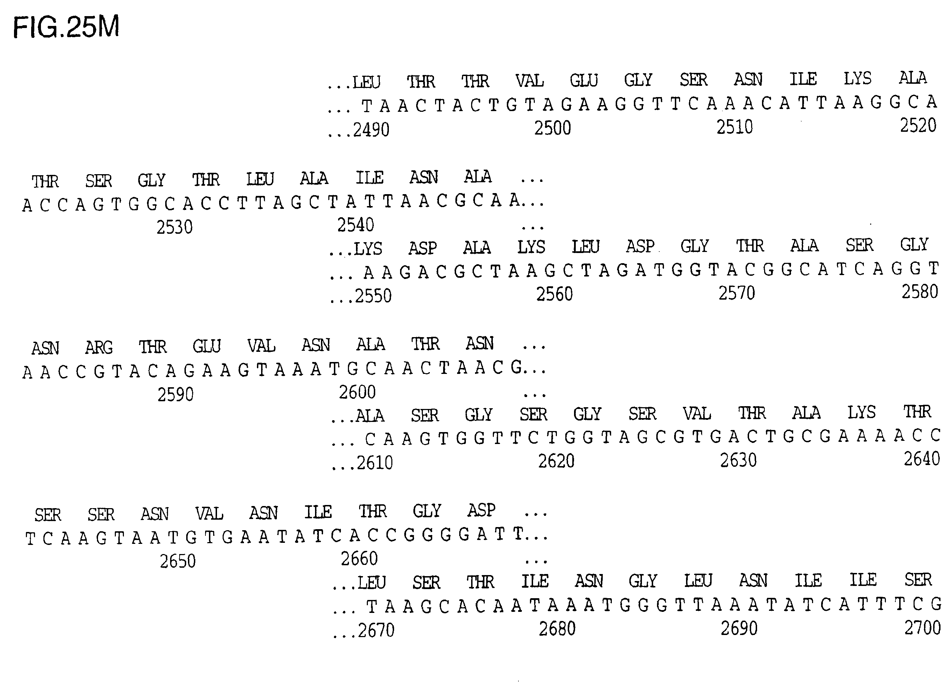

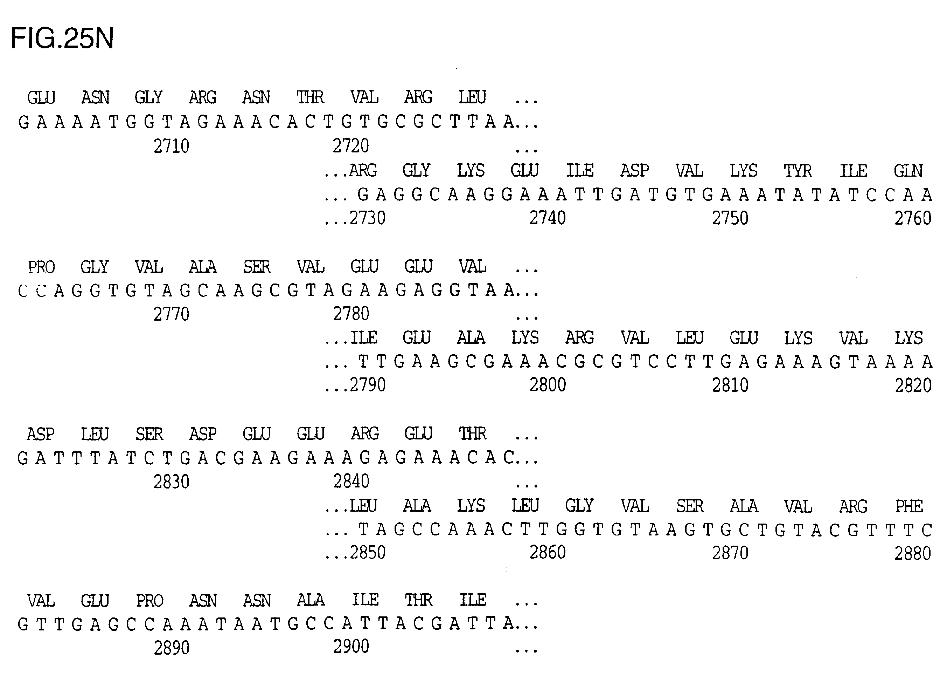

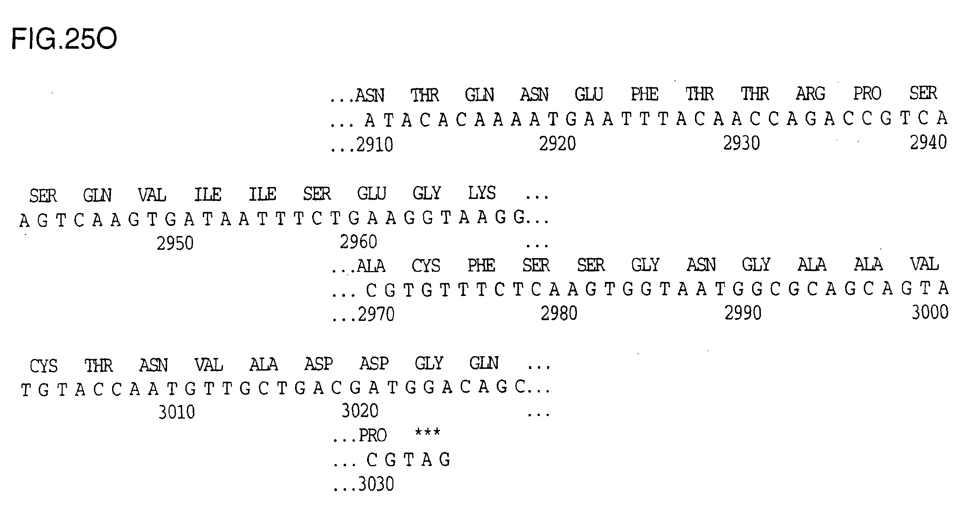

The nucleotide (SEQ ID NO: 50) and deduced amino acid (SEQ ID NO: 51) sequences of the *hmw1A* gene from strain PMH1 are shown in Figure 24. The predicted mature HMW1A protein from strain PMH1 (encoding sequence SEQ ID NO: 52, amino acid sequence ID NO: 53) has a molecular weight of 102.4 kDa and a pI of 6.73. There are two RGD motifs found in PMH1 HMW1A, the first at residues 19 to 21 and the second at residues 505 to 507. The nucleotide (SEQ ID NO: 54) and deduced amino acid (SEQ ID NO: 55) sequences of the *hmw2A* gene from strain PMH1 are shown in Figure 25. The predicted mature HMW2A protein from strain PMH1 (encoding sequence SEQ ID NO: 56, amino acid sequence SEQ ID NO: 57) has a molecular weight of 103.9 kDa and a pI of 9.07. There are two RGD motifs found in PMH1 HMW2A, the first at residues 26 to 28 and the second at residues 532 to 534.

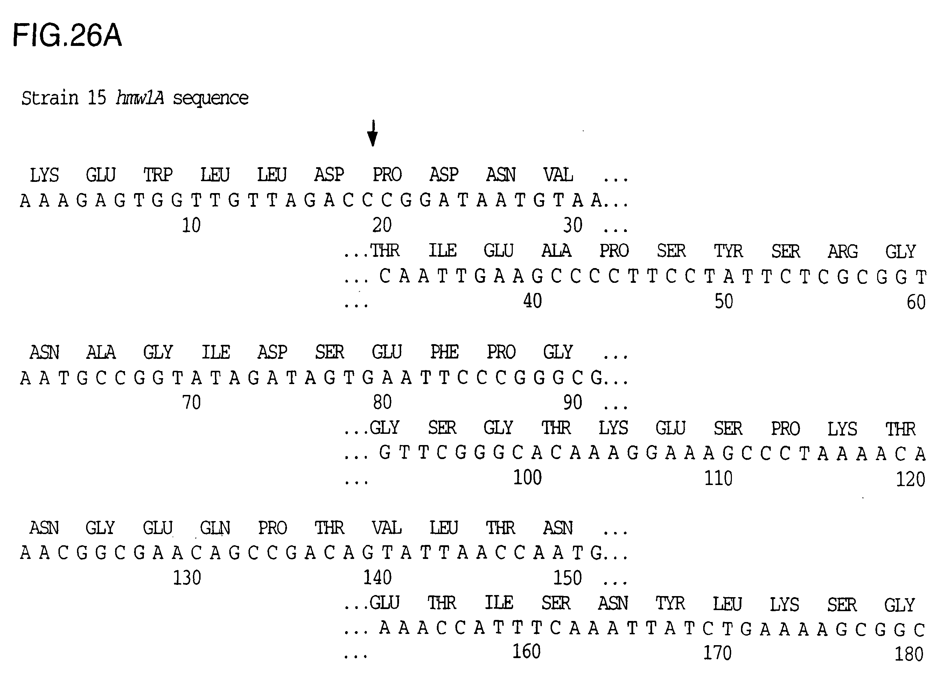

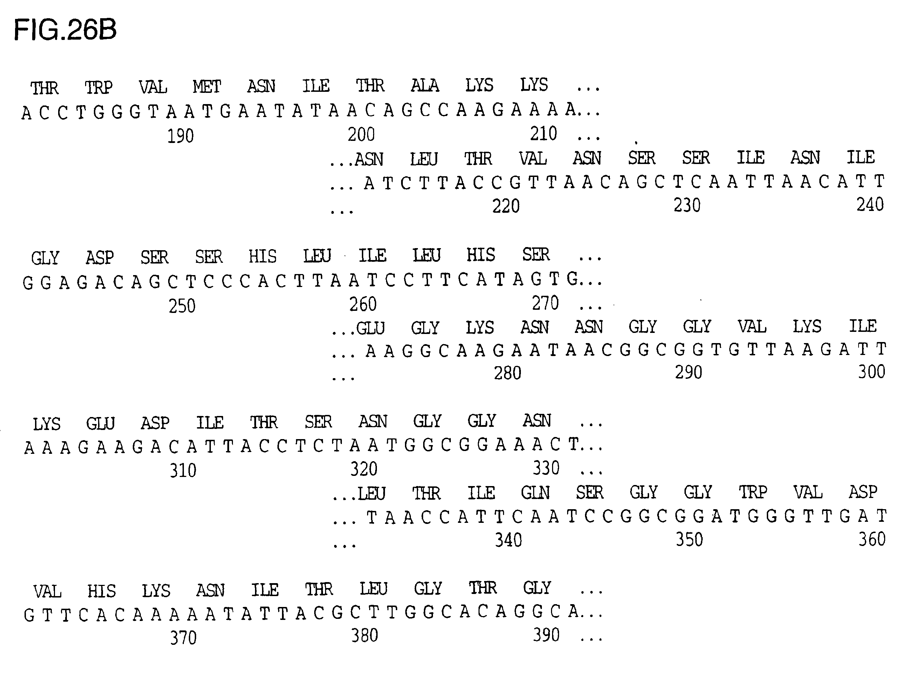

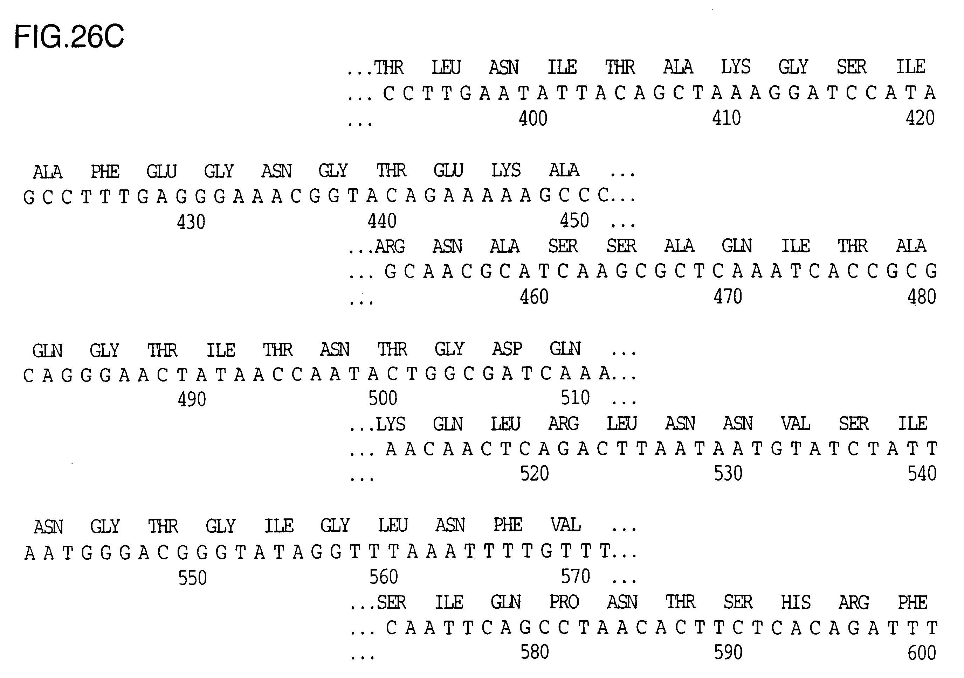

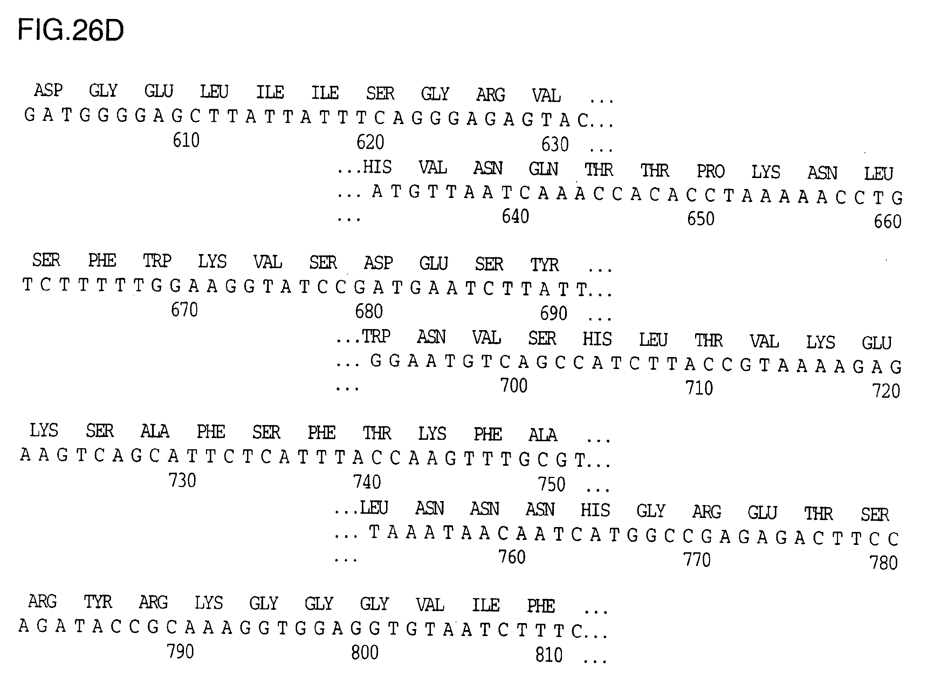

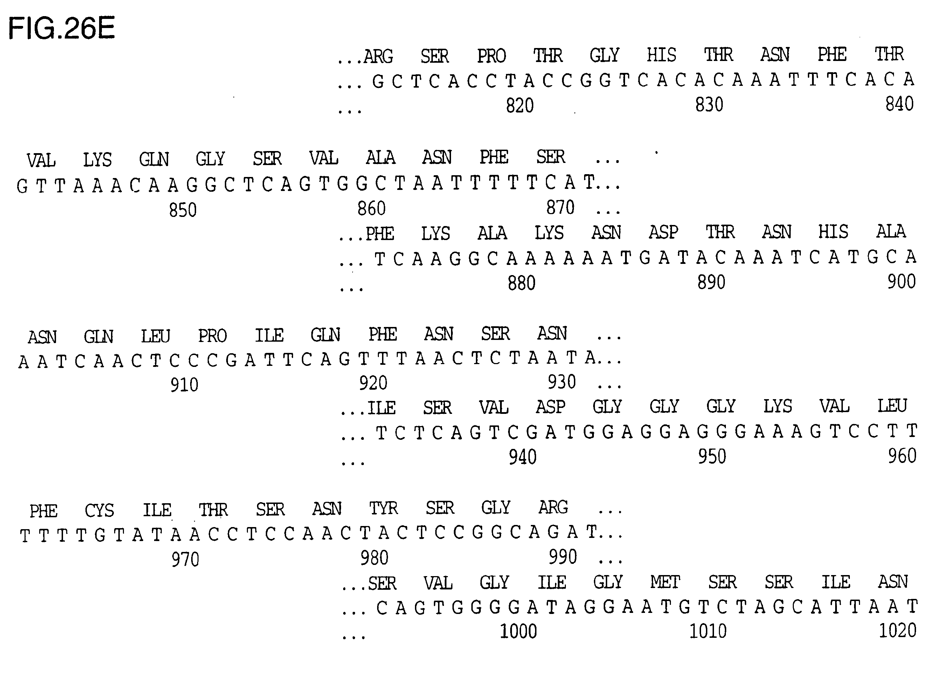

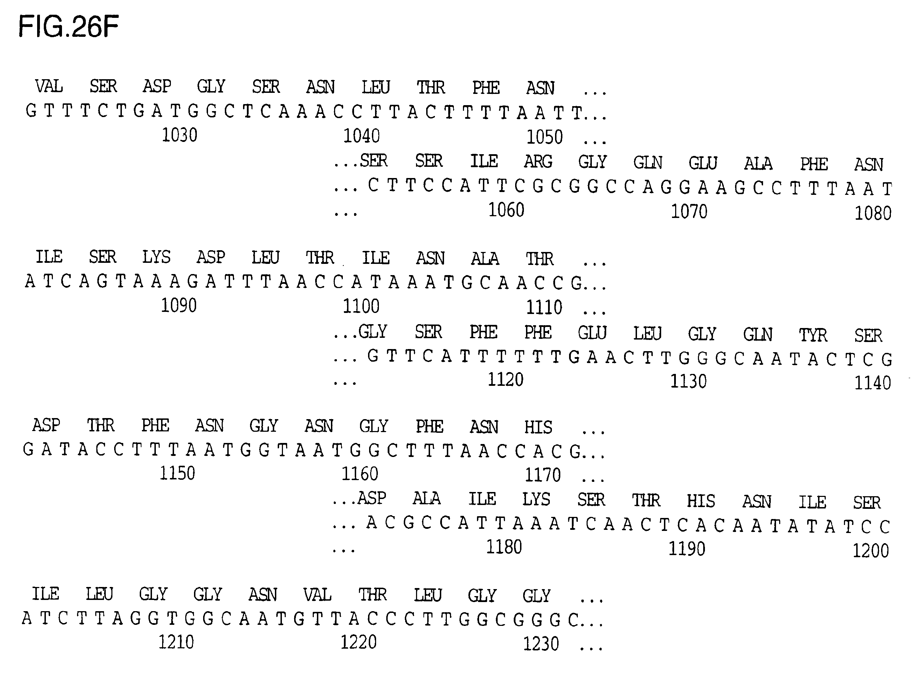

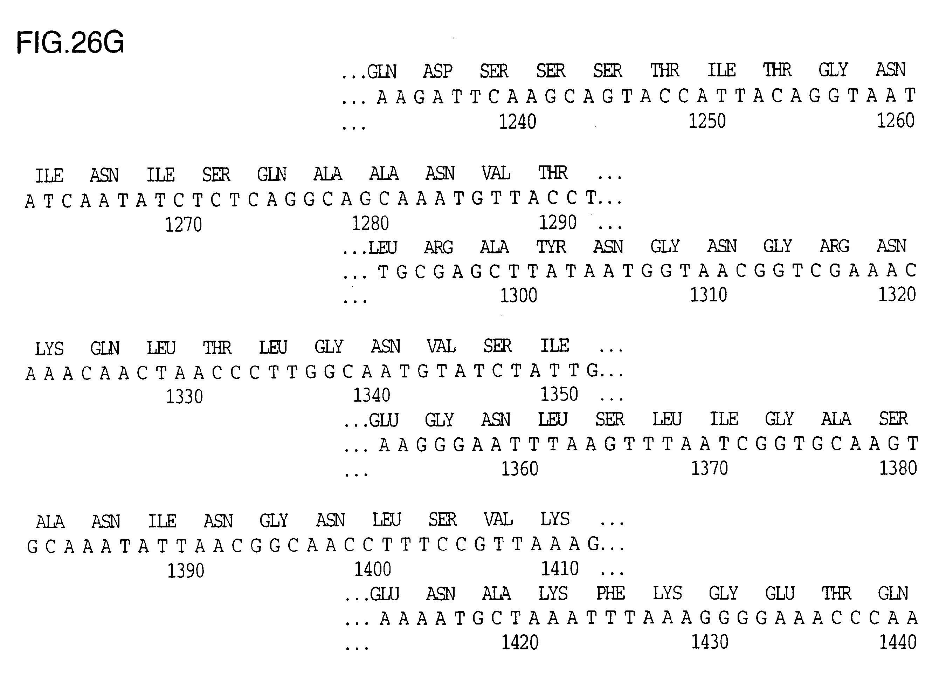

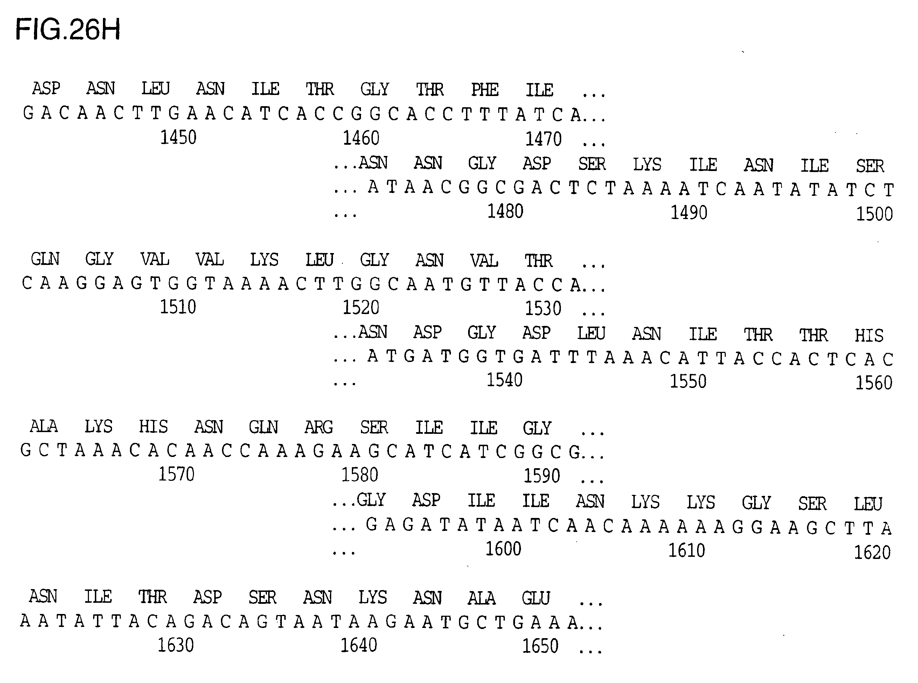

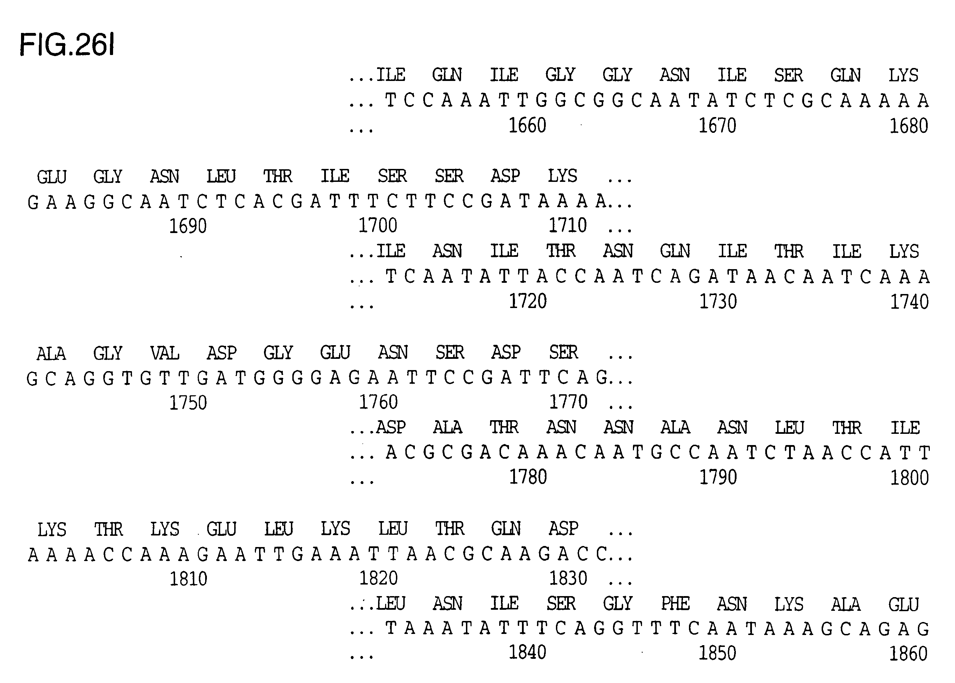

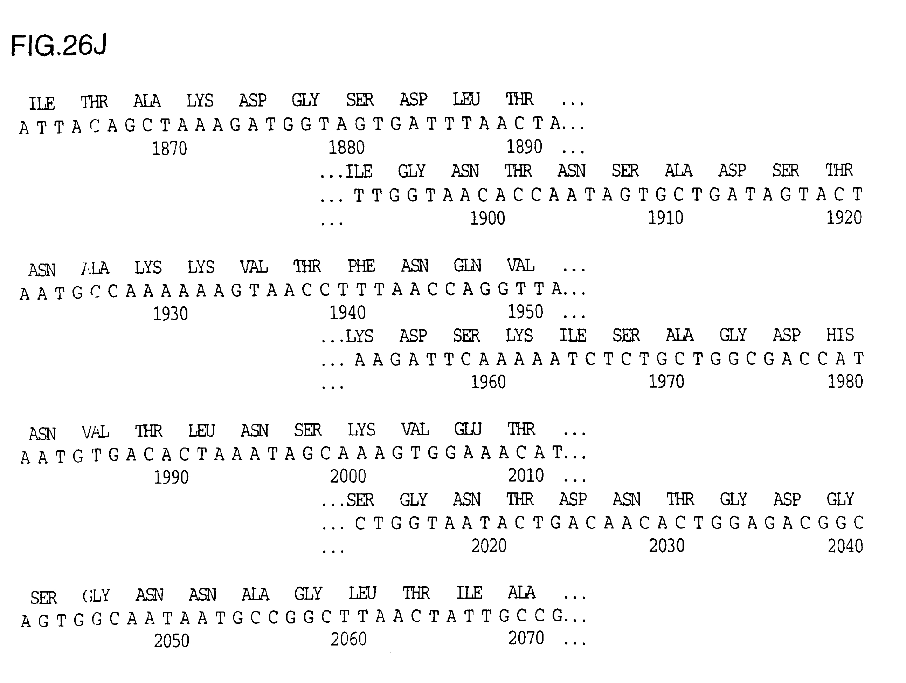

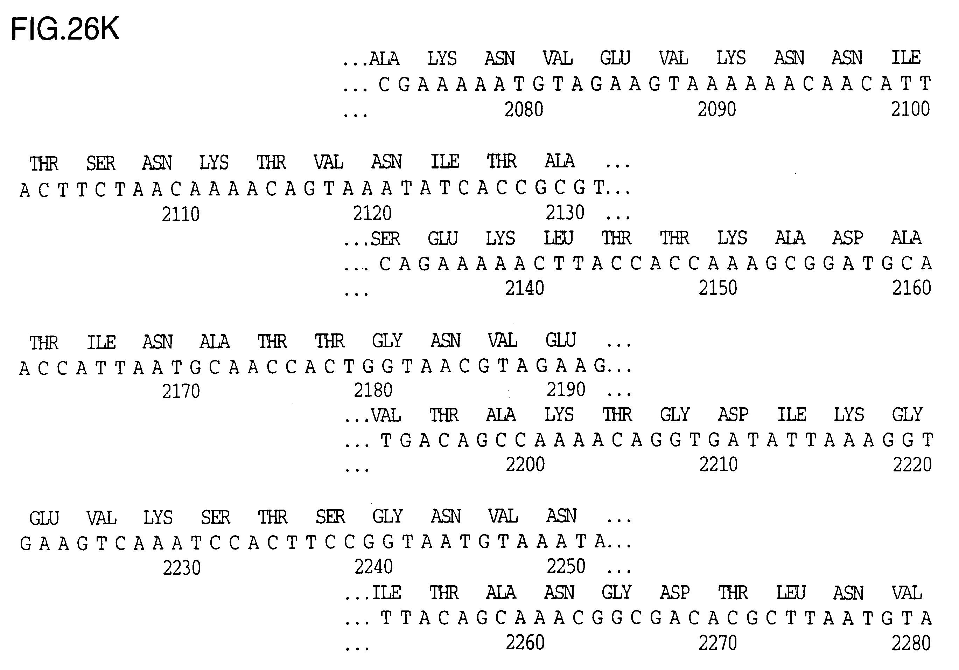

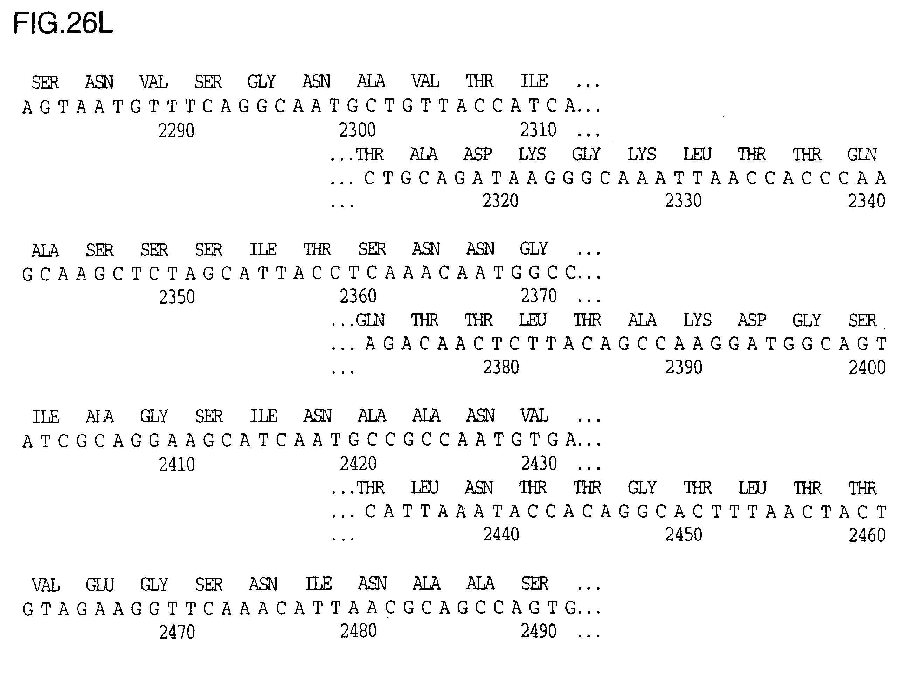

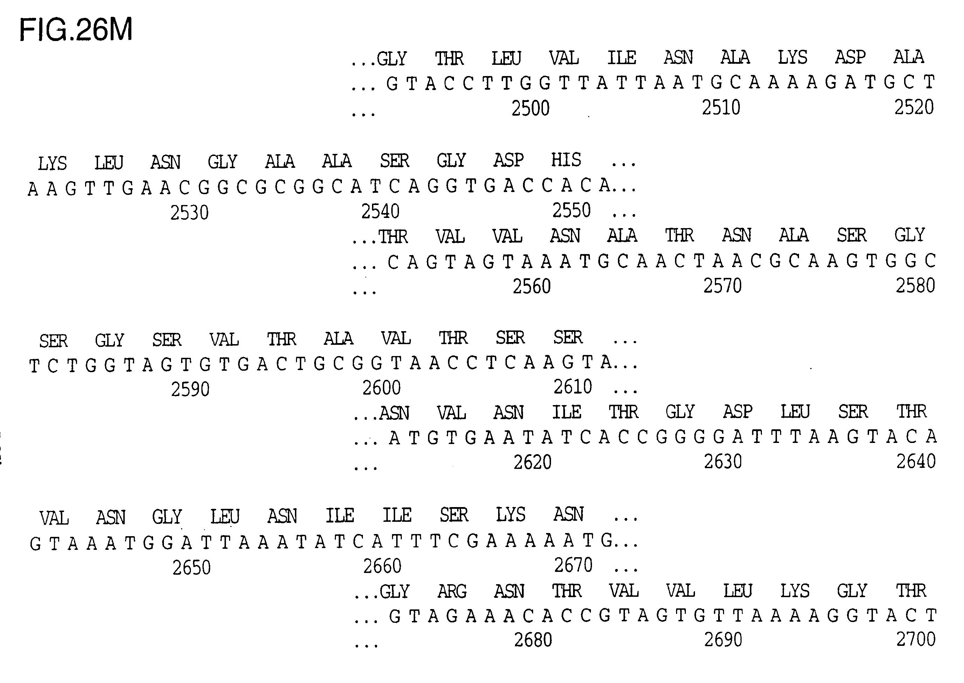

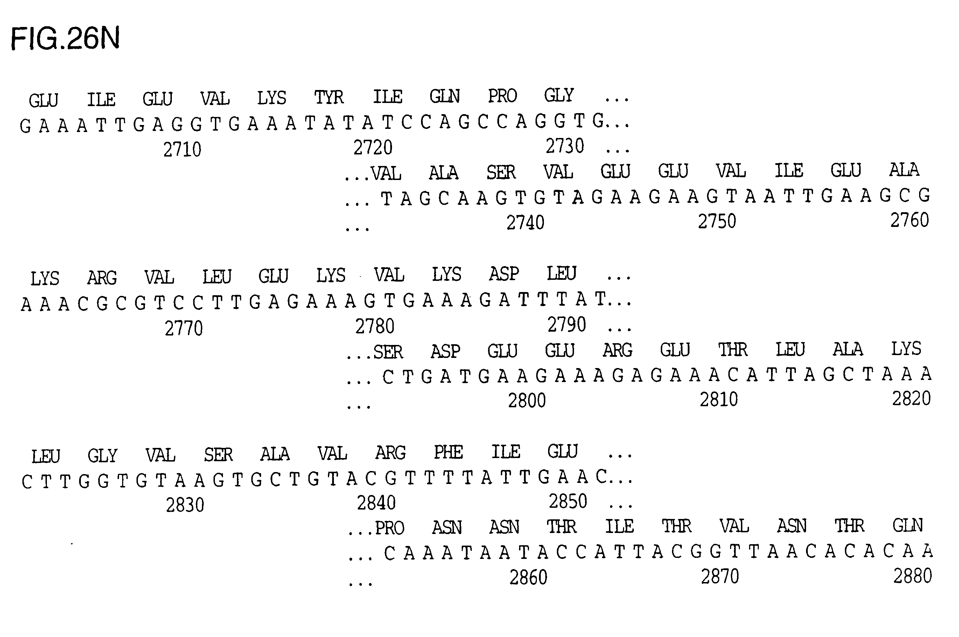

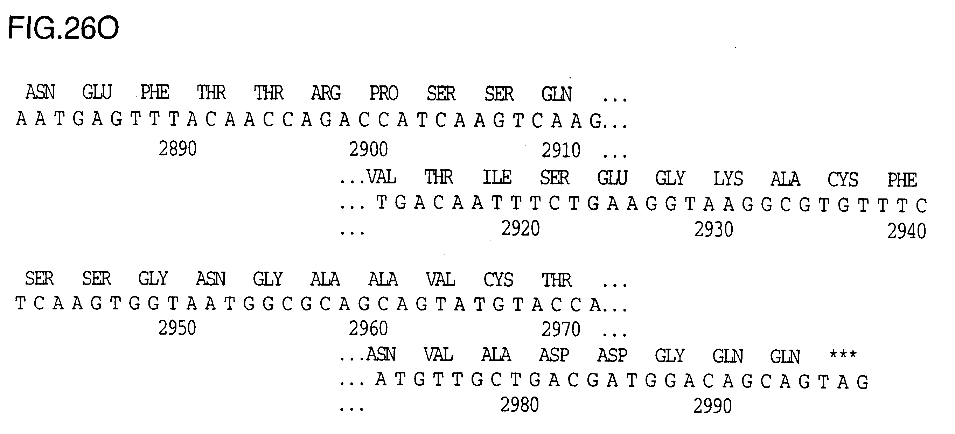

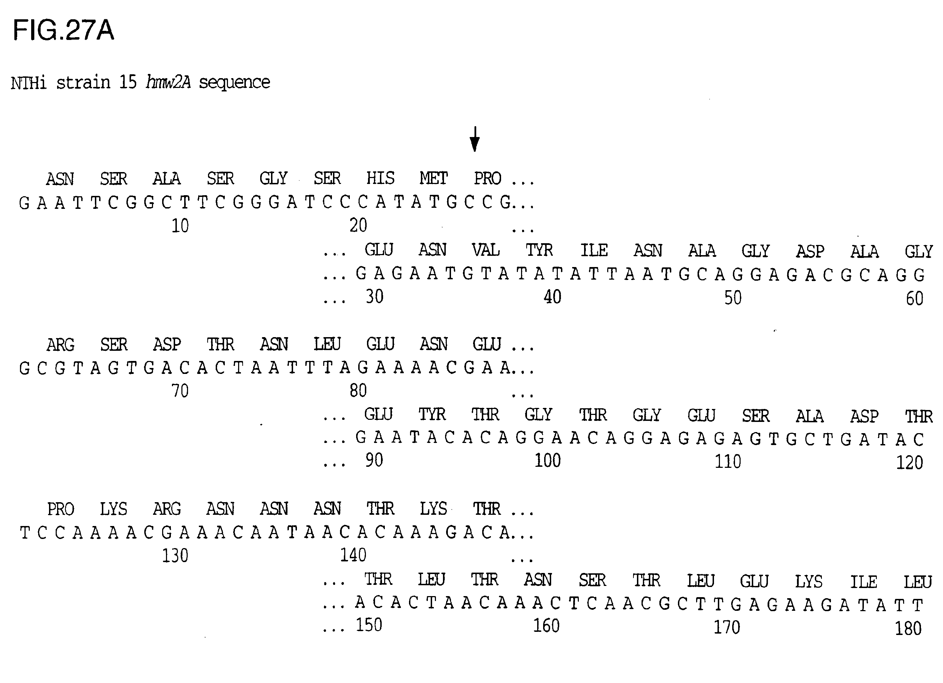

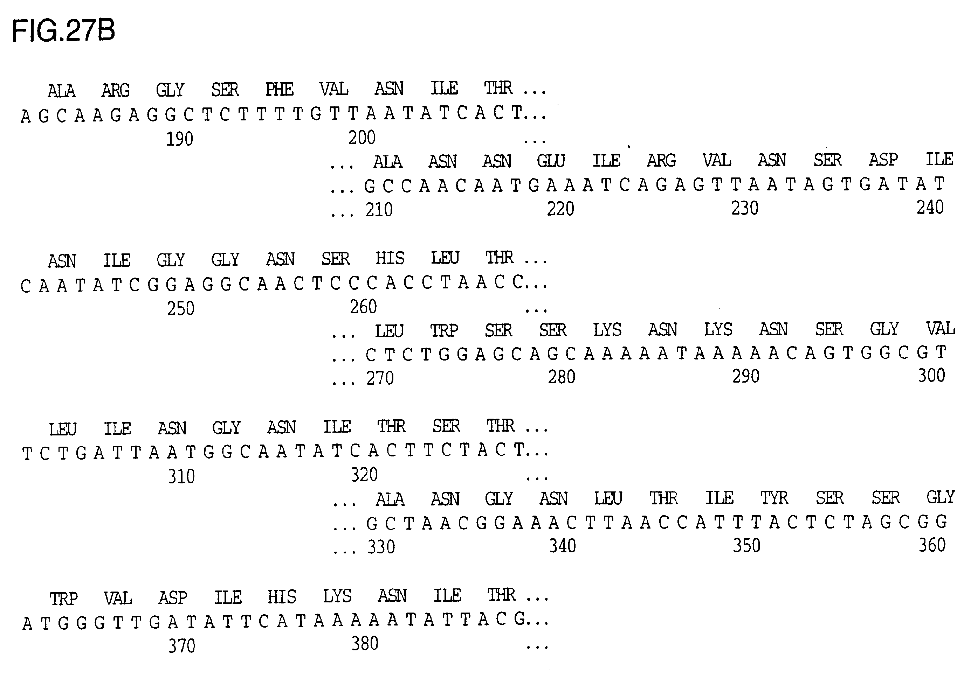

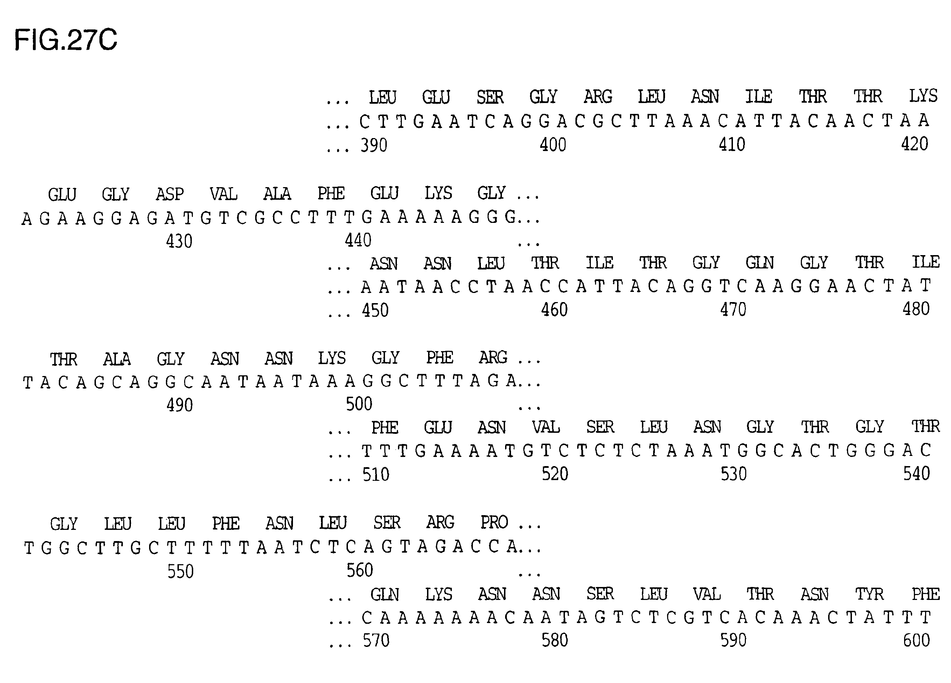

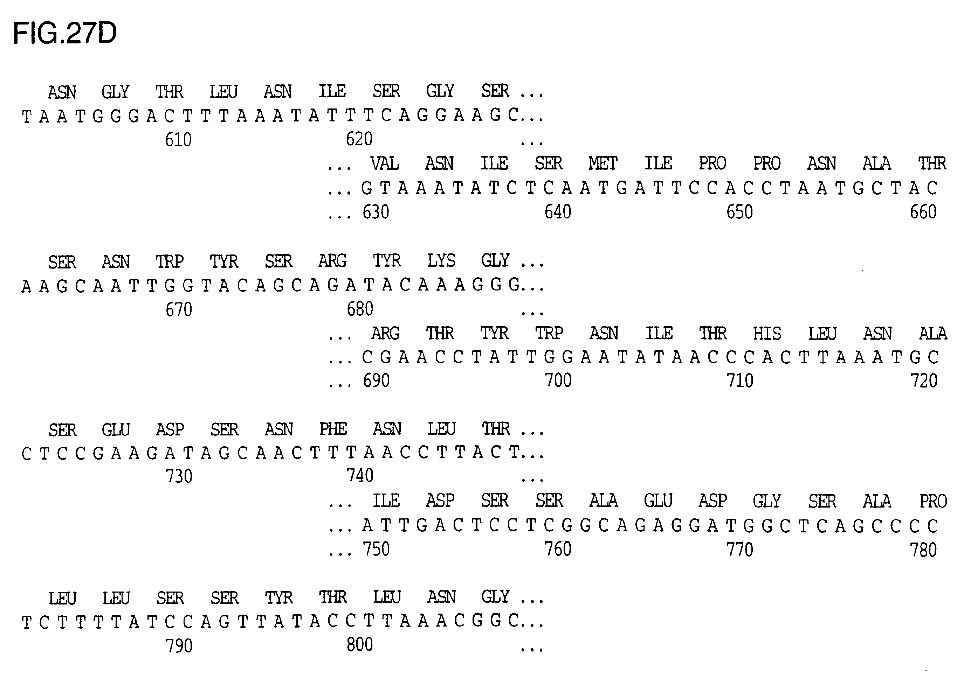

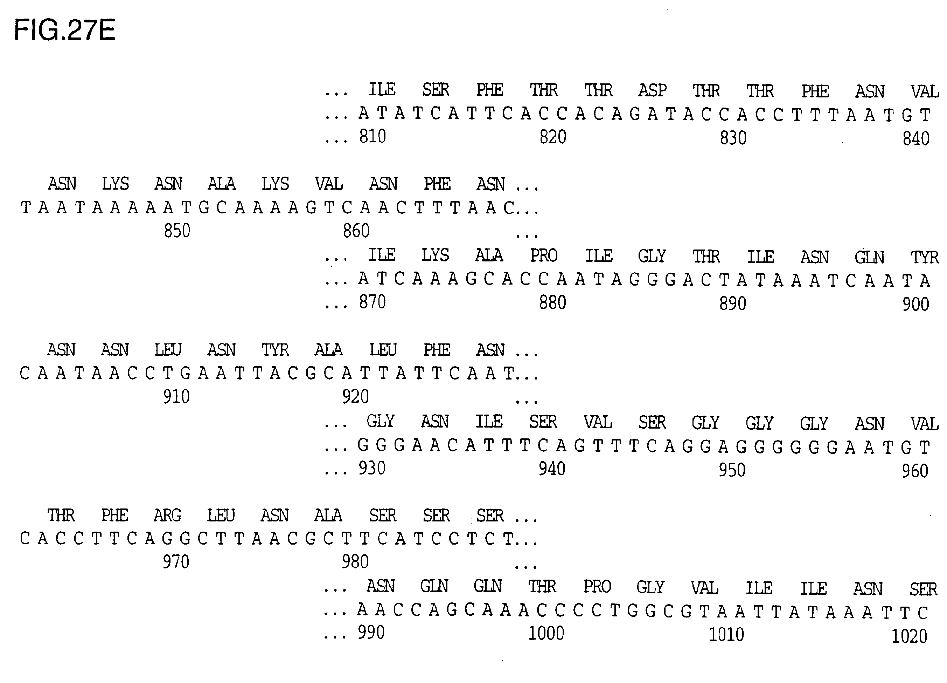

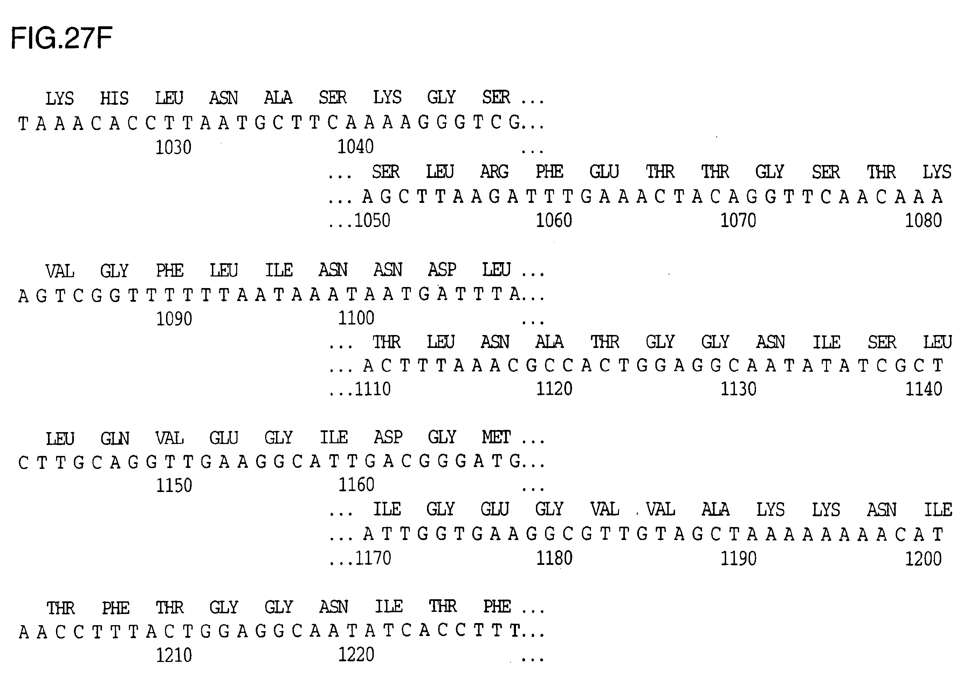

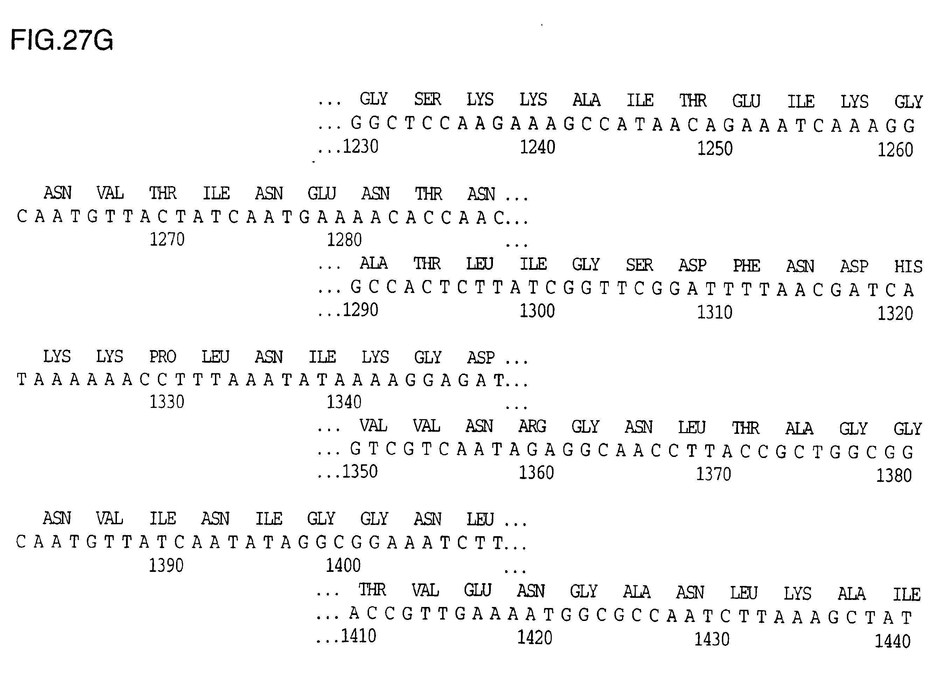

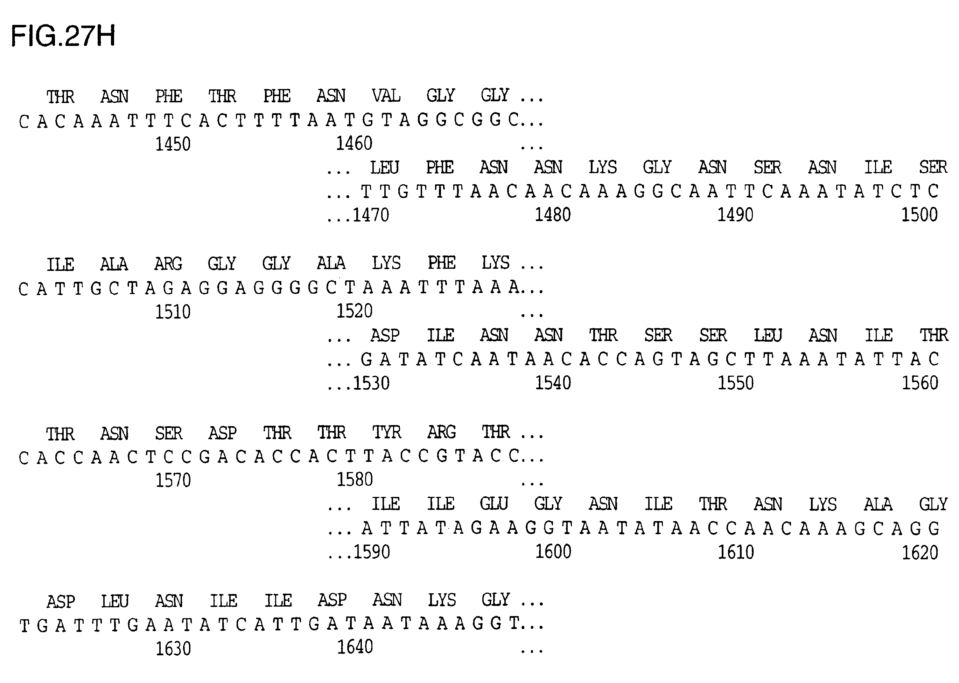

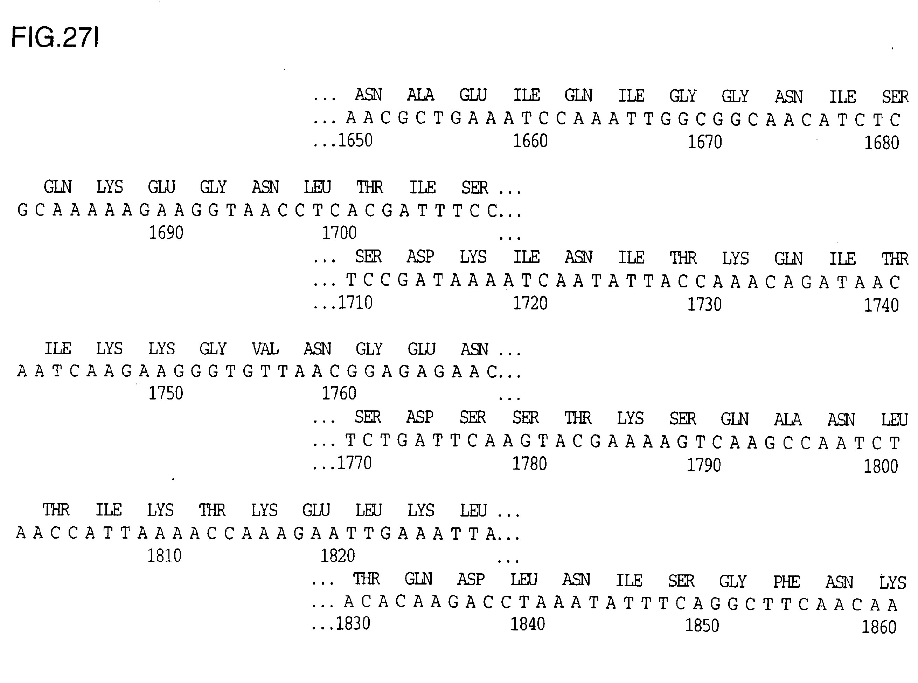

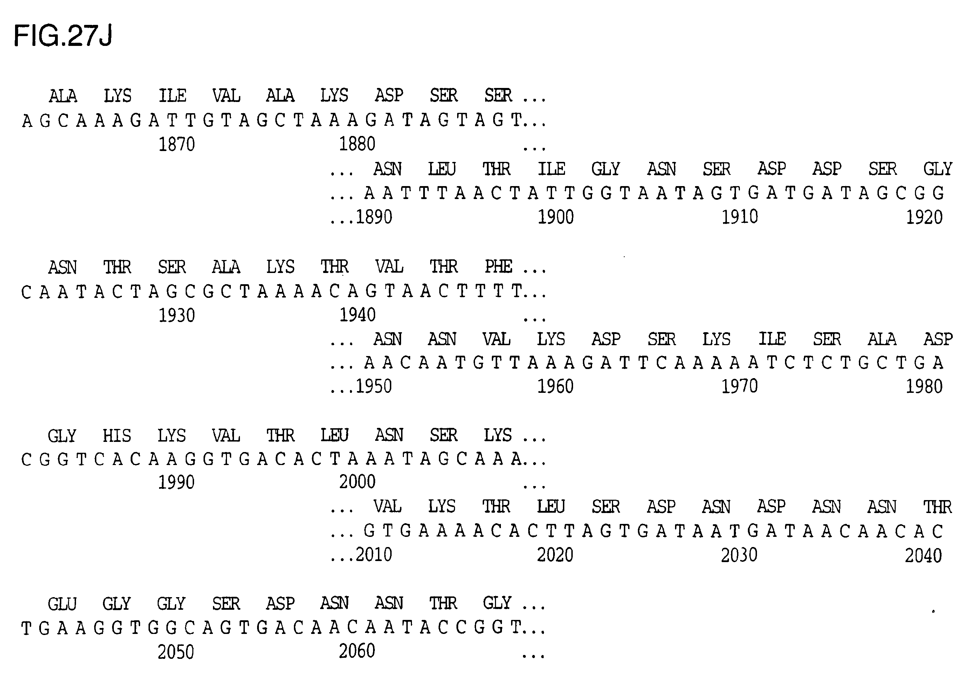

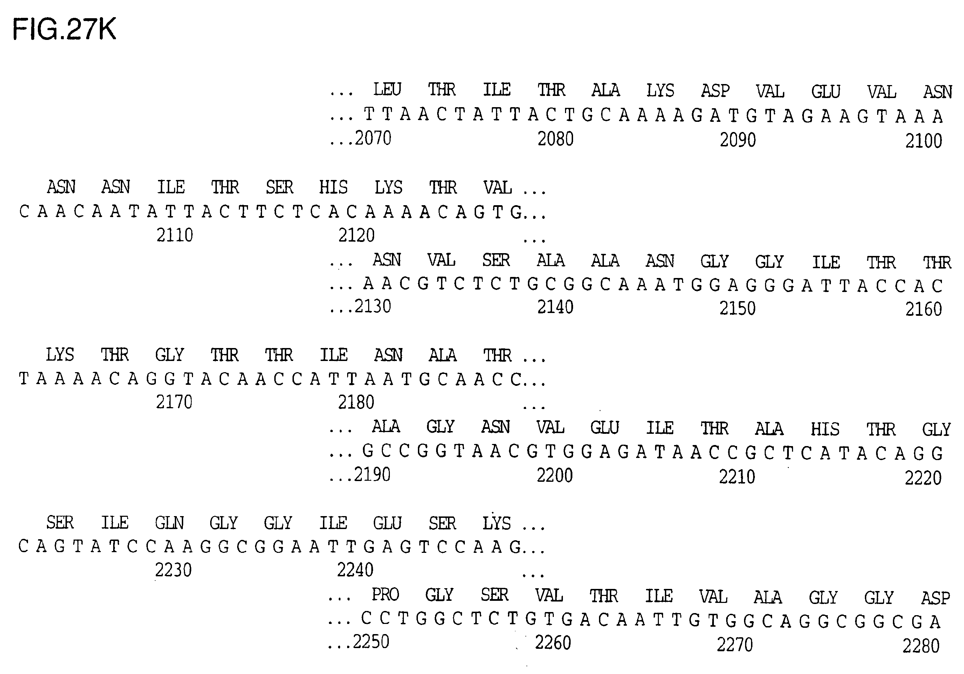

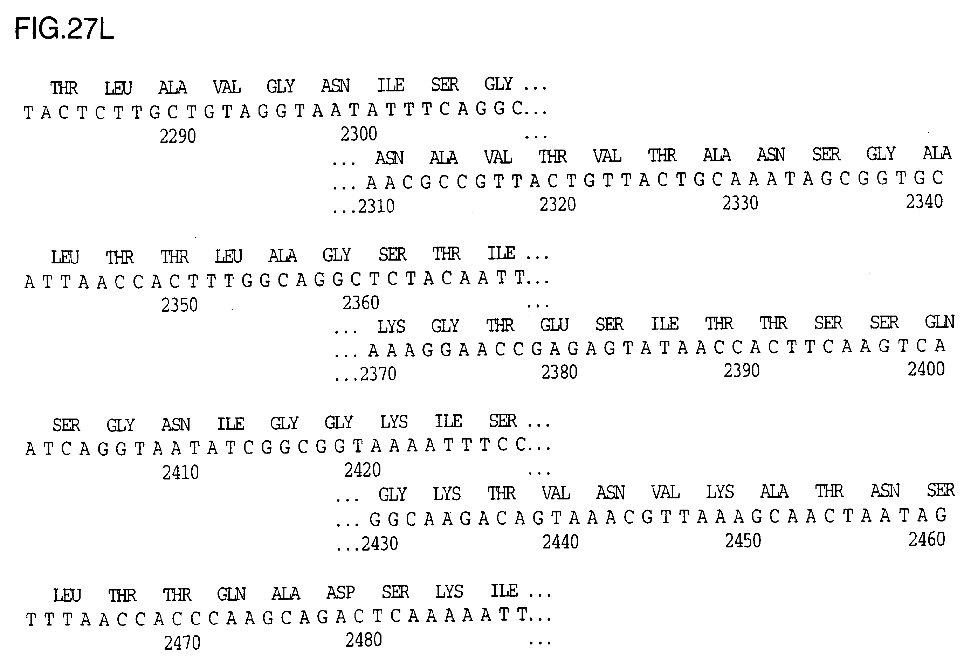

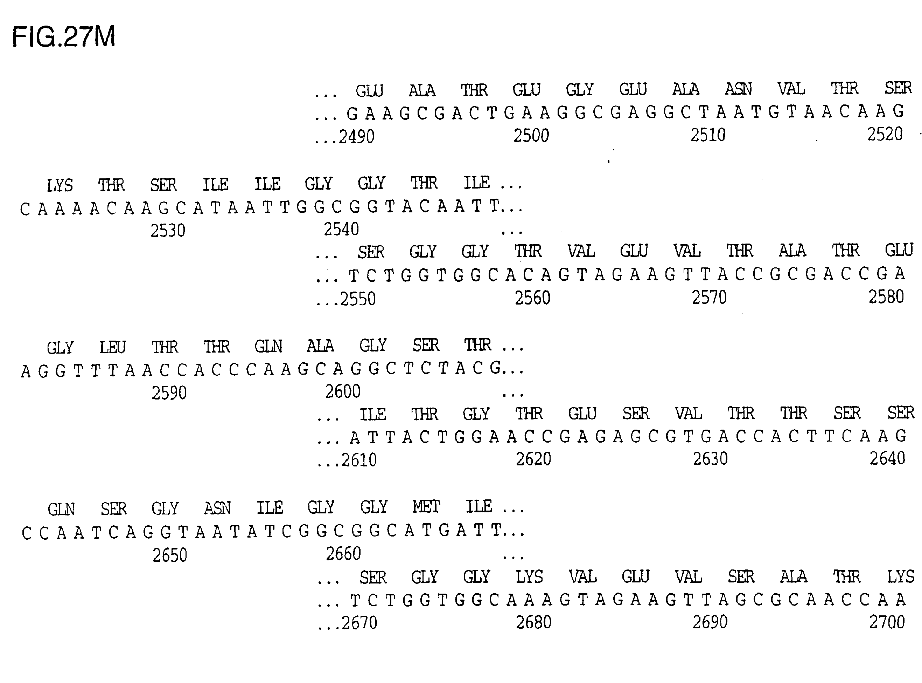

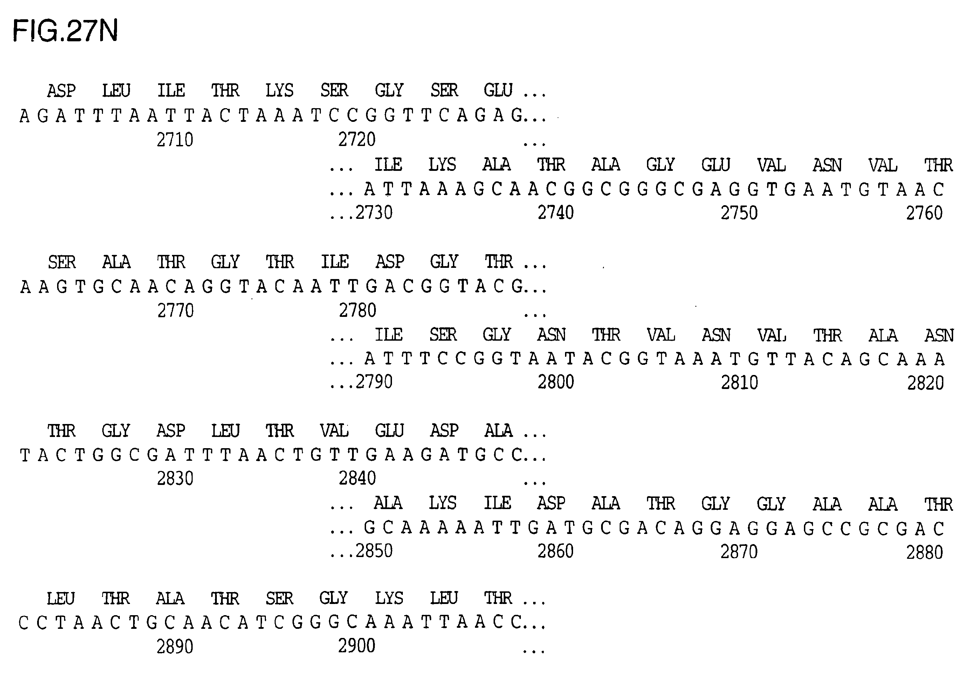

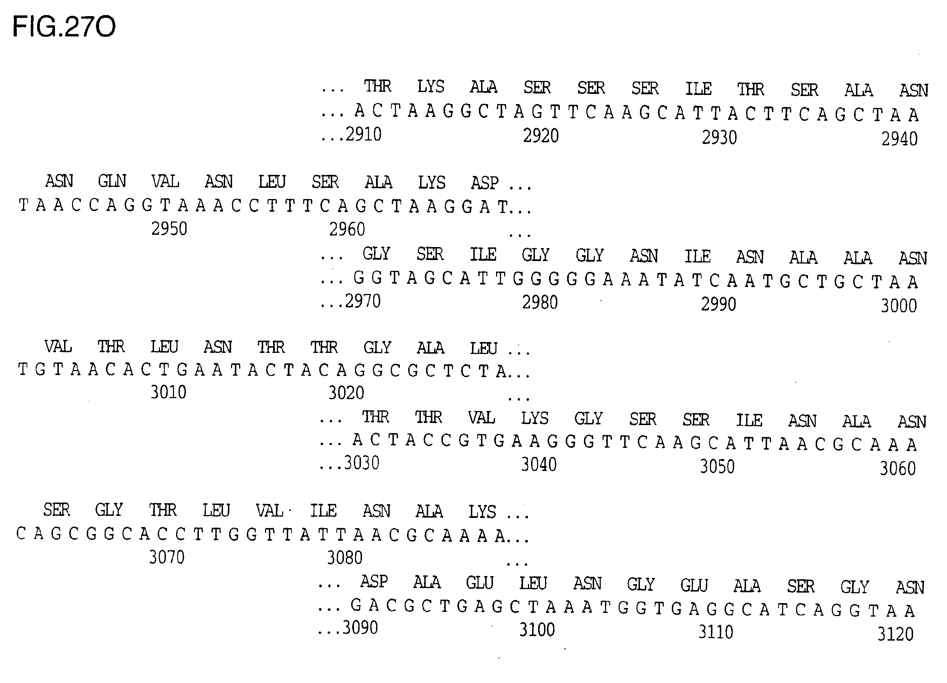

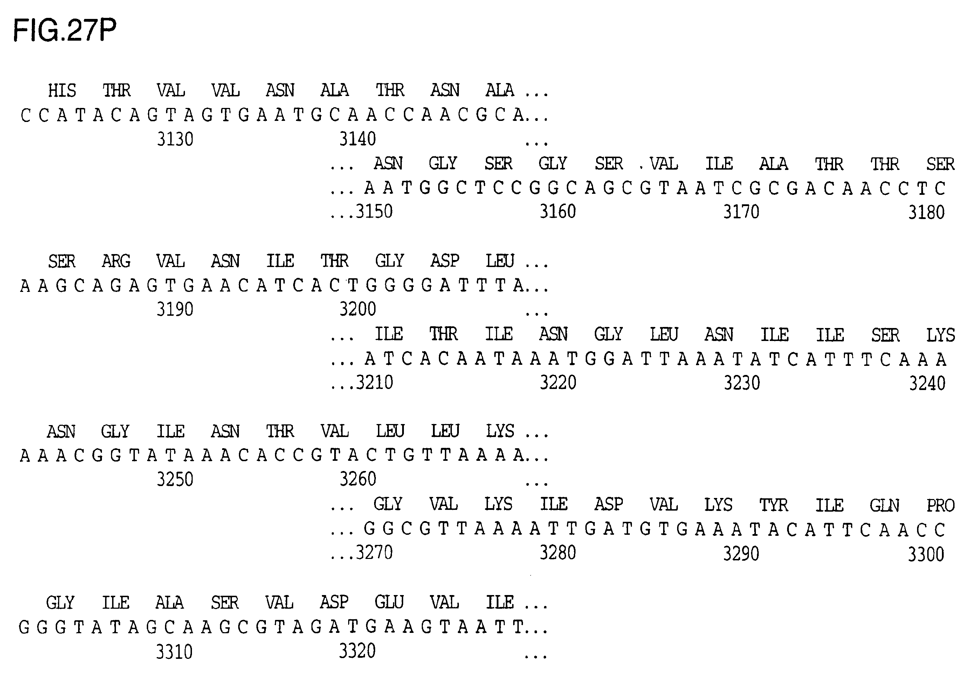

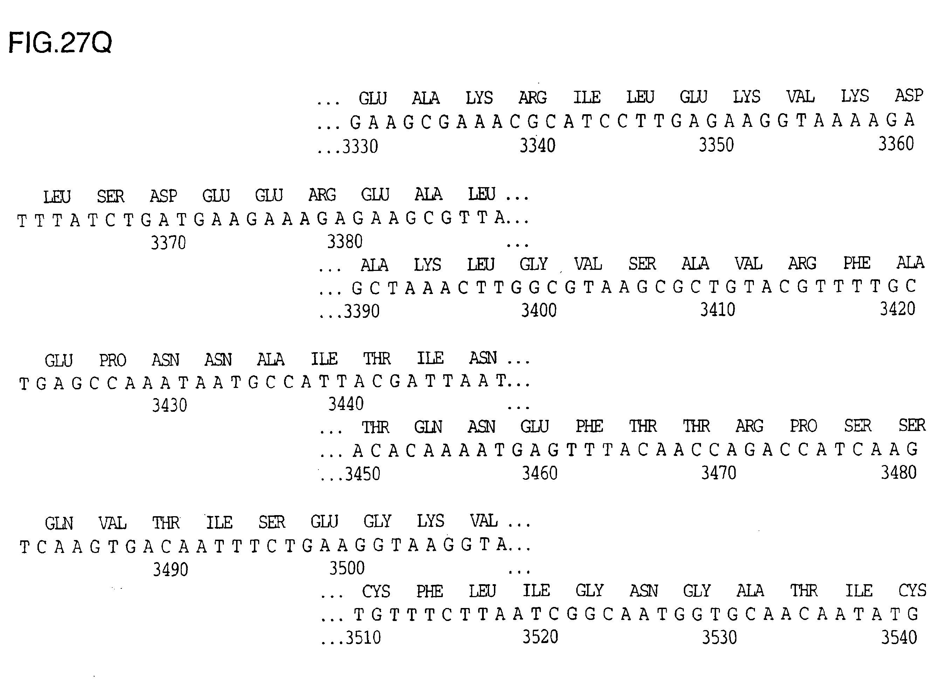

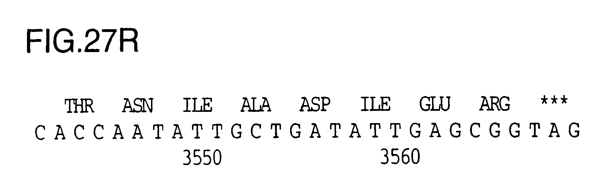

The nucleotide (SEQ ID NO: 58) and deduced amino acid (SEQ ID NO: 59) sequences of the *hmw1A* gene from strain 15 are shown in Figure 26. The predicted mature HMW1A protein from strain 15 (encoding seqeunce SEQ ID NO: 60, amino acid sequence SEQ ID NO: 61) has a molecular weight of 103.5 kDa and a pI of 8.06. There are no RGD motifs found in strain 15 HMW1A. The nucleotide (SEQ ID NO: 62) and deduced amino acid (SEQ ID NO: 63) sequences of the *hmw2A* gene from strain 15 are shown in Figure 27. The predicted mature HMW2A protein from strain 15 (encoding sequence SEQ ID NO: 64, amino acid sequence SEQ ID NO: 65) has a molecular weight of 121.9 kDa and a pI of 8.22. There are no RGD motifs in strain 15 HMW2A.

The nucleotide (SEQ ID NOS: 66, 70) and deduced amino acid sequence (SEQ ID NOS: 67, 71) for the *hmw1A* and *himw2A* genes, from strain 12, as contained in USP 5,603,938, are shown in Figures 28 and 29 respectively.

An alignment of the deduced HMW1A and HMW2A protein sequences with the published HMW1A and HMW2A protein sequences from strain 12 (SEQ ID NOS: 67, 71) is shown in Figure 30. The cleavage site for the mature proteins is shown by the arrow. Regions of similarity can be identified especially between residues about 980 to 1168 and, at the carboxyl terminal, from about residue 1360 to the end. There appear to be repeats in some proteins inserted around residue 1219, most notably in Joyc HMW1A and K1 HMW1A, that appear to have two tandem inserted repeats, while K21 HMW1A and LCDC2 HMW2A contain single copies of the repeat. Strain 15 HMW2A contains a different repeat segment located in the same area. There is a short segment of semi-conserved sequence inserted at residue 583 that is found in all of the HMW2A proteins, except strain 15 HMW2A. However, it is found in the strain 15 HMW1A protein.

### Example 17

This Example illustrates the PCR amplification used to determine whether *hmw1A* or *hmw2A* genes had been amplified.

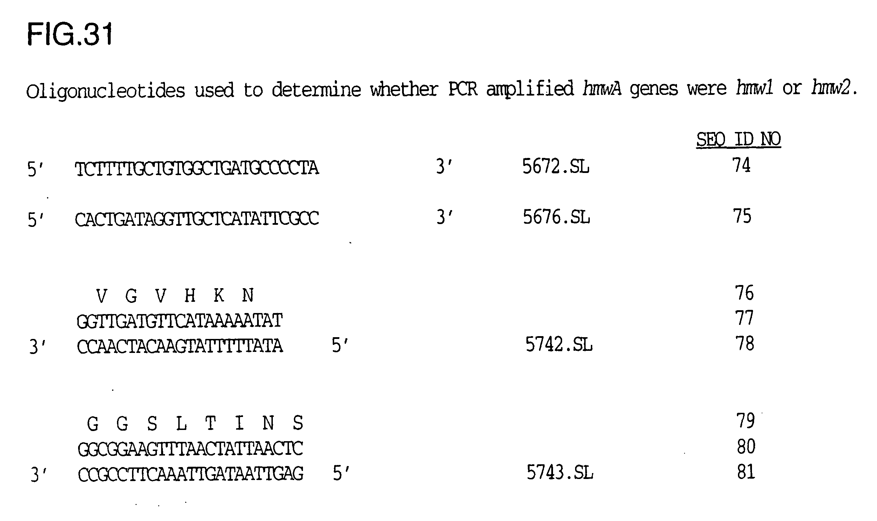

The *hmwA* genes were PCR amplified using primers based upon sequences conserved between *hmw1* and *hmw2* operons and thus amplified genes could be either *hmw1* or *hmw2*. Although the *hmw* genes do not occur in encapsulated strains, the 5'- and 3'-flanking sequences can be found in the genomic sequence of *H. influenzae* strain Rd (ref. 16). Oligonucleotide sense primers were generated based upon the *5'-hmw1* flanking sequence from strain Rd gene HI1679 (primer 5672.SL, SEQ ID NO: 74) and the *5'-hmw2* flanking region from strain Rd gene HI1598 (primer 5676.SL (SEQ ID NO: 75)). Antisense primers were generated based upon internal sequences of the amplified *hmwA* genes. The oligonucleotide primers are shown in Figure 31. Primer 5742.SL (SEQ ID NO: 78) was used to amplify *hmwA* genes from strains K1, K21, PMH1 and 15, while primer 5743.SL (SEQ ID NO: 81) was used to PCR *hmwA* genes from strains Joyc and LCDC2. Amplified fragments were directly sequenced using the *hmwA*-specific primers (5742.SL and 5743.SL) and the sequence compared to the sequence of the genes cloned in Example 16. After the PCR amplified *hmwA* genes were identified as *hmw1a* or *hmw2A,* specific PCR primers were used to PCR amplify a second copy of the gene with a start codon engineered at the start of the mature protein. A representative pair of PCR primers, used to amplify the LCDC2 *hmw2A* gene for expression, are illustrated in Figure 33B (5972.SL, SEQ ID NO: 92; 5973. SL, SEQ ID NO: 95).

### Example 18

This Example illustrates the construction of a generic plasmid for expression of *hmwABC* genes in *E. coli*.

As shown in Example 16, the *hmw1A* and *hmw2A* genes can be PCR amplified from any *hmw*-containing strain of non-typeable *H. influenzae*, but to produce protective recombinant antigen, they must be expressed in the presence of *hmwBC* genes. A generic expression plasmid was constructed that contains the T7 promoter, strain 12 *hmw1BC* genes, the *E. coli* cer gene, a kanamycin resistance gene, and a cloning site to insert any *hmwA* gene.

Plasmid BK-76-1-1 (Figure 10, Example 10) was digested with *EcoR* I and re-ligated to generate plasmid JB-2581-2-1, which has the 2 kb *EcoR* I fragment containing the 3'-end of *hmw1A* and the 5'-end of *hmw*1B deleted (Figure 32A). The 2 kb *EcoR* I fragment from BK-76-1-1 was subcloned into pUC-BgXb for further manipulation, creating plasmid JB-2581-1-1. Figure 32B shows the oligonucleotide primers used to amplify the 3'-end of *hmw*1A (5947.SL, SEQ ID NO: 83; 5948.SL, SEQ ID NO: 86) and the 5'-end of *hmw*1B (5949.SL, SEQ ID NO: 87; 5950.SL, SEQ ID NO: 90), introducing a *Xba* I site at the junction of the two genes. Plasmid JB-2603-1-1 contains a 1.5 kb *EcoR* I-*Xba* I 3'-fragment of the *hmw*1A gene and plasmid JB-2603-2-1 contains the approximately 550 bp *Xba* I-*EcoR* I fragment of the *hmw A-B* intergenic sequence and 5'-end of *hmw*1B*.* Plasmid JB-2581-2-1 was linearized with *EcoR* I, dephosphorylated, and ligated with the *EcoR* I-*Xba* I inserts from JB-2603-1-1 and JB-2603-2-1, generating plasmid JB-2641-1. This plasmid is identical to BK-76-1-1, except that it contains an extra *Xba* I site between the *hmw1A* and *hmw1B* genes. Plasmid JB-2641-1 was digested with *Xba* I which deleted the complete *hmw1A* gene, but left the *hmw1 BC* genes intact. Re-ligation of the vector fragment generated plasmid JB-2646-1 that is the generic expression vector into which *hmwA* genes can be cloned at the *Xba* I site (Figure 32A).

To demonstrate the utility of the generic expression vector, a chimeric *T7 hmwABC* gene cassette was generated containing the LCDC2 *hmw2A* gene combined with the strain 12 *hmw1BC* genes. The LCDC2 *hmw2A* gene was PCR amplified using the primers illustrated in Figure 33B and cloned into pCR II, generating plasmid BK-137-3-10, that contains the *hmw2A* gene in an anticlockwise orientation. In order to change the orientation of the *hmw2A* gene for cloning purposes, the plasmid was digested with *BamH* I to release the *hmw2A* insert, then both fragments re-ligated. Plasmid BK-177-3-2 contains the LCDC2 *hmw2A* gene in a clockwise orientation. Plasmid BK-177-3-2 was digested with *Nde* I and *Xho* I and the *hmw2A* fragment was ligated into pT7-7 that had been digested with *Nde* I and *Sal* I, to generate plasmid BK-189-2-5. The generic expression plasmid JB-2646-1 (Figure 32A) was linearized with *Xba* I and dephosphorylated. Plasmid BK-189-2-5 was digested with *Xba* I that released the *hmw2A* gene ready to be inserted into the expression vector. Plasmid DS2334-5 thus contains a *T7 hmwABC* gene cassette comprised of the *hmw2A* gene from LCDC2 and the *hmw1BC* genes from strain 12.

### Example 19

This Example illustrates the construction of plasmid DS-2400-13, that contains a T7 hmwA/T7 *hmwABC* cassette, the *E. coli cer* gene, and a kanamycin resistance gene.

Plasmid DS-1843-2 is a tetracycline resistant pBR328-based vector containing a multiple cloning site inserted between the *EcoR* I and *Pst* I sites. DS-1843-2 was linearized with *Xho* I and dephosphorylated and the kanamycin resistance gene from pUC-4K was inserted on a *Sal* I fragment, generating plasmid DS-2372-31 that is both tetracycline and kanamycin resistant. Plasmid DS-2372-31 was linearized with *Bgl* II and dephosphorylated, and the synthetic *E. coli cer* gene from DS-2224-1-4 was inserted on a *BamH* I fragment, generating plasmid DS-2379-2-6. Plasmid DS-1046-1-1 (Figure 3A, Example 3) was digested with *Bgl* II and *Sal* I and the T7 *hmwABC* gene fragment was inserted into DS-2379-2-6 that had been digested with *BamH* I and *Sal* I. The resulting plasmid (DS-2391-1) is a pBR-based kanamycin resistant and tetracycline sensitive vector containing the T7 *hmwABC* genes and the *E. coli cer* gene. JB-2369-6 (Figure 7, Example 7) was digested with *BamH* I to release an internal 3' *hmwA*/*T7 5' hmwA* fragment that was inserted into the unique *BamH* I site of the *pBR T7 hmwABC*/*cer*/*kanR* vector. The resulting *pBR T7 hmwA*/*T7 hmwABC*/*cer*/*kanR* plasmid (DS-2400-13) thus contains multiple *hmwA* genes (see Figure 34).

### SUMMARY OF THE DISCLOSURE

In summary of this disclosure, the present invention provides nucleic acid molecules and constructs incorporating the same which permit the recombinant production of high molecular weight proteins of non-typeable *Haemophilus influenzae* which are protective. Modifications are possible within the scope of the invention.

**Table 1. Immunogenicity of various forms of HMW1 and HMW2 in chinchillas.**

| HMW preparations | Anti-HMW antibody titers Log2 (Titers/100) |
|---|---|
| HMW1/HMW2 native | 7.11 ± 0.78 |
| | 7.75 ± 0.66 |
| HMW1 abc | 9.67 ± 1.12 |
| | 10.78 ± 0.83 |
| HMW1 a/abc | 8.44 ± 0.88 |
| | |
| HMW1 abc/cer | 7.11 ± 0.93 |
| | 7.44 ± 0.88 |
| HMW1 abΔ | 1.00 ± 0.50 |
| | 2.17 ± 1.67 |
| HMW1 2xa | 12.29 ± 0.49 |
| | |
| HMW2 abc | 9.22 ± 1.48 |
| | 11.44 ± 0.78 |
| HMW2 2xa | 12.89 ± 0.78 |
| | |
| alum | < 0.05 |
| | < 0.05 |

Groups of 9 chinchillas were immunized (i.m.) on days 1, 14 and 28 with 30 µg of the indicated antigens adsorbed to alum. Blood samples were collected on day 42. The reactive titer of an antiserum was defined as the reciprocal of the dilution consistently showing a two fold increase in absorbance over that obtained with the prebleed serum sample. Two sets of numbers indicate two sets of experiments.

**Table 2. Protective abilities of various forms of HMW1 and HMW2 against NP colonization with NTHi strain 12 in chinchillas.**

| HMW preparations | # of infected animals/ # of total animals challenged (%) | median cfu/25 µl nasal lavage |
|---|---|---|
| HMW1/HMW2 native + alum | 2 / 9 (22.2 %) | 11* |
| alum | 7 / 9 (77.8 %) | 800 |
| HMWlabc + alum | 2 / 9 (22.2 %) | 6* |
| alum | 7 / 9 (77.8 %) | 800 |
| HMW1 a/abc + alum | 2 / 9 (22.2 %) | 20* |
| alum | 6 / 9 (66.7 %) | 300 |
| HMW1 abc/cer + alum | 3 / 9 (33.3 %) | 60* |
| alum | 7 / 9 (77.8 %) | 1000 |
| HMW1 abΔ + alum | 8 / 9 (89.9 %) | 500 |
| alum | 7 / 8 (87.5 %) | 1270 |
| HMW1 2xa + alum | 5 / 7 (71.5 %) | 400 |
| alum | 7 / 9 (77.8 %) | 630 |
| HMW2 abc + alum | 2 / 9 22.2 %) | 7* |
| HMW2 2xa + alum | 7 / 9 (77.8 %) | 800 |
| alum | 7 / 9 (77.8 %) | 1000 |

Groups of 9 chinchillas were immunized (i.m.) on days 1, 14 and 28 with 30 µg of indicated antigens adsorbed to alum. Blood samples were collected on day 42. On day 44, animals were challenged by intranasal inoculations with freshly cultured streptomycin-resistant NTHi strain 12. The dose of bacterial challenge was 1x 10⁸ cfu per animal. Nasopharyngeal lavages were performed 4 days post inoculation and 25 µl of the nasal lavage were plated on chocolate agar plates.

An animal was defined as infected if >50 cfu of bacteria were recovered from 25 µl nasal lavage fluid. * Statistical significance was found when compared to the control animals by Mann-Whitney Rank Sum Test (p <0.05).

**TABLE 3**

| Molecular weights of Mature HMW Proteins from Various *H*. *influenzae* non-typeable Strains | | | | | | | |
|---|---|---|---|---|---|---|---|
| Molecular Weight (kDa) | | Non-typeable *H. influenzae* Strain | | | | | |
| | | 12 | JoyC | K21 | LCDC2 | PMH1 | 15 |
| | | | | | | | |
| Mature Protein: | HMW1 | 125 | 125.9 | 104.4 | 114.0 | 102.4 | 103.5 |
| | HMW2 | 120 | 100.9 | | 111.7 | 103.9 | 121.9 |

### REFERENCES

1. Berkowitz et al. 1987. J. Pediatr. 110:509.
2. Claesson et al. 1989. J. Pediatr. 114:97.
3. Black, S.B., H.R. Shinefield, B. Fireman, R. Hiatt, M. Polen, E. Vittinghoff, The Northern California Kaiser Permanent Vaccine Study Center Pediatrics Group. Efficacy in infancy of oligosaccharide conjugate Haemophilus influenzae type b (HbOC) vaccine in a United States population of 61,080 children. 1991. Pediatr. Infect. Dis. J. 10:97-104.
4. Madore, D.V. 1996. Impact of immunization on Haemophilus influenzae type b disease. Infectious Agents and Disease 5:8-20.
5. Bluestone, C.D. 1982. Current concepts in otolaryngology. Otitis media in children: to treat or not to treat? N. Engl. J. Med. 306:1399-1404.
6. Barenkamp, S.J., and F.F. Bodor. 1990. Development of serum bactericidal activity following nontypable Haemophilus influenzae acute otitis media. Pediatr. Infect. Dis. 9:333-339.
7. Barenkamp, S.J., and E. Leininger. 1992. Cloning, expression, and DNA sequence analysis of genes encoding nontypeable Haemophilus influenzae high-molecular-weight surface-exposed proteins related to filamentous hemagglutinin of Bordetella pertussis. Infect. Immun. 60:1302-1313.
8. Barenkamp, S.J., and J.W. St. Geme III. 1994. Genes encoding high-molecular-weight adhesion proteins of nontypeable Haemophilus influenzae are part of gene clusters. Infect. Immun. 62:3320-3328.
9. St. Geme III, J.W. and S. Grass. 1998. Secretion of the Haemophilus influenzae HMW1 and HMW2 adhesins involves a periplasmic intermediate and requires the HMWB and HMWC proteins. Molec. Microbiol. 27:617-630.
10. St. Geme III, J.W., S. Falkow, and S.J. Barenkamp. 1993. High-molecular-weight proteins of nontypeable Haemophilus influenzae mediate attachment to human epithelial cells. Proc. Natl. Acad. Sci. USA 90:2875-2879.
11. Barenkamp, S.J. 1996. Immunization with high-molecular-weight adhesion proteins of nontypeable Haemophilus influenzae modifies experimental otitis media in chinchillas. Infect. Immun. 64:1246-1251.
12. Tabor, S., and C.C. Richardson. 1985. A bacteriophage T7 RNA polymerase/promoter system for controlled exclusive expression of specific genes. Proc. Natl. Acad. Sci. USA 82:1074-1078.
13. Patient, M.E., and D.K. Summers. 1993. ColE1 multimer formation triggers inhibition of Escherichia coli cell division. Molec. Microbiol. 9:1089-1095.
14. Barenkamp, S. 1986. Protection by serum antibodies in experimental nontypeable Haemophilus influenzae otitis media. Infect. Immun. 52:572-578.
15. Yang, Y.-P., S.M. Loosmore, B. Underdown, and M.H. Klein. 1998. Nasopharyngeal colonization with nontypeable H. influenzae in chinchillas. Infect. Immun. 66:1973-1980.
16. Fleischmann et al. 1995. Whole-genome random sequencing and assembly of Haemophilus influenzae Rd. Science 269:496-512.
17. O'Hagan, DT. 1992. Oral delivery of vaccines. Formulation and clinical pharmaco kinetic considerations. Clin. Pharmacokinet 22(t): 1-10.
18. Ulmer et al. 1993. Curr. Opinion Invest. Drugs 2:983-989.
19. Lockhoff, O., 1991. Glycolipids as immunomodulators: Synthesis and properties.
20. Nixon-George A., et al., 1990. The adjuvant effect of stearyl tyrosine on a recombinant subunit hepatitis B surface antigen. J. Immunol 144 (12):4798-4802.

## Claims

1. A nucleic acid molecule comprising a promoter functional in *E. coli* and operatively coupled to a modified operon of a non-typeable strain of *Haemophilus* comprising hmw A, B and C genes, wherein the A gene of the operon contains only a nucleic acid sequence which codes for a mature high molecular weight protein of the non-typeable strain of *Haemophilus.*

2. The nucleic acid molecule claimed in claim 1, wherein said promoter is the T7 promoter.

3. The nucleic acid molecule claimed in claim 1 or 2, wherein said operon encodes the high molecular weight protein 1 (HMW1) of the non-typeable strain of *Haemophilus* or encodes the high molecular weight protein 2 (HMW2) of the non-typeable strain of *Haemophilus.*

4. The nucleic acid molecule claimed in any one of claims 1 to 3, wherein said a non-typeable strain of *Haemophilus* is selected from the group consisting of strains 12, Joyc, K21, PMH1 and 15 of non-typeable *Haemophilus influenzae.*

5. The nucleic acid molecule claimed in any one of claims 1 to 3, wherein said nucleic acid sequence which codes for a mature high molecular weight protein has a nucleic acid sequence selected from those having SQ ID NOS: 27, 31, 36, 40, 44, 48, 52, 56, 60, 64, 68, 72 or encodes a HMW1 or HMW2 protein having an amino acid sequence selected from those having SEQ ID NOS: 28, 32, 37, 41, 45, 49, 53, 57, 61, 65, 69, 73.

6. The nucleic acid molecule claimed in any one of claims 1 to 5 further including an additional nucleic acid sequence encoding the mature high molecular weight protein HMWA of a non-typeable strain of *Haemophilus.*

7. The nucleic acid molecule claimed in any one of claims 1 to 6 further including the cer gene of *E. coli.*

8. A vector comprising a nucleic acid molecule claimed in any one of claims 1 to 7, preferably a plasmid vector, adapted for transformation of a host including a nucleic acid molecule claimed in any one of claims 1 to 7.

9. A plasmid vector as claimed in claim 8 comprising the T7 promoter and a cloning site for insertion of a nucleic acid molecule into the plasmid vector.

10. A vector claimed in claim 8, wherein said plasmid vector has the identifying characteristic of a plasmid vector which is selected from group consisting of:
| | |
|---|---|
| DS-1046-1-1 | (ATCC No.: 203263) |
| JB-2507-7 | (ATCC No.: 203262) |
| BK-86-1-1 | (ATCC No.: 203258) |
| BK-35-4 | (ATCC No.: 203259) |
| BK-76-1-1 | (ATCC No.: 203261) |
| DS-2334-5 | (ATCC No.: 203260) |
| DS-2400-13 | (ATCC No.: 203257) |

11. A strain of *E. coli* transformed by an expression vector as claimed in claim 8 or 9 and expressing a protective high molecular weight protein of a non-typeable strain of *Haemophilus.*

12. The plasmid vector claimed in claim 9 further comprising the *E. coli cer* gene.

13. A method of the production of a protective high molecular weight protein of a non-typeable strain of *Haemophilus,* which comprises:
transforming *E. coli* with a vector as claimed in claim 8 or 9,
growing *E. coli* to express the encoded mature high molecular weight (HMW) protein, and
isolating and purifying the expressed HMW protein.

14. The method claimed in claim 13, wherein said isolation and purification procedure includes separating the HMWA protein from the B and C proteins.

## Patentansprüche

1. Ein Nukleinsäuremolekül, welches einen Promoter umfasst, der in *E. coli* funktionsfähig ist und operativ mit einem modifizierten Operon eines nicht typisierbaren Stammes von *Haemophilus,* das hmw A-, B- und C-Gene umfasst, gekoppelt ist, wobei das A-Gen des Operons nur eine Nukleinsäuresequenz enthält, welche für ein reifes Protein mit hohem Molekulargewicht des nicht typisierbaren Stammes von *Haemophilus* codiert.

2. Das Nukleinsäuremolekül, das in Anspruch 1 beansprucht wird, wobei der Promoter der T7-Promoter ist.

3. Das Nukleinsäuremolekül, das in Anspruch 1 oder 2 beansprucht wird, wobei das Operon das Protein mit hohem Molekulargewicht 1 (HMW1) des nicht typisierbaren Stammes von *Haemophilus* codiert oder das Protein mit hohem Molekulargewicht 2 (HMW2) des nicht typisierbaren Stammes von *Haemophilus* codiert.

4. Das Nukleinsäuremolekül, das in irgendeinem der Ansprüche 1 bis 3 beansprucht wird, wobei der nicht typisierbare Stamm von *Haemophilus* ausgewählt wird aus der Gruppe, die aus den Stämmen 12, Joyc, K21, PMH1 und 15 des nicht typisierbaren *Haemophilus influenzae* besteht.

5. Das Nukleinsäuremolekül, das in irgendeinem der Ansprüche 1 bis 3 beansprucht wird, wobei die Nukleinsäuresequenz, welche für ein reifes Protein mit hohem Molekulargewicht codiert, eine Nukleinsäuresequenz aufweist, die ausgewählt ist aus denen mit den SQ ID NOS: 27, 31, 36, 40, 44, 48, 52, 56, 60, 64, 68, 72, oder ein HMW1- oder HMW2-Protein codiert, das eine Aminosäuresequenz aufweist, die ausgewählt ist aus denen mit den SEQ ID NOS: 28, 32, 37, 41, 45, 49, 53, 57, 61, 65, 68, 73.

6. Das Nukleinsäuremolekül, das in irgendeinem der Ansprüche 1 bis 5 beansprucht wird, welches weiterhin eine zusätzliche Nukleinsäuresequenz einschließt, die das reife Protein mit hohem Molekulargewicht HMW eines nicht typisierbaren Stammes von *Haemophilus* codiert.

7. Das Nukleinsäuremolekül, das in irgendeinem der Ansprüche 1 bis 6 beansprucht wird, welches weiterhin das cer-Gen von *E. coli* einschließt.

8. Ein Vektor, welcher ein Nukleinsäuremolekül, das in irgendeinem der Ansprüche 1 bis 7 beansprucht wird, umfasst, vorzugsweise ein Plasmidvektor, welcher zur Transformation eines Wirtes ausgelegt ist, welcher ein Nukleinsäuremolekül, das in irgendeinem der Ansprüche 1 bis 7 beansprucht wird, einschließt.

9. Ein Plasmidvektor wie in Anspruch 8 beansprucht, welcher den T7-Promoter und eine Klonierungsstelle zur Insertion eines Nukleinsäuremoleküls in den Plasmidvektor umfasst.

10. Ein Vektor, der in Anspruch 8 beansprucht wird, wobei der Plasmidvektor die Identifizierungscharakteristika eines Plasmidvektors aufweist, welcher ausgewählt wird aus der Gruppe, bestehend aus:
| | |
|---|---|
| DS-1046-1-1 | (ATCC Nr.: 203263) |
| JB-2507-7 | (ATCC Nr.: 203262) |
| BK-86-1-1 | (ATCC Nr.: 203258) |
| BK-35-4 | (ATCC Nr.: 203259) |
| BK-76-1-1 | (ATCC Nr.: 203261) |
| DS-2334-5 | (ATCC Nr.: 203260) |
| DS-2400-13 | (ATCC Nr.: 203257) |

11. Ein Stamm von *E. coli,* welcher mit einem Expressionsvektor, wie dieser in Anspruch 8 oder 9 beansprucht wird, transformiert ist und welcher ein schützendes Protein mit hohem Molekulargewicht eines nicht typisierbaren Stammes von *Haemophilus* exprimiert.

12. Der Plasmidvektor, der in Anspruch 9 beansprucht wird, welcher weiterhin das *E. coli* cer-Gen umfasst.

13. Ein Verfahren zur Produktion eines schützenden Proteins mit hohem Molekulargewicht eines nicht typisierbaren Stammes von *Haemophilus,* welches umfasst:
das Transformieren von *E. coli* mit einem Vektor wie in Anspruch 8 oder 9 beansprucht,
das Wachsen lassen von *E. coli,* um das codierte reife Protein mit hohem Molekulargewicht (HMW) zu exprimieren, und
das Isolieren und Reinigen des exprimierten HMW-Proteins.

14. Das Verfahren, das in Anspruch 13 beansprucht wird, wobei die Isolations- und Reinigungsprozedur das Abtrennen des HMWA-Proteins von den B- und C-Proteinen einschließt.

## Revendications

1. Molécule d'acide nucléique comprenant un promoteur Fonctionnel dans *E. coli* et couplée de manière fonctionnelle à un opéron modifié d'une souche non typable de *Haemophilus* comprenant des gènes hmw A, B et C, dans laquelle le gène A de l'opéron contient uniquement une séquence d'acide nucléique qui code pour une protéine mature à poids moléculaire élevé de la souche non typable de *Haemophilus.*

2. Molécule d'acide nucléique selon la revendication 1, où ledit promoteur est le promoteur T7,

3. Molécule d'acide nucléique selon la revendication 1 ou 2, où ledit opéron code pour la protéine à poids moléculaire élevé 1 (HMW1) de la souche non typable de *Haemophilus* ou code pour la protéine à poids moléculaire élevé 2 (HMW2) de la souche non typable de *Haemophilus.*

4. Molécule d'acide nucléique selon l'une quelconque des revendications 1 à 3, ou ladite souche non typable de *Haemophilus* est choisie parmi le groupe consistant en les souches 12, Joyc, K21, PMH1 et 15 de *Haemophilus influenzae* non typable.

5. Molécule d'acide nucléique selon l'une quelconque des revendications 1 à 3, où ladite séquence d'acide nucléique qui code pour une protéine mature à poids moléculaire élevé possède une séquence d'acide nucléique choisie parmi celles ayant les SQ ID NO: 27, 31, 36, 40, 44, 48, 52, 56, 60, 64, 68, 72 ou code pour une protéine HMW1 ou HMW2 ayant une séquence d'acide aminé choisie parmi celles ayant les SEQ ID NO: 28, 32, 37, 41,45,49, 53, 57, 61, 65, 69, 73.

6. Molécule d'acide nucléique selon l'une quelconque des revendications 1 à 5, comprenant en outre une séquence d'acide nucléique supplémentaire codant pour la protéine mature à poids moléculaire élevé HMWA d'une souche non typable de *Haemophilus.*

7. Molécule d'acide nucléique selon l'une quelconque des revendications 1 à 6, comprenant en outre le gène cer de *E. coli.*

8. Vecteur comprenant une molécule d'acide nucléique selon l'une quelconque des revendications 1 à 7, de préférence un vecteur plasmidique, adapté pour la transformation d'un hôte comprenant une molécule d'acide nucléique selon l'une quelconque des revendications 1 à 7.

9. Vecteur plasmidique selon la revendication 8, comprenant le promoteur T7 et un site de clonage pour l'insertion d'une molécule d'acide nucléique dans le vecteur plasmidique.

10. Vecteur selon la revendication 8, ou ledit vecteur plasmidique possède la caractéristique d'identification d'un vecteur plasmidique qui est choisie parmi le groupe consistant en :
| | |
|---|---|
| DS-1046-1-1 | (ATCC No.: 203263) |
| JB-2507-7 | (ATCC No.: 203262) |
| BK-86-1-1 | (ATCC No.: 203258) |
| BIC-35-4 | (ATCC No.: 203259) |
| BK-76-1 -1 | (ATCC No.: 203261) |
| DS-2334-5 | (ATCC No.: 203260) |
| DS-2400-13 | (ATCC No.: 203257) |

11. Souche de *E. coli* transformée par un vecteur d'expression selon la revendication 8 ou 9 et exprimant une protéine protectrice à poids moléculaire élevé d'une souche non typable de *Haemophilus.*

12. Vecteur plasmidique selon la revendication 9, comprenant en outre le gène *cer E. coli.*

13. Procédé de production d'une protéine protectrice poids moléculaire élevé d'une souche non typable de *Haemophilus,* qui comprend :
la transformation de *E. coli* avec un vecteur selon la revendication 8 ou 9,
la mise en croissance de *E. coli* pour exprimer la protéine mature codée à poids moléculaire élevé (HMW), et
l'isolement et la purification de la protéine HMW exprimée.

14. Procédé selon la revendication 13, où ladite procédure d'isolement et de purification comprend la séparation de la protéine HMW des protéines B et C.
